(19) Europäisches Patentamt / European Patent Office / Office européen des brevets

(11) EP 1 820 860 A2

(12) EUROPEAN PATENT APPLICATION

(43) Date of publication:
**22.08.2007 Bulletin 2007/34**

(21) Application number: **07004855.8**

(22) Date of filing: **30.05.2000**

(51) Int Cl.:
***C12N 15/12*** *(2006.01)*
***A61K 38/17*** *(2006.01)*
***C12Q 1/68*** *(2006.01)*
***C07K 16/18*** *(2006.01)*
***C07K 14/47*** *(2006.01)*
***G01N 33/53*** *(2006.01)*
***C12N 15/62*** *(2006.01)*

(84) Designated Contracting States:
**AT BE CH CY DE DK ES FI FR GB GR IE IT LI LU MC NL PT SE**

(30) Priority:
**02.06.1999 WOPCT/US99/12252**
**22.06.1999 US 140650 P**
**23.06.1999 US 141037 P**
**20.07.1999 US 144758 P**
**01.09.1999 WOPCT/US99/20111**
**08.09.1999 WOPCT/US99/20594**
**29.10.1999 US 162506 P**
**30.11.1999 WOPCT/US99/28313**
**01.12.1999 WOPCT/US99/28634**
**02.12.1999 WOPCT/US99/28551**
**09.12.1999 US 170262 P**
**16.12.1999 WOPCT/US99/30095**
**20.12.1999 WOPCT/US99/30999**
**06.01.2000 WOPCT/US00/00376**
**11.02.2000 WOPCT/US00/03565**
**18.02.2000 WOPCT/US00/04341**
**18.02.2000 WOPCT/US00/04342**
**02.03.2000 WOPCT/US00/05841**
**03.03.2000 US 187202 P**
**10.03.2000 WOPCT/US00/06319**
**15.03.2000 WOPCT/US00/06884**
**30.03.2000 WOPCT/US00/08439**
**17.05.2000 WOPCT/US00/13705**

(62) Document number(s) of the earlier application(s) in accordance with Art. 76 EPC:
**00941164.6 / 1 185 648**

(71) Applicant: **Genentech, Inc.**
**South San Francisco CA 94080-4990 (US)**

(72) Inventors:
- **Baker, Kevin P.**
  **Darnestown, MD 20878 (US)**
- **Goddard, Audrey**
  **San Francisco, CA 94127 (US)**
- **Gurney, Austin L.**
  **San Francisco, CA 94114 (US)**
- **Hebert, Caroline**
  **Berkeley, CA 94703 (US)**
- **Henzel, William**
  **San Mateo, CA 94030 (US)**
- **Kabakoff, Rhona C.**
  **Pacifica, CA 94044 (US)**
- **Shelton, David L.**
  **Oakland, CA 94618 (US)**
- **Smith, Victoria**
  **Burlingame, CA 94010 (US)**
- **Watanabe, Colin K.**
  **San Francisco, CA 94110 (US)**
- **Wood, William I.**
  **Hillsborough, CA 94010 (US)**

(74) Representative: **Woolley, Lindsey Claire et al**
**Mewburn Ellis LLP**
**York House**
**23 Kingsway**
**London WC2B 6HP (GB)**

Remarks:
- This application was filed on 09 - 03 - 2007 as a divisional application to the application mentioned under INID code 62.
- The sequence listing, which is published as annex to the application documents, was filed after the date of filing. The applicant has declared that it does not include matter which goes beyond the content of the application as filed.

# (54) Methods and compositions for inhibiting neoplastic cell growth

(57) The present invention concerns methods and compositions for inhibiting neoplastic cell growth. In particular, the present invention concerns antitumor compositions and methods for the treatment of tumors. The invention further concerns screening methods for identifying growth inhibitory, e.g., antitumor compounds. The present invention is directed to novel polypeptides and to nucleic acid molecules encoding those polypeptides. Also provided herein are vectors and host cells comprising those nucleic acid sequences, chimeric polypeptide molecules comprising the polypeptides of the present invention fused to heterologous polypeptide sequences,



Printed by Jouve, 75001 PARIS (FR)

(Cont. next page)

antibodies which bind to the polypeptides of the present invention and to methods for producing the polypeptides of the present invention.

## FIGURE 48

MEFLWAPLLGLCCSLAAADRHTVFWNSSNPKFRNEDYTIHVQLNDYVDIICPHYEDHSADAAMEQYILYLV
EHEEYQLCQPQSKDQVRWQCNRPSAKHGPEKLSEKFQRFTPFTLGKEFKEGHSYYYISKPIHQHEDRCLRL
KVTVSGKITHSPQAHDNPQEKRLAADDPEVRVLHSIGHSAAPRLFPLAWTVLLLPLLLLQTP

Signal sequence: Amino acids 1-17

N-glycosylation site: Amino acids 26-30

Tyrosine kinase phosphorylation site: Amino acids 118-127

N-myristoylation site: Amino acids 10-16

Annex to the application documents - subsequently filed sequences listing

Sequence Listing

<110> Genentech, Inc.

<120> METHODS AND COMPOSITIONS FOR INHIBITING NEOPLASTIC CELL GROWTH

<130> LCW/FP6418180

<140> EP07004855.8
<141> 2000-05-30

<150> EP00941164.6
<151> 2000-05-30

<150> PCT/US00/14941
<151> 2000-05-30

<150> PCT/US99/12252
<151> 1999-06-02

<150> US 60/140,650
<151> 1999-06-22

<150> US 60/141,037
<151> 1999-06-23

<150> US 60/144,758
<151> 1999-07-20

<150> PCT/US99/20111
<151> 1999-09-01

<150> PCT/US99/20594
<151> 1999-09-08

<150> US 60/162,506
<151> 1999-10-29

<150> PCT/US99/28313
<151> 1999-11-30

<150> PCT/US99/28634
<151> 1999-12-01

<150> PCT/US99/28551
<151> 1999-12-02

<150> US 60/170,262
<151> 1999-12-09

<150> PCT/US99/30095
<151> 1999-12-16

<150> PCT/US99/30999
<151> 1999-12-20

<150> PCT/US00/00376

<151> 2000-01-06

<150> PCT/US00/03565
<151> 2000-02-11

<150> PCT/US00/04341
<151> 2000-02-18

<150> PCT/US00/04342
<151> 2000-02-18

<150> PCT/US00/05841
<151> 2000-03-02

<150> US 60/187,202
<151> 2000-03-03

<150> PCT/US00/06319
<151> 2000-03-10

<150> PCT/US00/06884
<151> 2000-03-15

<150> PCT/US00/08439
<151> 2000-03-30

<150> PCT/US00/13705
<151> 2000-05-17

<160> 91

<210> 1
<211> 932
<212> DNA
<213> Homo Sapien

<220>
<221> unsure
<222> 911
<223> unknown base

<400> 1
gggaacggaa aatggcgcct cacggcccgg gtagtcttac gaccctggtg 50

ccctgggctg ccgccctgct cctcgctctg ggcgtggaaa gggctctggc 100

gctacccgag atatgcaccc aatgtccagg gagcgtgcaa aatttgtcaa 150

aagtggcctt ttattgtaaa acgacacgag agctaatgct gcatgcccgt 200

tgctgcctga atcagaaggg caccatcttg gggctggatc tccagaactg 250

ttctctggag gaccctggtc caaactttca tcaggcacat accactgtca 300

tcatagacct gcaagcaaac cccctcaaag gtgacttggc caacaccttc 350

cgtggcttta ctcagctcca gactctgata ctgccacaac atgtcaactg 400

tcctggagga attaatgcct ggaatactat cacctcttat atagacaacc 450

aaatctgtca agggcaaaag aacctttgca ataacactgg ggacccagaa 500

atgtgtcctg agaatggatc ttgtgtacct gatggtccag gtcttttgca 550

gtgtgtttgt gctgatggtt tccatggata caagtgtatg cgccagggct 600

cgttctcact gcttatgttc ttcgggattc tgggagccac cactctatcc 650

gtctccattc tgctttgggc gacccagcgc cgaaaagcca agacttcatg 700

aactacatag gtcttaccat tgacctaaga tcaatctgaa ctatcttagc 750

ccagtcaggg agctctgctt cctagaaagg catctttcgc cagtggattc 800

gcctcaaggt tgaggccgcc attggaagat gaaaaattgc actcccttgg 850

tgtagacaaa taccagttcc cattggtgtt gttgcctata ataaacactt 900

tttctttttt naaaaaaaaa aaaaaaaaaa aa 932

<210> 2
<211> 229
<212> PRT
<213> Homo Sapien

<400> 2
Met Ala Pro His Gly Pro Gly Ser Leu Thr Thr Leu Val Pro Trp
1 5 10 15

Ala Ala Ala Leu Leu Leu Ala Leu Gly Val Glu Arg Ala Leu Ala
20 25 30

Leu Pro Glu Ile Cys Thr Gln Cys Pro Gly Ser Val Gln Asn Leu
35 40 45

Ser Lys Val Ala Phe Tyr Cys Lys Thr Thr Arg Glu Leu Met Leu
50 55 60

His Ala Arg Cys Cys Leu Asn Gln Lys Gly Thr Ile Leu Gly Leu
65 70 75

Asp Leu Gln Asn Cys Ser Leu Glu Asp Pro Gly Pro Asn Phe His
80 85 90

Gln Ala His Thr Thr Val Ile Ile Asp Leu Gln Ala Asn Pro Leu
95 100 105

Lys Gly Asp Leu Ala Asn Thr Phe Arg Gly Phe Thr Gln Leu Gln
110 115 120

Thr Leu Ile Leu Pro Gln His Val Asn Cys Pro Gly Gly Ile Asn
125 130 135

Ala Trp Asn Thr Ile Thr Ser Tyr Ile Asp Asn Gln Ile Cys Gln
140 145 150

Gly Gln Lys Asn Leu Cys Asn Asn Thr Gly Asp Pro Glu Met Cys
155 160 165

Pro Glu Asn Gly Ser Cys Val Pro Asp Gly Pro Gly Leu Leu Gln
170 175 180

Cys Val Cys Ala Asp Gly Phe His Gly Tyr Lys Cys Met Arg Gln
185 190 195

Gly Ser Phe Ser Leu Leu Met Phe Phe Gly Ile Leu Gly Ala Thr
200 205 210

Thr Leu Ser Val Ser Ile Leu Leu Trp Ala Thr Gln Arg Arg Lys
215 220 225

Ala Lys Thr Ser

<210> 3
<211> 2336
<212> DNA
<213> Homo Sapien

<220>
<221> unsure
<222> 1620, 1673
<223> unknown base

<400> 3
ttcgtgaccc ttgagaaaag agttggtggt aaatgtgcca cgtcttctaa 50

gaagggggag tcctgaactt gtctgaagcc cttgtccgta agccttgaac 100

tacgttctta aatctatgaa gtcgagggac ctttcgctgc ttttgtaggg 150

acttctttcc ttgcttcagc aacatgaggc ttttcttgtg gaacgcggtc 200

ttgactctgt tcgtcacttc tttgattggg gctttgatcc ctgaaccaga 250

agtgaaaatt gaagttctcc agaagccatt catctgccat cgcaagacca 300

aaggagggga tttgatgttg gtccactatg aaggctactt agaaaaggac 350

ggctccttat ttcactccac tcacaaacat aacaatggtc agcccatttg 400

gtttaccctg ggcatcctgg aggctctcaa aggttgggac cagggcttga 450

aaggaatgtg tgtaggagag aagagaaagc tcatcattcc tcctgctctg 500

ggctatggaa aagaaggaaa aggtaaaatt cccccagaaa gtacactgat 550

atttaatatt gatctcctgg agattcgaaa tggaccaaga tcccatgaat 600

cattccaaga aatggatctt aatgatgact ggaaactctc taaagatgag 650

gttaaagcat atttaaagaa ggagtttgaa aaacatggtg cggtggtgaa 700

tgaaagtcat catgatgctt tggtggagga tatttttgat aaagaagatg 750

aagacaaaga tgggtttata tctgccagag aatttacata taaacacgat 800
gagttataga gatacatcta cccttttaat atagcactca tctttcaaga 850
gagggcagtc atctttaaag aacattttat ttttatacaa tgttcttct 900
tgctttgttt tttattttta tatatttttt ctgactccta tttaaagaac 950
cccttaggtt tctaagtacc catttctttc tgataagtta ttgggaagaa 1000
aaagctaatt ggtctttgaa tagaagactt ctggacaatt tttcactttc 1050
acagatatga agctttgttt tactttctca cttataaatt taaaatgttg 1100
caactgggaa tataccacga catgagacca ggttatagca caaattagca 1150
ccctatattt ctgcttccct ctattttctc caagttagag gtcaacattt 1200
gaaaagcctt ttgcaatagc ccaaggcttg ctattttcat gttataatga 1250
aatagtttat gtgtaactgg ctctgagtct ctgcttgagg accagaggaa 1300
aatggttgtt ggacctgact tgttaatggc tactgcttta ctaaggagat 1350
gtgcaatgct gaagttagaa acaaggttaa tagccaggca tggtggctca 1400
tgcctgtaat cccagcactt tgggaggctg aggcgggcgg atcacctgag 1450
gttgggagtt cgagaccagc ctgaccaaca cggagaaacc ctatctctac 1500
taaaaataca aagtagcccg gcgtggtgat gcgtgcctgt aatcccagct 1550
acccaggaag gctgaggcgg cagaatcact tgaacccgag gccgaggttg 1600
cggtaagccg agatcacctn cagcctggac actctgtctc gaaaaaagaa 1650
aagaacacgg ttaataccat atnaatatgt atgcattgag acatgctacc 1700
taggacttaa gctgatgaag cttggctcct agtgattggt ggcctattat 1750
gataaatagg acaaatcatt tatgtgtgag tttctttgta ataaaatgta 1800
tcaatatgtt atagatgagg tagaaagtta tatttatatt caatatttac 1850
ttcttaaggc tagcggaata tccttcctgg ttctttaatg ggtagtctat 1900
agtatattat actacaataa cattgtatca taagataaag tagtaaacca 1950
gtctacattt tcccatttct gtctcatcaa aaactgaagt tagctgggtg 2000
tggtggctca tgcctgtaat cccagcactt tgggggccaa ggagggtgga 2050
tcacttgaga tcaggagttc aagaccagcc tggccaacat ggtgaaacct 2100
tgtctctact aaaaatacaa aaattagcca ggcgtggtgg tgcacacctg 2150
tagtcccagc tactcgggag gctgagacag gagatttgct tgaacccggg 2200
aggcggaggt tgcagtgagc caagattgtg ccactgcact ccagcctggg 2250

tgacagagca agactccatc tcaaaaaaaa aaaaaagaag cagacctaca 2300

gcagctacta ttgaataaat acctatcctg gatttt 2336

<210> 4
<211> 211
<212> PRT
<213> Homo Sapien

<400> 4
Met Arg Leu Phe Leu Trp Asn Ala Val Leu Thr Leu Phe Val Thr
1 5 10 15

Ser Leu Ile Gly Ala Leu Ile Pro Glu Pro Glu Val Lys Ile Glu
20 25 30

Val Leu Gln Lys Pro Phe Ile Cys His Arg Lys Thr Lys Gly Gly
35 40 45

Asp Leu Met Leu Val His Tyr Glu Gly Tyr Leu Glu Lys Asp Gly
50 55 60

Ser Leu Phe His Ser Thr His Lys His Asn Asn Gly Gln Pro Ile
65 70 75

Trp Phe Thr Leu Gly Ile Leu Glu Ala Leu Lys Gly Trp Asp Gln
80 85 90

Gly Leu Lys Gly Met Cys Val Gly Glu Lys Arg Lys Leu Ile Ile
95 100 105

Pro Pro Ala Leu Gly Tyr Gly Lys Glu Gly Lys Gly Lys Ile Pro
110 115 120

Pro Glu Ser Thr Leu Ile Phe Asn Ile Asp Leu Leu Glu Ile Arg
125 130 135

Asn Gly Pro Arg Ser His Glu Ser Phe Gln Glu Met Asp Leu Asn
140 145 150

Asp Asp Trp Lys Leu Ser Lys Asp Glu Val Lys Ala Tyr Leu Lys
155 160 165

Lys Glu Phe Glu Lys His Gly Ala Val Val Asn Glu Ser His His
170 175 180

Asp Ala Leu Val Glu Asp Ile Phe Asp Lys Glu Asp Glu Asp Lys
185 190 195

Asp Gly Phe Ile Ser Ala Arg Glu Phe Thr Tyr Lys His Asp Glu
200 205 210

Leu

<210> 5
<211> 1379
<212> DNA

<213> Homo Sapien

<400> 5
acccacgcgt ccgcccacgc gtccgcccac gcgtccgccc acgcgtccgc 50

gcgtagccgt gcgccgattg cctctcggcc tgggcaatgg tcccggctgc 100

cggtcgacga ccgccccgcg tcatgcggct cctcggctgg tggcaagtat 150

tgctgtgggt gctgggactt cccgtccgcg gcgtggaggt tgcagaggaa 200

agtggtcgct tatggtcaga ggagcagcct gctcaccctc tccaggtggg 250

ggctgtgtac ctgggtgagg aggagctcct gcatgacccg atgggccagg 300

acagggcagc agaagaggcc aatgcggtgc tggggctgga cacccaaggc 350

gatcacatgg tgatgctgtc tgtgattcct ggggaagctg aggacaaagt 400

gagttcagag cctagcggcg tcacctgtgg tgctggagga gcggaggact 450

caaggtgcaa cgtccgagag agccttttct ctctggatgg cgctggagca 500

cacttccctg acagagaaga ggagtattac acagagccag aagtggcgga 550

atctgacgca gccccgacag aggactccaa taacactgaa agtctgaaat 600

ccccaaaggt gaactgtgag gagagaaaca ttacaggatt agaaaatttc 650

actctgaaaa ttttaaatat gtcacaggac cttatggatt ttctgaaccc 700

aaacggtagt gactgtactc tagtcctgtt ttacaccccg tggtgccgct 750

tttctgccag tttggcccct cactttaact ctctgccccg ggcatttcca 800

gctcttcact ttttggcact ggatgcatct cagcacagca gcctttctac 850

caggtttggc accgtagctg ttcctaatat tttattattt caaggagcta 900

aaccaatggc cagatttaat catacagatc gaacactgga aacactgaaa 950

atcttcattt ttaatcagac aggtatagaa gccaagaaga atgtggtggt 1000

aactcaagcc gaccaaatag gccctcttcc cagcactttg ataaaaagtg 1050

tggactggtt gcttgtattt tccttattct ttttaattag ttttattatg 1100

tatgctacca ttcgaactga gagtattcgg tggctaattc caggacaaga 1150

gcaggaacat gtggagtagt gatggtctga aagaagttgg aaagaggaac 1200

ttcaatcctt cgtttcagaa attagtgcta cagtttcata cattttctcc 1250

agtgacgtgt tgacttgaaa cttcaggcag attaaaagaa tcatttgttg 1300

aacaactgaa tgtataaaaa aattataaac tggtgtttta actagtattg 1350

caataagcaa atgcaaaaat attcaatag 1379

<210> 6
<211> 360
<212> PRT
<213> Homo Sapien

<400> 6

```
Met Val Pro Ala Ala Gly Arg Arg Pro Pro Arg Val Met Arg Leu
1               5                   10                  15

Leu Gly Trp Trp Gln Val Leu Leu Trp Val Leu Gly Leu Pro Val
                20                  25                  30

Arg Gly Val Glu Val Ala Glu Glu Ser Gly Arg Leu Trp Ser Glu
                35                  40                  45

Glu Gln Pro Ala His Pro Leu Gln Val Gly Ala Val Tyr Leu Gly
                50                  55                  60

Glu Glu Glu Leu Leu His Asp Pro Met Gly Gln Asp Arg Ala Ala
                65                  70                  75

Glu Glu Ala Asn Ala Val Leu Gly Leu Asp Thr Gln Gly Asp His
                80                  85                  90

Met Val Met Leu Ser Val Ile Pro Gly Glu Ala Glu Asp Lys Val
                95                  100                 105

Ser Ser Glu Pro Ser Gly Val Thr Cys Gly Ala Gly Gly Ala Glu
                110                 115                 120

Asp Ser Arg Cys Asn Val Arg Glu Ser Leu Phe Ser Leu Asp Gly
                125                 130                 135

Ala Gly Ala His Phe Pro Asp Arg Glu Glu Glu Tyr Tyr Thr Glu
                140                 145                 150

Pro Glu Val Ala Glu Ser Asp Ala Ala Pro Thr Glu Asp Ser Asn
                155                 160                 165

Asn Thr Glu Ser Leu Lys Ser Pro Lys Val Asn Cys Glu Glu Arg
                170                 175                 180

Asn Ile Thr Gly Leu Glu Asn Phe Thr Leu Lys Ile Leu Asn Met
                185                 190                 195

Ser Gln Asp Leu Met Asp Phe Leu Asn Pro Asn Gly Ser Asp Cys
                200                 205                 210

Thr Leu Val Leu Phe Tyr Thr Pro Trp Cys Arg Phe Ser Ala Ser
                215                 220                 225

Leu Ala Pro His Phe Asn Ser Leu Pro Arg Ala Phe Pro Ala Leu
                230                 235                 240

His Phe Leu Ala Leu Asp Ala Ser Gln His Ser Ser Leu Ser Thr
                245                 250                 255

Arg Phe Gly Thr Val Ala Val Pro Asn Ile Leu Leu Phe Gln Gly
                260                 265                 270
```

Ala Lys Pro Met Ala Arg Phe Asn His Thr Asp Arg Thr Leu Glu
275 280 285

Thr Leu Lys Ile Phe Ile Phe Asn Gln Thr Gly Ile Glu Ala Lys
290 295 300

Lys Asn Val Val Val Thr Gln Ala Asp Gln Ile Gly Pro Leu Pro
305 310 315

Ser Thr Leu Ile Lys Ser Val Asp Trp Leu Leu Val Phe Ser Leu
320 325 330

Phe Phe Leu Ile Ser Phe Ile Met Tyr Ala Thr Ile Arg Thr Glu
335 340 345

Ser Ile Arg Trp Leu Ile Pro Gly Gln Glu Gln Glu His Val Glu
350 355 360

<210> 7
<211> 2180
<212> DNA
<213> Homo Sapien

<400> 7
tatgactggc gccgagcccc aaatgaaaac gggccctact tcctggccct 50

ccgcgagatg atcgaggaga tgtaccagct gtatgggggc cccgtggtgc 100

tggttgccca cagtatgggc aacatgtaca cgctctactt tctgcagcgg 150

cagccgcagg cctggaagga caagtatatc cgggccttcg tgtcactggg 200

tgcgccctgg gggggcgtgg ccaagaccct gcgcgtcctg gcttcaggag 250

acaacaaccg gatcccagtc atcgggcccc tgaagatccg ggagcagcag 300

cggtcagctg tctccaccag ctggctgctg ccctacaact acacatggtc 350

acctgagaag gtgttcgtgc agacacccac aatcaactac acactgcggg 400

actaccgcaa gttcttccag gacatcggct ttgaagatgg ctggctcatg 450

cggcaggaca cagaagggct ggtggaagcc acgatgccac ctggcgtgca 500

gctgcactgc ctctatggta ctggcgtccc cacaccagac tccttctact 550

atgagagctt ccctgaccgt gaccctaaaa tctgctttgg tgacggcgat 600

ggtactgtga acttgaagag tgccctgcag tgccaggcct ggcagagccg 650

ccaggagcac caagtgttgc tgcaggagct gccaggcagc gagcacatcg 700

agatgctggc caacgccacc accctggcct atctgaaacg tgtgctcctt 750

gggccctgac tcctgtgcca caggactcct gtggctcggc cgtggacctg 800

ctgttggcct ctggggctgt catggcccac gcgttttgca aagtttgtga 850

ctcaccattc aaggccccga gtcttggact gtgaagcatc tgccatgggg 900

aagtgctgtt tgttatcctt tctctgtggc agtgaagaag gaagaaatga 950

gagtctagac tcaagggaca ctggatggca agaatgctgc tgatggtgga 1000

actgctgtga ccttaggact ggctccacag ggtggactgg ctgggccctg 1050

gtcccagtcc ctgcctgggg ccatgtgtcc ccctattcct gtgggctttt 1100

catacttgcc tactgggccc tggccccgca gccttcctat gagggatgtt 1150

actgggctgt ggtcctgtac ccagaggtcc cagggatcgg ctcctggccc 1200

ctcgggtgac ccttcccaca caccagccac agataggcct gccactggtc 1250

atgggtagct agagctgctg gcttccctgt ggcttagctg gtggccagcc 1300

tgactggctt cctgggcgag cctagtagct cctgcaggca ggggcagttt 1350

gttgcgttct tcgtggttcc caggccctgg gacatctcac tccactccta 1400

cctcccttac caccaggagc attcaagctc tggattgggc agcagatgtg 1450

cccccagtcc cgcaggctgt gttccagggg ccctgatttc ctcggatgtg 1500

ctattggccc caggactgaa gctgcctccc ttcaccctgg gactgtggtt 1550

ccaaggatga gagcaggggt tggagccatg gccttctggg aacctatgga 1600

gaaagggaat ccaaggaagc agccaaggct gctcgcagct tccctgagct 1650

gcacctcttg ctaaccccac catcacactg ccaccctgcc ctagggtctc 1700

actagtacca agtgggtcag cacagggctg aggatggggc tcctatccac 1750

cctggccagc acccagctta gtgctgggac tagcccagaa acttgaatgg 1800

gaccctgaga gagccagggg tcccctgagg cccccctagg ggctttctgt 1850

ctgccccagg gtgctccatg gatctccctg tggcagcagg catggagagt 1900

cagggctgcc ttcatggcag taggctctaa gtgggtgact ggccacaggc 1950

cgagaaaagg gtacagcctc taggtggggt tcccaaagac gccttcaggc 2000

tggactgagc tgctctccca cagggtttct gtgcagctgg attttctctg 2050

ttgcatacat gcctggcatc tgtctcccct tgttcctgag tggccccaca 2100

tggggctctg agcaggctgt atctggattc tggcaataaa agtactctgg 2150

atgctgtaaa aaaaaaaaaa aaaaaaaaaa 2180

<210> 8
<211> 412
<212> PRT
<213> Homo Sapien

<400> 8

```
Met Gly Leu His Leu Arg Pro Tyr Arg Val Gly Leu Leu Pro Asp
 1               5                  10                  15

Gly Leu Leu Phe Leu Leu Leu Leu Leu Met Leu Leu Ala Asp Pro
                20                  25                  30

Ala Leu Pro Ala Gly Arg His Pro Pro Val Val Leu Val Pro Gly
                35                  40                  45

Asp Leu Gly Asn Gln Leu Glu Ala Lys Leu Asp Lys Pro Thr Val
                50                  55                  60

Val His Tyr Leu Cys Ser Lys Lys Thr Glu Ser Tyr Phe Thr Ile
                65                  70                  75

Trp Leu Asn Leu Glu Leu Leu Leu Pro Val Ile Ile Asp Cys Trp
                80                  85                  90

Ile Asp Asn Ile Arg Leu Val Tyr Asn Lys Thr Ser Arg Ala Thr
                95                  100                 105

Gln Phe Pro Asp Gly Val Asp Val Arg Val Pro Gly Phe Gly Lys
                110                 115                 120

Thr Phe Ser Leu Glu Phe Leu Asp Pro Ser Lys Ser Ser Val Gly
                125                 130                 135

Ser Tyr Phe His Thr Met Val Glu Ser Leu Val Gly Trp Gly Tyr
                140                 145                 150

Thr Arg Gly Glu Asp Val Arg Gly Ala Pro Tyr Asp Trp Arg Arg
                155                 160                 165

Ala Pro Asn Glu Asn Gly Pro Tyr Phe Leu Ala Leu Arg Glu Met
                170                 175                 180

Ile Glu Glu Met Tyr Gln Leu Tyr Gly Gly Pro Val Val Leu Val
                185                 190                 195

Ala His Ser Met Gly Asn Met Tyr Thr Leu Tyr Phe Leu Gln Arg
                200                 205                 210

Gln Pro Gln Ala Trp Lys Asp Lys Tyr Ile Arg Ala Phe Val Ser
                215                 220                 225

Leu Gly Ala Pro Trp Gly Gly Val Ala Lys Thr Leu Arg Val Leu
                230                 235                 240

Ala Ser Gly Asp Asn Asn Arg Ile Pro Val Ile Gly Pro Leu Lys
                245                 250                 255

Ile Arg Glu Gln Gln Arg Ser Ala Val Ser Thr Ser Trp Leu Leu
                260                 265                 270

Pro Tyr Asn Tyr Thr Trp Ser Pro Glu Lys Val Phe Val Gln Thr
                275                 280                 285

Pro Thr Ile Asn Tyr Thr Leu Arg Asp Tyr Arg Lys Phe Phe Gln
```

290 295 300

Asp Ile Gly Phe Glu Asp Gly Trp Leu Met Arg Gln Asp Thr Glu
305 310 315

Gly Leu Val Glu Ala Thr Met Pro Pro Gly Val Gln Leu His Cys
320 325 330

Leu Tyr Gly Thr Gly Val Pro Thr Pro Asp Ser Phe Tyr Tyr Glu
335 340 345

Ser Phe Pro Asp Arg Asp Pro Lys Ile Cys Phe Gly Asp Gly Asp
350 355 360

Gly Thr Val Asn Leu Lys Ser Ala Leu Gln Cys Gln Ala Trp Gln
365 370 375

Ser Arg Gln Glu His Gln Val Leu Leu Gln Glu Leu Pro Gly Ser
380 385 390

Glu His Ile Glu Met Leu Ala Asn Ala Thr Thr Leu Ala Tyr Leu
395 400 405

Lys Arg Val Leu Leu Gly Pro
410

<210> 9
<211> 2854
<212> DNA
<213> Homo Sapien

<400> 9
ctaagaggac aagatgaggc ccggcctctc atttctccta gcccttctgt 50

tcttccttgg ccaagctgca ggggatttgg gggatgtggg acctccaatt 100

cccagccccg gcttcagctc tttcccaggt gttgactcca gctccagctt 150

cagctccagc tccaggtcgg gctccagctc cagccgcagc ttaggcagcg 200

gaggttctgt gtcccagttg ttttccaatt tcaccggctc cgtggatgac 250

cgtgggacct gccagtgctc tgtttccctg ccagacacca cctttcccgt 300

ggacagagtg gaacgcttgg aattcacagc tcatgttctt tctcagaagt 350

ttgagaaaga actttctaaa gtgagggaat atgtccaatt aattagtgtg 400

tatgaaaaga aactgttaaa cctaactgtc cgaattgaca tcatggagaa 450

ggataccatt tcttacactg aactggactt cgagctgatc aaggtagaag 500

tgaaggagat ggaaaaactg gtcatacagc tgaaggagag ttttggtgga 550

agctcagaaa ttgttgacca gctggaggtg gagataagaa atatgactct 600

cttggtagag aagcttgaga cactagacaa aaacaatgtc cttgccattc 650

gccgagaaat cgtggctctg aagaccaagc tgaaagagtg tgaggcctct 700

aaagatcaaa acaccccctgt cgtccaccct cctcccactc cagggagctg 750
tggtcatggt ggtgtggtga acatcagcaa accgtctgtg gttcagctca 800
actggagagg gttttcttat ctatatggtg cttggggtag ggattactct 850
ccccagcatc caaacaaagg actgtattgg gtggcgccat tgaatacaga 900
tgggagactg ttggagtatt atagactgta caacacactg gatgatttgc 950
tattgtatat aaatgctcga gagttgcgga tcacctatgg ccaaggtagt 1000
ggtacagcag tttacaacaa caacatgtac gtcaacatgt acaacaccgg 1050
gaatattgcc agagttaacc tgaccaccaa cacgattgct gtgactcaaa 1100
ctctccctaa tgctgcctat aataaccgct tttcatatgc taatgttgct 1150
tggcaagata ttgactttgc tgtggatgag aatggattgt gggttattta 1200
ttcaactgaa gccagcactg gtaacatggt gattagtaaa ctcaatgaca 1250
ccacacttca ggtgctaaac acttggtata ccaagcagta taaaccatct 1300
gcttctaacg ccttcatggt atgtggggtt ctgtatgcca cccgtactat 1350
gaacaccaga acagaagaga tttttacta ttatgacaca aacacaggga 1400
aagagggcaa actagacatt gtaatgcata agatgcagga aaaagtgcag 1450
agcattaact ataacccttt tgaccagaaa ctttatgtct ataacgatgg 1500
ttaccttctg aattatgatc tttctgtctt gcagaagccc cagtaagctg 1550
tttaggagtt agggtgaaag agaaaatgtt tgttgaaaaa atagtcttct 1600
ccacttactt agatatctgc aggggtgtct aaaagtgtgt tcattttgca 1650
gcaatgttta ggtgcatagt tctaccacac tagagatcta ggacatttgt 1700
cttgatttgg tgagttctct tgggaatcat ctgcctcttc aggcgcattt 1750
tgcaataaag tctgtctagg gtgggattgt cagaggtcta ggggcactgt 1800
gggcctagtg aagcctactg tgaggaggct tcactagaag ccttaaatta 1850
ggaattaagg aacttaaaac tcagtatggc gtctagggat tctttgtaca 1900
ggaaatattg cccaatgact agtcctcatc catgtagcac cactaattct 1950
tccatgcctg gaagaaacct ggggacttag ttaggtagat taatatctgg 2000
agctcctcga gggaccaaat ctccaacttt tttttcccct cactagcacc 2050
tggaatgatg ctttgtatgt ggcagataag taaatttggc atgcttatat 2100
attctacatc tgtaaagtgc tgagttttat ggagagaggc ctttttatgc 2150

attaaattgt acatggcaaa taaatcccag aaggatctgt agatgaggca 2200

cctgcttttt cttttctctc attgtccacc ttactaaaag tcagtagaat 2250

cttctacctc ataacttcct tccaaaggca gctcagaaga ttagaaccag 2300

acttactaac caattccacc ccccaccaac ccccttctac tgcctacttt 2350

aaaaaaatta atagttttct atggaactga tctaagatta gaaaaattaa 2400

ttttctttaa tttcattatg gacttttatt tacatgactc taagactata 2450

agaaaatctg atggcagtga caaagtgcta gcatttattg ttatctaata 2500

aagaccttgg agcatatgtg caacttatga gtgtatcagt tgttgcatgt 2550

aatttttgcc tttgtttaag cctggaactt gtaagaaaat gaaaatttaa 2600

tttttttttc taggacgagc tatagaaaag ctattgagag tatctagtta 2650

atcagtgcag tagttggaaa ccttgctggt gtatgtgatg tgcttctgtg 2700

cttttgaatg actttatcat ctagtctttg tctatttttc ctttgatgtt 2750

caagtcctag tctataggat tggcagttta aatgctttac tccccctttt 2800

aaaataaatg attaaaatgt gctttgaaaa aaaaaaaaaa aaaaaaaaaa 2850

aaaa 2854

<210> 10
<211> 510
<212> PRT
<213> Homo Sapien

<400> 10
Met Arg Pro Gly Leu Ser Phe Leu Leu Ala Leu Leu Phe Phe Leu
1               5                   10                  15

Gly Gln Ala Ala Gly Asp Leu Gly Asp Val Gly Pro Pro Ile Pro
                20                  25                  30

Ser Pro Gly Phe Ser Ser Phe Pro Gly Val Asp Ser Ser Ser Ser
                35                  40                  45

Phe Ser Ser Ser Ser Arg Ser Gly Ser Ser Ser Ser Arg Ser Leu
                50                  55                  60

Gly Ser Gly Gly Ser Val Ser Gln Leu Phe Ser Asn Phe Thr Gly
                65                  70                  75

Ser Val Asp Asp Arg Gly Thr Cys Gln Cys Ser Val Ser Leu Pro
                80                  85                  90

Asp Thr Thr Phe Pro Val Asp Arg Val Glu Arg Leu Glu Phe Thr
                95                  100                 105

Ala His Val Leu Ser Gln Lys Phe Glu Lys Glu Leu Ser Lys Val
                110                 115                 120

Arg Glu Tyr Val Gln Leu Ile Ser Val Tyr Glu Lys Lys Leu Leu
125 130 135

Asn Leu Thr Val Arg Ile Asp Ile Met Glu Lys Asp Thr Ile Ser
140 145 150

Tyr Thr Glu Leu Asp Phe Glu Leu Ile Lys Val Glu Val Lys Glu
155 160 165

Met Glu Lys Leu Val Ile Gln Leu Lys Glu Ser Phe Gly Gly Ser
170 175 180

Ser Glu Ile Val Asp Gln Leu Glu Val Glu Ile Arg Asn Met Thr
185 190 195

Leu Leu Val Glu Lys Leu Glu Thr Leu Asp Lys Asn Asn Val Leu
200 205 210

Ala Ile Arg Arg Glu Ile Val Ala Leu Lys Thr Lys Leu Lys Glu
215 220 225

Cys Glu Ala Ser Lys Asp Gln Asn Thr Pro Val Val His Pro Pro
230 235 240

Pro Thr Pro Gly Ser Cys Gly His Gly Gly Val Val Asn Ile Ser
245 250 255

Lys Pro Ser Val Val Gln Leu Asn Trp Arg Gly Phe Ser Tyr Leu
260 265 270

Tyr Gly Ala Trp Gly Arg Asp Tyr Ser Pro Gln His Pro Asn Lys
275 280 285

Gly Leu Tyr Trp Val Ala Pro Leu Asn Thr Asp Gly Arg Leu Leu
290 295 300

Glu Tyr Tyr Arg Leu Tyr Asn Thr Leu Asp Asp Leu Leu Leu Tyr
305 310 315

Ile Asn Ala Arg Glu Leu Arg Ile Thr Tyr Gly Gln Gly Ser Gly
320 325 330

Thr Ala Val Tyr Asn Asn Asn Met Tyr Val Asn Met Tyr Asn Thr
335 340 345

Gly Asn Ile Ala Arg Val Asn Leu Thr Thr Asn Thr Ile Ala Val
350 355 360

Thr Gln Thr Leu Pro Asn Ala Ala Tyr Asn Asn Arg Phe Ser Tyr
365 370 375

Ala Asn Val Ala Trp Gln Asp Ile Asp Phe Ala Val Asp Glu Asn
380 385 390

Gly Leu Trp Val Ile Tyr Ser Thr Glu Ala Ser Thr Gly Asn Met
395 400 405

Val Ile Ser Lys Leu Asn Asp Thr Thr Leu Gln Val Leu Asn Thr

410 415 420

Trp Tyr Thr Lys Gln Tyr Lys Pro Ser Ala Ser Asn Ala Phe Met
425 430 435

Val Cys Gly Val Leu Tyr Ala Thr Arg Thr Met Asn Thr Arg Thr
440 445 450

Glu Glu Ile Phe Tyr Tyr Tyr Asp Thr Asn Thr Gly Lys Glu Gly
455 460 465

Lys Leu Asp Ile Val Met His Lys Met Gln Glu Lys Val Gln Ser
470 475 480

Ile Asn Tyr Asn Pro Phe Asp Gln Lys Leu Tyr Val Tyr Asn Asp
485 490 495

Gly Tyr Leu Leu Asn Tyr Asp Leu Ser Val Leu Gln Lys Pro Gln
500 505 510

<210> 11
<211> 662
<212> DNA
<213> Homo Sapien

<400> 11
attctcctag agcatctttg gaagcatgag gccacgatgc tgcatcttgg 50

ctcttgtctg ctggataaca gtcttcctcc tccagtgttc aaaaggaact 100

acagacgctc ctgttggctc aggactgtgg ctgtgccagc cgacacccag 150

gtgtgggaac aagatctaca acccttcaga gcagtgctgt tatgatgatg 200

ccatcttatc cttaaaggag acccgccgct gtggctccac ctgcaccttc 250

tggccctgct ttgagctctg ctgtcccgag tcttttggcc cccagcagaa 300

gtttcttgtg aagttgaggg ttctgggtat gaagtctcag tgtcacttat 350

ctcccatctc ccggagctgt accaggaaca ggaggcacgt cctgtaccca 400

taaaaacccc aggctccact ggcagacggc agacaagggg agaagagacg 450

aagcagctgg acatcggaga ctacagttga acttcggaga gaagcaactt 500

gacttcagag ggatggctca atgacatagc tttggagagg agcccagctg 550

gggatggcca gacttcaggg gaagaatgcc ttcctgcttc atcccctttc 600

cagctcccct tcccgctgag agccactttc atcggcaata aaatccccca 650

catttaccat ct 662

<210> 12
<211> 125
<212> PRT
<213> Homo Sapien

<400> 12

```
Met Arg Pro Arg Cys Cys Ile Leu Ala Leu Val Cys Trp Ile Thr
1               5                   10                  15

Val Phe Leu Leu Gln Cys Ser Lys Gly Thr Thr Asp Ala Pro Val
                20                  25                  30

Gly Ser Gly Leu Trp Leu Cys Gln Pro Thr Pro Arg Cys Gly Asn
                35                  40                  45

Lys Ile Tyr Asn Pro Ser Glu Gln Cys Cys Tyr Asp Asp Ala Ile
                50                  55                  60

Leu Ser Leu Lys Glu Thr Arg Arg Cys Gly Ser Thr Cys Thr Phe
                65                  70                  75

Trp Pro Cys Phe Glu Leu Cys Cys Pro Glu Ser Phe Gly Pro Gln
                80                  85                  90

Gln Lys Phe Leu Val Lys Leu Arg Val Leu Gly Met Lys Ser Gln
                95                  100                 105

Cys His Leu Ser Pro Ile Ser Arg Ser Cys Thr Arg Asn Arg Arg
                110                 115                 120

His Val Leu Tyr Pro
                125
```

<210> 13
<211> 745
<212> DNA
<213> Homo Sapien

<400> 13

```
cctctgtcca ctgctttcgt gaagacaaga tgaagttcac aattgtcttt 50

gctggacttc ttggagtctt tctagctcct gccctagcta actataatat 100

caacgtcaat gatgacaaca acaatgctgg aagtgggcag cagtcagtga 150

gtgtcaacaa tgaacacaat gtggccaatg ttgacaataa caacggatgg 200

gactcctgga attccatctg ggattatgga aatggctttg ctgcaaccag 250

actctttcaa aagaagacat gcattgtgca caaaatgaac aaggaagtca 300

tgccctccat tcaatccctt gatgcactgg tcaaggaaaa gaagcttcag 350

ggtaagggac caggaggacc acctcccaag ggcctgatgt actcagtcaa 400

cccaaacaaa gtcgatgacc tgagcaagtt cggaaaaaac attgcaaaca 450

tgtgtcgtgg gattccaaca tacatggctg aggagatgca agaggcaagc 500

ctgttttttt actcaggaac gtgctacacg accagtgtac tatggattgt 550

ggacatttcc ttctgtggag acacggtgga gaactaaaca atttttttaaa 600

gccactatgg atttagtcat ctgaatatgc tgtgcagaaa aaatatgggc 650
```

tccagtggtt tttaccatgt cattctgaaa tttttctcta ctagttatgt 700

ttgatttctt taagtttcaa taaaatcatt tagcattgaa aaaaa 745

<210> 14
<211> 185
<212> PRT
<213> Homo Sapien

<400> 14

```
Met Lys Phe Thr Ile Val Phe Ala Gly Leu Leu Gly Val Phe Leu
  1               5                  10                  15

Ala Pro Ala Leu Ala Asn Tyr Asn Ile Asn Val Asn Asp Asp Asn
                 20                  25                  30

Asn Asn Ala Gly Ser Gly Gln Gln Ser Val Ser Val Asn Asn Glu
                 35                  40                  45

His Asn Val Ala Asn Val Asp Asn Asn Asn Gly Trp Asp Ser Trp
                 50                  55                  60

Asn Ser Ile Trp Asp Tyr Gly Asn Gly Phe Ala Ala Thr Arg Leu
                 65                  70                  75

Phe Gln Lys Lys Thr Cys Ile Val His Lys Met Asn Lys Glu Val
                 80                  85                  90

Met Pro Ser Ile Gln Ser Leu Asp Ala Leu Val Lys Glu Lys Lys
                 95                 100                 105

Leu Gln Gly Lys Gly Pro Gly Gly Pro Pro Pro Lys Gly Leu Met
                110                 115                 120

Tyr Ser Val Asn Pro Asn Lys Val Asp Asp Leu Ser Lys Phe Gly
                125                 130                 135

Lys Asn Ile Ala Asn Met Cys Arg Gly Ile Pro Thr Tyr Met Ala
                140                 145                 150

Glu Glu Met Gln Glu Ala Ser Leu Phe Phe Tyr Ser Gly Thr Cys
                155                 160                 165

Tyr Thr Thr Ser Val Leu Trp Ile Val Asp Ile Ser Phe Cys Gly
                170                 175                 180

Asp Thr Val Glu Asn
                185
```

<210> 15
<211> 1575
<212> DNA
<213> Homo Sapien

<400> 15

gagcaggacg gagccatgga ccccgccagg aaagcaggtg cccaggccat 50

gatctggact gcaggctggc tgctgctgct gctgcttcgc ggaggagcgc 100

aggccctgga gtgctacagc tgcgtgcaga aagcagatga cggatgctcc 150
ccgaacaaga tgaagacagt gaagtgcgcg ccgggcgtgg acgtctgcac 200
cgaggccgtg gggcggtgg agaccatcca cggacaattc tcgctggcag 250
tgcggggttg cggttcggga ctccccggca agaatgaccg cggcctggat 300
cttcacgggc ttctggcgtt catccagctg cagcaatgcg ctcaggatcg 350
ctgcaacgcc aagctcaacc tcacctcgcg ggcgctcgac ccggcaggta 400
atgagagtgc atacccgccc aacggcgtgg agtgctacag ctgtgtgggc 450
ctgagccggg aggcgtgcca gggtacatcg ccgccggtcg tgagctgcta 500
caacgccagc gatcatgtct acaagggctg cttcgacggc aacgtcacct 550
tgacggcagc taatgtgact gtgtccttgc ctgtccgggg ctgtgtccag 600
gatgaattct gcactcggga tggagtaaca ggcccagggt tcacgctcag 650
tggctcctgt tgccaggggt cccgctgtaa ctctgacctc cgcaacaaga 700
cctacttctc ccctcgaatc ccaccccttg tccggctgcc ccctccagag 750
cccacgactg tggcctcaac cacatctgtc accacttcta cctcggcccc 800
agtgagaccc acatccacca ccaaacccat gccagcgcca accagtcaga 850
ctccgagaca gggagtagaa cacgaggcct cccgggatga ggagcccagg 900
ttgactggag gcgccgctgg ccaccaggac cgcagcaatt cagggcagta 950
tcctgcaaaa ggggggcccc agcagcccca taataaaggc tgtgtggctc 1000
ccacagctgg attggcagcc cttctgttgg ccgtggctgc tggtgtccta 1050
ctgtgagctt ctccacctgg aaatttccct ctcacctact tctctggccc 1100
tgggtacccc tcttctcatc acttcctgtt cccaccactg gactgggctg 1150
gcccagcccc tgtttttcca acattcccca gtatccccag cttctgctgc 1200
gctggtttgc ggctttggga aataaaatac cgttgtatat attctgccag 1250
gggtgttcta gctttttgag gacagctcct gtatccttct catccttgtc 1300
tctccgcttg tcctcttgtg atgttaggac agagtgagag aagtcagctg 1350
tcacggggaa ggtgagagag aggatgctaa gcttcctact cactttctcc 1400
tagccagcct ggactttgga gcgtggggtg ggtgggacaa tggctcccca 1450
ctctaagcac tgcctcccct actccccgca tctttgggga atcggttccc 1500
catatgtctt ccttactaga ctgtgagctc ctcgaggggg ggcccggtac 1550

ccaattcgcc ctatagtgag tcgta 1575

<210> 16
<211> 346
<212> PRT
<213> Homo Sapien

<400> 16
```
Met Asp Pro Ala Arg Lys Ala Gly Ala Gln Ala Met Ile Trp Thr
 1               5                  10                  15

Ala Gly Trp Leu Leu Leu Leu Leu Leu Arg Gly Gly Ala Gln Ala
                20                  25                  30

Leu Glu Cys Tyr Ser Cys Val Gln Lys Ala Asp Asp Gly Cys Ser
                35                  40                  45

Pro Asn Lys Met Lys Thr Val Lys Cys Ala Pro Gly Val Asp Val
                50                  55                  60

Cys Thr Glu Ala Val Gly Ala Val Glu Thr Ile His Gly Gln Phe
                65                  70                  75

Ser Leu Ala Val Arg Gly Cys Gly Ser Gly Leu Pro Gly Lys Asn
                80                  85                  90

Asp Arg Gly Leu Asp Leu His Gly Leu Leu Ala Phe Ile Gln Leu
                95                 100                 105

Gln Gln Cys Ala Gln Asp Arg Cys Asn Ala Lys Leu Asn Leu Thr
               110                 115                 120

Ser Arg Ala Leu Asp Pro Ala Gly Asn Glu Ser Ala Tyr Pro Pro
               125                 130                 135

Asn Gly Val Glu Cys Tyr Ser Cys Val Gly Leu Ser Arg Glu Ala
               140                 145                 150

Cys Gln Gly Thr Ser Pro Pro Val Val Ser Cys Tyr Asn Ala Ser
               155                 160                 165

Asp His Val Tyr Lys Gly Cys Phe Asp Gly Asn Val Thr Leu Thr
               170                 175                 180

Ala Ala Asn Val Thr Val Ser Leu Pro Val Arg Gly Cys Val Gln
               185                 190                 195

Asp Glu Phe Cys Thr Arg Asp Gly Val Thr Gly Pro Gly Phe Thr
               200                 205                 210

Leu Ser Gly Ser Cys Cys Gln Gly Ser Arg Cys Asn Ser Asp Leu
               215                 220                 225

Arg Asn Lys Thr Tyr Phe Ser Pro Arg Ile Pro Pro Leu Val Arg
               230                 235                 240

Leu Pro Pro Pro Glu Pro Thr Thr Val Ala Ser Thr Thr Ser Val
               245                 250                 255
```

Thr Thr Ser Thr Ser Ala Pro Val Arg Pro Thr Ser Thr Thr Lys
260 265 270

Pro Met Pro Ala Pro Thr Ser Gln Thr Pro Arg Gln Gly Val Glu
275 280 285

His Glu Ala Ser Arg Asp Glu Glu Pro Arg Leu Thr Gly Gly Ala
290 295 300

Ala Gly His Gln Asp Arg Ser Asn Ser Gly Gln Tyr Pro Ala Lys
305 310 315

Gly Gly Pro Gln Gln Pro His Asn Lys Gly Cys Val Ala Pro Thr
320 325 330

Ala Gly Leu Ala Ala Leu Leu Leu Ala Val Ala Ala Gly Val Leu
335 340 345

Leu

<210> 17
<211> 1841
<212> DNA
<213> Homo Sapien

<400> 17
gcagtcagag acttcccctg cccctcgctg ggaaagaaca ttaggaatgc 50

cttttagtgc cttgcttcct gaactagctc acagtagccc ggcggcccag 100

ggcaatccga ccacatttca ctctcaccgc tgtaggaatc cagatgcagg 150

ccaagtacag cagcacgagg gacatgctgg atgatgatgg ggacaccacc 200

atgagcctgc attctcaagc ctctgccaca actcggcatc cagagccccg 250

gcgcacagag cacagggctc cctcttcaac gtggcgacca gtggccctga 300

ccctgctgac tttgtgcttg gtgctgctga tagggctggc agccctgggg 350

cttttgtttt ttcagtacta ccagctctcc aatactggtc aagacaccat 400

ttctcaaatg gaagaaagat taggaaatac gtcccaagag ttgcaatctc 450

ttcaagtcca gaatataaag cttgcaggaa gtctgcagca tgtggctgaa 500

aaactctgtc gtgagctgta taacaaagct ggagcacaca ggtgcagccc 550

ttgtacagaa caatggaaat ggcatggaga caattgctac cagttctata 600

aagacagcaa aagttgggag gactgtaaat atttctgcct tagtgaaaac 650

tctaccatgc tgaagataaa caaacaagaa gacctggaat ttgccgcgtc 700

tcagagctac tctgagtttt tctactctta ttggacaggg cttttgcgcc 750

ctgacagtgg caaggcctgg ctgtggatgg atggaacccc tttcacttct 800

gaactgttcc atattataat agatgtcacc agcccaagaa gcagagactg 850
tgtggccatc ctcaatggga tgatcttctc aaaggactgc aaagaattga 900
agcgttgtgt ctgtgagaga agggcaggaa tggtgaagcc agagagcctc 950
catgtccccc ctgaaacatt aggcgaaggt gactgattcg ccctctgcaa 1000
ctacaaatag cagagtgagc caggcggtgc caaagcaagg gctagttgag 1050
acattgggaa atggaacata atcaggaaag actatctctc tgactagtac 1100
aaaatgggtt ctcgtgtttc ctgttcagga tcaccagcat ttctgagctt 1150
gggtttatgc acgtatttaa cagtcacaag aagtcttatt tacatgccac 1200
caaccaacct cagaaaccca taatgtcatc tgccttcttg gcttagagat 1250
aacttttagc tctctttctt ctcaatgtct aatatcacct ccctgttttc 1300
atgtcttcct tacacttggt ggaataagaa actttttgaa gtagaggaaa 1350
tacattgagg taacatcctt ttctctgaca gtcaagtagt ccatcagaaa 1400
ttggcagtca cttcccagat tgtaccagca aatacacaag gaattctttt 1450
tgtttgtttc agttcatact agtcccttcc caatccatca gtaaagaccc 1500
catctgcctt gtccatgccg tttcccaaca gggatgtcac ttgatatgag 1550
aatctcaaat ctcaatgcct tataagcatt ccttcctgtg tccattaaga 1600
ctctgataat tgtctcccct ccataggaat ttctcccagg aaagaaatat 1650
atccccatct ccgtttcata tcagaactac cgtccccgat attcccttca 1700
gagagattaa agaccagaaa aaagtgagcc tcttcatctg cacctgtaat 1750
agtttcagtt cctattttct tccattgacc catatttata cctttcaggt 1800
actgaagatt taataataat aaatgtaaat actgtgaaaa a 1841

<210> 18
<211> 280
<212> PRT
<213> Homo Sapien

<400> 18
Met Gln Ala Lys Tyr Ser Ser Thr Arg Asp Met Leu Asp Asp Asp
1 5 10 15

Gly Asp Thr Thr Met Ser Leu His Ser Gln Ala Ser Ala Thr Thr
20 25 30

Arg His Pro Glu Pro Arg Arg Thr Glu His Arg Ala Pro Ser Ser
35 40 45

Thr Trp Arg Pro Val Ala Leu Thr Leu Leu Thr Leu Cys Leu Val
50 55 60

Leu Leu Ile Gly Leu Ala Ala Leu Gly Leu Leu Phe Phe Gln Tyr
65 70 75

Tyr Gln Leu Ser Asn Thr Gly Gln Asp Thr Ile Ser Gln Met Glu
80 85 90

Glu Arg Leu Gly Asn Thr Ser Gln Glu Leu Gln Ser Leu Gln Val
95 100 105

Gln Asn Ile Lys Leu Ala Gly Ser Leu Gln His Val Ala Glu Lys
110 115 120

Leu Cys Arg Glu Leu Tyr Asn Lys Ala Gly Ala His Arg Cys Ser
125 130 135

Pro Cys Thr Glu Gln Trp Lys Trp His Gly Asp Asn Cys Tyr Gln
140 145 150

Phe Tyr Lys Asp Ser Lys Ser Trp Glu Asp Cys Lys Tyr Phe Cys
155 160 165

Leu Ser Glu Asn Ser Thr Met Leu Lys Ile Asn Lys Gln Glu Asp
170 175 180

Leu Glu Phe Ala Ala Ser Gln Ser Tyr Ser Glu Phe Phe Tyr Ser
185 190 195

Tyr Trp Thr Gly Leu Leu Arg Pro Asp Ser Gly Lys Ala Trp Leu
200 205 210

Trp Met Asp Gly Thr Pro Phe Thr Ser Glu Leu Phe His Ile Ile
215 220 225

Ile Asp Val Thr Ser Pro Arg Ser Arg Asp Cys Val Ala Ile Leu
230 235 240

Asn Gly Met Ile Phe Ser Lys Asp Cys Lys Glu Leu Lys Arg Cys
245 250 255

Val Cys Glu Arg Arg Ala Gly Met Val Lys Pro Glu Ser Leu His
260 265 270

Val Pro Pro Glu Thr Leu Gly Glu Gly Asp
275 280

<210> 19
<211> 446
<212> DNA
<213> Homo Sapien

<400> 19
tggacttctc tggaccacag tcctctgcca gacccctgcc agaccccagt 50

ccaccatgat ccatctgggt cacatcctct tcctgctttt gctcccagtg 100

gctgcagctc agacgactcc aggagagaga tcatcactcc ctgcctttta 150

ccctggcact tcaggctctt gttccggatg tgggtccctc tctctgccgc 200

tcctggcagg cctcgtggct gctgatgcgg tggcatcgct gctcatcgtg 250

ggggcggtgt tcctgtgcgc acgcccacgc cgcagccccg cccaagatgg 300

caaagtctac atcaacatgc caggcagggg ctgaccctcc tgcagcttgg 350

acctttgact tctgaccctc tcatcctgga tggtgtgtgg tggcacagga 400

acccccgccc caacttttgg attgtaataa aacaattgaa acacca 446

<210> 20
<211> 92
<212> PRT
<213> Homo Sapien

<400> 20
Met Ile His Leu Gly His Ile Leu Phe Leu Leu Leu Leu Pro Val
1 5 10 15

Ala Ala Ala Gln Thr Thr Pro Gly Glu Arg Ser Ser Leu Pro Ala
20 25 30

Phe Tyr Pro Gly Thr Ser Gly Ser Cys Ser Gly Cys Gly Ser Leu
35 40 45

Ser Leu Pro Leu Leu Ala Gly Leu Val Ala Ala Asp Ala Val Ala
50 55 60

Ser Leu Leu Ile Val Gly Ala Val Phe Leu Cys Ala Arg Pro Arg
65 70 75

Arg Ser Pro Ala Gln Asp Gly Lys Val Tyr Ile Asn Met Pro Gly
80 85 90

Arg Gly

<210> 21
<211> 462
<212> DNA
<213> Homo Sapien

<400> 21
agcccaccga gaggcgcctg caggatgaaa gctctctgtc tcctcctcct 50

ccctgtcctg gggctgttgg tgtctagcaa gaccctgtgc tccatggaag 100

aagccatcaa tgagaggatc caggaggtcg ccggctccct aatatttagg 150

gcaataagca gcattggcct ggagtgccag agcgtcacct ccaggggga 200

cctggctact tgcccccgag gcttcgccgt caccggctgc acttgtggct 250

ccgcctgtgg ctcgtgggat gtgcgcgccg agaccacatg tcactgccag 300

tgcgcgggca tggactggac cggagcgcgc tgctgtcgtg tgcagccctg 350

aggtcgcgcg cagcgcgtgc acagcgcggg cggaggcggc tccaggtccg 400

gaggggttgc gggggagctg gaaataaacc tggagatgat gatgatgatg 450

atgatggaaa aa 462

<210> 22
<211> 108
<212> PRT
<213> Homo Sapien

<400> 22
Met Lys Ala Leu Cys Leu Leu Leu Leu Pro Val Leu Gly Leu Leu
1 5 10 15

Val Ser Ser Lys Thr Leu Cys Ser Met Glu Glu Ala Ile Asn Glu
20 25 30

Arg Ile Gln Glu Val Ala Gly Ser Leu Ile Phe Arg Ala Ile Ser
35 40 45

Ser Ile Gly Leu Glu Cys Gln Ser Val Thr Ser Arg Gly Asp Leu
50 55 60

Ala Thr Cys Pro Arg Gly Phe Ala Val Thr Gly Cys Thr Cys Gly
65 70 75

Ser Ala Cys Gly Ser Trp Asp Val Arg Ala Glu Thr Thr Cys His
80 85 90

Cys Gln Cys Ala Gly Met Asp Trp Thr Gly Ala Arg Cys Cys Arg
95 100 105

Val Gln Pro

<210> 23
<211> 1844
<212> DNA
<213> Homo Sapien

<400> 23
gacagtggag ggcagtggag aggaccgcgc tgtcctgctg tcaccaagag 50

ctggagacac catctcccac cgagagtcat ggccccattg gccctgcacc 100

tcctcgtcct cgtccccatc ctcctcagcc tggtggcctc ccaggactgg 150

aaggctgaac gcagccaaga ccccttcgag aaatgcatgc aggatcctga 200

ctatgagcag ctgctcaagg tggtgacctg gggctcaat cggaccctga 250

agccccagag ggtgattgtg gttggcgctg gtgtggccgg gctggtggcc 300

gccaaggtgc tcagcgatgc tggacacaag gtcaccatcc tggaggcaga 350

taacaggatc gggggccgca tcttcaccta ccgggaccag aacacgggct 400

ggattgggga gctgggagcc atgcgcatgc ccagctctca caggatcctc 450

cacaagctct gccagggcct ggggctcaac ctgaccaagt tcacccagta 500
cgacaagaac acgtggacgg aggtgcacga agtgaagctg cgcaactatg 550
tggtggagaa ggtgcccgag aagctgggct acgccttgcg tccccaggaa 600
aagggccact cgcccgaaga catctaccag atggctctca accaggccct 650
caaagacctc aaggcactgg gctgcagaaa ggcgatgaag aagtttgaaa 700
ggcacacgct cttggaatat cttctcgggg aggggaacct gagccggccg 750
gccgtgcagc ttctgggaga cgtgatgtcc gaggatggct tcttctatct 800
cagcttcgcc gaggccctcc gggcccacag ctgcctcagc gacagactcc 850
agtacagccg catcgtgggt ggctgggacc tgctgccgcg cgcgctgctg 900
agctcgctgt ccgggcttgt gctgttgaac gcgcccgtgg tggcgatgac 950
ccagggaccg cacgatgtgc acgtgcagat cgagacctct cccccggcgc 1000
ggaatctgaa ggtgctgaag gccgacgtgg tgctgctgac ggcgagcgga 1050
ccggcggtga agcgcatcac cttctcgccg ccgctgcccc gccacatgca 1100
ggaggcgctg cggaggctgc actacgtgcc ggccaccaag gtgttcctaa 1150
gcttccgcag gcccttctgg cgcgaggagc acattgaagg cggccactca 1200
aacaccgatc gcccgtcgcg catgattttc tacccgccgc cgcgcgaggg 1250
cgcgctgctg ctggcctcgt acacgtggtc ggacgcggcg gcagcgttcg 1300
ccggcttgag ccgggaagag gcgttgcgct tggcgctcga cgacgtggcg 1350
gcattgcacg ggcctgtcgt gcgccagctc tgggacggca ccggcgtcgt 1400
caagcgttgg gcggaggacc agcacagcca gggtggcttt gtggtacagc 1450
cgccggcgct ctggcaaacc gaaaaggatg actggacggt cccttatggc 1500
cgcatctact ttgccggcga gcacaccgcc tacccgcacg gctgggtgga 1550
gacggcggtc aagtcggcgc tgcgcgccgc catcaagatc aacagccgga 1600
aggggcctgc atcggacacg gccagccccg aggggcacgc atctgacatg 1650
gaggggcagg ggcatgtgca tggggtggcc agcagcccct cgcatgacct 1700
ggcaaaggaa gaaggcagcc accctccagt ccaaggccag ttatctctcc 1750
aaaacacgac ccacacgagg acctcgcatt aaagtatttt cggaaaaaaa 1800
aaaaaaaaaa aaaaaaaaaa aaaaaaaaaa aaaaaaaaaa aaaa 1844

<210> 24
<211> 567
<212> PRT

<213> Homo Sapien

<400> 24

```
Met Ala Pro Leu Ala Leu His Leu Leu Val Leu Val Pro Ile Leu
1               5                   10                  15

Leu Ser Leu Val Ala Ser Gln Asp Trp Lys Ala Glu Arg Ser Gln
                20                  25                  30

Asp Pro Phe Glu Lys Cys Met Gln Asp Pro Asp Tyr Glu Gln Leu
                35                  40                  45

Leu Lys Val Val Thr Trp Gly Leu Asn Arg Thr Leu Lys Pro Gln
                50                  55                  60

Arg Val Ile Val Val Gly Ala Gly Val Ala Gly Leu Val Ala Ala
                65                  70                  75

Lys Val Leu Ser Asp Ala Gly His Lys Val Thr Ile Leu Glu Ala
                80                  85                  90

Asp Asn Arg Ile Gly Gly Arg Ile Phe Thr Tyr Arg Asp Gln Asn
                95                  100                 105

Thr Gly Trp Ile Gly Glu Leu Gly Ala Met Arg Met Pro Ser Ser
                110                 115                 120

His Arg Ile Leu His Lys Leu Cys Gln Gly Leu Gly Leu Asn Leu
                125                 130                 135

Thr Lys Phe Thr Gln Tyr Asp Lys Asn Thr Trp Thr Glu Val His
                140                 145                 150

Glu Val Lys Leu Arg Asn Tyr Val Val Glu Lys Val Pro Glu Lys
                155                 160                 165

Leu Gly Tyr Ala Leu Arg Pro Gln Glu Lys Gly His Ser Pro Glu
                170                 175                 180

Asp Ile Tyr Gln Met Ala Leu Asn Gln Ala Leu Lys Asp Leu Lys
                185                 190                 195

Ala Leu Gly Cys Arg Lys Ala Met Lys Lys Phe Glu Arg His Thr
                200                 205                 210

Leu Leu Glu Tyr Leu Leu Gly Glu Gly Asn Leu Ser Arg Pro Ala
                215                 220                 225

Val Gln Leu Leu Gly Asp Val Met Ser Glu Asp Gly Phe Phe Tyr
                230                 235                 240

Leu Ser Phe Ala Glu Ala Leu Arg Ala His Ser Cys Leu Ser Asp
                245                 250                 255

Arg Leu Gln Tyr Ser Arg Ile Val Gly Gly Trp Asp Leu Leu Pro
                260                 265                 270

Arg Ala Leu Leu Ser Ser Leu Ser Gly Leu Val Leu Leu Asn Ala
                275                 280                 285
```

```
Pro Val Val Ala Met Thr Gln Gly Pro His Asp Val His Val Gln
                290                 295                 300

Ile Glu Thr Ser Pro Pro Ala Arg Asn Leu Lys Val Leu Lys Ala
                305                 310                 315

Asp Val Val Leu Leu Thr Ala Ser Gly Pro Ala Val Lys Arg Ile
                320                 325                 330

Thr Phe Ser Pro Pro Leu Pro Arg His Met Gln Glu Ala Leu Arg
                335                 340                 345

Arg Leu His Tyr Val Pro Ala Thr Lys Val Phe Leu Ser Phe Arg
                350                 355                 360

Arg Pro Phe Trp Arg Glu Glu His Ile Glu Gly Gly His Ser Asn
                365                 370                 375

Thr Asp Arg Pro Ser Arg Met Ile Phe Tyr Pro Pro Pro Arg Glu
                380                 385                 390

Gly Ala Leu Leu Leu Ala Ser Tyr Thr Trp Ser Asp Ala Ala Ala
                395                 400                 405

Ala Phe Ala Gly Leu Ser Arg Glu Glu Ala Leu Arg Leu Ala Leu
                410                 415                 420

Asp Asp Val Ala Ala Leu His Gly Pro Val Val Arg Gln Leu Trp
                425                 430                 435

Asp Gly Thr Gly Val Val Lys Arg Trp Ala Glu Asp Gln His Ser
                440                 445                 450

Gln Gly Gly Phe Val Val Gln Pro Pro Ala Leu Trp Gln Thr Glu
                455                 460                 465

Lys Asp Asp Trp Thr Val Pro Tyr Gly Arg Ile Tyr Phe Ala Gly
                470                 475                 480

Glu His Thr Ala Tyr Pro His Gly Trp Val Glu Thr Ala Val Lys
                485                 490                 495

Ser Ala Leu Arg Ala Ala Ile Lys Ile Asn Ser Arg Lys Gly Pro
                500                 505                 510

Ala Ser Asp Thr Ala Ser Pro Glu Gly His Ala Ser Asp Met Glu
                515                 520                 525

Gly Gln Gly His Val His Gly Val Ala Ser Ser Pro Ser His Asp
                530                 535                 540

Leu Ala Lys Glu Glu Gly Ser His Pro Pro Val Gln Gly Gln Leu
                545                 550                 555

Ser Leu Gln Asn Thr Thr His Thr Arg Thr Ser His
                560                 565
```

<210> 25

<211> 693
<212> DNA
<213> Homo Sapien

<400> 25
ctagcctgcg ccaaggggta gtgagaccgc gcggcaacag cttgcggctg 50

cggggagctc ccgtgggcgc tccgctggct gtgcaggcgg ccatggattc 100

cttgcggaaa atgctgatct cagtcgcaat gctgggcgca ggggctggcg 150

tgggctacgc gctcctcgtt atcgtgaccc cgggagagcg gcggaagcag 200

gaaatgctaa aggagatgcc actgcaggac ccaaggagca gggaggaggc 250

ggccaggacc cagcagctat tgctggccac tctgcaggag gcagcgacca 300

cgcaggagaa cgtggcctgg aggaagaact ggatggttgg cggcgaaggc 350

ggcgccagcg ggaggtcacc gtgagaccgg acttgcctcc gtgggcgccg 400

gaccttggct tgggcgcagg aatccgaggc agcctttctc cttcgtgggc 450

ccagcggaga gtccggaccg agataccatg ccaggactct ccggggtcct 500

gtgagctgcc gtcgggtgag cacgtttccc ccaaaccctg gactgactgc 550

tttaaggtcc gcaaggcggg ccagggccga gacgcgagtc ggatgtggtg 600

aactgaaaga accaataaaa tcatgttcct ccaaaaaaaa aaaaaaaaaa 650

aaaaaaaaaa aaaaaaaaaa aaaaaaaaaa aaaaaaaaaa aaa 693

<210> 26
<211> 93
<212> PRT
<213> Homo Sapien

<400> 26
Met Asp Ser Leu Arg Lys Met Leu Ile Ser Val Ala Met Leu Gly
1 5 10 15

Ala Gly Ala Gly Val Gly Tyr Ala Leu Leu Val Ile Val Thr Pro
20 25 30

Gly Glu Arg Arg Lys Gln Glu Met Leu Lys Glu Met Pro Leu Gln
35 40 45

Asp Pro Arg Ser Arg Glu Glu Ala Ala Arg Thr Gln Gln Leu Leu
50 55 60

Leu Ala Thr Leu Gln Glu Ala Ala Thr Thr Gln Glu Asn Val Ala
65 70 75

Trp Arg Lys Asn Trp Met Val Gly Gly Glu Gly Gly Ala Ser Gly
80 85 90

Arg Ser Pro

<210> 27
<211> 1181
<212> DNA
<213> Homo Sapien

<400> 27
aagaccctct ctttcgctgt ttgagagtct ctcggctcaa ggaccgggag 50

gtaagaggtt tgggactgcc ccggcaactc cagggtgtct ggtccacgac 100

ctatcctagg cgccatgggt gtgataggta tacagctggt tgttaccatg 150

gtgatggcca gtgtcatgca gaagattata cctcactatt ctcttgctcg 200

atggctactc tgtaatggca gtttgaggtg gtatcaacat cctacagaag 250

aagaattaag aattcttgca gggaaacaac aaaaagggaa aaccaaaaaa 300

gataggaaat ataatggtca cattgaaagt aagccattaa ccattccaaa 350

ggatattgac cttcatctag aaacaaagtc agttacagaa gtggatactt 400

tagcattgca ttactttcca gaataccagt ggctggtgga tttcacagtg 450

gctgctacag ttgtgtatct agtaactgaa gtctactaca attttatgaa 500

gcctacacag gaaatgaata tcagcttagt ctggtgccta cttgttttgt 550

cttttgcaat caaagttcta ttttcattaa ctacacacta ttttaaagta 600

gaagatggtg gtgaaagatc tgtttgtgtc acctttggat ttttttctt 650

tgtcaaagca atggcagtgt tgattgtaac agaaaattat ctggaatttg 700

gacttgaaac agggtttaca aatttttcag acagtgcgat gcagtttctt 750

gaaaagcaag gtttagaatc tcagagtcct gtttcaaaac ttactttcaa 800

atttttcctg gctattttct gttcattcat tggggctttt ttgacatttc 850

ctggattacg actggctcaa atgcatctgg atgccctgaa tttggcaaca 900

gaaaaaatta cacaaacttt acttcatatc aacttcttgg cacctttatt 950

tatggttttg ctctgggtaa aaccaatcac caaagactac attatgaacc 1000

caccactggg caaagaaatt tccccatctg gaagatgaag ataatagtat 1050

ctaactcaca aggttatcat tggaataaat gaaagaacac atgtaatgca 1100

accagctgga attaagtgct taataaatgt tcttttcact gctttgcctc 1150

atcagaatta aaatagaaat acttgactag t 1181

<210> 28
<211> 307
<212> PRT
<213> Homo Sapien

<400> 28

```
Met Gly Val Ile Gly Ile Gln Leu Val Val Thr Met Val Met Ala
1               5                   10                  15

Ser Val Met Gln Lys Ile Ile Pro His Tyr Ser Leu Ala Arg Trp
                20                  25                  30

Leu Leu Cys Asn Gly Ser Leu Arg Trp Tyr Gln His Pro Thr Glu
                35                  40                  45

Glu Glu Leu Arg Ile Leu Ala Gly Lys Gln Gln Lys Gly Lys Thr
                50                  55                  60

Lys Lys Asp Arg Lys Tyr Asn Gly His Ile Glu Ser Lys Pro Leu
                65                  70                  75

Thr Ile Pro Lys Asp Ile Asp Leu His Leu Glu Thr Lys Ser Val
                80                  85                  90

Thr Glu Val Asp Thr Leu Ala Leu His Tyr Phe Pro Glu Tyr Gln
                95                  100                 105

Trp Leu Val Asp Phe Thr Val Ala Ala Thr Val Val Tyr Leu Val
                110                 115                 120

Thr Glu Val Tyr Tyr Asn Phe Met Lys Pro Thr Gln Glu Met Asn
                125                 130                 135

Ile Ser Leu Val Trp Cys Leu Leu Val Leu Ser Phe Ala Ile Lys
                140                 145                 150

Val Leu Phe Ser Leu Thr Thr His Tyr Phe Lys Val Glu Asp Gly
                155                 160                 165

Gly Glu Arg Ser Val Cys Val Thr Phe Gly Phe Phe Phe Phe Val
                170                 175                 180

Lys Ala Met Ala Val Leu Ile Val Thr Glu Asn Tyr Leu Glu Phe
                185                 190                 195

Gly Leu Glu Thr Gly Phe Thr Asn Phe Ser Asp Ser Ala Met Gln
                200                 205                 210

Phe Leu Glu Lys Gln Gly Leu Glu Ser Gln Ser Pro Val Ser Lys
                215                 220                 225

Leu Thr Phe Lys Phe Phe Leu Ala Ile Phe Cys Ser Phe Ile Gly
                230                 235                 240

Ala Phe Leu Thr Phe Pro Gly Leu Arg Leu Ala Gln Met His Leu
                245                 250                 255

Asp Ala Leu Asn Leu Ala Thr Glu Lys Ile Thr Gln Thr Leu Leu
                260                 265                 270

His Ile Asn Phe Leu Ala Pro Leu Phe Met Val Leu Leu Trp Val
                275                 280                 285
```

Lys Pro Ile Thr Lys Asp Tyr Ile Met Asn Pro Pro Leu Gly Lys
290 295 300

Glu Ile Ser Pro Ser Gly Arg
305

<210> 29
<211> 2668
<212> DNA
<213> Homo Sapien

<400> 29
gactttgctt gaatgtttac attttctgct cgctgtccta catatcacaa 50

tatagtgttc acgttttgtt aaaactttgg ggtgtcagga gttgagcttg 100

ctcagcaagc cagcatggct aggatgagct ttgttatagc agcttgccaa 150

ttggtgctgg gcctactaat gacttcatta accgagtctt ccatacagaa 200

tagtgagtgt ccacaacttt gcgtatgtga aattcgtccc tggtttaccc 250

cacagtcaac ttacagagaa gccaccactg ttgattgcaa tgacctccgc 300

ttaacaagga ttcccagtaa cctctctagt gacacacaag tgcttctctt 350

acagagcaat aacatcgcga agactgtgga tgagctgcag cagcttttca 400

acttgactga actagatttc tcccaaaaca actttactaa cattaaggag 450

gtcgggctgg caaacctaac ccagctcaca acgctgcatt tggaggaaaa 500

tcagattacc gagatgactg attactgtct acaagacctc agcaaccttc 550

aagaactcta catcaaccac aaccaaatta gcactatttc tgctcatgct 600

tttgcaggct taaaaaatct attaaggctc cacctgaact ccaacaaatt 650

gaaagttatt gatagtcgct ggtttgattc tacacccaac ctggaaattc 700

tcatgatcgg agaaaaccct gtgattggaa ttctggatat gaacttcaaa 750

cccctcgcaa atttgagaag cttagttttg gcaggaatgt atctcactga 800

tattcctgga aatgctttgg tgggtctgga tagccttgag agcctgtctt 850

tttatgataa caaactggtt aaagtccctc aacttgccct gcaaaaagtt 900

ccaaatttga aattcttaga cctcaacaaa aaccccattc acaaaatcca 950

agaaggggac ttcaaaaata tgcttcggtt aaaagaactg ggaatcaaca 1000

atatgggcga gctcgtttct gtcgaccgct atgccctgga taacttgcct 1050

gaactcacaa agctggaagc caccaataac cctaaactct cttacatcca 1100

ccgcttggct ttccgaagtg tccctgctct ggaaagcttg atgctgaaca 1150

acaatgcctt gaatgccatt taccaaaaga cagtcgaatc cctccccaat 1200

ctgcgtgaga tcagtatcca tagcaatccc ctcaggtgtg actgtgtgat 1250
ccactggatt aactccaaca aaaccaacat ccgcttcatg gagcccctgt 1300
ccatgttctg tgccatgccg cccgaatata aagggcacca ggtgaaggaa 1350
gttttaatcc aggattcgag tgaacagtgc ctcccaatga tatctcacga 1400
cagcttccca aatcgtttaa acgtggatat cggcacgacg gttttcctag 1450
actgtcgagc catggctgag ccagaacctg aaatttactg ggtcactccc 1500
attggaaata agataactgt ggaaaccctt tcagataaat acaagctaag 1550
tagcgaaggt accttggaaa tatctaacat acaaattgaa gactcaggaa 1600
gatacacatg tgttgcccag aatgtccaag ggcagacac tcgggtggca 1650
acaattaagg ttaacgggac ccttctggat ggtacccagg tgctaaaaat 1700
atacgtcaag cagacagaat cccattccat cttagtgtcc tggaaagtta 1750
attccaatgt catgacgtca aacttaaaat ggtcgtctgc caccatgaag 1800
attgataacc ctcacataac atatactgcc agggtcccag tcgatgtcca 1850
tgaatacaac ctaacgcatc tgcagccttc cacagattat gaagtgtgtc 1900
tcacagtgtc caatattcat cagcagactc aaaagtcatg cgtaaatgtc 1950
acaaccaaaa atgccgcctt cgcagtggac atctctgatc aagaaaccag 2000
tacagccctt gctgcagtaa tggggtctat gtttgccgtc attagccttg 2050
cgtccattgc tgtgtacttt gccaaaagat ttaagagaaa aaactaccac 2100
cactcattaa aaaagtatat gcaaaaaacc tcttcaatcc cactaaatga 2150
gctgtaccca ccactcatta acctctggga aggtgacagc gagaaagaca 2200
aagatggttc tgcagacacc aagccaaccc aggtcgacac atccagaagc 2250
tattacatgt ggtaactcag aggatatttt gcttctggta gtaaggagca 2300
caaagacgtt tttgctttat tctgcaaaag tgaacaagtt gaagactttt 2350
gtatttttga ctttgctagt ttgtggcaga gtggagagga cgggtggata 2400
tttcaaattt ttttagtata gcgtatcgca agggtttgac acggctgcca 2450
gcgactctag gcttccagtc tgtgtttggt ttttattctt atcattatta 2500
tgattgttat tatattatta ttttatttta gttgttgtgc taaactcaat 2550
aatgctgttc taactacagt gctcaataaa atgattaatg acaggaaaaa 2600
aaaaaaaaaa aaaaaaaaaa aaaaaaaaaa aaaaaaaaaa aaaaaaaaaa 2650

aaaaaaaaaa aaaaaaaa 2668

<210> 30
<211> 716
<212> PRT
<213> Homo Sapien

<400> 30

```
Met Ala Arg Met Ser Phe Val Ile Ala Ala Cys Gln Leu Val Leu
1               5                   10                  15

Gly Leu Leu Met Thr Ser Leu Thr Glu Ser Ser Ile Gln Asn Ser
                20                  25                  30

Glu Cys Pro Gln Leu Cys Val Cys Glu Ile Arg Pro Trp Phe Thr
                35                  40                  45

Pro Gln Ser Thr Tyr Arg Glu Ala Thr Thr Val Asp Cys Asn Asp
                50                  55                  60

Leu Arg Leu Thr Arg Ile Pro Ser Asn Leu Ser Ser Asp Thr Gln
                65                  70                  75

Val Leu Leu Leu Gln Ser Asn Asn Ile Ala Lys Thr Val Asp Glu
                80                  85                  90

Leu Gln Gln Leu Phe Asn Leu Thr Glu Leu Asp Phe Ser Gln Asn
                95                  100                 105

Asn Phe Thr Asn Ile Lys Glu Val Gly Leu Ala Asn Leu Thr Gln
                110                 115                 120

Leu Thr Thr Leu His Leu Glu Glu Asn Gln Ile Thr Glu Met Thr
                125                 130                 135

Asp Tyr Cys Leu Gln Asp Leu Ser Asn Leu Gln Glu Leu Tyr Ile
                140                 145                 150

Asn His Asn Gln Ile Ser Thr Ile Ser Ala His Ala Phe Ala Gly
                155                 160                 165

Leu Lys Asn Leu Leu Arg Leu His Leu Asn Ser Asn Lys Leu Lys
                170                 175                 180

Val Ile Asp Ser Arg Trp Phe Asp Ser Thr Pro Asn Leu Glu Ile
                185                 190                 195

Leu Met Ile Gly Glu Asn Pro Val Ile Gly Ile Leu Asp Met Asn
                200                 205                 210

Phe Lys Pro Leu Ala Asn Leu Arg Ser Leu Val Leu Ala Gly Met
                215                 220                 225

Tyr Leu Thr Asp Ile Pro Gly Asn Ala Leu Val Gly Leu Asp Ser
                230                 235                 240

Leu Glu Ser Leu Ser Phe Tyr Asp Asn Lys Leu Val Lys Val Pro
                245                 250                 255
```

Gln Leu Ala Leu Gln Lys Val Pro Asn Leu Lys Phe Leu Asp Leu
260 265 270

Asn Lys Asn Pro Ile His Lys Ile Gln Glu Gly Asp Phe Lys Asn
275 280 285

Met Leu Arg Leu Lys Glu Leu Gly Ile Asn Asn Met Gly Glu Leu
290 295 300

Val Ser Val Asp Arg Tyr Ala Leu Asp Asn Leu Pro Glu Leu Thr
305 310 315

Lys Leu Glu Ala Thr Asn Asn Pro Lys Leu Ser Tyr Ile His Arg
320 325 330

Leu Ala Phe Arg Ser Val Pro Ala Leu Glu Ser Leu Met Leu Asn
335 340 345

Asn Asn Ala Leu Asn Ala Ile Tyr Gln Lys Thr Val Glu Ser Leu
350 355 360

Pro Asn Leu Arg Glu Ile Ser Ile His Ser Asn Pro Leu Arg Cys
365 370 375

Asp Cys Val Ile His Trp Ile Asn Ser Asn Lys Thr Asn Ile Arg
380 385 390

Phe Met Glu Pro Leu Ser Met Phe Cys Ala Met Pro Pro Glu Tyr
395 400 405

Lys Gly His Gln Val Lys Glu Val Leu Ile Gln Asp Ser Ser Glu
410 415 420

Gln Cys Leu Pro Met Ile Ser His Asp Ser Phe Pro Asn Arg Leu
425 430 435

Asn Val Asp Ile Gly Thr Thr Val Phe Leu Asp Cys Arg Ala Met
440 445 450

Ala Glu Pro Glu Pro Glu Ile Tyr Trp Val Thr Pro Ile Gly Asn
455 460 465

Lys Ile Thr Val Glu Thr Leu Ser Asp Lys Tyr Lys Leu Ser Ser
470 475 480

Glu Gly Thr Leu Glu Ile Ser Asn Ile Gln Ile Glu Asp Ser Gly
485 490 495

Arg Tyr Thr Cys Val Ala Gln Asn Val Gln Gly Ala Asp Thr Arg
500 505 510

Val Ala Thr Ile Lys Val Asn Gly Thr Leu Leu Asp Gly Thr Gln
515 520 525

Val Leu Lys Ile Tyr Val Lys Gln Thr Glu Ser His Ser Ile Leu
530 535 540

Val Ser Trp Lys Val Asn Ser Asn Val Met Thr Ser Asn Leu Lys
545 550 555

```
Trp Ser Ser Ala Thr Met Lys Ile Asp Asn Pro His Ile Thr Tyr
                560                 565                 570

Thr Ala Arg Val Pro Val Asp Val His Glu Tyr Asn Leu Thr His
                575                 580                 585

Leu Gln Pro Ser Thr Asp Tyr Glu Val Cys Leu Thr Val Ser Asn
                590                 595                 600

Ile His Gln Gln Thr Gln Lys Ser Cys Val Asn Val Thr Thr Lys
                605                 610                 615

Asn Ala Ala Phe Ala Val Asp Ile Ser Asp Gln Glu Thr Ser Thr
                620                 625                 630

Ala Leu Ala Ala Val Met Gly Ser Met Phe Ala Val Ile Ser Leu
                635                 640                 645

Ala Ser Ile Ala Val Tyr Phe Ala Lys Arg Phe Lys Arg Lys Asn
                650                 655                 660

Tyr His His Ser Leu Lys Lys Tyr Met Gln Lys Thr Ser Ser Ile
                665                 670                 675

Pro Leu Asn Glu Leu Tyr Pro Pro Leu Ile Asn Leu Trp Glu Gly
                680                 685                 690

Asp Ser Glu Lys Asp Lys Asp Gly Ser Ala Asp Thr Lys Pro Thr
                695                 700                 705

Gln Val Asp Thr Ser Arg Ser Tyr Tyr Met Trp
                710                 715
```

```
<210> 31
<211> 1728
<212> DNA
<213> Homo Sapien

<400> 31
cagccgggtc ccaagcctgt gcctgagcct gagcctgagc ctgagcccga 50

gccgggagcc ggtcgcgggg gctccgggct gtgggaccgc tgggccccca 100

gcgatggcga ccctgtgggg aggccttctt cggcttggct ccttgctcag 150

cctgtcgtgc ctggcgcttt ccgtgctgct gctggcgcag ctgtcagacg 200

ccgccaagaa tttcgaggat gtcagatgta aatgtatctg ccctccctat 250

aaagaaaatt ctgggcatat ttataataag aacatatctc agaaagattg 300

tgattgcctt catgttgtgg agcccatgcc tgtgcggggg cctgatgtag 350

aagcatactg tctacgctgt gaatgcaaat atgaagaaag aagctctgtc 400

acaatcaagg ttaccattat aatttatctc tccattttgg gccttctact 450

tctgtacatg gtatatctta ctctggttga gcccatactg aagaggcgcc 500
```

tctttggaca tgcacagttg atacagagtg atgatgatat tggggatcac 550

cagccttttg caaatgcaca cgatgtgcta gcccgctccc gcagtcgagc 600

caacgtgctg aacaaggtag aatatgcaca gcagcgctgg aagcttcaag 650

tccaagagca gcgaaagtct gtctttgacc ggcatgttgt cctcagctaa 700

ttgggaattg aattcaaggt gactagaaag aaacaggcag acaactggaa 750

agaactgact gggttttgct gggtttcatt ttaatacctt gttgatttca 800

ccaactgttg ctggaagatt caaaactgga agcaaaaact tgcttgattt 850

ttttttcttg ttaacgtaat aatagagaca tttttaaaag cacacagctc 900

aaagtcagcc aataagtctt ttcctatttg tgacttttac taataaaaat 950

aaatctgcct gtaaattatc ttgaagtcct ttacctggaa caagcactct 1000

ctttttcacc acatagtttt aacttgactt tcaagataat tttcagggtt 1050

tttgttgttg ttgtttttttg tttgtttgtt ttggtgggag aggggaggga 1100

tgcctgggaa gtggttaaca actttttttca agtcacttta ctaaacaaac 1150

ttttgtaaat agaccttacc ttctattttc gagtttcatt tatattttgc 1200

agtgtagcca gcctcatcaa agagctgact tactcatttg acttttgcac 1250

tgactgtatt atctgggtat ctgctgtgtc tgcacttcat ggtaaacggg 1300

atctaaaatg cctggtggct tttcacaaaa agcagatttt cttcatgtac 1350

tgtgatgtct gatgcaatgc atcctagaac aaactggcca tttgctagtt 1400

tactctaaag actaaacata gtcttggtgt gtgtggtctt actcatcttc 1450

tagtaccttt aaggacaaat cctaaggact tggacacttg caataaagaa 1500

attttatttt aaacccaagc ctccctggat tgataatata tacacatttg 1550

tcagcatttc cggtcgtggt gagaggcagc tgtttgagct ccaatatgtg 1600

cagctttgaa ctagggctgg ggttgtgggt gcctcttctg aaaggtctaa 1650

ccattattgg ataactggct tttttcttcc tatgtcctct ttggaatgta 1700

acaataaaaa taatttttga aacatcaa 1728

<210> 32
<211> 198
<212> PRT
<213> Homo Sapien

<400> 32
Met Ala Thr Leu Trp Gly Gly Leu Leu Arg Leu Gly Ser Leu Leu
1 5 10 15

Ser Leu Ser Cys Leu Ala Leu Ser Val Leu Leu Leu Ala Gln Leu
20 25 30

Ser Asp Ala Ala Lys Asn Phe Glu Asp Val Arg Cys Lys Cys Ile
35 40 45

Cys Pro Pro Tyr Lys Glu Asn Ser Gly His Ile Tyr Asn Lys Asn
50 55 60

Ile Ser Gln Lys Asp Cys Asp Cys Leu His Val Val Glu Pro Met
65 70 75

Pro Val Arg Gly Pro Asp Val Glu Ala Tyr Cys Leu Arg Cys Glu
80 85 90

Cys Lys Tyr Glu Glu Arg Ser Ser Val Thr Ile Lys Val Thr Ile
95 100 105

Ile Ile Tyr Leu Ser Ile Leu Gly Leu Leu Leu Leu Tyr Met Val
110 115 120

Tyr Leu Thr Leu Val Glu Pro Ile Leu Lys Arg Arg Leu Phe Gly
125 130 135

His Ala Gln Leu Ile Gln Ser Asp Asp Asp Ile Gly Asp His Gln
140 145 150

Pro Phe Ala Asn Ala His Asp Val Leu Ala Arg Ser Arg Ser Arg
155 160 165

Ala Asn Val Leu Asn Lys Val Glu Tyr Ala Gln Gln Arg Trp Lys
170 175 180

Leu Gln Val Gln Glu Gln Arg Lys Ser Val Phe Asp Arg His Val
185 190 195

Val Leu Ser

<210> 33
<211> 1170
<212> DNA
<213> Homo Sapien

<400> 33
gtcgaaggtt ataaaagctt ccagccaaac ggcattgaag ttgaagatac 50

aacctgacag cacagcctga gatcttgggg atccctcagc ctaacaccca 100

cagacgtcag ctggtggatt cccgctgcat caaggcctac ccactgtctc 150

catgctgggc tctccctgcc ttctgtggct cctggccgtg accttcttgg 200

ttcccagagc tcagcccttg gcccctcaag actttgaaga agaggaggca 250

gatgagactg agacggcgtg gccgcctttg ccggctgtcc cctgcgacta 300

cgaccactgc cgacacctgc aggtgccctg caaggagcta cagagggtcg 350

ggccggcggc ctgcctgtgc ccaggactct ccagccccgc ccagccgccc 400

gacccgccgc gcatgggaga agtgcgcatt gcggccgaag agggccgcgc 450

agtggtccac tggtgtgccc ccttctcccc ggtcctccac tactggctgc 500

tgctttggga cggcagcgag gctgcgcaga aggggccccc gctgaacgct 550

acggtccgca gagccgaact gaaggggctg aagccagggg gcatttatgt 600

cgtttgcgta gtggccgcta acgaggccgg ggcaagccgc gtgccccagg 650

ctggaggaga gggcctcgag gggccgaca tccctgcctt cgggccttgc 700

agccgccttg cggtgccgcc caaccccgc actctggtcc acgcggccgt 750

cggggtgggc acggccctgg ccctgctaag ctgtgccgcc ctggtgtggc 800

acttctgcct gcgcgatcgc tggggctgcc cgcgccgagc cgccgcccga 850

gccgcagggg cgctctgaaa ggggcctggg ggcatctcgg gcacagacag 900

ccccacctgg ggcgctcagc ctggcccccg ggaaagagga aaacccgctg 950

cctccaggga gggctggacg gcgagctggg agccagcccc aggctccagg 1000

gccacggcgg agtcatggtt ctcaggactg agcgcttgtt taggtccggt 1050

acttggcgct ttgtttcctg gctgaggtct gggaaggaat agaaaggggc 1100

ccccaatttt tttttaagcg gccagataat aaataatgta acctttgcgg 1150

ttaaaaaaaa aaaaaaaaaa 1170

<210> 34
<211> 238
<212> PRT
<213> Homo Sapien

<400> 34
Met Leu Gly Ser Pro Cys Leu Leu Trp Leu Leu Ala Val Thr Phe
1 5 10 15

Leu Val Pro Arg Ala Gln Pro Leu Ala Pro Gln Asp Phe Glu Glu
20 25 30

Glu Glu Ala Asp Glu Thr Glu Thr Ala Trp Pro Pro Leu Pro Ala
35 40 45

Val Pro Cys Asp Tyr Asp His Cys Arg His Leu Gln Val Pro Cys
50 55 60

Lys Glu Leu Gln Arg Val Gly Pro Ala Ala Cys Leu Cys Pro Gly
65 70 75

Leu Ser Ser Pro Ala Gln Pro Pro Asp Pro Pro Arg Met Gly Glu
80 85 90

Val Arg Ile Ala Ala Glu Glu Gly Arg Ala Val Val His Trp Cys
95 100 105

Ala Pro Phe Ser Pro Val Leu His Tyr Trp Leu Leu Leu Trp Asp
110 115 120

Gly Ser Glu Ala Ala Gln Lys Gly Pro Pro Leu Asn Ala Thr Val
125 130 135

Arg Arg Ala Glu Leu Lys Gly Leu Lys Pro Gly Gly Ile Tyr Val
140 145 150

Val Cys Val Val Ala Ala Asn Glu Ala Gly Ala Ser Arg Val Pro
155 160 165

Gln Ala Gly Gly Glu Gly Leu Glu Gly Ala Asp Ile Pro Ala Phe
170 175 180

Gly Pro Cys Ser Arg Leu Ala Val Pro Pro Asn Pro Arg Thr Leu
185 190 195

Val His Ala Ala Val Gly Val Gly Thr Ala Leu Ala Leu Leu Ser
200 205 210

Cys Ala Ala Leu Val Trp His Phe Cys Leu Arg Asp Arg Trp Gly
215 220 225

Cys Pro Arg Arg Ala Ala Ala Arg Ala Ala Gly Ala Leu
230 235

<210> 35
<211> 1315
<212> DNA
<213> Homo Sapien

<400> 35
tcgccatggc ctctgccgga atgcagatcc tgggagtcgt cctgacactg 50

ctgggctggg tgaatggcct ggtctcctgt gccctgccca tgtggaaggt 100

gaccgctttc atcggcaaca gcatcgtggt ggcccaggtg gtgtgggagg 150

gcctgtggat gtcctgcgtg gtgcagagca ccggccagat gcagtgcaag 200

gtgtacgact cactgctggc gctgccacag gacctgcagg ctgcacgtgc 250

cctctgtgtc atcgccctcc ttgtggccct gttcggcttg ctggtctacc 300

ttgctggggc caagtgtacc acctgtgtgg aggagaagga ttccaaggcc 350

cgcctggtgc tcacctctgg gattgtcttt gtcatctcag ggtcctgac 400

gctaatcccc gtgtgctgga cggcgcatgc catcatccgg gacttctata 450

accccctggt ggctgaggcc caaaagcggg agctgggggc ctccctctac 500

ttgggctggg cggcctcagg cctttgttg ctgggtgggg ggttgctgtg 550

ctgcacttgc ccctcggggg ggtcccaggg ccccagccat tacatggccc 600

gctactcaac atctgcccct gccatctctc gggggccctc tgagtaccct 650

accaagaatt acgtctgacg tggaggggaa tgggggctcc gctggcgcta 700

gagccatcca gaagtggcag tgcccaacag ctttgggatg ggttcgtacc 750

ttttgtttct gcctcctgct atttttcttt tgactgagga tatttaaaat 800

tcatttgaaa actgagccaa ggtgttgact cagactctca cttaggctct 850

gctgtttctc acccttggat gatggagcca aagaggggat gctttgagat 900

tctggatctt gacatgccca tcttagaagc cagtcaagct atggaactaa 950

tgcggaggct gcttgctgtg ctggctttgc aacaagacag actgtcccca 1000

agagttcctg ctgctgctgg gggctgggct tccctagatg tcactggaca 1050

gctgcccccc atcctactca ggtctctgga gctcctctct tcacccctgg 1100

aaaaacaaat catctgttaa caaaggactg cccacctccg gaacttctga 1150

cctctgtttc ctccgtcctg ataagacgtc caccccccag ggccaggtcc 1200

cagctatgta gacccccgcc cccacctcca acactgcacc cttctgccct 1250

gcccccctcg tctcaccccc tttacactca catttttatc aaataaagca 1300

tgttttgtta gtgca 1315

<210> 36
<211> 220
<212> PRT
<213> Homo Sapien

<400> 36

Met Ala Ser Ala Gly Met Gln Ile Leu Gly Val Val Leu Thr Leu
1 5 10 15

Leu Gly Trp Val Asn Gly Leu Val Ser Cys Ala Leu Pro Met Trp
20 25 30

Lys Val Thr Ala Phe Ile Gly Asn Ser Ile Val Val Ala Gln Val
35 40 45

Val Trp Glu Gly Leu Trp Met Ser Cys Val Val Gln Ser Thr Gly
50 55 60

Gln Met Gln Cys Lys Val Tyr Asp Ser Leu Leu Ala Leu Pro Gln
65 70 75

Asp Leu Gln Ala Ala Arg Ala Leu Cys Val Ile Ala Leu Leu Val
80 85 90

Ala Leu Phe Gly Leu Leu Val Tyr Leu Ala Gly Ala Lys Cys Thr
95 100 105

Thr Cys Val Glu Glu Lys Asp Ser Lys Ala Arg Leu Val Leu Thr

110 115 120

Ser Gly Ile Val Phe Val Ile Ser Gly Val Leu Thr Leu Ile Pro
125 130 135

Val Cys Trp Thr Ala His Ala Ile Ile Arg Asp Phe Tyr Asn Pro
140 145 150

Leu Val Ala Glu Ala Gln Lys Arg Glu Leu Gly Ala Ser Leu Tyr
155 160 165

Leu Gly Trp Ala Ala Ser Gly Leu Leu Leu Leu Gly Gly Gly Leu
170 175 180

Leu Cys Cys Thr Cys Pro Ser Gly Gly Ser Gln Gly Pro Ser His
185 190 195

Tyr Met Ala Arg Tyr Ser Thr Ser Ala Pro Ala Ile Ser Arg Gly
200 205 210

Pro Ser Glu Tyr Pro Thr Lys Asn Tyr Val
215 220

<210> 37
<211> 1240
<212> DNA
<213> Homo Sapien

<400> 37
tgcatcagtg cccaggcaag cccaggagtt gacatttctc tgcccagcca 50

tgggcctcac cctgctcttg ctgctgctcc tgggactaga aggtcagggc 100

atagttggca gcctccctga ggtgctgcag gcacccgtgg gaagctccat 150

tctggtgcag tgccactaca ggctccagga tgtcaaagct cagaaggtgt 200

ggtgccggtt cttgccggag gggtgccagc ccctggtgtc ctcagctgtg 250

gatcgcagag ctccagcggg caggcgtacg tttctcacag acctgggtgg 300

gggcctgctg caggtggaaa tggttaccct gcaggaagag gatgctggcg 350

agtatggctg catggtggat ggggccaggg ggccccagat tttgcacaga 400

gtctctctga acatactgcc cccagaggaa gaagaagaga cccataagat 450

tggcagtctg gctgagaacg cattctcaga ccctgcaggc agtgccaacc 500

ctttggaacc cagccaggat gagaagagca tccccttgat ctggggtgct 550

gtgctcctgg taggtctgct ggtggcagcg gtggtgctgt ttgctgtgat 600

ggccaagagg aaacaagaat ccctcctcag tggtccacca cgtcagtgac 650

tctggaccgg ctgctgaatt gcctttggat gtaccacaca ttaggcttga 700

ctcaccacct tcatttgaca ataccaccta caccagccta cctcttgatt 750

ccccatcagg aaaaccttca ctcccagctc catcctcatt gccccctcta 800

cctcctaagg tcctggtctg ctccaagcct gtgacatatg ccacagtaat 850

cttcccggga gggaacaagg gtggagggac ctcgtgtggg ccagcccaga 900

atccacctaa caatcagact ccatccagct aagctgctca tcacacttta 950

aactcatgag gaccatccct aggggttctg tgcatccatc cagccagctc 1000

atgccctagg atccttagga tatctgagca accagggact ttaagatcta 1050

atccaatgtc ctaactttac tagggaaagt gacgctcaga catgactgag 1100

atgtcttggg gaagacctcc ctgcacccaa ctcccccact ggttcttcta 1150

ccattacaca ctgggctaaa taaaccctaa taatgatgtg caaaaaaaaa 1200

aaaaaaaaaa aaaaaaaaaa aaaaaaaaaa aaaaaaaaaa 1240

<210> 38
<211> 199
<212> PRT
<213> Homo Sapien

<400> 38

```
Met Gly Leu Thr Leu Leu Leu Leu Leu Leu Leu Gly Leu Glu Gly
 1               5                  10                  15

Gln Gly Ile Val Gly Ser Leu Pro Glu Val Leu Gln Ala Pro Val
                20                  25                  30

Gly Ser Ser Ile Leu Val Gln Cys His Tyr Arg Leu Gln Asp Val
                35                  40                  45

Lys Ala Gln Lys Val Trp Cys Arg Phe Leu Pro Glu Gly Cys Gln
                50                  55                  60

Pro Leu Val Ser Ser Ala Val Asp Arg Arg Ala Pro Ala Gly Arg
                65                  70                  75

Arg Thr Phe Leu Thr Asp Leu Gly Gly Gly Leu Leu Gln Val Glu
                80                  85                  90

Met Val Thr Leu Gln Glu Glu Asp Ala Gly Glu Tyr Gly Cys Met
                95                 100                 105

Val Asp Gly Ala Arg Gly Pro Gln Ile Leu His Arg Val Ser Leu
               110                 115                 120

Asn Ile Leu Pro Pro Glu Glu Glu Glu Glu Thr His Lys Ile Gly
               125                 130                 135

Ser Leu Ala Glu Asn Ala Phe Ser Asp Pro Ala Gly Ser Ala Asn
               140                 145                 150

Pro Leu Glu Pro Ser Gln Asp Glu Lys Ser Ile Pro Leu Ile Trp
               155                 160                 165
```

```
Gly Ala Val Leu Leu Val Gly Leu Leu Val Ala Ala Val Val Leu
                170                 175                 180

Phe Ala Val Met Ala Lys Arg Lys Gln Glu Ser Leu Leu Ser Gly
                185                 190                 195

Pro Pro Arg Gln
```

<210> 39
<211> 1088
<212> DNA
<213> Homo Sapien

<400> 39

```
gggtgattga actaaacctt cgccgcaccg agtttgcagt acggccgtca 50
cccgcaccgc tgcctgcttg cggttggaga aatcaaggcc ctaccgggcc 100
tccgtagtca cctctctata gtgggcgtgg ccgaggccgg ggtgaccctg 150
ccggagcctc cgctgccagc gacatgttca aggtaattca gaggtccgtg 200
gggccagcca gcctgagctt gctcaccttc aaagtctatg cagcaccaaa 250
aaaggactca cctcccaaaa attccgtgaa ggttgatgag ctttcactct 300
actcagttcc tgagggtcaa tcgaagtatg tggaggaggc aaggagccag 350
cttgaagaaa gcatctcaca gctccgacac tattgcgagc catacacaac 400
ctggtgtcag gaaacgtact cccaaactaa gcccaagatg caaagtttgg 450
ttcaatgggg gttagacagc tatgactatc tccaaaatgc acctcctgga 500
tttttttccga gacttggtgt tattggtttt gctggcctta ttggactcct 550
tttggctaga ggttcaaaaa taaagaagct agtgtatccg cctggtttca 600
tgggattagc tgcctccctc tattatccac aacaagccat cgtgtttgcc 650
caggtcagtg gggagagatt atatgactgg ggtttacgag gatatatagt 700
catagaagat ttgtggaagg agaactttca aaagccagga aatgtgaaga 750
attcacctgg aactaagtag aaaactccat gctctgccat cttaatcagt 800
tataggtaaa cattggaaac tccatagaat aaatcagtat ttctacagaa 850
aaatggcata gaagtcagta ttgaatgtat taaattggct ttcttcttca 900
ggaaaaacta gaccagacct ctgttatctt ctgtgaaatc atcctacaag 950
caaactaacc tggaatccct tcacctagag ataatgtaca agccttagaa 1000
ctcctcattc tcatgttgct atttatgtac ctaattaaaa cccaagttta 1050
aaaaaaaaaa aaaaaaaaaa aaaaaaaaaa aaaaaaaa 1088
```

<210> 40
<211> 198
<212> PRT
<213> Homo Sapien

<400> 40
Met Phe Lys Val Ile Gln Arg Ser Val Gly Pro Ala Ser Leu Ser
1 5 10 15

Leu Leu Thr Phe Lys Val Tyr Ala Ala Pro Lys Lys Asp Ser Pro
20 25 30

Pro Lys Asn Ser Val Lys Val Asp Glu Leu Ser Leu Tyr Ser Val
35 40 45

Pro Glu Gly Gln Ser Lys Tyr Val Glu Glu Ala Arg Ser Gln Leu
50 55 60

Glu Glu Ser Ile Ser Gln Leu Arg His Tyr Cys Glu Pro Tyr Thr
65 70 75

Thr Trp Cys Gln Glu Thr Tyr Ser Gln Thr Lys Pro Lys Met Gln
80 85 90

Ser Leu Val Gln Trp Gly Leu Asp Ser Tyr Asp Tyr Leu Gln Asn
95 100 105

Ala Pro Pro Gly Phe Phe Pro Arg Leu Gly Val Ile Gly Phe Ala
110 115 120

Gly Leu Ile Gly Leu Leu Leu Ala Arg Gly Ser Lys Ile Lys Lys
125 130 135

Leu Val Tyr Pro Pro Gly Phe Met Gly Leu Ala Ala Ser Leu Tyr
140 145 150

Tyr Pro Gln Gln Ala Ile Val Phe Ala Gln Val Ser Gly Glu Arg
155 160 165

Leu Tyr Asp Trp Gly Leu Arg Gly Tyr Ile Val Ile Glu Asp Leu
170 175 180

Trp Lys Glu Asn Phe Gln Lys Pro Gly Asn Val Lys Asn Ser Pro
185 190 195

Gly Thr Lys

<210> 41
<211> 1840
<212> DNA
<213> Homo Sapien

<400> 41
gtgggccgcc cctgctgctg ccgtccatgc tgatgtttgc ggtgatcgtg 50

gcctccagcg ggctgctgct catgatcgag cggggcatcc tggccgagat 100

gaagcccctg cccctgcacc cgcccggccg cgagggcaca gcctggcgcg 150

ggaaagcccc caagcctggg ggcctgtccc tcagggctgg ggacgcggac 200

ttgcaagtgc ggcaggacgt ccggaacagg accctgcggg cggtgtgcgg 250

acagccaggc atgccccggg acccctggga cttgccggtg gggcagcggc 300

gcaccctgct gcgccacatc ctcgtaagtg accgttaccg cttcctctac 350

tgctacgtcc ccaaggtggc ctgctctaac tggaagcggg tgatgaaggt 400

gctggcaggc gtcctggaca gcgtggacgt ccgcctcaag atggaccacc 450

gcagtgacct ggtgttcctg gccgacctgc ggcctgagga gattcgctac 500

cgcctgcagc actactttaa gttcctgttt gtgcgggagc ccttggaacg 550

cctcctctct gcctaccgca acaagtttgg cgagatccga gagtaccagc 600

aacgctatgg ggctgagata gtgaggcggt acagggctgg agcggggccc 650

agccctgcag gcgacgatgt cacattcccc gagttcctga gatacctggt 700

ggatgaggac cctgagcgca tgaatgagca ttggatgccc gtgtaccacc 750

tgtgccagcc ttgtgccgtg cactatgact ttgtgggctc ctatgagagg 800

ctggaggctg atgcaaatca ggtgctggag tgggtacggg caccacctca 850

cgtccgattt ccagctcgcc aggcctggta ccggccagcc agccccgaaa 900

gcctgcatta ccacttgtgc agtgcccccc gggccctgct gcaggatgtg 950

ctgcctaagt atatcctgga cttctccctc tttgcctacc cactgcctaa 1000

tgtcaccaag gaggcgtgtc agcagtgacc atgggtgtgg ggccagcagc 1050

tggtggggac tggtttcaac gccagctttc tgtgcttctg cctgtcattc 1100

ggagaaactc tggctctggg gcttggggct tctcaggatc ctggatggca 1150

gagactgccc tcagaagttc cttgtccagg gtgggcaccc acagtgactc 1200

agaggacagg gctaggcagg agacctgctg ctcctcattg gggggatctc 1250

ttgggggggca gacaccagtt tgccaatgaa gcaacacatc tgatctaaag 1300

actggctcca gaccccgggc tgccaggatt atgcagtcca cttggtctac 1350

cttaatttaa cctgtggcca aactcagaga tggtaccagc caggggcaag 1400

catgaccaga gccagggacc ctgtggctct gatcccccat ttatccaccc 1450

catgtgcctc aggactagag tgagcaatca taccttataa atgacttttg 1500

tgcctttctg ctccagtctc aaaatttcct acacctgcca gttctttaca 1550

tttttccaag gaaaggaaaa cggaagcagg gttcttgcct ggtagctcca 1600

```
ggacccagct ctgcaggcac ccaaagaccc tctgtgccca gcctcttcct 1650

tgagttctcg gaacctcctc cctaattctc ccttccttcc ccacaaggcc 1700

tttgaggttg tgactgtggc tggtatatct ggctgccatt tttctgatgc 1750

atttatttaa aatttgtact ttttgataga acccttgtaa gggctttgtt 1800

ttcctaatag ctgacttttt aataaagcag ttttatatat 1840

<210> 42
<211> 333
<212> PRT
<213> Homo Sapien

<400> 42
Met Leu Met Phe Ala Val Ile Val Ala Ser Ser Gly Leu Leu Leu
1               5                   10                  15

Met Ile Glu Arg Gly Ile Leu Ala Glu Met Lys Pro Leu Pro Leu
                20                  25                  30

His Pro Pro Gly Arg Glu Gly Thr Ala Trp Arg Gly Lys Ala Pro
                35                  40                  45

Lys Pro Gly Gly Leu Ser Leu Arg Ala Gly Asp Ala Asp Leu Gln
                50                  55                  60

Val Arg Gln Asp Val Arg Asn Arg Thr Leu Arg Ala Val Cys Gly
                65                  70                  75

Gln Pro Gly Met Pro Arg Asp Pro Trp Asp Leu Pro Val Gly Gln
                80                  85                  90

Arg Arg Thr Leu Leu Arg His Ile Leu Val Ser Asp Arg Tyr Arg
                95                  100                 105

Phe Leu Tyr Cys Tyr Val Pro Lys Val Ala Cys Ser Asn Trp Lys
                110                 115                 120

Arg Val Met Lys Val Leu Ala Gly Val Leu Asp Ser Val Asp Val
                125                 130                 135

Arg Leu Lys Met Asp His Arg Ser Asp Leu Val Phe Leu Ala Asp
                140                 145                 150

Leu Arg Pro Glu Glu Ile Arg Tyr Arg Leu Gln His Tyr Phe Lys
                155                 160                 165

Phe Leu Phe Val Arg Glu Pro Leu Glu Arg Leu Leu Ser Ala Tyr
                170                 175                 180

Arg Asn Lys Phe Gly Glu Ile Arg Glu Tyr Gln Gln Arg Tyr Gly
                185                 190                 195

Ala Glu Ile Val Arg Arg Tyr Arg Ala Gly Ala Gly Pro Ser Pro
                200                 205                 210

Ala Gly Asp Asp Val Thr Phe Pro Glu Phe Leu Arg Tyr Leu Val
```

```
                    215                 220                 225

Asp Glu Asp Pro Glu Arg Met Asn Glu His Trp Met Pro Val Tyr
                230                 235                 240

His Leu Cys Gln Pro Cys Ala Val His Tyr Asp Phe Val Gly Ser
                245                 250                 255

Tyr Glu Arg Leu Glu Ala Asp Ala Asn Gln Val Leu Glu Trp Val
                260                 265                 270

Arg Ala Pro Pro His Val Arg Phe Pro Ala Arg Gln Ala Trp Tyr
                275                 280                 285

Arg Pro Ala Ser Pro Glu Ser Leu His Tyr His Leu Cys Ser Ala
                290                 295                 300

Pro Arg Ala Leu Leu Gln Asp Val Leu Pro Lys Tyr Ile Leu Asp
                305                 310                 315

Phe Ser Leu Phe Ala Tyr Pro Leu Pro Asn Val Thr Lys Glu Ala
                320                 325                 330

Cys Gln Gln
```

<210> 43
<211> 633
<212> DNA
<213> Homo Sapien

<400> 43

```
ctcctgcact aggctctcag ccagggatga tgcgctgctg ccgccgccgc 50

tgctgctgcc ggcaaccacc ccatgccctg aggccgttgc tgttgctgcc 100

cctcgtcctt ttacctcccc tggcagcagc tgcagcgggc ccaaaccgat 150

gtgacaccat ataccagggc ttcgccgagt gtctcatccg cttgggggac 200

agcatgggcc gcggaggcga gctggagacc atctgcaggt cttggaatga 250

cttccatgcc tgtgcctctc aggtcctgtc aggctgtccg gaggaggcag 300

ctgcagtgtg ggaatcacta cagcaagaag ctcgccaggc cccccgtccg 350

aataacttgc acactctgtg cggtgccccg gtgcatgttc gggagcgcgg 400

cacaggctcc gaaaccaacc aggagacgct gcgggctaca gcgcctgcac 450

tccccatggc ccctgcgccc ccactgctgg cggctgctct ggctctggcc 500

tacctcctga ggcctctggc ctagcttgtt gggttgggta gcagcgcccg 550

tacctccagc cctgctctgg cggtggttgt ccaggctctg cagagcgcag 600

cagggctttt cattaaaggt atttatattt gta 633
```

<210> 44

<211> 165
<212> PRT
<213> Homo Sapien

<400> 44

Met Met Arg Cys Cys Arg Arg Arg Cys Cys Cys Arg Gln Pro Pro
1               5                   10                  15

His Ala Leu Arg Pro Leu Leu Leu Leu Pro Leu Val Leu Leu Pro
                20                  25                  30

Pro Leu Ala Ala Ala Ala Ala Gly Pro Asn Arg Cys Asp Thr Ile
                35                  40                  45

Tyr Gln Gly Phe Ala Glu Cys Leu Ile Arg Leu Gly Asp Ser Met
                50                  55                  60

Gly Arg Gly Gly Glu Leu Glu Thr Ile Cys Arg Ser Trp Asn Asp
                65                  70                  75

Phe His Ala Cys Ala Ser Gln Val Leu Ser Gly Cys Pro Glu Glu
                80                  85                  90

Ala Ala Ala Val Trp Glu Ser Leu Gln Gln Glu Ala Arg Gln Ala
                95                  100                 105

Pro Arg Pro Asn Asn Leu His Thr Leu Cys Gly Ala Pro Val His
                110                 115                 120

Val Arg Glu Arg Gly Thr Gly Ser Glu Thr Asn Gln Glu Thr Leu
                125                 130                 135

Arg Ala Thr Ala Pro Ala Leu Pro Met Ala Pro Ala Pro Pro Leu
                140                 145                 150

Leu Ala Ala Ala Leu Ala Leu Ala Tyr Leu Leu Arg Pro Leu Ala
                155                 160                 165

<210> 45
<211> 1231
<212> DNA
<213> Homo Sapien

<400> 45

cccacgcgtc cgcgcagtcg cgcagttctg cctccgcctg ccagtctcgc 50

ccgcgatccc ggcccggggc tgtggcgtcg actccgaccc aggcagccag 100

cagcccgcgc gggagccgga ccgccgccgg aggagctcgg acggcatgct 150

gagccccctc ctttgctgaa gcccgagtgc ggagaagccc gggcaaacgc 200

aggctaagga gaccaaagcg gcgaagtcgc gagacagcgg acaagcagcg 250

gaggagaagg aggaggaggc gaacccagag aggggcagca aaagaagcgg 300

tggtggtggg cgtcgtggcc atggcggcgg ctatcgccag ctcgctcatc 350

cgtcagaaga ggcaagcccg cgagcgcgag aaatccaacg cctgcaagtg 400

tgtcagcagc cccagcaaag gcaagaccag ctgcgacaaa aacaagttaa 450

atgtcttttc ccgggtcaaa ctcttcggct ccaagaagag gcgcagaaga 500

agaccagagc ctcagcttaa gggtatagtt accaagctat acagccgaca 550

aggctaccac ttgcagctgc aggcggatgg aaccattgat ggcaccaaag 600

atgaggacag cacttacact ctgtttaacc tcatccctgt gggtctgcga 650

gtggtggcta tccaaggagt tcaaaccaag ctgtacttgg caatgaacag 700

tgagggatac ttgtacacct cggaactttt cacacctgag tgcaaattca 750

aagaatcagt gtttgaaaat tattatgtga catattcatc aatgatatac 800

cgtcagcagc agtcaggccg agggtggtat ctgggtctga acaaagaagg 850

agagatcatg aaaggcaacc atgtgaagaa gaacaagcct gcagctcatt 900

ttctgcctaa accactgaaa gtggccatgt acaaggagcc atcactgcac 950

gatctcacgg agttctcccg atctggaagc gggaccccaa ccaagagcag 1000

aagtgtctct ggcgtgctga acggaggcaa atccatgagc cacaatgaat 1050

caacgtagcc agtgagggca aaagaagggc tctgtaacag aaccttacct 1100

ccaggtgctg ttgaattctt ctagcagtcc ttcacccaaa agttcaaatt 1150

tgtcagtgac atttaccaaa caaacaggca gagttcacta ttctatctgc 1200

cattagacct tcttatcatc catactaaag c 1231

<210> 46
<211> 245
<212> PRT
<213> Homo Sapien

<400> 46
Met Ala Ala Ala Ile Ala Ser Ser Leu Ile Arg Gln Lys Arg Gln
1 5 10 15

Ala Arg Glu Arg Glu Lys Ser Asn Ala Cys Lys Cys Val Ser Ser
20 25 30

Pro Ser Lys Gly Lys Thr Ser Cys Asp Lys Asn Lys Leu Asn Val
35 40 45

Phe Ser Arg Val Lys Leu Phe Gly Ser Lys Lys Arg Arg Arg Arg
50 55 60

Arg Pro Glu Pro Gln Leu Lys Gly Ile Val Thr Lys Leu Tyr Ser
65 70 75

Arg Gln Gly Tyr His Leu Gln Leu Gln Ala Asp Gly Thr Ile Asp
80 85 90

```
Gly Thr Lys Asp Glu Asp Ser Thr Tyr Thr Leu Phe Asn Leu Ile
                95                  100                 105

Pro Val Gly Leu Arg Val Val Ala Ile Gln Gly Val Gln Thr Lys
                110                 115                 120

Leu Tyr Leu Ala Met Asn Ser Glu Gly Tyr Leu Tyr Thr Ser Glu
                125                 130                 135

Leu Phe Thr Pro Glu Cys Lys Phe Lys Glu Ser Val Phe Glu Asn
                140                 145                 150

Tyr Tyr Val Thr Tyr Ser Ser Met Ile Tyr Arg Gln Gln Gln Ser
                155                 160                 165

Gly Arg Gly Trp Tyr Leu Gly Leu Asn Lys Glu Gly Glu Ile Met
                170                 175                 180

Lys Gly Asn His Val Lys Lys Asn Lys Pro Ala Ala His Phe Leu
                185                 190                 195

Pro Lys Pro Leu Lys Val Ala Met Tyr Lys Glu Pro Ser Leu His
                200                 205                 210

Asp Leu Thr Glu Phe Ser Arg Ser Gly Ser Gly Thr Pro Thr Lys
                215                 220                 225

Ser Arg Ser Val Ser Gly Val Leu Asn Gly Gly Lys Ser Met Ser
                230                 235                 240

His Asn Glu Ser Thr
                245

<210> 47
<211> 1496
<212> DNA
<213> Homo Sapien

<400> 47
cagcgctgac tgcgccgcgg agaaagccag tgggaaccca gacccatagg 50

agacccgcgt ccccgctcgg cctggccagg ccccgcgcta tggagttcct 100

ctgggcccct ctcttgggtc tgtgctgcag tctggccgct gctgatcgcc 150

acaccgtctt ctggaacagt tcaaatccca agttccggaa tgaggactac 200

accatacatg tgcagctgaa tgactacgtg gacatcatct gtccgcacta 250

tgaagatcac tctgtggcag acgctgccat ggagcagtac atactgtacc 300

tggtggagca tgaggagtac cagctgtgcc agccccagtc caaggaccaa 350

gtccgctggc agtgcaaccg gcccagtgcc aagcatggcc cggagaagct 400

gtctgagaag ttccagcgct tcacaccttt caccctgggc aaggagttca 450

aagaaggaca cagctactac tacatctcca aacccatcca ccagcatgaa 500
```

gaccgctgct tgaggttgaa ggtgactgtc agtggcaaaa tcactcacag 550

tcctcaggcc catgacaatc cacaggagaa gagacttgca gcagatgacc 600

cagaggtgcg ggttctacat agcatcggtc acagtgctgc cccacgcctc 650

ttcccacttg cctggactgt gctgctcctt ccacttctgc tgctgcaaac 700

cccgtgaagg tgtgtgccac acctggcctt aaagagggac aggctgaaga 750

gagggacagg cactccaaac ctgtcttggg gccactttca gagcccccag 800

ccctgggaac cactcccacc acaggcataa gctatcacct agcagcctca 850

aaacgggtca atattaaggt tttcaaccgg aaggaggcca accagcccga 900

cagtgccatc cccaccttca cctcggaggg atggagaaag aagtggagac 950

agtcctttcc caccattcct gcctttaagc caaagaaaca agctgtgcag 1000

gcatggtccc ttaaggcaca gtgggagctg agctggaagg ggccacgtgg 1050

atgggcaaag cttgtcaaag atgccccctt caggagagag ccaggatgcc 1100

cagatgaact gactgaagga aaagcaagaa acagtttctt gcttggaagc 1150

caggtacagg agaggcagca tgcttgggct gacccagcat ctcccagcaa 1200

gacctcatct gtggagctgc cacagagaag tttgtagcca ggtactgcat 1250

tctctcccat cctggggcag cactccccag agctgtgcca gcaggggggc 1300

tgtgccaacc tgttcttaga gtgtagctgt aagggcagtg cccatgtgta 1350

cattctgcct agagtgtagc ctaaagggca gggcccacgt gtatagtatc 1400

tgtatataag ttgctgtgtg tctgtcctga tttctacaac tggagttttt 1450

ttatacaatg ttctttgtct caaaataaag caatgtgttt tttcgg 1496

<210> 48
<211> 204
<212> PRT
<213> Homo Sapien

<400> 48
Met Glu Phe Leu Trp Ala Pro Leu Leu Gly Leu Cys Cys Ser Leu
1               5                   10                  15

Ala Ala Ala Asp Arg His Thr Val Phe Trp Asn Ser Ser Asn Pro
                20                  25                  30

Lys Phe Arg Asn Glu Asp Tyr Thr Ile His Val Gln Leu Asn Asp
                35                  40                  45

Tyr Val Asp Ile Ile Cys Pro His Tyr Glu Asp His Ser Ala Asp
                50                  55                  60

Ala Ala Met Glu Gln Tyr Ile Leu Tyr Leu Val Glu His Glu Glu

```
                    65                  70                  75

Tyr Gln Leu Cys Gln Pro Gln Ser Lys Asp Gln Val Arg Trp Gln
                80                  85                  90

Cys Asn Arg Pro Ser Ala Lys His Gly Pro Glu Lys Leu Ser Glu
                95                  100                 105

Lys Phe Gln Arg Phe Thr Pro Phe Thr Leu Gly Lys Glu Phe Lys
                110                 115                 120

Glu Gly His Ser Tyr Tyr Tyr Ile Ser Lys Pro Ile His Gln His
                125                 130                 135

Glu Asp Arg Cys Leu Arg Leu Lys Val Thr Val Ser Gly Lys Ile
                140                 145                 150

Thr His Ser Pro Gln Ala His Asp Asn Pro Gln Glu Lys Arg Leu
                155                 160                 165

Ala Ala Asp Asp Pro Glu Val Arg Val Leu His Ser Ile Gly His
                170                 175                 180

Ser Ala Ala Pro Arg Leu Phe Pro Leu Ala Trp Thr Val Leu Leu
                185                 190                 195

Leu Pro Leu Leu Leu Leu Gln Thr Pro
                200
```

<210> 49
<211> 2795
<212> DNA
<213> Homo Sapien

<400> 49

```
ctgggcccag ctcccccgag aggtggtcgg atcctctggg ctgctcggtc 50

gatgcctgtg ccactgacgt ccaggcatga ggtggttcct gccctggacg 100

ctggcagcag tgacagcagc agccgccagc accgtcctgg ccacggccct 150

ctctccagcc cctacgacca tggactttac tccagctcca ctggaggaca 200

cctcctcacg cccccaattc tgcaagtggc catgtgagtg cccgccatcc 250

ccacccccgct gcccgctggg ggtcagcctc atcacagatg gctgtgagtg 300

ctgtaagatg tgcgctcagc agcttgggga caactgcacg gaggctgcca 350

tctgtgaccc ccaccggggc ctctactgtg actacagcgg ggaccgcccg 400

aggtacgcaa taggagtgtg tgcacaggtg gtcggtgtgg gctgcgtcct 450

ggatggggtg cgctacaaca acggccagtc cttccagcct aactgcaagt 500

acaactgcac gtgcatcgac ggcgcggtgg gctgcacacc actgtgcctc 550

cgagtgcgcc ccccgcgtct ctggtgcccc cacccgcggc gcgtgagcat 600
```

acctggccac tgctgtgagc agtgggtatg tgaggacgac gccaagaggc 650
cacgcaagac cgcaccccgt gacacaggag ccttcgatgc tgtgggtgag 700
gtggaggcat ggcacaggaa ctgcatagcc tacacaagcc cctggagccc 750
ttgctccacc agctgcggcc tgggggtctc cactcggatc tccaatgtta 800
acgcccagtg ctggcctgag caagagagcc gcctctgcaa cttgcggcca 850
tgcgatgtgg acatccatac actcattaag gcagggaaga agtgtctggc 900
tgtgtaccag ccagaggcat ccatgaactt cacacttgcg ggctgcatca 950
gcacacgctc ctatcaaccc aagtactgtg gagtttgcat ggacaatagg 1000
tgctgcatcc cctacaagtc taagactatc gacgtgtcct tccagtgtcc 1050
tgatgggctt ggcttctccc gccaggtcct atggattaat gcctgcttct 1100
gtaacctgag ctgtaggaat cccaatgaca tctttgctga cttggaatcc 1150
taccctgact tctcagaaat tgccaactag gcaggcacaa atcttgggtc 1200
ttggggacta acccaatgcc tgtgaagcag tcagccctta tggccaataa 1250
cttttcacca atgagcctta gttaccctga tctggaccct tggcctccat 1300
ttctgtctct aaccattcaa atgacgcctg atggtgctgc tcaggcccat 1350
gctatgagtt ttctccttga tatcattcag catctactct aaagaaaaat 1400
gcctgtctct agctgttctg gactacaccc aagcctgatc cagcctttcc 1450
aagtcactag aagtcctgct ggatcttgcc taaatcccaa gaaatggaat 1500
caggtagact tttaatatca ctaatttctt ctttagatgc caaaccacaa 1550
gactctttgg gtccattcag atgaatagat ggaatttgga acaatagaat 1600
aatctattat ttggagcctg ccaagaggta ctgtaatggg taattctgac 1650
gtcagcgcac caaaactatc ctgattccaa atatgtatgc acctcaaggt 1700
catcaaacat ttgccaagtg agttgaatag ttgcttaatt ttgattttta 1750
atggaaagtt gtatccatta acctgggcat tgttgaggtt aagtttctct 1800
tcacccctac actgtgaagg gtacagatta ggtttgtccc agtcagaaat 1850
aaaatttgat aaacattcct gttgatggga aaagccccca gttaatactc 1900
cagagacagg gaaaggtcag cccatttcag aaggaccaat tgactctcac 1950
actgaatcag ctgctgactg gcagggcttt gggcagttgg ccaggctctt 2000
ccttgaatct tctcccttgt cctgcttggg ttcataggaa ttggtaaggc 2050
ctctggactg gcctgtctgg cccctgagag tggtgccctg gaacactcct 2100

ctactcttac agagccttga gagacccagc tgcagaccat gccagaccca 2150

ctgaaatgac caagacaggt tcaggtaggg gtgtgggtca aaccaagaag 2200

tgggtgccct tggtagcagc ctggggtgac ctctagagct ggaggctgtg 2250

ggactccagg ggcccccgtg ttcaggacac atctattgca gagactcatt 2300

tcacagcctt tcgttctgct gaccaaatgg ccagttttct ggtaggaaga 2350

tggaggttta ccagttgttt agaaacagaa atagacttaa taaaggttta 2400

aagctgaaga ggttgaagct aaaaggaaaa ggttgttgtt aatgaatatc 2450

aggctattat ttattgtatt aggaaaatat aatatttact gttagaattc 2500

ttttatttag ggccttttct gtgccagaca ttgctctcag tgctttgcat 2550

gtattagctc actgaatctt cacgacaatg ttgagaagtt cccattatta 2600

tttctgttct tacaaatgtg aaacggaagc tcatagaggt gagaaaactc 2650

aaccagagtc acccagttgg tgactgggaa agttaggatt cagatcgaaa 2700

ttggactgtc tttataaccc atattttccc cctgttttta gagcttccaa 2750

atgtgtcaga ataggaaaac attgcaataa atggcttgat ttttt 2795

<210> 50
<211> 367
<212> PRT
<213> Homo Sapien

<400> 50

Met Arg Trp Phe Leu Pro Trp Thr Leu Ala Ala Val Thr Ala Ala
1 5 10 15

Ala Ala Ser Thr Val Leu Ala Thr Ala Leu Ser Pro Ala Pro Thr
20 25 30

Thr Met Asp Phe Thr Pro Ala Pro Leu Glu Asp Thr Ser Ser Arg
35 40 45

Pro Gln Phe Cys Lys Trp Pro Cys Glu Cys Pro Pro Ser Pro Pro
50 55 60

Arg Cys Pro Leu Gly Val Ser Leu Ile Thr Asp Gly Cys Glu Cys
65 70 75

Cys Lys Met Cys Ala Gln Gln Leu Gly Asp Asn Cys Thr Glu Ala
80 85 90

Ala Ile Cys Asp Pro His Arg Gly Leu Tyr Cys Asp Tyr Ser Gly
95 100 105

Asp Arg Pro Arg Tyr Ala Ile Gly Val Cys Ala Gln Val Val Gly
110 115 120

Val Gly Cys Val Leu Asp Gly Val Arg Tyr Asn Asn Gly Gln Ser
125 130 135

Phe Gln Pro Asn Cys Lys Tyr Asn Cys Thr Cys Ile Asp Gly Ala
140 145 150

Val Gly Cys Thr Pro Leu Cys Leu Arg Val Arg Pro Pro Arg Leu
155 160 165

Trp Cys Pro His Pro Arg Arg Val Ser Ile Pro Gly His Cys Cys
170 175 180

Glu Gln Trp Val Cys Glu Asp Asp Ala Lys Arg Pro Arg Lys Thr
185 190 195

Ala Pro Arg Asp Thr Gly Ala Phe Asp Ala Val Gly Glu Val Glu
200 205 210

Ala Trp His Arg Asn Cys Ile Ala Tyr Thr Ser Pro Trp Ser Pro
215 220 225

Cys Ser Thr Ser Cys Gly Leu Gly Val Ser Thr Arg Ile Ser Asn
230 235 240

Val Asn Ala Gln Cys Trp Pro Glu Gln Glu Ser Arg Leu Cys Asn
245 250 255

Leu Arg Pro Cys Asp Val Asp Ile His Thr Leu Ile Lys Ala Gly
260 265 270

Lys Lys Cys Leu Ala Val Tyr Gln Pro Glu Ala Ser Met Asn Phe
275 280 285

Thr Leu Ala Gly Cys Ile Ser Thr Arg Ser Tyr Gln Pro Lys Tyr
290 295 300

Cys Gly Val Cys Met Asp Asn Arg Cys Cys Ile Pro Tyr Lys Ser
305 310 315

Lys Thr Ile Asp Val Ser Phe Gln Cys Pro Asp Gly Leu Gly Phe
320 325 330

Ser Arg Gln Val Leu Trp Ile Asn Ala Cys Phe Cys Asn Leu Ser
335 340 345

Cys Arg Asn Pro Asn Asp Ile Phe Ala Asp Leu Glu Ser Tyr Pro
350 355 360

Asp Phe Ser Glu Ile Ala Asn
365

<210> 51
<211> 2185
<212> DNA
<213> Homo Sapien

<400> 51
cgccgcccgc cgcctgcctg ggccgggccg aggatgcggc gcagcgcctc 50

ggcggccagg ctcgctcccc tccggcacgc ctgctaactt cccccgctac 100
gtccccgttc gcccgccggg ccgccccgtc tccccgcgcc ctccgggtcg 150
ggtcctccag gagcgccagg cgctgccgcc gtgtgccctc cgccgctcgc 200
ccgcgcgccc gcgctccccg cctgcgccca gcgccccgcg cccgcgccca 250
gtcctcgggc ggtcatgctg cccctctgcc tcgtggccgc cctgctgctg 300
gccgccgggc ccgggccgag cctgggcgac gaagccatcc actgcccgcc 350
ctgctccgag gagaagctgg cgcgctgccg cccccccgtg ggctgcgagg 400
agctggtgcg agagccgggc tgcggctgtt gcgccacttg cgccctgggc 450
ttggggatgc cctgcggggt gtacaccccc cgttgcggct cgggcctgcg 500
ctgctacccg ccccgagggg tggagaagcc cctgcacaca ctgatgcacg 550
ggcaaggcgt gtgcatggag ctggcggaga tcgaggccat ccaggaaagc 600
ctgcagccct ctgacaagga cgagggtgac caccccaaca acagcttcag 650
cccctgtagc gcccatgacc gcaggtgcct gcagaagcac ttcgccaaaa 700
ttcgagaccg gagcaccagt gggggcaaga tgaaggtcaa tggggcgccc 750
cgggaggatg cccggcctgt gccccagggc tcctgccaga gcgagctgca 800
ccgggcgctg gagcggctgg ccgcttcaca gagccgcacc cacgaggacc 850
tctacatcat ccccatcccc aactgcgacc gcaacggcaa cttccacccc 900
aagcagtgtc acccagctct ggatgggcag cgtggcaagt gctggtgtgt 950
ggaccggaag acgggggtga agcttccggg gggcctggag ccaaaggggg 1000
agctggactg ccaccagctg gctgacagct ttcgagagtg aggcctgcca 1050
gcaggccagg gactcagcgt cccctgctac tcctgtgctc tggaggctgc 1100
agagctgacc cagagtggag tctgagtctg agtcctgtct ctgcctgcgg 1150
cccagaagtt tccctcaaat gcgcgtgtgc acgtgtgcgt gtgcgtgcgt 1200
gtgtgtgtgt ttgtgagcat gggtgtgccc ttggggtaag ccagagcctg 1250
gggtgttctc tttggtgtta cacagcccaa gaggactgag actggcactt 1300
agcccaagag gtctgagccc tggtgtgttt ccagatcgat cctggattca 1350
ctcactcact cattccttca ctcatccagc cacctaaaaa catttactga 1400
ccatgtacta cgtgccagct ctagttttca gccttgggag gttttattct 1450
gacttcctct gattttggca tgtggagaca ctcctataag gagagttcaa 1500
gcctgtggga gtagaaaaat ctcattccca gagtcagagg agaagagaca 1550

```
tgtaccttga ccatcgtcct tcctctcaag ctagccagag ggtgggagcc 1600
taaggaagcg tggggtagca gatggagtaa tggtcacgag gtccagaccc 1650
actcccaaag ctcagacttg ccaggctccc tttctcttct tccccaggtc 1700
cttcctttag gtctggttgt tgcaccatct gcttggttgg ctggcagctg 1750
agagccctgc tgtgggagag cgaagggggt caaaggaaga cttgaagcac 1800
agagggctag ggaggtgggg tacatttctc tgagcagtca gggtgggaag 1850
aaagaatgca agagtggact gaatgtgcct aatggagaag acccacgtgc 1900
taggggatga ggggcttcct gggtcctgtt ccctacccca tttgtggtca 1950
cagccatgaa gtcaccggga tgaacctatc cttccagtgg ctcgctccct 2000
gtagctctgc ctccctctcc atatctcctt cccctacacc tccctcccca 2050
cacctcccta ctcccctggg catcttctgg cttgactgga tggaaggaga 2100
cttaggaacc taccagttgg ccatgatgtc ttttcttctt tttctttttt 2150
ttaacaaaac agaacaaaac caaaaaatgt ccaaa 2185
```

<210> 52
<211> 258
<212> PRT
<213> Homo Sapien

<400> 52

```
Met Leu Pro Leu Cys Leu Val Ala Ala Leu Leu Leu Ala Ala Gly
 1               5                  10                  15

Pro Gly Pro Ser Leu Gly Asp Glu Ala Ile His Cys Pro Pro Cys
                20                  25                  30

Ser Glu Glu Lys Leu Ala Arg Cys Arg Pro Pro Val Gly Cys Glu
                35                  40                  45

Glu Leu Val Arg Glu Pro Gly Cys Gly Cys Cys Ala Thr Cys Ala
                50                  55                  60

Leu Gly Leu Gly Met Pro Cys Gly Val Tyr Thr Pro Arg Cys Gly
                65                  70                  75

Ser Gly Leu Arg Cys Tyr Pro Pro Arg Gly Val Glu Lys Pro Leu
                80                  85                  90

His Thr Leu Met His Gly Gln Gly Val Cys Met Glu Leu Ala Glu
                95                 100                 105

Ile Glu Ala Ile Gln Glu Ser Leu Gln Pro Ser Asp Lys Asp Glu
               110                 115                 120

Gly Asp His Pro Asn Asn Ser Phe Ser Pro Cys Ser Ala His Asp
               125                 130                 135
```

Arg Arg Cys Leu Gln Lys His Phe Ala Lys Ile Arg Asp Arg Ser
140 145 150

Thr Ser Gly Gly Lys Met Lys Val Asn Gly Ala Pro Arg Glu Asp
155 160 165

Ala Arg Pro Val Pro Gln Gly Ser Cys Gln Ser Glu Leu His Arg
170 175 180

Ala Leu Glu Arg Leu Ala Ala Ser Gln Ser Arg Thr His Glu Asp
185 190 195

Leu Tyr Ile Ile Pro Ile Pro Asn Cys Asp Arg Asn Gly Asn Phe
200 205 210

His Pro Lys Gln Cys His Pro Ala Leu Asp Gly Gln Arg Gly Lys
215 220 225

Cys Trp Cys Val Asp Arg Lys Thr Gly Val Lys Leu Pro Gly Gly
230 235 240

Leu Glu Pro Lys Gly Glu Leu Asp Cys His Gln Leu Ala Asp Ser
245 250 255

Phe Arg Glu

<210> 53
<211> 1042
<212> DNA
<213> Homo Sapien

<400> 53
tttcctcact gactataaaa gaatagagaa ggaagggctt cagtgaccgg 50

ctgcctggct gacttacagc agtcagactc tgacaggatc atggctatga 100

tggaggtcca ggggggaccc agcctgggac agacctgcgt gctgatcgtg 150

atcttcacag tgctcctgca gtctctctgt gtggctgtaa cttacgtgta 200

ctttaccaac gagctgaagc agatgcagga caagtactcc aaaagtggca 250

ttgcttgttt cttaaaagaa gatgacagtt attgggaccc caatgacgaa 300

gagagtatga acagcccctg ctggcaagtc aagtggcaac tccgtcagct 350

cgttagaaag atgattttga gaacctctga ggaaaccatt tctacagttc 400

aagaaaagca acaaaatatt tctcccctag tgagagaaag aggtcctcag 450

agagtagcag ctcacataac tgggaccaga ggaagaagca acacattgtc 500

ttctccaaac tccaagaatg aaaaggctct gggccgcaaa ataaactcct 550

gggaatcatc aaggagtggg cattcattcc tgagcaactt gcacttgagg 600

aatggtgaac tggtcatcca tgaaaaaggg ttttactaca tctattccca 650

aacatacttt cgatttcagg aggaaataaa agaaaacaca aagaacgaca 700

aacaaatggt ccaatatatt tacaaataca caagttatcc tgaccctata 750

ttgttgatga aaagtgctag aaatagttgt tggtctaaag atgcagaata 800

tggactctat tccatctatc aagggggaat atttgagctt aaggaaaatg 850

acagaatttt tgtttctgta acaaatgagc acttgataga catggaccat 900

gaagccagtt ttttcggggc ctttttagtt ggctaactga cctggaaaga 950

aaaagcaata acctcaaagt gactattcag ttttcaggat gatacactat 1000

gaagatgttt caaaaaatct gaccaaaaca aacaaacaga aa 1042

<210> 54
<211> 281
<212> PRT
<213> Homo Sapien

<400> 54
Met Ala Met Met Glu Val Gln Gly Gly Pro Ser Leu Gly Gln Thr
1 5 10 15

Cys Val Leu Ile Val Ile Phe Thr Val Leu Leu Gln Ser Leu Cys
20 25 30

Val Ala Val Thr Tyr Val Tyr Phe Thr Asn Glu Leu Lys Gln Met
35 40 45

Gln Asp Lys Tyr Ser Lys Ser Gly Ile Ala Cys Phe Leu Lys Glu
50 55 60

Asp Asp Ser Tyr Trp Asp Pro Asn Asp Glu Glu Ser Met Asn Ser
65 70 75

Pro Cys Trp Gln Val Lys Trp Gln Leu Arg Gln Leu Val Arg Lys
80 85 90

Met Ile Leu Arg Thr Ser Glu Glu Thr Ile Ser Thr Val Gln Glu
95 100 105

Lys Gln Gln Asn Ile Ser Pro Leu Val Arg Glu Arg Gly Pro Gln
110 115 120

Arg Val Ala Ala His Ile Thr Gly Thr Arg Gly Arg Ser Asn Thr
125 130 135

Leu Ser Ser Pro Asn Ser Lys Asn Glu Lys Ala Leu Gly Arg Lys
140 145 150

Ile Asn Ser Trp Glu Ser Ser Arg Ser Gly His Ser Phe Leu Ser
155 160 165

Asn Leu His Leu Arg Asn Gly Glu Leu Val Ile His Glu Lys Gly
170 175 180

Phe Tyr Tyr Ile Tyr Ser Gln Thr Tyr Phe Arg Phe Gln Glu Glu
185 190 195

Ile Lys Glu Asn Thr Lys Asn Asp Lys Gln Met Val Gln Tyr Ile
200 205 210

Tyr Lys Tyr Thr Ser Tyr Pro Asp Pro Ile Leu Leu Met Lys Ser
215 220 225

Ala Arg Asn Ser Cys Trp Ser Lys Asp Ala Glu Tyr Gly Leu Tyr
230 235 240

Ser Ile Tyr Gln Gly Gly Ile Phe Glu Leu Lys Glu Asn Asp Arg
245 250 255

Ile Phe Val Ser Val Thr Asn Glu His Leu Ile Asp Met Asp His
260 265 270

Glu Ala Ser Phe Phe Gly Ala Phe Leu Val Gly
275 280

<210> 55
<211> 663
<212> DNA
<213> Homo Sapien

<400> 55
atggaacttg gacttggagg cctctccacg ctgtcccact gcccctggcc 50

taggcggcag cctgccctgt ggcccaccct ggccgctctg gctctgctga 100

gcagcgtcgc agaggcctcc ctgggctccg cgccccgcag ccctgccccc 150

cgcgaaggcc ccccgcctgt cctggcgtcc cccgccggcc acctgccggg 200

gggacgcacg gcccgctggt gcagtggaag agcccggcgg ccgccgccgc 250

agccttctcg gcccgcgccc ccgccgcctg cacccccatc tgctcttccc 300

cgcgggggcc gcgcggcgcg ggctgggggc ccgggcagcc gcgctcgggc 350

agcgggggcg cggggctgcc gcctgcgctc gcagctggtg ccggtgcgcg 400

cgctcggcct gggccaccgc tccgacgagc tggtgcgttt ccgcttctgc 450

agcggctcct gccgccgcgc gcgctctcca cacgacctca gcctggccag 500

cctactgggc gccggggccc tgcgaccgcc cccgggctcc cggcccgtca 550

gccagccctg ctgccgaccc acgcgctacg aagcggtctc cttcatggac 600

gtcaacagca cctggagaac cgtggaccgc ctctccgcca ccgcctgcgg 650

ctgcctgggc tga 663

<210> 56
<211> 220
<212> PRT
<213> Homo Sapien

<400> 56

```
Met Glu Leu Gly Leu Gly Gly Leu Ser Thr Leu Ser His Cys Pro
1               5                   10                  15

Trp Pro Arg Arg Gln Pro Ala Leu Trp Pro Thr Leu Ala Ala Leu
                20                  25                  30

Ala Leu Leu Ser Ser Val Ala Glu Ala Ser Leu Gly Ser Ala Pro
                35                  40                  45

Arg Ser Pro Ala Pro Arg Glu Gly Pro Pro Pro Val Leu Ala Ser
                50                  55                  60

Pro Ala Gly His Leu Pro Gly Gly Arg Thr Ala Arg Trp Cys Ser
                65                  70                  75

Gly Arg Ala Arg Arg Pro Pro Pro Gln Pro Ser Arg Pro Ala Pro
                80                  85                  90

Pro Pro Pro Ala Pro Pro Ser Ala Leu Pro Arg Gly Gly Arg Ala
                95                  100                 105

Ala Arg Ala Gly Gly Pro Gly Ser Arg Ala Arg Ala Ala Gly Ala
                110                 115                 120

Arg Gly Cys Arg Leu Arg Ser Gln Leu Val Pro Val Arg Ala Leu
                125                 130                 135

Gly Leu Gly His Arg Ser Asp Glu Leu Val Arg Phe Arg Phe Cys
                140                 145                 150

Ser Gly Ser Cys Arg Arg Ala Arg Ser Pro His Asp Leu Ser Leu
                155                 160                 165

Ala Ser Leu Leu Gly Ala Gly Ala Leu Arg Pro Pro Pro Gly Ser
                170                 175                 180

Arg Pro Val Ser Gln Pro Cys Cys Arg Pro Thr Arg Tyr Glu Ala
                185                 190                 195

Val Ser Phe Met Asp Val Asn Ser Thr Trp Arg Thr Val Asp Arg
                200                 205                 210

Leu Ser Ala Thr Ala Cys Gly Cys Leu Gly
                215                 220
```

<210> 57
<211> 43
<212> DNA
<213> Artificial Sequence

<220>
<223> Synthetic oligonucleotide probe.

<400> 57
tgtaaaacga cggccagtta aatagacctg caattattaa tct 43

<210> 58

<211> 41
<212> DNA
<213> Artificial Sequence

<220>
<223> Synthetic oligonucleotide probe.

<400> 58
caggaaacag ctatgaccac ctgcacacct gcaaatccat t 41

<210> 59
<211> 24
<212> DNA
<213> Artificial Sequence

<220>
<223> Synthetic oligonucleotide probe.

<400> 59
tcagctccag actctgatac tgcc 24

<210> 60
<211> 24
<212> DNA
<213> Artificial Sequence

<220>
<223> Synthetic oligonucleotide probe.

<400> 60
tgcctttcta ggaggcagag ctcc 24

<210> 61
<211> 50
<212> DNA
<213> Artificial Sequence

<220>
<223> Synthetic oligonucleotide probe.

<400> 61
ggacccagaa atgtgtcctg agaatggatc ttgtgtacct gatggtccag 50

<210> 62
<211> 26
<212> DNA
<213> Artificial Sequence

<220>
<223> Synthetic oligonucleotide probe.

<400> 62
ctttccttgc ttcagcaaca tgaggc 26

<210> 63
<211> 25
<212> DNA
<213> Artificial Sequence

<220>
<223> Synthetic oligonucleotide probe.

<400> 63
gcccagagca ggaggaatga tgagc 25

<210> 64
<211> 49
<212> DNA
<213> Artificial Sequence

<220>
<223> Synthetic oligonucleotide probe.

<400> 64
gtggaacgcg gtcttgactc tgttcgtcac ttctttgatt ggggctttg 49

<210> 65
<211> 24
<212> DNA
<213> Artificial Sequence

<220>
<223> Synthetic oligonucleotide probe.

<400> 65
cacagagcca gaagtggcgg aatc 24

<210> 66
<211> 25
<212> DNA
<213> Artificial Sequence

<220>
<223> Synthetic oligonucleotide probe.

<400> 66
ccacatgttc ctgctcttgt cctgg 25

<210> 67
<211> 45
<212> DNA
<213> Artificial Sequence

<220>
<223> Synthetic oligonucleotide probe.

<400> 67
cggtagtgac tgtactctag tcctgtttta caccccgtgg tgccg 45

<210> 68
<211> 23
<212> DNA
<213> Artificial Sequence

<220>
<223> Synthetic oligonucleotide probe.

<400> 68

ctggggctac acacggggtg agg 23

<210> 69
<211> 24
<212> DNA
<213> Artificial Sequence

<220>
<223> Synthetic oligonucleotide probe.

<400> 69
ggtgccgctg cagaaagtag agcg 24

<210> 70
<211> 45
<212> DNA
<213> Artificial Sequence

<220>
<223> Synthetic oligonucleotide probe.

<400> 70
gccccaaatg aaaacgggcc ctacttcctg gccctccgcg agatg 45

<210> 71
<211> 24
<212> DNA
<213> Artificial Sequence

<220>
<223> Synthetic oligonucleotide probe.

<400> 71
agctgtggtc atggtggtgt ggtg 24

<210> 72
<211> 24
<212> DNA
<213> Artificial Sequence

<220>
<223> Synthetic oligonucleotide probe.

<400> 72
ctaccttggc cataggtgat ccgc 24

<210> 73
<211> 42
<212> DNA
<213> Artificial Sequence

<220>
<223> Synthetic oligonucleotide probe.

<400> 73
catcagcaaa ccgtctgtgg ttcagctcaa ctggagaggg tt 42

<210> 74
<211> 23

<212> DNA
<213> Artificial Sequence

<220>
<223> Synthetic oligonucleotide probe.

<400> 74
atgcaggcca agtacagcag cac 23

<210> 75
<211> 23
<212> DNA
<213> Artificial Sequence

<220>
<223> Synthetic oligonucleotide probe.

<400> 75
catgctgacg acttcctgca agc 23

<210> 76
<211> 23
<212> DNA
<213> Artificial Sequence

<220>
<223> Synthetic oligonucleotide probe.

<400> 76
ccacacagtc tctgcttctt ggg 23

<210> 77
<211> 40
<212> DNA
<213> Artificial Sequence

<220>
<223> Synthetic oligonucleotide probe.

<400> 77
atgctggatg atgatgggga caccaccatg agcctgcatt 40

<210> 78
<211> 27
<212> DNA
<213> Artificial Sequence

<220>
<223> Synthetic oligonucleotide probe.

<400> 78
ggatttggtt agctgagccc accgaga 27

<210> 79
<211> 26
<212> DNA
<213> Artificial Sequence

<220>

<223> Synthetic oligonucleotide probe.

<400> 79
gcactgcgcg cgacctcagg gctgca 26

<210> 80
<211> 50
<212> DNA
<213> Artificial Sequence

<220>
<223> Synthetic oligonucleotide probe.

<400> 80
cttattgccc taaatattag ggagccggcg acctcctgga tcctctcatt 50

<210> 81
<211> 22
<212> DNA
<213> Artificial Sequence

<220>
<223> Synthetic oligonucleotide probe.

<400> 81
gctgctgccg tccatgctga tg 22

<210> 82
<211> 23
<212> DNA
<213> Artificial Sequence

<220>
<223> Synthetic oligonucleotide probe.

<400> 82
ctcggggaat gtgacatcgt cgc 23

<210> 83
<211> 35
<212> DNA
<213> Artificial Sequence

<220>
<223> Synthetic oligonucleotide probe.

<400> 83
gctgccgtcc atgctgatgt ttgcggtgat cgtgg 35

<210> 84
<211> 48
<212> DNA
<213> Artificial Sequence

<220>
<223> Synthetic oligonucleotide probe.

<400> 84
ggattctaat acgactcact atagggcccg agatatgcac ccaatgtc 48

<210> 85
<211> 47
<212> DNA
<213> Artificial Sequence

<220>
<223> Synthetic oligonucleotide probe.

<400> 85
ctatgaaatt aaccctcact aaagggatcc cagaatcccg aagaaca 47

<210> 86
<211> 48
<212> DNA
<213> Artificial Sequence

<220>
<223> Synthetic oligonucleotide probe.

<400> 86
ggattctaat acgactcact atagggccct ctgtccactg ctttcgtg 48

<210> 87
<211> 48
<212> DNA
<213> Artificial Sequence

<220>
<223> Synthetic oligonucleotide probe.

<400> 87
ctatgaaatt aaccctcact aaagggagtt ctccaccgtg tctccaca 48

<210> 88
<211> 48
<212> DNA
<213> Artificial Sequence

<220>
<223> Synthetic oligonucleotide probe.

<400> 88
ggattctaat acgactcact atagggccgc gctgtcctgc tgtcacca 48

<210> 89
<211> 48
<212> DNA
<213> Artificial Sequence

<220>
<223> Synthetic oligonucleotide probe.

<400> 89
ctatgaaatt aaccctcact aaagggagtt cccctccccg agaagata 48

<210> 90
<211> 47
<212> DNA

<213> Artificial Sequence

<220>
<223> Synthetic oligonucleotide probe.

<400> 90
ggattctaat acgactcact atagggccag caaaagaagc ggtggtg 47

<210> 91
<211> 48
<212> DNA
<213> Artificial Sequence

<220>
<223> Synthetic oligonucleotide probe.

<400> 91
ctatgaaatt aaccctcact aaagggattc agcacgccag agacactt 48

**Description**

## FIELD OF THE INVENTION

The present invention concerns methods and compositions for inhibiting neoplastic cell growth. In particular, the present invention concerns antitumor compositions and methods for the treatment of tumors. The invention further concerns screening methods for identifying growth inhibitory, *e.g.*, antitumor compounds.

## BACKGROUND OF THE INVENTION

Malignant tumors (cancers) are the second leading cause of death in the United States, after heart disease (Boring et al., CA Cancel J. Clin., 43:7 (1993)).
Cancer is characterized by the increase in the number of abnormal, or neoplastic, cells derived from a normal tissue which proliferate to form a tumor mass, the invasion of adjacent tissues by these neoplastic tumor cells, and the generation of malignant cells which eventually spread via the blood or lymphatic system to regional lymph nodes and to distant sites (metastasis). In a cancerous state a cell proliferates under conditions in which normal cells would not grow. Cancer manifests itself in a wide variety of forms, characterized by different degrees of invasiveness and aggressiveness.
Despite recent advances in cancer therapy, there is a great need for new therapeutic agents capable of inhibiting neoplastic cell growth. Accordingly, it is the objective of the present invention to identify compounds capable of inhibiting the growth of neoplastic cells, such as cancer cells.

## SUMMARY OF THE INVENTION

A. Embodiments

The present invention relates to methods and compositions for inhibiting neoplastic cell growth. More particularly, the invention concerns methods and compositions for the treatment of tumors, including cancers, such as breast, prostate, colon, lung, ovarian, renal and CNS cancers, leukemia, melanoma, etc., in mammalian patients, preferably humans.
In one aspect, the present invention concerns compositions of matter useful for the inhibition of neoplastic cell growth comprising an effective amount of a PRO polypeptide as herein defined, or an agonist thereof, in admixture with a pharmaceutically acceptable carrier. In a preferred embodiment, the composition of matter comprises a growth inhibitory amount of a PRO polypeptide, or an agonist thereof. In another preferred embodiment, the composition comprises a cytotoxic amount of a PRO polypeptide, or an agonist thereof. Optionally, the compositions of matter maycontain one or more additional growth inhibitoryand/orcytotoxic and/or other chemotherapeutic agents.
In a further aspect, the present invention concerns compositions of matter useful for the treatment of a tumor in a mammal comprising a therapeutically effective amount of a PRO polypeptide as herein defined, or an agonist thereof. The tumor is preferably a cancer.
In another aspect, the invention concerns a method for inhibiting the growth of a tumor cell comprising exposing the cell to an effective amount of a PRO polypeptide as herein defined, or an agonist thereof. In a particular embodiment, the agonist is an anti-PRO agonist antibody. In another embodiment, the agonist is a small molecule that mimics the biological activity of a PRO polypeptide. The method may be performed *in vitro* or *in vivo.*
In a still further embodiment, the invention concerns an article of manufacture comprising:

(a) a container;
(b) a composition comprising an active agent contained within the container; wherein the composition is effective for inhibiting the neoplastic cell growth, *e.g.,* growth of tumor cells, and the active agent in the composition is a PRO polypeptide as herein defined, or an agonist thereof; and
(c) a label affixed to said container, or a package insert included in said container referring to the use of said PRO polypeptide or agonist thereof, for the inhibition of neoplastic cell growth, wherein the agonist may be an antibody which binds to the PRO polypeptide.
In a particular embodiment, the agonist is an anti-PRO agonist antibody. In another embodiment, the agonist is a small molecule that mimics the biological activity of a PRO polypeptide. Similar articles of manufacture comprising a PRO polypeptide as herein defined, or an agonist thereof in an amount that is therapeutically effective for the treatment of tumor are also within the scope of the present invention. Also within the scope of the invention are articles of manufacture comprising a PRO polypeptide as herein defined, or an agonist thereof, and a further growth inhibitory agent, cytotoxic agent or chemotherapeutic agent.

B. Additional Embodiments

In other embodiments of the present invention, the invention provides an isolated nucleic acid molecule comprising a nucleotide sequence that encodes a PRO polypeptide. In one aspect, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule encoding a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule comprising the coding sequence of a full-length PRO polypeptide cDNA as disclosed herein, the coding sequence of a PRO polypeptide lacking the signal peptide as disclosed herein, the coding sequence of an extracellular domain of a transmembrane PRO polypeptide, with or without the signal peptide, as disclosed herein or the coding sequence of any other specifically defined fragment of the full-length amino acid sequence as disclosed herein, or (b) the complement of the DNA molecule of (a).

In a further aspect, the invention concerns an isolated nucleic acid molecule comprising a nucleotide sequence having at least about 80% nucleic acid sequence identity, alternatively at least about 81 % nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule that encodes the same mature polypeptide encoded by any of the human protein cDNAs deposited with the ATCC as disclosed herein, or (b) the complement of the DNA molecule of (a).

Another aspect of the invention provides an isolated nucleic acid molecule comprising a nucleotide sequence encoding a PRO polypeptide which is either transmembrane domain-deleted or transmembrane domain-inactivated, or is complementary to such encoding nucleotide sequence, wherein the transmembrane domain(s) of such polypeptides are disclosed herein. Therefore, soluble extracellular domains of the herein described PRO polypeptides are contemplated.

Another embodiment is directed to fragments of a PRO polypeptide coding sequence, or the complement thereof, that may find use as, for example, hybridization probes, for encoding fragments of a PRO polypeptide that may optionally encode a polypeptide comprising a binding site for an anti-PRO antibody or as antisense oligonucleotide probes. Such nucleic acid fragments are usually at least about 20 nucleotides in length, alternatively at least about 30 nucleotides in length, alternatively at least about 40 nucleotides in length, alternatively at least about 50 nucleotides in length, alternatively at least about 60 nucleotides in length, alternatively at least about 70 nucleotides in length, alternatively at least about 80 nucleotides in length, alternatively at least about 90 nucleotides in length, alternatively at least about 100 nucleotides in length, alternatively at least about 110 nucleotides in length, alternatively at least about 120 nucleotides in length, alternatively at least about 130 nucleotides in length, alternatively at least about 140 nucleotides in length, alternatively at least about 150 nucleotides in length, alternatively at least about 160 nucleotides in length, alternatively at least about 170 nucleotides in length, alternatively at least about 180 nucleotides in length, alternatively at least about 190 nucleotides in length, alternatively at least about 200 nucleotides in length, alternatively at least about 250 nucleotides in length, alternatively at least about 300 nucleotides in length, alternatively at least about 350 nucleotides in length, alternatively at least about 400 nucleotides in length, alternatively at least about 450 nucleotides in length, alternatively at least about 500 nucleotides in length, alternatively at least about 600 nucleotides in length, alternatively at least about 700 nucleotides in length, alternatively at least about 800 nucleotides in length, alternatively at least about 900 nucleotides in length and alternatively at least about 1000 nucleotides in length, wherein in this context the term "about" means the referenced nucleotide sequence length plus or minus 10% of that referenced length. It is noted that novel fragments of a PRO polypeptide-encoding nucleotide sequence may be determined in a routine manner by aligning the PRO polypeptide-encoding nucleotide sequence with other known nucleotide sequences using any of a number of well known sequence alignment programs and determining which PRO polypeptide-encoding nucleotide sequence fragment(s) are novel. All of such PRO polypeptide-encoding nucleotide sequences are contemplated herein. Also contemplated are the PRO polypeptide fragments encoded by these nucleotide molecule fragments, preferably those PRO polypeptide fragments that comprise a binding site for an anti-PRO antibody.

In another embodiment, the invention provides an isolated PRO polypeptide encoded by any of the isolated nucleic acid sequences hereinabove identified.

In a certain aspect, the invention concerns an isolated PRO polypeptide, comprising an amino acid sequence having at least about 80% amino acid sequence identity, alternatively at least about 81 % amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91 % amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a further aspect, the invention concerns an isolated PRO polypeptide comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91 % amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to an amino acid sequence encoded by any of the human protein cDNAs deposited with the ATCC as disclosed herein.

In a further aspect, the invention concerns an isolated PRO polypeptide comprising an aminoacid sequence scoring at least about 80% positives, alternatively at least about 81 % positives, alternatively at least about 82% positives, alternatively at least about 83% positives, alternatively at least about 84% positives, alternatively at least about 85% positives, alternatively at least about 86% positives, alternatively at least about 87% positives, alternatively at least about 88% positives, alternatively at least about 89% positives, alternatively at least about 90%. positives, alternatively at least about 91 % positives, alternatively at least about 92% positives, alternatively at least about 93% positives, alternatively at least about 94% positives, alternatively at least about 95% positives, alternatively at least about 96% positives, alternatively at least about 97% positives, alternatively at least about 98% positives and alternatively at least about 99% positives when compared with the amino acid sequence of a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a specific aspect, the invention provides an isolated PRO polypeptide without the N-terminal signal sequence and/or the initiating methionine and is encoded by a nucleotide sequence that encodes such an amino acid sequence as hereinbefore described. Processes for producing the same are also herein described, wherein those processes comprise culturing a host cell comprising a vector which comprises the appropriate encoding nucleic acid molecule underconditions suitable for expression of the PRO polypeptide and recovering the PRO polypeptide from the cell culture.

Another aspect of the invention provides an isolated PRO polypeptide which is either transmembrane domain-deleted or transmembrane domain-inactivated. Processes for producing the same are also herein described,

wherein those processes comprise culturing a host cell comprising a vector which comprises the appropriate encoding nucleic acid molecule under conditions suitable for expression of the PRO polypeptide and recovering the PRO polypeptide from the cell culture.

In yet another embodiment, the invention concerns agonists of a native PRO polypeptide as defined herein.

In a particular embodiment, the agonist is an anti-PRO antibody or a small molecule.

In a further embodiment, the invention concerns a method of identifying agonists to a PRO polypeptide which comprise contacting the PRO polypeptide with a candidate molecule and monitoring a biological activity mediated by said PRO polypeptide. Preferably, the PRO polypeptide is a native PRO polypeptide.

In a still further embodiment, the invention concerns a composition of matter comprising a PRO polypeptide, or an agonist of a PRO polypeptide as herein described, or an anti-PRO antibody, in combination with a carrier. Optionally, the carrier is a pharmaceutically acceptable carrier.

Another embodiment of the present invention is directed to the use of a PRO polypeptide, or an agonist thereof as hereinbefore described, or an anti-PRO antibody, for the preparation of a medicament useful in the treatment of a condition which is responsive to the PRO polypeptide, an agonist thereof or an anti-PRO antibody.

In additional embodiments of the present invention, the invention provides vectors comprising DNA encoding any of the herein described polypeptides. Host cells comprising any such vector are also provided. By way of example, the host cells may be CHO cells, *E. coli,* yeast, or Baculovirus-infected insect cells. A process for producing any of the herein described polypeptides is further provided and comprises culturing host cells under conditions suitable for expression of the desired polypeptide and recovering the desired polypeptide from the cell culture.

In other embodiments, the invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

In yet another embodiment, the invention provides an antibody which specifically binds to any of the above or below described polypeptides. Optionally, the antibody is a monoclonal antibody, humanized antibody, antibody fragment or single-chain antibody.

In yet other embodiments, the invention provides oligonucleotide probes useful for isolating genomic and cDNA nucleotide sequences or as antisense probes, wherein those probes may be derived from any of the above or below described nucleotide sequences.

## BRIEF DESCRIPTION OF THE DRAWINGS

Figure 1 shows a nucleotide sequence (SEQ ID NO:1) of a native sequence PRO240 cDNA, wherein SEQ ID NO: 1 is a clone designated herein as "DNA34387-1138".

Figure 2 shows the amino acid sequence (SEQ ID NO:2) derived from the coding sequence of SEQ ID NO:1 shown in Figure 1.

Figure 3 shows a nucleotide sequence (SEQ ID NO:3) of a native sequence PRO381 cDNA, wherein SEQ ID NO: 3 is a clone designated herein as "DNA44194-1317".

Figure 4 shows the amino acid sequence (SEQ ID NO:4) derived from the coding sequence of SEQ ID NO:3 shown in Figure 3.

Figure 5 shows a nucleotide sequence (SEQ ID NO:5) of a native sequence PRO534 cDNA, wherein SEQ ID NO: 5 is a clone designated herein as "DNA48333-1321".

Figure 6 shows the amino acid sequence (SEQ ID NO:6) derived from the coding sequence of SEQ ID NO:5 shown in Figure 5.

Figure 7 shows a nucleotide sequence (SEQ ID NO:7) of a native sequence PRO540 cDN wherein SEQ ID NO:7 is a clone designated herein as "DNA44189-1322".

Figure 8 shows the amino acid sequence (SEQ ID NO:8) derived from the coding sequence of SEQ ID NO:7 shown in Figure 7.

Figure 9 shows a nucleotide sequence (SEQ ID NO:9) of a native sequence PRO698 cDNA, wherein SEQ ID NO: 9 is a clone designated herein as "DNA48320-1433".

Figure 10 shows the amino acid sequence (SEQ ID NO:10) derived from the coding sequence of SEQ ID NO:9 shown in Figure 9.

Figure 11 shows a nucleotide sequence (SEQ ID NO:11) of a native sequence PRO982 cDNA, wherein SEQ ID NO:11 is a clone designated herein as "DNA57700-1408".

Figure 12 shows the amino acid sequence (SEQ ID NO:12) derived from the coding sequence of SEQ ID NO:11 shown in Figure 11.

Figure 13 shows a nucleotide sequence (SEQ ID NO: 13) of a native sequence PRO1005 cDNA, wherein SEQ ID NO: 13 is a clone designated herein as "DNA57708-1411".

Figure 14 shows the amino acid sequence (SEQ ID NO:14) derived from the coding sequence of SEQ ID NO:13 shown in Figure 13.

Figure 15 shows a nucleotide sequence (SEQ ID NO: 15) of a native sequence PRO 1007 cDNA, wherein SEQ ID NO:15 is a clone designated herein as "DNA57690-1374".

Figure 16 shows the amino acid sequence (SEQ ID NO:16) derived from the coding sequence of SEQ ID NO:15 shown in Figure 15.

Figure 17 shows a nucleotide sequence (SEQ ID NO:17) of a native sequence PRO 1131 cDNA, wherein SEQ ID NO:17 is a clone designated herein as "DNA59777-1480".

Figure 18 shows the amino acid sequence (SEQ ID NO:18) derived from the coding sequence of SEQ ID NO:17 shown in Figure 17.

Figure 19 shows a nucleotide sequence (SEQ ID NO:19) of a native sequence PRO1157 cDNA, wherein SEQ ID NO:19 is a clone designated herein as "DNA60292-1506".

Figure 20 shows the amino acid sequence (SEQ ID NO:20) derived from the coding sequence of SEQ ID NO:19 shown in Figure 19.

Figure 21 shows a nucleotide sequence (SEQ ID NO:21) of a native sequence PRO1199 cDNA. wherein SEQ ID NO:21 is a clone designated herein as "DNA65351-1366-1".

Figure 22 shows the amino acid sequence (SEQ ID NO:22) derived from the coding sequence of SEQ ID NO:21 shown in Figure 21.

Figure 23 shows a nucleotide sequence (SEQ ID NO:23) of a native sequence PRO1265 cDNA, wherein SEQ ID NO:23 is a clone designated herein as "DNA60764-1533".

Figure 24 shows the amino acid sequence (SEQ ID NO:24) derived from the coding sequence of SEQ ID NO:23 shown in Figure 23.

Figure 25 shows a nucleotide sequence (SEQ ID NO:25) of a native sequence PRO 1286 cDNA, wherein SEQ ID NO:25 is a clone designated herein as "DNA64903-1553".

Figure 26 shows the amino acid sequence (SEQ ID NO:26) derived from the coding sequence of SEQ ID NO:25 shown in Figure 25.

Figure 27 shows a nucleotide sequence (SEQ ID NO:27) of a native sequence PRO1313 cDNA, wherein SEQ ID NO:27 is a clone designated herein as "DNAC4966-1575".

Figure 28 shows the amino acid sequence (SEQ ID NO:28) derived from the coding sequence of SEQ ID NO:27 shown in Figure 27.

Figure 29 shows a nucleotide sequence (SEQ ID NO:29) of a native sequence PRO1338 cDNA, wherein SEQ ID NO:29 is a clone designated herein as "DNA66667".

Figure 30 shows the amino acid sequence (SEQ ID NO:30) derived from the coding sequence of SEQ ID NO:29 shown in Figure 29.

Figure 31 shows a nucleotide sequence (SEQ ID NO:31) of a native sequence PRO1375 cDNA, wherein SEQ ID NO:31 is a clone designated herein as "DNA67004-1614".

Figure 32 shows the amino acid sequence (SEQ ID NO:32) derived from the coding sequence of SEQ ID NO:31 shown in Figure 31.

Figure 33 shows a nucleotide sequence (SEQ ID NO:33) of a native sequence PRO1410 cDNA, wherein SEQ ID NO:33 is a clone designated herein as "DNA68874-1622".

Figure 34 shows the amino acid sequence (SEQ ID NO:34) derived from the coding sequence of SEQ ID NO:33 shown in Figure 33.

Figure 35 shows a nucleotide sequence (SEQ ID NO:35) of a native sequence PRO 1488 cDNA, wherein SEQ ID NO:35 is a clone designated herein as "DNA73736-1657".

Figure 36 shows the amino acid sequence (SEQ ID NO:36) derived from the coding sequence of SEQ ID NO:35 shown in Figure 35.

Figure 37 shows a nucleotide sequence (SEQ ID NO:37) of a native sequence PRO3438 cDNA, wherein SEQ ID NO:37 is a clone designated herein as "DNA82364-2538".

Figure 38 shows the amino acid sequence (SEQ ID NO:38) derived from the coding sequence of SEQ ID NO:37 shown in Figure 37.

Figure 39 shows a nucleotide sequence (SEQ ID NO:39) of a native sequence PRO4302 cDNA. wherein SEQ ID NO:39 is a clone designated herein as "DNA92218-2554".

Figure 40 shows the amino acid sequence (SEQ ID NO:40) derived from the coding sequence of SEQ ID NO:39 shown in Figure 39.

Figure 41 shows a nucleotide sequence (SEQ ID NO:41) of a native sequence PRO4400 cDNA. wherein SEQ ID NO:41 is a clone designated herein as "DNA87974-2609".

Figure 42 shows the amino acid sequence (SEQ ID NO:42) derived from the coding sequence of SEQ ID NO:41 shown in Figure 41.

Figure 43 shows a nucleotide sequence (SEQ ID NO:43) of a native sequence PRO5725 cDNA, wherein SEQ ID NO:43 is a clone designated herein as "DNA92265-2669".

Figure 44 shows the amino acid sequence (SEQ ID NO:44) derived from the coding sequence of SEQ ID NO:43 shown in Figure 43.

Figure 45 shows a nucleotide sequence (SEQ ID NO:45) of a native sequence PRO183 cDNA, wherein SEQ ID NO:45 is a clone designated herein as "DNA28498".

Figure 46 shows the amino acid sequence (SEQ ID NO:46) derived from the coding sequence of SEQ ID NO:45 shown in Figure 45.

Figure 47 shows a nucleotide sequence (SEQ ID NO:47) of a native sequence PRO202 cDNA, wherein SEQ ID NO:47 is a clone designated herein as "DNA30869".

Figure 48 shows the amino acid sequence (SEQ ID NO:48) derived from the coding sequence of SEQ ID NO:47 shown in Figure 47.

Figure 49 shows a nucleotide sequence (SEQ ID NO:49) of a native sequence PRO542 cDNA, wherein SEQ ID NO:49 is a clone designated herein as "DNA56505".

Figure 50 shows the amino acid sequence (SEQ ID NO:50) derived from the coding sequence of SEQ ID NO:49 shown in Figure 49.

Figure 51 shows a nucleotide sequence (SEQ ID NO:51) of a native sequence PRO861 cDNA, wherein SEQ ID NO:51 is a clone designated herein as "DNA50798".

Figure 52 shows the amino acid sequence (SEQ ID NO:52) derived from the coding sequence of SEQ ID NO:51 shown in Figure 51.

Figure 53 shows a nucleotide sequence (SEQ ID NO:53) of a native sequence PRO1096 cDNA, wherein SEQ ID NO:53 is a clone designated herein as "DNA61870".

Figure 54 shows the amino acid sequence (SEQ ID NO:54) derived from the coding sequence of SEQ ID NO:53 shown in Figure 53.

Figure 55 shows a nucleotide sequence (SEQ ID NO:55) of a native sequence PRO3562 cDNA, wherein SEQ ID NO:55 is a clone designated herein as "DNA96791 ".

Figure 56 shows the amino acid sequence (SEQ ID NO:56) derived from the coding sequence of SEQ ID NO:55 shown in Figure 55.

## DETAILED DESCRIPTION OF THE INVENTION

The terms "PRO polypeptide", and "PRO" as used herein and when immediately followed by a numerical designation refer to various polypeptides, wherein the complete designation (*i.e.,* PRO/number) refers to specific polypeptide sequences as described herein. The terms "PRO/number polypeptide" and "PRO/number" wherein the term "number" is provided as an actual numerical designation as used herein encompass native sequence polypeptides and polypeptide variants (which are further defined herein). The PRO polypeptides described herein may be isolated from a variety of sources, such as from human tissue types or from another source, or prepared by recombinant or synthetic methods. A "native sequence PRO polypeptide" comprises a polypeptide having the same amino acid sequence as the corresponding PRO polypeptide derived from nature. Such native sequence PRO polypeptides can be isolated from nature or can be produced by recombinant or synthetic means. The term "native sequence PRO polypeptide" specifically encompasses naturally-occurring truncated or secreted forms of the specific PRO polypeptide (e.g., an extracellular domain sequence), naturally-occurring variant forms (e.g., alternatively spliced forms) and naturally-occurring allelic variants of the polypeptide. In various embodiments of the invention, the native sequence PRO polypeptides disclosed herein are mature or full-length native sequence polypeptides comprising the full-length amino acid sequences shown in the accompanying figures. Start and stop codons are shown in bold font and underlined in the figures. However, while the PRO polypeptide disclosed in the accompanying figures are shown to begin with methionine residues designated herein as amino acid position 1 in the figures, it is conceivable and possible that other methionine residues located either upstream or downstream from the amino acid position 1 in the figures may be employed as the starting amino acid residue for the PRO polypeptides.

The PRO polypeptide "extracellular domain" or "ECD" refers to a form of the PRO polypeptide which is essentially free of the transmembrane and cytoplasmic domains. Ordinarily, a PRO polypeptide ECD will have less than 1% of such transmembrane and/or cytoplasmic domains and preferably, will have less than 0.5% of such domains. It will be understood that any transmembrane domains identified for the PRO polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a PRO polypeptide may contain from about 5 or fewer amino acids on either side of the transmembrane domain/extracellular domain boundary as identified in the Examples or specification and such polypeptides, with or without the associated signal peptide, and nucleic acid encoding them, are comtemplated by the present invention.

The approximate location of the "signal peptides" of the various PRO polypeptides disclosed herein are shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (e.g., Nielsen et al., Prot. Eng., 10:1-6 (1997) and von Heinje et al., Nucl. Acids Res., 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"PRO polypeptide variant" means an active PRO polypeptide as defined above or below having at least about 80% amino acid sequence identity with a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Such PRO polypeptide variants include, for instance, PRO polypeptides wherein one or more amino acid residues are added, or deleted, at the N- or C-terminus of the full-length native amino acid sequence. Ordinarily, a PRO polypeptide variant will have at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity. alternatively at least about 90% amino acid sequence identity, alternatively at least about 91 % amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other specifically defined fragment of a full-length PRO polypeptide sequence as disclosed herein. Ordinarily, PRO variant polypeptides are at least about 10 amino acids in length, alternatively at least about 20 amino acids in length, alternatively at least about 30 amino acids in length, alternatively at least about 40 amino acids in length, alternatively at least about 50 amino acids in length, alternatively at least about 60 amino acids in length, alternatively at least about 70 amino acids in length, alternatively at least about 80 amino acids in length, alternatively at least about 90 amino acids in length, alternatively at least about 100 amino acids in length, alternatively at least about 150 amino acids in length, alternatively at least about 200 amino acids in length, alternatively at least about 300 amino acids in length, or more.

"Percent (%) amino acid sequence identity" with respect to the PRO polypeptide sequences identified herein is defined as the percentage of amino acid residues in a candidate sequence that are identical with the amino acid residues in a PRO sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared.

For purposes herein, however, % amino acid sequence identity values are obtained as described below by using the sequence comparison computer program ALIGN-2. wherein the complete source code for the ALIGN-2 program is provided in Table 1. The ALIGN-2 sequence comparison computer program was authored by Genentech, Inc., and the source code shown in Table I has been filed with user documentation in the U.S. Copyright Office. Washington D.C., 20559, where it is registered under U.S. Copyright Registration No. TXU510087. The ALIGN-2 program is publicly available through Genentech, Inc., South San Francisco, California or may be compiled from the source code provided in Table 1. The ALIGN-2 program should be compiled for use on a UNIX operating system, preferably digital UNIX V4.0D. All sequence comparison parameters are set by the ALIGN-2 program and do not vary.

For purposes herein, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program ALIGN-2 in that program's alignment of A and B,. and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations. Tables 2-3 demonstrate how to calculate the % amino acid sequence identity of the amino acid sequence designated "Comparison Protein" to the amino acid sequence designated "PRO".

Unless specifically stated otherwise, all % amino acid sequence identity values used herein are obtained as described above using the ALIGN-2 sequence comparison computer program. However, % amino acid sequence identity may also be determined using the sequence comparison program NCBI-BLAST2 (Altschul et al., Nucleic Acids Res., 25: 3389-3402 (1997)). The NCBI-BLAST2 sequence comparison program may be downloaded from http://www.ncbi.nlm.nih.gov, or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

In addition, % amino acid sequence identity may also be determined using the WU-BLAST-2 computer program (Altschul et al., Methods in Enzymology, 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. For purposes herein, a % amino acid sequence identity value is determined by dividing (a) the number of matching identical amino acids residues between the amino acid sequence of the PRO polypeptide of interest having a sequence derived from the native PRO polypeptide and the comparison amino acid sequence of interest (i.e., the sequence against which the PRO polypeptide of interest is being compared which may be a PRO variant polypeptide) as determined by WU-BLAST-2 by (b) the total number of amino acid residues of the PRO polypeptide of interest. For example, in the statement "a polypeptide comprising an amino acid sequence A which has or having at least 80% amino acid sequence identity to the amino acid sequence B", the amino acid sequence A is the comparison amino acid sequence of interest and the amino acid sequence B is the amino acid sequence of the PRO polypeptide of interest.

"PRO variant polynucleotide" or "PRO variant nucleic acid sequence" means a nucleic acid molecule which encodes an active PRO polypeptide as defined below and which has at least about 80% nucleic acid sequence identity with a nucleic acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal peptide, as disclosed herein or any other fragment of a fulf-length PRO polypeptide sequence as disclosed herein. Ordinarily, a PRO variant polynucleotide will have at least about 80% nucleic acid sequence identity, alternatively at least about 81 % nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91 % nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity with a nucleic acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal sequence, as disclosed herein or any other fragment of a full-length PRO polypeptide sequence as disclosed herein. Variants do not encompass the native nucleotide sequence.

Ordinarily, PRO variant polynucleotides are at least about 30 nucleotides in length, alternatively at least about 60 nucleotides in length, alternatively at least about 90 nucleotides in length, alternatively at least about 120 nucleotides in length, alternatively at least about 150 nucleotides in length, alternatively at least about 180 nucleotides in length, alternatively at least about 210 nucleotides in length, alternatively at least about 240 nucleotides in length, alternatively at least about 270 nucleotides in length, alternatively at least about 300 nucleotides in length, alternatively at least about 450 nucleotides in length, alternatively at least about 600 nucleotides in length, alternatively at least about 900 nucleotides in length, or more.

"Percent (%) nucleic acid sequence identity" with respect to the PRO polypeptide-encoding nucleic acid sequences identified herein is defined as the percentage of nucleotides in a candidate sequence that are identical with the nucleotides in a PRO polypeptide-encoding nucleic acid sequence, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however,% nucleic acid sequence identity values are obtained as described below by using the sequence comparison computer program ALIGN-2, wherein the complete source code for the ALIGN-2 program is provided in Table 1. The ALIGN-2 sequence comparison computer program was authored by Genentech, Inc., and the source code shown in Table I has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1. The ALIGN-2 program should be compiled for use on a UNIX operating system, preferably digital UNIX V4.0D. All sequence comparison parameters are set by the ALIGN-2 program and do not vary.

For purposes herein, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

$$100 \text{ times the fraction } W/Z$$

where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of % nucleic acid sequence identity calculations, Tables 4-5 demonstrate how to calculate the % nucleic acid sequence identity of the nucleic acid sequence designated "Comparison DNA" to the nucleic acid sequence designated "PRO-DNA".

Unless specifically stated otherwise, all % nucleic acid sequence identity values used herein are obtained as described above using the ALIGN-2 sequence comparison computer program. However, % nucleic acid sequence identity may also be determined using the sequence comparison program NCBI-BLAST2 (Altschul et al., Nucleic Acids Res., 25:

3389-3402 (1997)). The NCBI-BLAST2 sequence comparison program may be downloaded from http://www.ncbi.nlm.nih.gov, or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:

100 times the fraction W/Z

where W is the number of nucleotides scored as identical matches by the sequence alignment program NCBI-BLAST2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

In addition, % nucleic acid sequence identity values may also be generated using the WU-BLAST-2 computer program (Altschul et al., Methods in Enzymology, 266:460-480 (1996)). Most of the WU-BLAST-2 search parameters are set to the default values. Those not set to default values, *i.e.,* the adjustable parameters, are set with the following values: overlap span = 1, overlap fraction = 0.125, word threshold (T) = 11, and scoring matrix = BLOSUM62. For purposes herein, a % nucleic acid sequence identity value is determined by dividing (a) the number of matching identical nucleotides between the nucleic acid sequence of the PRO polypeptide-encoding nucleic acid molecule of interest having a sequence derived from the native sequence PRO polypeptide-encoding nucleic acid and the comparison nucleic acid molecule of interest (i.e., the sequence against which the PRO polypeptide-encoding nucleic acid molecule of interest is being compared which may be a variant PRO polynucleotide) as determined by WU-BLAST-2 by (b) the total number of nucleotides of the PRO polypeptide-encoding nucleic acid molecule of interest. For example, in the statement "an isolated nucleic acid molecule comprising a nucleic acid sequence A which has or having at least 80% nucleic acid sequence identity to the nucleic acid sequence B", the nucleic acid sequence A is the comparison nucleic acid molecule of interest and the nucleic acid sequence B is the nucleic acid sequence of the PRO polypeptide-encoding nucleic acid molecule of interest.

In other embodiments, PRO variant polynucleotides are nucleic acid molecules that encode an active PRO polypeptide and which are capable of hybridizing, preferably under stringent hybridization and wash conditions, to nucleotide sequences encoding the full-length PRO polypeptide shown in the accompanying figures herein. PRO variant polypeptides may be those that are encoded by a PRO variant polynucleotide.

The term "positives", in the context of the amino acid sequence identity comparisons performed as described above, includes amino acid residues in the sequences compared that are not only identical, but also those that have similar properties. Amino acid residues that score a positive value to an amino acid residue of interest are those that are either identical to the amino acid residue of interest or are a preferred substitution (as defined in Table 6 below) of the amino acid residue of interest.

For purposes herein, the % value of positives of a given amino acid sequence A to, with, or against a given amino acid sequence B (which can alternatively be phrased as a given amino acid sequence A that has or comprises a certain % positives to, with, or against a given amino acid sequence B) is calculated as follows:

100 times the fraction X/Y

where X is the number of amino acid residues scoring a positive value by the sequence alignment program ALIGN-2 in that program's alignment of A and B, and where Y is the total number of amino acid residues in B. It will be appreciated that where the length of amino acid sequence A is not equal to the length of amino acid sequence B, the % positives of A to B will not equal the % positives of B to A.

"Isolated", when used to describe the various polypeptides disclosed herein, means a polypeptide that has been identified and separated and/or recovered from a component of its natural environment. Preferably, the isolated polypeptide is free of association with all components with which it is naturally associated. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptides includes polypeptides *in situ* within recombinant cells, since at least one component of the PRO polypeptide natural environment will not be present. Ordinarily, however, isolated polypeptides will be prepared by at least one purification step.

An "isolated" nucleic acid molecule encoding a PRO polypeptide or an "isolated" nucleic acid molecule encoding an anti-PRO antibody is a nucleic acid molecule that is identified and separated from at least one contaminant nucleic acid molecule with which it is ordinarily associated in the natural source of the PRO-encoding nucleic acid or the natural source of the anti-PRO-encoding nucleic acid. Preferably, the isolated nucleic acid is free of association with all components with which it is naturally associated. An isolated PRO-encoding nucleic acid molecule or an isolated anti-PRO-encoding nucleic acid molecule is other than in the form or setting in which it is found in nature. Isolated nucleic acid molecules therefore are distinguished from the PRO-encoding nucleic acid molecule or from the anti-PRO-encoding nucleic acid molecule as it exists in natural cells. However, an isolated nucleic acid molecule encoding a PRO polypeptide or an isolated nucleic acid molecule encoding an anti-PRO antibody includes PRO-nucleic acid molecules or anti-PRO-nucleic acid molecules contained in cells that ordinarily express PRO polypeptides or anti-PRO antibodies where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a PRO polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers, for example, single anti-PRO monoclonal antibodies (including agonist antibodies), anti-PRO antibody compositions with polyepitopic specificity, single chain anti-PRO antibodies, and fragments of anti-PRO antibodies (see below). The term "monoclonal antibody" as used herein refers to an antibody obtained from a population of substantially homogeneous antibodies, i.e., the individual antibodies comprising the population are identical except for possible naturally-occurring mutations that may be present in minor amounts.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature that can be used. As a result, it follows that higher relative temperatures would tend to make the reaction conditions more stringent, while lower temperatures less so. For additional details and explanation of stringency of hybridization reactions, *see*, Ausubel et al., Current Protocols in Molecular Biology (Wiley Interscience Publishers, 1995).

"Stringent conditions" or "high-stringency conditions", as defined herein, may be identified by those that: (1) employ low ionic strength and high temperature for washing, for example, 0.015 M sodium chloride/0.0015 M sodium citrate/0.1 % sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml 0.1 % SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55 °C.

"Moderately-stringent conditions" may be identified as described by Sambrook et al., Molecular Cloning: A Laboratory Manual (New York: Cold Spring Harbor Press, 1989), and include the use of washing solution and hybridization conditions (*e.g.,* temperature, ionic strength, and % SDS) less stringent than those described above. An example of moderately stringent conditions is overnight incubation at 37°C in a solution comprising: 20% fonnamide,5 x SSC (150 mM NaCl, 15 mM trisodium citrate). 50 mM sodium phosphate (pH 7.6). 5 x Denhardt's solution.109c dextran sulfate, and 20 mg/ml denatured sheared salmon sperm DNA. followed by washing the filters in I x SSC at about 37°-50°C. The skilled artisan will recognize how to adjust the temperature, ionic strength, etc. as necessary to accommodate factors such as probe length and the like.

The term "epitope tagged" when used herein refers to a chimeric polypeptide comprising a PRO polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with otherepitopes. Suitable tag polypeptides generally have at least six amino acid residues and usually between about 8 and 50 amino acid residues (preferably, between about 10 and 20 amino acid residues).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1,IgG-2. IgG-3, or IgG-4 subtypes, IgA (including IgA-1 and IgA-2), IgE, IgD or IgM.

"Active" or "activity" for the purposes herein refers to form(s) of PRO polypeptides which retain a biological and/or an immunological activity of native or naturally-occurring PRO polypeptides, wherein "biological" activity refers to a biological function (either inhibitory or stimulatory) caused by a native or naturally-occurring PRO polypeptide other than the ability to induce the production of an antibody against an antigenic epitope possessed by a native or naturally-occurring PRO polypeptide and an "immunological" activity refers to the ability to induce the production of an antibody against an antigenic epitope possessed by a native or naturally-occurring PRO polypeptide.

"Biological activity" in the context of an antibody or another agonist that can be identified by the screening assays disclosed herein (e.g., an organic or inorganic small molecule, peptide, etc.) is used to refer to the ability of such molecules to invoke one or more of the effects listed herein in connection with the definition of a "therapeutically effective amount." In a specific embodiment, "biological activity" is the ability to inhibit neoplastic cell growth or proliferation. A preferred biological activity is inhibition, including slowing or complete stopping, of the growth of a target tumor (e.g., cancer) cell. Another preferred biological activity is cytotoxic activity resulting in the death of the target tumor (e.g., cancer) cell. Yet another preferred biological activity is the induction of apoptosis of a target tumor (e.g., cancer) cell.

The phrase "immunological activity" means immunological cross-reactivity with at least one epitope of a PRO polypeptide. "Immunological cross-reactivity" as used herein means that the candidate polypeptide is capable of competitively inhibiting the qualitative biological activity of a PRO polypeptide having this activity with polyclonal antisera raised against the known active PRO polypeptide. Such antisera are prepared in conventional fashion by injecting goats or rabbits, for example, subcutaneously with the known active analogue in complete Freund's adjuvant, followed by booster intraperitoneal or subcutaneous injection in incomplete Freunds. The immunological cross-reactivity preferably is "specific", which means that the binding affinity of the immunologically cross-reactive molecule (e.g., antibody) identified, to the corresponding PRO polypeptide is significantly higher (preferably at least about 2-times, more preferably at least about 4-times, even more preferably at least about 6-times, most preferably at least about 8-times higher) than the binding affinity of that molecule to any other known native polypeptide.

"Tumor", as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers include breast cancer, prostate cancer, colon cancer, squamous cell cancer, small-cell lung cancer, non-small cell lung cancer, ovarian cancer, cervical cancer, gastrointestinal cancer, pancreatic cancer, glioblastoma, liver cancer, bladder cancer, hepatoma, colorectal cancer, endometrial carcinoma, salivary gland carcinoma, kidney cancer, vulval cancer, thyroid cancer, hepatic carcinoma and various types of head and neck cancer.

"Treatment" is an intervention performed with the intention of preventing the development or altering the pathology of a disorder. Accordingly, "treatment" refers to both therapeutic treatment and prophylactic or preventative measures. Those in need of treatment include those already with the disorder as well as those in which the disorder is to be prevented. In tumor (e.g., cancer) treatment, a therapeutic agent may directly decrease the pathology of tumor cells, or render the tumor cells more susceptible to treatment by other therapeutic agents, *e.g.*, radiation and/or chemotherapy.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, etc.

An "effective amount" of a polypeptide disclosed herein or an agonist thereof, in reference to inhibition of neoplastic cell growth, is an amount capable of inhibiting, to some extent, the growth of target cells. The term includes an amount capable of invoking a growth inhibitory, cytostatic and/or cytotoxic effect and/or apoptosis of the target cells. An "effective amount" of a PRO polypeptide or an agonist thereof for purposes of inhibiting neoplastic cell growth may be determined empirically and in a routine manner.

A "therapeutically effective amount", in reference to the treatment of tumor, refers to an amount capable of invoking one or more of the following effects: (1) inhibition, to some extent, of tumor growth, including, slowing down and complete growth arrest; (2) reduction in the number of tumor cells; (3) reduction in tumor size; (4) inhibition (*i.e.*, reduction, slowing down or complete stopping) of tumor cell infiltration into peripheral organs; (5) inhibition (i.e., reduction, slowing down or complete stopping) of metastasis; (6) enhancement of anti-tumor immune response, which may, but does not have to, result in the regression or rejection of the tumor; and/or (7) relief, to some extent, of one or more symptoms associated with the disorder. A "therapeutically effective amount" of a PRO polypeptide or an agonist thereof for purposes of treatment of tumor may be determined empirically and in a routine manner.

A "growth inhibitory amount" of a PRO polypeptide or an agonist thereof is an amount capable of inhibiting the growth of a cell, especially tumor, *e.g.,* cancer cell, either *in vitro* or *in vivo.* A "growth inhibitory amount" of a PRO polypeptide or an agonist thereof for purposes of inhibiting neoplastic cell growth may be determined empirically and in a routine manner.

A "cytotoxic amount" of a PRO polypeptide or an agonist thereof is an amount capable of causing the destruction of a cell, especially tumor, *e.g.*, cancer cell, either *in vitro* or *in vivo.* A "cytotoxic amount" of a PRO polypeptide or an agonist thereof for purposes of inhibiting neoplastic cell growth may be determined empirically and in a routine manner.

The term "cytotoxic agent" as used herein refers to a substance that inhibits or prevents the function of cells and/or causes destruction of cells. The term is intended to include radioactive isotopes *(e.g.,* $I^{131}$, $I^{125}$, $Y^{90}$ and $Re^{186}$), chemotherapeutic agents, and toxins such as enzymatically active toxins of bacterial, fungal, plant or animal origin, or fragments thereof.

A "chemotherapeutic agent" is a chemical compound useful in the treatment of tumor, *e.g.*, cancer. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, *e.g.*, paclitaxel (Taxol, Bristol-Myers Squibb Oncology, Princeton, NJ), and doxetaxel (Taxotere, Rhône-Poulenc Rorer, Antony, Rnace), toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins (*see*, U.S. Patent No. 4,675,187), melphalan and other related nitrogen mustards. Also included in this definition are hormonal agents that act to regulate or inhibit hormone action on tumors such as tamoxifen and onapristone.

A "growth inhibitory agent" when used herein refers to a compound or composition which inhibits growth of a cell, especially tumor, *e.g.*, cancer cell, either *in vitro* or *in vivo.* Thus, the growth inhibitory agent is one which significantly reduces the percentage of the target cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al., (WB Saunders: Philadelphia, 1995), especially p. 13.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine: insulin; proinsulin; relaxin: prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-$\alpha$ and -$\beta$; mullerian-inhibiting substance; mouse gonadotropin-associated peptide; inhibin; activin; vascular endothelial growth factor; integrin; thrombopoietin (TPO); nerve growth factors such as NGF-$\beta$; platelet-growth factor; transforming growth factors (TGFs) such as TGF-$\alpha$ and TGF-$\beta$: insulin-like growth factor-I and -II; erythropoietin (EPO); osteoinductive factors; interferons such as interferon-$\alpha$, -$\beta$, and -$\gamma$; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1$\alpha$, IL-2, IL-3, IL-4, IL-5, IL-6, IL-7, IL-8, IL-9, IL-11, IL-12; a tumor necrosis factor such as TNF-$\alpha$ or TNF-$\beta$; and other polypeptide factors including LIF and kit ligand (KL). As used herein, the term cytokine includes proteins from natural sources or from recombinant cell culture and biologically active equivalents of the native sequence cytokines.

The term "prodrug" as used in this application refers to a precursor or derivative form of a pharmaceutically active substance that is less cytotoxic to tumor cells compared to the parent drug and is capable of being enzymatically activated or converted into the more active parent form. *See, e.g.,* Wilman, "Prodrugs in Cancer Chemotherapy", Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs of this invention include, but are not limited to, phosphate-containing prodrugs, thiophosphate-containing prodrugs, glycosylated prodrugs or optionally substituted phenylacetamide-containing prodrugs, 5-fluorocytosine and other 5-fluorouridine prodrugs which can be derivatized into a prodrug form for use in this invention include, but are not limited to, those chemotherapeutic agents described above.

The term "agonist" is used in the broadest sense and includes any molecule that mimics a biological activity of a native PRO polypeptide disclosed herein. Suitable agonist molecules specifically include agonist antibodies or antibody fragments, fragments or amino acid sequence variants of native PRO polypeptides, peptides, small organic molecules, etc. Methods for identifying agonists of a PRO polypeptide may comprise contacting a tumor cell with a candidate agonist molecule and measuring the inhibition of tumor cell growth.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of treatment refers to any animal classified as a mammal, including humans, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins: chelating agents such as EDTA; sugar alcohols such as mannitol or sorbitol; salt-forming counterions such as sodium; and/or nonionic surfactants such as TWEEN™, polyethylene glycol (PEG), and PLURONICST™.

"Native antibodies" and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain ($V_H$) followed by a number of constant domains. Each light chain has a variable domain at one end ($V_L$) and a constant domain at its other end; the constant domain of the light chain is aligned with the first constant domain of the heavy chain, and the light-chain variable domain is aligned with the variable domain of the heavy chain. Particular amino acid residues are believed to form an interface between the light- and heavy-chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework regions (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies *(see,* Kabat et al., NIH Publ. No.91-3242, Vol. I, pages 647-669 (1991)). The constant domains are not involved directly in binding an antibody to an antigen, but exhibit various effector functions, such as participation of the antibody in antibody-dependent cellular toxicity.

The term "hypervariable region" when used herein refers to the amino acid residues of an antibody which are responsible for antigen-binding. The hypervariable region comprises amino acid residues from a "complementarity determining region" or "CDR" *(i.e.,* residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institute of Health, Bethesda, MD. [1991]) and/or those residues from a "hypervariable loop" *(i.e.,* residues 26-32 (L1), 50-52 (L2) and 91-96 (L3) in the light chain variable domain and 26-32 (H1), 53-55 (H2) and 96-101 (H3) in the heavy chain variable domain; Clothia and Lesk, J. Mol. Biol., 196:901-917 [1987]). "Framework" or "FR" residues are those variable domain residues other than the hypervariable region residues as herein defined.

"Antibody fragments" comprise a portion of an intact antibody, preferably the antigen binding or variable region of the intact antibody. Examples of antibody fragments include Fab, Fab', $F(ab')_2$, and Fv fragments; diabodies; linear antibodies *(*Zapata et al., Protein Eng., 8(10): 1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an $F(ab')_2$ fragment that has two antigen-combining sites and is still capable of cross-linking antigen.

"Fv" is the minimum antibody fragment which contains a complete antigen-recognition and -binding site. This region consists of a dimerofone heavy- and one light-chain variable domain in tight, non-covalent association. It is in this configuration that the three CDRs of each variable domain interact to define an antigen-binding site on the surface of the $V_H$-$V_L$ dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CHI domain including one or more cysteines from the antibody hinge region. Fab'-SH is the designation herein for Fab' in which the cysteine residue(s) of the constant domains bear a free thiol group. $F(ab')_2$ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), *e.g.,* IgG1, IgG2, IgG3, IgG4, IgA, and IgA2.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 [1975], or may be made by recombinant DNA methods *(see, e.g.,* U.S. Patent No. 4,816,567). The "monoclonal antibodies" may also be isolated from phage antibody libraries using the techniques described in Clackson et al., Nature, 352:624-628 [1991] and Marks et al., J. Mol. Biol., 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 [1984]).

"Humanized" forms of non-human (*e.g*., murine) antibodies are chimeric immunoglobulins. immunoglobulin chains or fragments thereof (such as Fv, Fab, Fab', $F(ab')_2$ or other antigen-binding subsequences of antibodies) which contain minimal sequence derived from non-human immunoglobulin. For the most part, humanized antibodies are human immunoglobulins (recipient antibody) in which residues from a CDR of the recipient are replaced by residues from a CDR of a non-human species (donor antibody) such as mouse, rat or rabbit having the desired specificity, affinity, and capacity. In some instances, Fv FR residues of the human immunoglobulin are replaced by corresponding non-human residues. Furthermore, humanized antibodies may comprise residues which are found neither in the recipient antibody nor in the imported CDR or framework sequences. These modifications are made to further refine and maximize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, *see,* Jones et al., Nature, 321 :522-525 (1986); Reichmann et al., Nature, 332: 323-329 [1988]; and Presta, Curr. Op. Struct. Biol., 2:593-596 (1992). The humanized antibody includes a PRIMATIZED™ antibody wherein the antigen-binding region of the antibody is derived from an antibody produced by immunizing macaque monkeys with the antigen of interest.

"Single-chain Fv" or "sFv" antibody fragments comprise the $V_H$ and $V_L$ domains of antibody, wherein these domains are present in a single polypeptide chain. Preferably, the Fv polypeptide further comprises a polypeptide linker between the $V_H$ and $V_L$ domains which enables the sFv to form the desired structure for antigen binding. For a review of sFv, *see,* Pluckthun in The Pharmacology of Monoclonal Antibodies, Vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain ($V_H$) connected to a light-chain variable domain ($V_L$) in the same polypeptide chain ($V_H$ - $V_L$). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90: 6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

The word "label" when used herein refers to a detectable compound or composition which is conjugated directly or indirectly to the antibody so as to generate a "labeled" antibody. The label may be detectable by itself (e.g., radioisotope labels or fluorescent labels) or, in the case of an enzymatic label, may catalyze chemical alteration of a substrate compound or composition which is detectable. The label may also be a non-detectable entity such as a toxin.

By "solid phase" is meant a non-aqueous matrix to which the antibody of the present invention can adhere. Examples of solid phases encompassed herein include those formed partially or entirely of glass (*e.g.*, controlled pore glass), polysaccharides *(e.g.,* agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PRO polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

As shown below, Table 1 provides the complete source code for the ALIGN-2 sequence comparison computer program. This source code may be routinely compiled for use on a UNIX operating system to provide the ALIGN-2 sequence comparison computer program.

In addition, Tables 2-5 show hypothetical exemplifications for using the below described method to determine % amino acid sequence identity (Tables 2-3) and % nucleic acid sequence identity (Tables 4-5) using the ALIGN-2 sequence comparison computer program, wherein "PRO" represents the amino acid sequence of a hypothetical PRO polypeptide of interest, "Comparison Protein" represents the amino acid sequence of a polypeptide against which the "PRO" polypeptide of interest is being compared, "PRO-DNA" represents a hypothetical PRO-encoding nucleic acid sequence of interest, "Comparison DNA" represents the nucleotide sequence of a nucleic acid molecule against which the "PRO-DNA" nucleic acid molecule of interest is being compared, "X", "Y", and "Z" each represent different hypothetical amino acid residues and "N", "L" and "V" each represent different hypothetical nucleotides.

## Table 1

```
/*
 *
 * C-C increased from 12 to 15
 * Z is average of EQ
/ * B is average of ND
 * match with stop is _M; stop-stop = 0; J (joker) match = 0
 */
#define_M       -8        /* value of a match with a stop */

int     _day[26][26] = {
/*      A B C D E F G H I J K L M N O P Q R S T U V W X Y Z */
/* A */  { 2, 0,-2, 0, 0,-4, 1,-1,-1, 0,-1,-2,-1, 0,_M, 1, 0,-2, 1, 1, 0, 0,-6, 0,-3, 0},
/* B */  { 0, 3,-4, 3, 2,-5, 0, 1,-2, 0, 0,-3,-2, 2,_M,-1, 1, 0, 0, 0, 0,-2,-5, 0,-3, 1},
/* C */  {-2,-4,15,-5,-5,-4,-3,-3,-2, 0,-5,-6,-5,-4,_M,-3,-5,-4, 0,-2, 0,-2,-8, 0, 0,-5},
/* D */  { 0, 3,-5, 4, 3,-6, 1, 1,-2, 0, 0,-4,-3, 2,_M,-1, 2,-1, 0, 0, 0,-2,-7, 0,-4, 2},
/* E */  { 0, 2,-5, 3, 4,-5, 0, 1,-2, 0, 0,-3,-2, 1,_M,-1, 2,-1, 0, 0, 0,-2,-7, 0,-4, 3},
/* F */  {-4,-5,-4,-6,-5, 9,-5,-2, 1, 0,-5, 2, 0,-4,_M,-5,-5,-4,-3,-3, 0,-1, 0, 0, 7,-5},
/* G */  { 1, 0,-3, 1, 0,-5, 5,-2,-3, 0,-2,-4,-3, 0,_M,-1,-1,-3, 1, 0, 0,-1,-7, 0,-5, 0},
/* H */  {-1, 1,-3, 1, 1,-2,-2, 6,-2, 0, 0,-2,-2, 2,_M, 0, 3, 2,-1,-1, 0,-2,-3, 0, 0, 2},
/* I */  {-1,-2,-2,-2,-2, 1,-3,-2, 5, 0,-2, 2, 2,-2,_M,-2,-2,-2,-1, 0, 0, 4,-5, 0,-1,-2},
/* J */  { 0, 0, 0, 0, 0, 0, 0, 0, 0, 0, 0, 0, 0, 0,_M, 0, 0, 0, 0, 0, 0, 0, 0, 0, 0, 0},
/* K */  {-1, 0,-5, 0, 0,-5,-2, 0,-2, 0, 5,-3, 0, 1,_M,-1, 1, 3, 0, 0, 0,-2,-3, 0,-4, 0},
/* L */  {-2,-3,-6,-4,-3, 2,-4,-2, 2, 0,-3, 6, 4,-3,_M,-3,-2,-3,-3,-1, 0, 2,-2, 0,-1,-2},
/* M */  {-1,-2,-5,-3,-2, 0,-3,-2, 2, 0, 0, 4, 6,-2,_M,-2,-1, 0,-2,-1, 0, 2,-4, 0,-2,-1},
/* N */  { 0, 2,-4, 2, 1,-4, 0, 2,-2, 0, 1,-3,-2, 2,_M,-1, 1, 0, 1, 0, 0,-2,-4, 0,-2, 1},
/* O */  {_M,_M,_M,_M,_M,_M,_M,_M,_M,_M,_M,_M,_M,_M, 0,_M,_M,_M,_M,_M,_M,_M,_M,_M,_M,_M},
/* P */  { 1,-1,-3,-1,-1,-5,-1, 0,-2, 0,-1,-3,-2,-1,_M, 6, 0, 0, 1, 0, 0,-1,-6, 0,-5, 0},
/* Q */  { 0, 1,-5, 2, 2,-5,-1, 3,-2, 0, 1,-2,-1, 1,_M, 0, 4, 1,-1,-1, 0,-2,-5, 0,-4, 3},
/* R */  {-2, 0,-4,-1,-1,-4,-3, 2,-2, 0, 3,-3, 0, 0,_M, 0, 1, 6, 0,-1, 0,-2, 2, 0,-4, 0},
/* S */  { 1, 0, 0, 0, 0,-3, 1,-1,-1, 0, 0,-3,-2, 1,_M, 1,-1, 0, 2, 1, 0,-1,-2, 0,-3, 0},
/* T */  { 1, 0,-2, 0, 0,-3, 0,-1, 0, 0, 0,-1,-1, 0,_M, 0,-1,-1, 1, 3, 0, 0,-5, 0,-3, 0},
/* U */  { 0, 0, 0, 0, 0, 0, 0, 0, 0, 0, 0, 0, 0, 0,_M, 0, 0, 0, 0, 0, 0, 0, 0, 0, 0, 0},
/* V */  { 0,-2,-2,-2,-2,-1,-1,-2, 4, 0,-2, 2, 2,-2,_M,-1,-2,-2,-1, 0, 0, 4,-6, 0,-2,-2},
/* W */  {-6,-5,-8,-7,-7, 0,-7,-3,-5, 0,-3,-2,-4,-4,_M,-6,-5, 2,-2,-5, 0,-6,17, 0, 0,-6},
/* X */  { 0, 0, 0, 0, 0, 0, 0, 0, 0, 0, 0, 0, 0, 0,_M, 0, 0, 0, 0, 0, 0, 0, 0, 0, 0, 0},
/* Y */  {-3,-3, 0,-4,-4, 7,-5, 0,-1, 0,-4,-1,-2,-2,_M,-5,-4,-4,-3,-3, 0,-2, 0, 0,10,-4},
/* Z */  { 0, 1,-5, 2, 3,-5, 0, 2,-2, 0, 0,-2,-1, 1,_M, 0, 3, 0, 0, 0, 0,-2,-6, 0,-4, 4}
};
```

Table 1 (cont')

```
/*
 */
#include <stdio.h>
#include <ctype.h>

#define MAXJMP      16      /* max jumps in a diag */
#define MAXGAP      24      /* don't continue to penalize gaps larger than this */
#define JMPS        1024    /* max jmps in an path */
#define MX          4       /* save if there's at least MX-1 bases since last jmp */

#define DMAT        3       /* value of matching bases */
#define DMIS        0       /* penalty for mismatched bases */
#define DINS0       8       /* penalty for a gap */
#define DINS1       1       /* penalty per base */
#define PINS0       8       /* penalty for a gap */
#define PINS1       4       /* penalty per residue */

struct jmp {
        short           n[MAXJMP];      /* size of jmp (neg for dely) */
        unsigned short  x[MAXJMP];      /* base no. of jmp in seq x */
};                                      /* limits seq to 2^16 -1 */

struct diag {
        int             score;          /* score at last jmp */
        long            offset;         /* offset of prev block */
        short           ijmp;           /* current jmp index */
        struct jmp      jp;             /* list of jmps */
};

struct path {
        int     spc;                    /* number of leading spaces */
        short   n[JMPS];/* size of jmp (gap) */
        int     x[JMPS];/* loc of jmp (last elem before gap) */
};

char            *ofile;                 /* output file name */
char            *namex[2];              /* seq names: getseqs() */
char            *prog;                  /* prog name for err msgs */
char            *seqx[2];               /* seqs: getseqs() */
int             dmax;                   /* best diag: nw() */
int             dmax0;                  /* final diag */
int             dna;                    /* set if dna: main() */
int             endgaps;                /* set if penalizing end gaps */
int             gapx, gapy;             /* total gaps in seqs */
int             len0, len1;             /* seq lens */
int             ngapx, ngapy;           /* total size of gaps */
int             smax;                   /* max score: nw() */
int             *xbm;                   /* bitmap for matching */
long            offset;                 /* current offset in jmp file */
struct  diag    *dx;                    /* holds diagonals */
struct  path    pp[2];                  /* holds path for seqs */

char            *calloc(), *malloc(), *index(), *strcpy();
char            *getseq(), *g_calloc();
```

## Table 1 (cont')

```
/* Needleman-Wunsch alignment program
 *
 * usage: progs file1 file2
 *   where file1 and file2 are two dna or two protein sequences.
 *   The sequences can be in upper- or lower-case an may contain ambiguity
 *   Any lines beginning with ';', '>' or '<' are ignored
 *   Max file length is 65535 (limited by unsigned short x in the jmp struct)
 *   A sequence with 1/3 or more of its elements ACGTU is assumed to be DNA
 *   Output is in the file "align.out"
 *
 * The program may create a tmp file in /tmp to hold info about traceback.
 * Original version developed under BSD 4.3 on a vax 8650
 */
#include "nw.h"
#include "day.h"

static  _dbval[26] = {
        1,14,2,13,0,0,4,11,0,0,12,0,3,15,0,0,0,5,6,8,8,7,9,0,10,0
};

static  _pbval[26] = {
        1, 2|(1<<('D'-'A'))|(1<<('N'-'A')), 4, 8, 16, 32, 64,
        128, 256, 0xFFFFFFF, 1<<10, 1<<11, 1<<12, 1<<13, 1<<14,
        1<<15, 1<<16, 1<<17, 1<<18, 1<<19, 1<<20, 1<<21, 1<<22,
        1<<23, 1<<24, 1<<25|(1<<('E'-'A'))|(1<<('Q'-'A'))
};

main(ac, av)                                                            main
        int     ac;
        char    *av[];
{
        prog = av[0];
        if (ac != 3) {
                fprintf(stderr,"usage: %s file1 file2\n", prog);
                fprintf(stderr,"where file1 and file2 are two dna or two protein sequences.\n");
                fprintf(stderr,"The sequences can be in upper- or lower-case\n");
                fprintf(stderr,"Any lines beginning with ';' or '<' are ignored\n");
                fprintf(stderr,"Output is in the file \"align.out\"\n");
                exit(1);
        }
        namex[0] = av[1];
        namex[1] = av[2];
        seqx[0] = getseq(namex[0], &len0);
        seqx[1] = getseq(namex[1], &len1);
        xbm = (dna)? _dbval : _pbval;

        endgaps = 0;                    /* 1 to penalize endgaps */
        ofile = "align.out";            /* output file */

        nw();           /* fill in the matrix, get the possible jmps */
        readjmps();     /* get the actual jmps */
        print();        /* print stats, alignment */

        cleanup(0);     /* unlink any tmp files */
}
```

## Table 1 (cont')

```
/* do the alignment, return best score: main()
 * dna: values in Fitch and Smith, PNAS, 80, 1382-1386, 1983
 * pro: PAM 250 values
 * When scores are equal, we prefer mismatches to any gap, prefer
 * a new gap to extending an ongoing gap, and prefer a gap in seqx
 * to a gap in seq y.
 */
nw()                                                                              nw
{
        char            *px, *py;           /* seqs and ptrs */
        int             *ndely, *dely;      /* keep track of dely */
        int             ndelx, delx;        /* keep track of delx */
        int             *tmp;               /* for swapping row0, row1 */
        int             mis;                /* score for each type */
        int             ins0, ins1;         /* insertion penalties */
        register        id;                 /* diagonal index */
        register        ij;                 /* jmp index */
        register        *col0, *col1;       /* score for curr, last row */
        register        xx, yy;             /* index into seqs */

        dx = (struct diag *)g_calloc("to get diags", len0+len1+1, sizeof(struct diag));

        ndely = (int *)g_calloc("to get ndely", len1+1, sizeof(int));
        dely = (int *)g_calloc("to get dely", len1+1, sizeof(int));
        col0 = (int *)g_calloc("to get col0", len1+1, sizeof(int));
        col1 = (int *)g_calloc("to get col1", len1+1, sizeof(int));
        ins0 = (dna)? DINS0 : PINS0;
        ins1 = (dna)? DINS1 : PINS1;

        smax = -10000;
        if (endgaps) {
                for (col0[0] = dely[0] = -ins0, yy = 1; yy <= len1; yy++) {
                        col0[yy] = dely[yy] = col0[yy-1] - ins1;
                        ndely[yy] = yy;
                }
                col0[0] = 0;        /* Waterman Bull Math Biol 84 */
        }
        else
                for (yy = 1; yy <= len1; yy++)
                        dely[yy] = -ins0;

        /* fill in match matrix
         */
        for (px = seqx[0], xx = 1; xx <= len0; px++, xx++) {
                /* initialize first entry in col
                 */
                if (endgaps) {
                        if (xx == 1)
                                col1[0] = delx = -(ins0+ins1);
                        else
                                col1[0] = delx = col0[0] - ins1;
                        ndelx = xx;
                }
                else {
                        col1[0] = 0;
                        delx = -ins0;
                        ndelx = 0;
                }
```

**Table 1 (cont')**

...nw

```
for (py = seqx[1], yy = 1; yy <= len1; py++, yy++) {
        mis = col0[yy-1];
        if (dna)
                mis += (xbm[*px-'A']&xbm[*py-'A'])? DMAT : DMIS;
        else
                mis += _day[*px-'A'][*py-'A'];

        /* update penalty for del in x seq;
         * favor new del over ongong del
         * ignore MAXGAP if weighting endgaps
         */
        if (endgaps || ndely[yy] < MAXGAP) {
                if (col0[yy] - ins0 >= dely[yy]) {
                        dely[yy] = col0[yy] - (ins0+ins1);
                        ndely[yy] = 1;
                } else {
                        dely[yy] -= ins1;
                        ndely[yy]++;
                }
        } else {
                if (col0[yy] - (ins0+ins1) >= dely[yy]) {
                        dely[yy] = col0[yy] - (ins0+ins1);
                        ndely[yy] = 1;
                } else
                        ndely[yy]++;
        }

        /* update penalty for del in y seq;
         * favor new del over ongong del
         */
        if (endgaps || ndelx < MAXGAP) {
                if (col1[yy-1] - ins0 >= delx) {
                        delx = col1[yy-1] - (ins0+ins1);
                        ndelx = 1;
                } else {
                        delx -= ins1;
                        ndelx++;
                }
        } else {
                if (col1[yy-1] - (ins0+ins1) >= delx) {
                        delx = col1[yy-1] - (ins0+ins1);
                        ndelx = 1;
                } else
                        ndelx++;
        }

        /* pick the maximum score; we're favoring
         * mis over any del and delx over dely
         */
```

## Table 1 (cont')

...nw

```
id = xx - yy + len1 - 1;
if (mis >= delx && mis >= dely[yy])
        col1[yy] = mis;
else if (delx >= dely[yy]) {
        col1[yy] = delx;
        ij = dx[id].ijmp;
        if (dx[id].jp.n[0] && (!dna || (ndelx >= MAXJMP
        && xx > dx[id].jp.x[ij]+MX) || mis > dx[id].score+DINS0)) {
                dx[id].ijmp++;
                if (++ij >= MAXJMP) {
                        writejmps(id);
                        ij = dx[id].ijmp = 0;
                        dx[id].offset = offset;
                        offset += sizeof(struct jmp) + sizeof(offset);
                }
        }
        dx[id].jp.n[ij] = ndelx;
        dx[id].jp.x[ij] = xx;
        dx[id].score = delx;
}
else {
        col1[yy] = dely[yy];
        ij = dx[id].ijmp;
if (dx[id].jp.n[0] && (!dna || (ndely[yy] >= MAXJMP
        && xx > dx[id].jp.x[ij]+MX) || mis > dx[id].score+DINS0)) {
                dx[id].ijmp++;
                if (++ij >= MAXJMP) {
                        writejmps(id);
                        ij = dx[id].ijmp = 0;
                        dx[id].offset = offset;
                        offset += sizeof(struct jmp) + sizeof(offset);
                }
        }
        dx[id].jp.n[ij] = -ndely[yy];
        dx[id].jp.x[ij] = xx;
        dx[id].score = dely[yy];
}
if (xx == len0 && yy < len1) {
        /* last col
         */
        if (endgaps)
                col1[yy] -= ins0+ins1*(len1-yy);
        if (col1[yy] > smax) {
                smax = col1[yy];
                dmax = id;
        }
}
        }
        if (endgaps && xx < len0)
                col1[yy-1] -= ins0+ins1*(len0-xx);
        if (col1[yy-1] > smax) {
                smax = col1[yy-1];
                dmax = id;
        }
        tmp = col0; col0 = col1; col1 = tmp;
}
(void) free((char *)ndely);
(void) free((char *)dely);
(void) free((char *)col0);
(void) free((char *)col1);                  }
```

- -

## Table 1 (cont')

```
/*
 *
 * print() -- only routine visible outside this module
 *
 * static:
 * getmat() -- trace back best path, count matches: print()
 * pr_align() -- print alignment of described in array p[]: print()
 * dumpblock() -- dump a block of lines with numbers, stars: pr_align()
 * nums() -- put out a number line: dumpblock()
 * putline() -- put out a line (name, [num], seq, [num]): dumpblock()
 * stars() - -put a line of stars: dumpblock()
 * stripname() -- strip any path and prefix from a seqname
 */

#include "nw.h"

#define SPC      3
#define P_LINE 256      /* maximum output line */
#define P_SPC   3       /* space between name or num and seq */

extern  _day[26][26];
int     olen;           /* set output line length */
FILE    *fx;            /* output file */

print()                                                      print
{
        int     lx, ly, firstgap, lastgap;      /* overlap */

        if ((fx = fopen(ofile, "w")) == 0) {
                fprintf(stderr,"%s: can't write %s\n", prog, ofile);
                cleanup(1);
        }
        fprintf(fx, "<first sequence: %s (length = %d)\n", namex[0], len0);
        fprintf(fx, "<second sequence: %s (length = %d)\n", namex[1], len1);
        olen = 60;
        lx = len0;
        ly = len1;
        firstgap = lastgap = 0;
        if (dmax < len1 - 1) {          /* leading gap in x */
                pp[0].spc = firstgap = len1 - dmax - 1;
                ly -= pp[0].spc;
        }
        else if (dmax > len1 - 1) {  /* leading gap in y */
                pp[1].spc = firstgap = dmax - (len1 - 1);
                lx -= pp[1].spc;
        }
        if (dmax0 < len0 - 1) {         /* trailing gap in x */
                lastgap = len0 - dmax0 -1;
                lx -= lastgap;
        }
        else if (dmax0 > len0 - 1) { /* trailing gap in y */
                lastgap = dmax0 - (len0 - 1);
                ly -= lastgap;
        }
        getmat(lx, ly, firstgap, lastgap);
        pr_align();
}
```

## Table 1 (cont')

getmat

```
/*
 * trace back the best path, count matches
 */
static
getmat(lx, ly, firstgap, lastgap)
        int     lx, ly;                 /* "core" (minus endgaps) */
        int     firstgap, lastgap;      /* leading trailing overlap */
{
        int             nm, i0, i1, siz0, siz1;
        char            outx[32];
        double          pct;
        register        n0, n1;
        register char   *p0, *p1;

        /* get total matches, score
         */
        i0 = i1 = siz0 = siz1 = 0;
        p0 = seqx[0] + pp[1].spc;
        p1 = seqx[1] + pp[0].spc;
        n0 = pp[1].spc + 1;
        n1 = pp[0].spc + 1;

        nm = 0;
        while ( *p0 && *p1 ) {
                if (siz0) {
                        p1++;
                        n1++;
                        siz0--;
                }
                else if (siz1) {
                        p0++;
                        n0++;
                        siz1--;
                }
                else {
                        if (xbm[*p0-'A']&xbm[*p1-'A'])
                                nm++;
                        if (n0++ == pp[0].x[i0])
                                siz0 = pp[0].n[i0++];
                        if (n1++ == pp[1].x[i1])
                                siz1 = pp[1].n[i1++];
                        p0++;
                        p1++;
                }
        }

        /* pct homology:
         * if penalizing endgaps, base is the shorter seq
         * else, knock off overhangs and take shorter core
         */
        if (endgaps)
                lx = (len0 < len1)? len0 : len1;
        else
                lx = (lx < ly)? lx : ly;
        pct = 100.*(double)nm/(double)lx;
        fprintf(fx, "\n");
        fprintf(fx, "<%d match%s in an overlap of %d: %.2f percent similarity\n",
                nm, (nm == 1)? "" : "es", lx, pct);
```

## Table 1 (cont')

...getmat

```
        fprintf(fx, "<gaps in first sequence: %d", gapx);
        if (gapx) {
                (void) sprintf(outx, " (%d %s%s)",
                        ngapx, (dna)? "base":"residue", (ngapx == 1)? "":"s");
                fprintf(fx,"%s", outx);

        fprintf(fx, ", gaps in second sequence: %d", gapy);
        if (gapy) {
                (void) sprintf(outx, " (%d %s%s)",
                        ngapy, (dna)? "base":"residue", (ngapy == 1)? "":"s");
                fprintf(fx,"%s", outx);
        }
        if (dna)
                fprintf(fx,
                "\n<score: %d (match = %d, mismatch = %d, gap penalty = %d + %d per base)\n",
                smax, DMAT, DMIS, DINS0, DINS1);
        else
                fprintf(fx,
                "\n<score: %d (Dayhoff PAM 250 matrix, gap penalty = %d + %d per residue)\n",
                smax, PINS0, PINS1);
        if (endgaps)
                fprintf(fx,
                "<endgaps penalized. left endgap: %d %s%s, right endgap: %d %s%s\n",
                firstgap, (dna)? "base" : "residue", (firstgap == 1)? "" : "s",
                lastgap, (dna)? "base" : "residue", (lastgap == 1)? "" : "s");
        else
                fprintf(fx, "<endgaps not penalized\n");
}

static          nm;             /* matches in core -- for checking */
static          lmax;           /* lengths of stripped file names */
static          ij[2];          /* jmp index for a path */
static          nc[2];          /* number at start of current line */
static          ni[2];          /* current elem number -- for gapping */
static          siz[2];
static char     *ps[2];         /* ptr to current element */
static char     *po[2];         /* ptr to next output char slot */
static char     out[2][P_LINE]; /* output line */
static char     star[P_LINE];   /* set by stars() */

/*
 * print alignment of described in struct path pp[]
 */
static
pr_align()
{
        int             nn;     /* char count */
        int             more;
        register        i;

        for (i = 0, lmax = 0; i < 2; i++) {
                nn = stripname(namex[i]);
                if (nn > lmax)
                        lmax = nn;

                nc[i] = 1;
                ni[i] = 1;
                siz[i] = ij[i] = 0;
                ps[i] = seqx[i];
                po[i] = out[i];                 }
```

pr_align

**Table 1 (cont')**

...pr_align

```
	for (nn = nm = 0, more = 1; more; ) {
		for (i = more = 0; i < 2; i++) {
			/*
			 * do we have more of this sequence?
			 */
			if (!*ps[i])
				continue;

			more++;

			if (pp[i].spc) {	/* leading space */
				*po[i]++ = ' ';
				pp[i].spc--;
			}
			else if (siz[i]) {	/* in a gap */
				*po[i]++ = '-';
				siz[i]--;
			}
			else {			/* we're putting a seq element
						 */
				*po[i] = *ps[i];
				if (islower(*ps[i]))
					*ps[i] = toupper(*ps[i]);
				po[i]++;
				ps[i]++;

				/*
				 * are we at next gap for this seq?
				 */
				if (ni[i] == pp[i].x[ij[i]]) {
					/*
					 * we need to merge all gaps
					 * at this location
					 */
					siz[i] = pp[i].n[ij[i]++];
					while (ni[i] == pp[i].x[ij[i]])
						siz[i] += pp[i].n[ij[i]++];
				}
				ni[i]++;
			}
		}
		if (++nn == olen || !more && nn) {
			dumpblock();
			for (i = 0; i < 2; i++)
				po[i] = out[i];
			nn = 0;
		}
	}
}

/*
 * dump a block of lines, including numbers, stars: pr_align()
 */
static
dumpblock()
{
	register i;

	for (i = 0; i < 2; i++)
		*po[i]-- = '\0';
```

dumpblock

Table 1 (cont')

...dumpblock

```
		(void) putc('\n', fx);
		for (i = 0; i < 2; i++) {
			if (*out[i] && (*out[i] != ' ' || *(po[i]) != ' ')) {
				if (i == 0)
					nums(i);
				if (i == 0 && *out[1])
					stars();
				putline(i);
				if (i == 0 && *out[1])
					fprintf(fx, star);
				if (i == 1)
					nums(i);
			}
		}
}

/*
 * put out a number line: dumpblock()
 */
static
nums(ix)
	int	ix;	/* index in out[] holding seq line */
{
	char		nline[P_LINE];
	register	i, j;
	register char	*pn, *px, *py;

	for (pn = nline, i = 0; i < lmax+P_SPC; i++, pn++)
		*pn = ' ';
	for (i = nc[ix], py = out[ix]; *py; py++, pn++) {
		if (*py == ' ' || *py == '-')
			*pn = ' ';
		else {
			if (i%10 == 0 || (i == 1 && nc[ix] != 1)) {
				j = (i < 0)? -i : i;
				for (px = pn; j; j /= 10, px--)
					*px = j%10 + '0';
				if (i < 0)
					*px = '-';
			}
			else
				*pn = ' ';
			i++;
		}
	}
	*pn = '\0';
	nc[ix] = i;
	for (pn = nline; *pn; pn++)
		(void) putc(*pn, fx);
	(void) putc('\n', fx);
}

/*
 * put out a line (name, [num], seq, [num]): dumpblock()
 */
static
putline(ix)
	int	ix;			{
```

nums

putline

## Table 1 (cont')

...putline

```
        int                 i;
        register char       *px;

        for (px = namex[ix], i = 0; *px && *px != ':'; px++, i++)
                (void) putc(*px, fx);
        for (; i < lmax+P_SPC; i++)
                (void) putc(' ', fx);

        /* these count from 1:
         * ni[] is current element (from 1)
         * nc[] is number at start of current line
         */
        for (px = out[ix]; *px; px++)
                (void) putc(*px&0x7F, fx);
        (void) putc('\n', fx);
}


/*
 * put a line of stars (seqs always in out[0], out[1]): dumpblock()
 */
static
stars()
{
        int                 i;
        register char       *p0, *p1, cx, *px;

        if (!*out[0] || (*out[0] == ' ' && *(po[0]) == ' ') ||
            !*out[1] || (*out[1] == ' ' && *(po[1]) == ' '))
                return;
        px = star;
        for (i = lmax+P_SPC; i; i--)
                *px++ = ' ';

        for (p0 = out[0], p1 = out[1]; *p0 && *p1; p0++, p1++) {
                if (isalpha(*p0) && isalpha(*p1)) {

                        if (xbm[*p0-'A']&xbm[*p1-'A']) {
                                cx = '*';
                                nm++;
                        }
                        else if (!dna && _day[*p0-'A'][*p1-'A'] > 0)
                                cx = '.';
                        else
                                cx = ' ';
                }
                else
                        cx = ' ';
                *px++ = cx;
        }
        *px++ = '\n';
        *px = '\0';
}
```

stars

## Table 1 (cont')

stripname

```
/*
 * strip path or prefix from pn, return len: pr_align()
 */
static
stripname(pn)
        char    *pn;    /* file name (may be path) */
{
        register char   *px, *py;

        py = 0;
        for (px = pn; *px; px++)
                if (*px == '/')
                        py = px + 1;
        if (py)
                (void) strcpy(pn, py);
        return(strlen(pn));

}
```

## Table 1 (cont')

```
/*
 * cleanup() -- cleanup any tmp file
 * getseq() -- read in seq, set dna, len, maxlen
 * g_calloc() -- calloc() with error checkin
 * readjmps() -- get the good jmps, from tmp file if necessary
 * writejmps() -- write a filled array of jmps to a tmp file: nw()
 */
#include "nw.h"
#include <sys/file.h>

char    *jname = "/tmp/homgXXXXXX";          /* tmp file for jmps */
FILE    *fj;

int     cleanup();                           /* cleanup tmp file */
long    lseek();

/*
 * remove any tmp file if we blow
 */
cleanup(i)                                                                    cleanup
        int     i;
{
        if (fj)
                (void) unlink(jname);
        exit(i);
}

/*
 * read, return ptr to seq, set dna, len, maxlen
 * skip lines starting with ';', '<', or '>'
 * seq in upper or lower case
 */
char    *
getseq(file, len)                                                             getseq
        char    *file;  /* file name */
        int     *len;   /* seq len */
{
        char            line[1024], *pseq;
        register char   *px, *py;
        int             natgc, tlen;
        FILE            *fp;

        if ((fp = fopen(file,"r")) == 0) {
                fprintf(stderr,"%s: can't read %s\n", prog, file);
                exit(1);
        }
        tlen = natgc = 0;
        while (fgets(line, 1024, fp)) {
                if (*line == ';' || *line == '<' || *line == '>')
                        continue;
                for (px = line; *px != '\n'; px++)
                        if (isupper(*px) || islower(*px))
                                tlen++;
        }
        if ((pseq = malloc((unsigned)(tlen+6))) == 0) {
                fprintf(stderr,"%s: malloc() failed to get %d bytes for %s\n", prog, tlen+6, file);
                exit(1);
        }
        pseq[0] = pseq[1] = pseq[2] = pseq[3] = '\0';
```

## Table 1 (cont')

**...getseq**

```
		py = pseq + 4;
		*len = tlen;
		rewind(fp);

		while (fgets(line, 1024, fp)) {
			if (*line == ';' || *line == '<' || *line == '>')
				continue;
			for (px = line; *px != '\n'; px++) {
				if (isupper(*px))
					*py++ = *px;
				else if (islower(*px))
					*py++ = toupper(*px);
				if (index("ATGCU",*(py-1)))
					natgc++;
			}
		}
		*py++ = '\0';
		*py = '\0';
		(void) fclose(fp);
		dna = natgc > (tlen/3);
		return(pseq+4);
}

char	*
g_calloc(msg, nx, sz)
	char	*msg;		/* program, calling routine */
	int	nx, sz;		/* number and size of elements */
{
	char		*px, *calloc();

	if ((px = calloc((unsigned)nx, (unsigned)sz)) == 0) {
		if (*msg) {
			fprintf(stderr, "%s: g_calloc() failed %s (n=%d, sz=%d)\n", prog, msg, nx, sz);
			exit(1);
		}
	}
	return(px);
}

/*
 * get final jmps from dx[] or tmp file, set pp[], reset dmax: main()
 */
readjmps()
{
	int		fd = -1;
	int		siz, i0, i1;
	register	i, j, xx;

	if (fj) {
		(void) fclose(fj);
		if ((fd = open(jname, O_RDONLY, 0)) < 0) {
			fprintf(stderr, "%s: can't open() %s\n", prog, jname);
			cleanup(1);
		}
	}
	for (i = i0 = i1 = 0, dmax0 = dmax, xx = len0; ; i++) {
		while (1) {
			for (j = dx[dmax].ijmp; j >= 0 && dx[dmax].jp.x[j] >= xx; j--)
				;
```

**g_calloc** / **readjmps** (section labels in right margin)

## Table 1 (cont')

**...readjmps**

```
                        if (j < 0 && dx[dmax].offset && fj) {
                                (void) lseek(fd, dx[dmax].offset, 0);
                                (void) read(fd, (char *)&dx[dmax].jp, sizeof(struct jmp));
                                (void) read(fd, (char *)&dx[dmax].offset, sizeof(dx[dmax].offset));
                                dx[dmax].ijmp = MAXJMP-1;
                        }
                        else
                                break;
                }
                if (i >= JMPS) {
                        fprintf(stderr, "%s: too many gaps in alignment\n", prog);
                        cleanup(1);
                }
                if (j >= 0) {
                        siz = dx[dmax].jp.n[j];
                        xx = dx[dmax].jp.x[j];
                        dmax += siz;
                        if (siz < 0) {                  /* gap in second seq */
                                pp[1].n[i1] = -siz;
                                xx += siz;
                                /* id = xx - yy + len1 - 1
                                 */
                                pp[1].x[i1] = xx - dmax + len1 - 1;
                                gapy++;
                                ngapy -= siz;
/* ignore MAXGAP when doing endgaps */
                                siz = (-siz < MAXGAP || endgaps)? -siz : MAXGAP;
                                i1++;
                        }
                        else if (siz > 0) {  /* gap in first seq */
                                pp[0].n[i0] = siz;
                                pp[0].x[i0] = xx;
                                gapx++;
                                ngapx += siz;
/* ignore MAXGAP when doing endgaps */
                                siz = (siz < MAXGAP || endgaps)? siz : MAXGAP;
                                i0++;
                        }
                }
                else
                        break;
        }

        /* reverse the order of jmps
         */
        for (j = 0, i0--; j < i0; j++, i0--) {
                i = pp[0].n[j]; pp[0].n[j] = pp[0].n[i0]; pp[0].n[i0] = i;
                i = pp[0].x[j]; pp[0].x[j] = pp[0].x[i0]; pp[0].x[i0] = i;
        }
        for (j = 0, i1--; j < i1; j++, i1--) {
                i = pp[1].n[j]; pp[1].n[j] = pp[1].n[i1]; pp[1].n[i1] = i;
                i = pp[1].x[j]; pp[1].x[j] = pp[1].x[i1]; pp[1].x[i1] = i;
        }
        if (fd >= 0)
                (void) close(fd);
        if (fj) {
                (void) unlink(jname);
                fj = 0;
                offset = 0;
        }                               }
```

**Table 1 (cont')**

```
/*
 * write a filled jmp struct offset of the prev one (if any): nw()
 */
writejmps(ix)                                                              writejmps
        int       ix;
{
        char      *mktemp();

        if (!fj) {
                  if (mktemp(jname) < 0) {
                          fprintf(stderr, "%s: can't mktemp() %s\n", prog, jname);
                          cleanup(1);
                  }
                  if ((fj = fopen(jname, "w")) == 0) {
                          fprintf(stderr, "%s: can't write %s\n", prog, jname);
                          exit(1);
                  }
        }
        (void) fwrite((char *)&dx[ix].jp, sizeof(struct jmp), 1, fj);
        (void) fwrite((char *)&dx[ix].offset, sizeof(dx[ix].offset), 1, fj);
}
```

Table 2

| | | |
|---|---|---|
| PRO | XXXXXXXXXXXXXXX | (Length = 15 amino acids) |
| Comparison Protein | XXXXXYYYYYYY | (Length = 12 amino acids) |

% amino acid sequence identity =
(the number of identically matching amino acid residues between the two polypeptide sequences as determined by ALIGN-2) divided by (the total number of amino acid residues of the PRO polypeptide) =
5 divided by 15 = 33.3%

Table 3

| | | |
|---|---|---|
| PRO | XXXXXXXXXX | (Length = 10 amino acids) |
| Comparison Protein | XXXXXYYYYYYZZYZ | (Length = 15 amino acids) |

% amino acid sequence identity =
(the number of identically matching amino acid residues between the two polypeptide sequences as determined by ALIGN-2) divided by (the total number of amino acid residues of the PRO polypeptide) =
5 divided by 10 = 50%

Table 4

| | | |
|---|---|---|
| PRO-DNA | NNNNNNNNNNNNNN | (Length = 14 nucleotides) |
| Comparison DNA | NNNNNNLLLLLLLLLL | (Length = 16 nucleotides) |

% nucleic acid sequence identity =
(the number of identically matching nucleotides between the two nucleic acid sequences as determined by ALIGN-2) divided by (the total number of nucleotides of the PRO-DNA nucleic acid sequence) =
6 divided by 14 = 42.9%

Table 5

| | | |
|---|---|---|
| PRO-DNA | NNNNNNNNNNNN | (Length = 12 nucleotides) |

(continued)

| | | |
|---|---|---|
| Comparison DNA | NNNNLLLVV | (Length = 9 nucleotides) |

% nucleic acid sequence identity =

(the number of identically matching nucleotides between the two nucleic acid sequences as determined by ALIGN-2) divided by (the total number of nucleotides of the PRO-DNA nucleic acid sequence) =

4 divided by 12 = 33.3%

II. Compositions and Methods of the Invention

A. Full-length PRO Polypeptides

The present invention provides newly identified and isolated nucleotide sequences encoding polypeptides referred to in the present application as PRO polypeptides. In particular, cDNAs encoding the PRO polypeptide has been identified and isolated, as disclosed in further detail in the Examples below.

As disclosed in the Examples below, cDNA clones encoding PRO polypeptides have been deposited with the ATCC. The actual nucleotide sequences of the clones can readily be determined by the skilled artisan by sequencing of the deposited clones using routine methods in the art. The predicted amino acid sequences can be determined from the nucleotide sequences using routine skill. For the PRO polypeptides and encoding nucleic acids described herein, Applicants have identified what is believed to be the reading frame best identifiable with the sequence information available at the time.

B. PRO Variants

In addition to the full-length native sequence PRO polypeptides described herein, it is contemplated that PRO variants can be prepared. PRO variants can be prepared by introducing appropriate nucleotide changes into the PRO DNA, and/or by synthesis of the desired PRO polypeptide. Those skilled in the art will appreciate that amino acid changes may alter post-translational processes of the PRO polypeptide, such as changing the number or position of glycosylation sites or altering the membrane anchoring characteristics.

Variations in the native full-length sequence PRO polypeptide or in various domains of the PRO polypeptide described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO polypeptide that results in a change in the amino acid sequence of the PRO polypeptide as compared with the native sequence PRO polypeptide. Optionally the variation is by substitution of at least one amino acid with any other amino acid in one or more of the domains of the PRO polypeptide. Guidance in determining which amino acid residue may be inserted, substituted or deleted without adversely affecting the desired activity may be found by comparing the sequence of the PRO polypeptide with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PRO polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the PRO polypeptide.

PRO fragments may be prepared by any of a number of conventional techniques. Desired peptide fragments may be chemically synthesized. An alternative approach involves generating PRO fragments by enzymatic digestion, *e.g.*, by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PRO polypeptide fragments share at least one biological and/or immunological activity with the native PRO polypeptide shown in the accompanying figures.

In particular embodiments, conservative substitutions of interest are shown in Table 6 under the heading of preferred substitutions. If such substitutions result in a change in biological activity, then more substantial changes, denominated exemplary substitutions in Table 6, or as further described below in reference to amino acid classes, are introduced and the products screened.

Table 6

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in function or immunological identity of the PRO polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:

(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PRO variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

C. Modifications of PRO Polypeptides

Covalent modifications of PRO polypeptides are included within the scope of this invention. One type of covalent modification includes reacting targeted amino acid residues of a PRO polypeptide with an organic derivatizing agent that is capable of reacting with selected side chains or the N- or C- terminal residues of the PRO polypeptide. Derivatization with bifunctional agents is useful, for instance, for crosslinking PRO polypeptides to a water-insoluble support matrix or surface for use in the method for purifying anti-PRO antibodies, and vice-versa. Commonly used crosslinking agents include, *e.g.*, 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the PRO polypeptide included within the scope of this invention comprises altering the native glycosylation pattern of the polypeptide. "Altering the native glycosylation pattern" is intended for purposes herein to mean deleting one or more carbohydrate moieties found in native sequence PRO polypeptides (either by removing the underlying glycosylation site or by deleting the glycosylation by chemical and/or enzymatic means), and/or adding one or more glycosylation sites that are not present in the native sequence PRO polypeptide. In addition, the phrase includes qualitative changes in the glycosylation of the native proteins, involving a change in the nature and proportions of the various carbohydrate moieties present.

Addition of glycosylation sites to the PRO polypeptide may be accomplished by altering the amino acid sequence. The alteration may be made, for example, by the addition of, or substitution by, one or more serine or threonine residues to the native sequence PRO polypeptide (for O-linked glycosylation sites). The PRO polypeptide amino acid sequence may optionally be altered through changes at the DNA level, particularly by mutating the DNA encoding the PRO polypeptide at preselected bases such that codons are generated that will translate into the desired amino acids.

Another means of increasing the number of carbohydrate moieties on the PRO polypeptide is by chemical or enzymatic coupling of glycosides to the polypeptide. Such methods are described in the art, *e.g.*, in WO 87/05330 published 11 September 1987, and in Aplin and Wriston, CRC Crit. Rev. Biochem., pp. 259-306 (1981).

Removal of carbohydrate moieties present on the PRO polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PRO polypeptides comprises linking the PRO polypeptide to one of a variety of nonproteinaceous polymers, *e.g.*, polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO polypeptide of the present invention may also be modified in a way to form a chimeric molecule comprising a PRO polypeptide fused to another, heterologous polypeptide or amino acid sequence.

In one embodiment, such a chimeric molecule comprises a fusion of the PRO polypeptide with a tag polypeptide which provides an epitope to which an anti-tag antibody can selectively bind. The epitope tag is generally placed at the amino- or carboxyl- terminus of the PRO polypeptide. The presence of such epitope-tagged forms of the PRO polypeptide can be detected using an antibody against the tag polypeptide. Also, provision of the epitope tag enables the PRO polypeptide to be readily purified by affinity purification using an anti-tag antibody or another type of affinity matrix that binds to the epitope tag. Various tag polypeptides and their respective antibodies are well known in the art. Examples include poly-histidine (poly-His) or poly-histidine-glycine (poly-His-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [ Hopp et al., BioTechnology, 6:1204-1210 (1988)]: the KT3 epitope peptide [Martin et al., Science, 255:192-194 (1992)]; an α-tubulin epitope peptide [Skinner et al., J. Biol. Chem., 266: 15163-15166 (1991)]; and the T7 gene 10 protein peptide tag [Lutz-Freyermuth et al., Proc. Natl. Acad. Sci. USA, 87:6393-6397 (1990)].

In an alternative embodiment, the chimeric molecule may comprise a fusion of the PRO polypeptide with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PRO polypeptide in place of at least one variable region within an Ig molecule. In a particularly preferred embodiment, the immunoglobulin fusion includes the hinge, CH2 and CH3. or the hinge, CH 1, CH2 and CH3 regions of an IgG 1 molecule. For the production of immunoglobulin fusions *see* also, U.S. Patent No. 5,428,130 issued June 27, 1995.

D. Preparation of PRO Polypeptides

The description below relates primarily to production of PRO polypeptides by culturing cells transformed or transfected with a vector containing PRO polypeptide nucleic acid. It is, of course, contemplated that alternative methods, which are well known in the art, may be employed to prepare PROpolypeptides. For instance, the PRO polypeptide sequence, or portions thereof, may be produced by direct peptide synthesis using solid-phase techniques *[see, e.g.,* Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO polypeptide may be chemically synthesized separately and combined using chemical or enzymatic methods to produce the full-length PRO polypeptide.

1. Isolation of DNA Encoding PRO Polypeptides

DNA encoding PRO polypeptides may be obtained from a cDNA library prepared from tissue believed to possess the PRO mRNA and to express it at a detectable level. Accordingly, human PRO DNA can be conveniently obtained from a cDNA library prepared from human tissue, such as described in the Examples. The PRO-encoding gene may also be obtained from a genomic library or by known synthetic procedures *(e.g.,* automated nucleic acid synthesis).

Libraries can be screened with probes (such as antibodies to the PRO polypeptide or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding the PRO polypeptide is to use PCR methodology [Sambrook *et al., supra;* Dieffenbach et al., PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like $^{32}P$-labeled ATP, biotinylation or enzyme labeling. Hybridization conditions, including moderate stringency and high stringency, are provided in Sambrook *et al., supra.*

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook *et al., supra,* to detect precursors and processing intermediates of mRNA that may not have been reverse-transcribed into cDNA.

2. Selection and Transformation of Host Cells

Host cells are transfected or transformed with expression or cloning vectors described herein for PRO polypeptide production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology; a Practical Approach, M. Butler, ed. (IRL Press, 1991) and Sambrook *et al., supra.*

Methods of eukaryotic cell transfection and prokaryotic cell transformation are known to the ordinarily skilled artisan, for example, $CaCl_2$, $CaPO_4$, liposome-mediated and electroporation. Depending on the host cell used, transformation is performed using standard techniques appropriate to such cells. The calcium treatment employing calcium chloride, as described in Sambrook *et al., supra,* or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, *see,* Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli* X 1776 (ATCC 31,537); *E. coli* strain W3110 (ATCC 27,325) and K5 772 (ATCC 53.635). Other suitable prokaryotic host cells include Enterobacteriaceae such as *Escherichia, e.g., E. coli, Enterobacter, Erwinia, Klebsiella, Proteus, Salmonella, e.g., Salmonella typhimurium*, *Serratia, e.g., Serratia marcescans,* and *Shigella,* as well as *Bacilli* such as *B. subtilis* and *B. licheniformis (e.g., B. lichenifomis* 41 P disclosed in DD 266,710 published 12 April 1989), *Pseudomonas* such as *P. aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA ; E. coli* W3110 strain 9E4, which has the complete genotype *tonA ptr3; E. coli* W31 10 strain 27C7 (ATCC 55,244), which has the complete genotype *tonA ptr3 phoA E15 (argF-lac)169 degP ompT kan'; E. coli* W3110 strain 37D6, which has the complete genotype *$ton_A$ ptr3 phoA E15 (argF-lac)169 degP ompT rbs7 ilvG kan'; E. coli* W3110 strain 40B4, which is strain 37D6 with a non-kanamycin resistant *degP* deletion mutation: and an *E. coli* strain having mutant periplasmic protease disclosed in U.S. Patent No. 4,946.783 issued 7 August 1990. Alternatively, *in vitro* methods of cloning, *e.g.,* PCR or other nucleic acid polymerase reactions, are suitable.

In addition to prokaryotes, eukaryotic microbes such as filamentous fungi or yeast are suitable cloning or expression hosts for PRO-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943.529; Fleer et al., Bio/Technology, 9:968-975 (1991)) such as, *e.g., K. lactis* (MW98-8C, CBS683, CBS4574; Louvencourt et al., J. Bacteriol., 737 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans,* and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); Neurospora crassa (Case et al., Proc. Natl. Acad. Sci. USA, 76:5259-5263 [1979]): *Schwanniomyces* such as *Schwanniomyces occidentalis* (EP 394,538 published 31 October 1990); and filamentous fungi such as, *e.g., Neurospora, Penicillium, Tolypocladium* (WO 91/00357 published 10 January 1991), and *Aspergillus* hosts such as *A. nidulans* (Ballance et al., Biochem. Biophys. Res. Commun., 112:284-289 [1983]; Tilburn et al., Gene, 26:205-221 119831; Yelton et al., Proc. Natl. Acad. Sci. USA, 81: 1470-1474 [1984]) and *A. niger* (Kelly and Hynes, EMBO J., 4: 475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated PRO polypeptides are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651 human embryonic kidney line (293 or 293 cells subcloned for growth in suspension culture, Graham *et al.,* J. Gen. Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

3. Selection and Use of a Replicable Vector

The nucleic acid (e.g., cDNA or genomic DNA) encoding PRO polypeptides may be inserted into a replicable vector for cloning (amplification of the DNA) or for expression. Various vectors are publicly available, The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO polypeptide may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, *e.g.,* the yeast invertase leader, alpha factor leader (including *Saccharomyces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2μ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, *e.g.,* ampicillin, neomycin, methotrexate, or tetracycline, (b) complement auxotrophic deficiencies, or (c) supply critical nutrients not available from complex media, *e.g.,* the gene encoding D-alanine racemase for *Bacilli.*

An example of suitable selectable markers for mammalian cells are those that enable the identification of cells competent to take up the PRO-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10: 157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the PRO-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding the PRO polypeptide.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7: 149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraidehyde-3-phosphate dehydrogenase, and enzymes responsible for maltose and galactose utilization. Suitable vectors and promoters for use in yeast expression are further described in EP 73,657.

PRO transcription from vectors in mammalian host cells is controlled, for example, by promoters obtained from the genomes of viruses such as polyoma virus, fowlpox virus (UK 2,211.504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, *e.g.*, the actin promoter or an immunoglobulin promoter, and from heat-shock promoters, provided such promoters are compatible with the host cell systems.

Transcription of a DNA encoding the PRO polypeptide by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO polypeptide coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs orcDNAs. These regions contain nucleotide segments transcribed as polyadenylated fragments in the untranslated portion of the mRNA encoding the PRO polypeptide.

Still other methods, vectors, and host cells suitable for adaptation to the synthesis of PRO polypeptides in recombinant vertebrate cell culture are described in Gething et al., Nature, 293:620-625 (1981); Mantel et al., Nature, 281:40-46 (1979), EP 117,060; and EP 117,058.

4. Detecting Gene Amplification/Expression

Gene amplification and/or expression may be measured in a sample directly, for example, by conventional Southern blotting, Northern blotting to quantitate the transcription of mRNA (Thomas, Proc. Natl. Acad. Sci. USA, 77: 5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO polypeptide or against a synthetic peptide based on the DNA sequences provided herein or against exogenous sequence fused to PRO DNA and encoding a specific antibody epitope.

5. Purification of PRO Polypeptides

Forms of PRO polypeptides may be recovered from culture medium or from host cell lysates. If membrane-bound, it can be released from the membrane using a suitable detergent solution *(e.g.,* Triton-X 100) or by enzymatic cleavage. Cells employed in expression of PRO polypeptides can be disrupted by various physical or chemical means, such as freeze-thaw cycling, sonication, mechanical disruption, or cell lysing agents.

It may be desired to purify PRO polypeptides from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PRO polypeptide.

Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular PRO polypeptide produced.

E. Antibodies

Some drug candidates for use in the compositions and methods of the present invention are antibodies and antibody fragments which mimic the biological activity of a PRO polypeptide.

1. Polyclonal Antibodies

Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

2. Monoclonal Antibodies

The antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PRO polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center. San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against the PRO polypeptide. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, *supra*]. Suitable culture media for this purpose include, for example, Dulbecco's Modified Eagle's Medium and RPMI-16400 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison *et al., supra]* or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

3. Human and Humanized Antibodies

The antibodies of the invention may further comprise humanized antibodies or human antibodies. Humanized forms of non-human *(e.g.,* murine) antibodies are chimeric immunoglobulins, immunoglobulin chains or fragments thereof (such as Fv, Fab, Fab', $F(ab')_2$ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].
Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.
Human antibodies can also be produced using various techniques known in the art, including phage display libraries [Hoogenboom and Winter, J. Mol. Biol., 227:381 (1991 Marks et al., J. Mol. Biol., 222:581 (1991)]. The techniques of Cole *et al.,* and Boerner *et al.,* are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by the introducing of human immunoglobulin loci into transgenic animals, *e.g.*, mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5.569.825; 5,625,126; 5,633,425; 5,661,016, and in the following scientific publications: Marks et al., Bio/Technology, 10: 779-783 (1992); Lonberg et al., Nature, 368: 856-859 (1994); Morrison, Nature, 368: 812-13 (1994); Fishwild et al., Nature Biotechnology, 14:845-51 (1996): Neuberger, Nature Biotechnology, 14: 826 (1996): Lonberg and Huszar, Intern. Rev. Immunol., 13 :65-93 (1995).

4. Bispecific Antibodies

Bispecific antibodies are monoclonal, preferably human or humanized, antibodies that have binding specificities for at least two different antigens. In the present case, one of the binding specificities is for the PRO polypeptide, the other one is for any other antigen, and preferably for a cell-surface protein or receptor or receptor subunit.
Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).
Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies *see,* for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g., tyrosine or tryptophan). Compensatory "cavities" of identical or similar size to the large side chain(s) are created on the interface of the second antibody molecule by replacing large amino acid side chains with smaller ones (e.g., alanine or threonine). This provides a mechanism for increasing the yield of the heterodimer over other unwanted end-products such as homodimers.

Bispecific antibodies can be prepared as full length antibodies or antibody fragments *(e.g.,* $F(ab')_2$ bispecific antibodies). Techniques for generating bispecific antibodies from antibody fragments have been described in the literature. For example, bispecific antibodies can be prepared using chemical linkage. Brennan et al., Science, 229:81 (1985) describe a procedure wherein intact antibodies are proteolytically cleaved to generate $F(ab')_2$ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from *E. coli* and chemically coupled to form bispecific antibodies. Shalaby et al., J. Exp. Med., 175:217-225 (1992) describe the production of a fully humanized bispecific antibody $F(ab')_2$ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various techniques for making and isolating bispecific antibody fragments directly from recombinant cell culture have also been described. For example, bispecific antibodies have been produced using leucine zippers. Kostelny et al., J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA, 90: 6444-6448 (1993) has provided an alternative mechanism for making bispecific antibody fragments. The fragments comprise a heavy-chain variable domain ($V_H$) connected to a light-chain variable domain ($V_L$) by a linker which is too short to allow pairing between the two domains on the same chain. Accordingly, the $V_H$ and $V_L$ domains of one fragment are forced to pair with the complementary $V_L$ and $V_H$ domains of another fragment, thereby forming two antigen-binding sites. Another strategy for making bispecific antibody fragments by the use of single-chain Fv (sFv) dimers has also been reported. *See*, Gruber et al., J. Immunol., 152:5368 (1994).

Antibodies with more than two valencies are contemplated. For example, trispecific antibodies can be prepared. Tutt et al., J. Immunol., 147:60 (1991).

Exemplary bispecific antibodies may bind to two different epitopes on a given PRO polypeptide herein. Alternatively, an anti-PRO polypeptide arm may be combined with an arm which binds to a triggering molecule on a leukocyte such as a T-cell receptor molecule (e.g., CD2, CD3, CD28, or B7), or Fc receptors for IgG (FcyR), such as FcγRI (CD64), FcγRII (CD32) and FcγRIII (CD16) so as to focus cellular defense mechanisms to the cell expressing the particular PRO polypeptide. Bispecific antibodies may also be used to localize cytotoxic agents to cells which express a particular PRO polypeptide. These antibodies possess a PRO-binding arm and an arm which binds a cytotoxic agent or a radionuclide chelator, such as EOTUBE, DPTA, DOTA, or TETA. Another bispecific antibody of interest binds the PRO polypeptide and further binds tissue factor (TF).

5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

6. Effector Function Engineering

It may be desirable to modify the antibody of the invention with respect to effector function, so as to enhance, *e.g.,* the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC). *See,* Caron et al., J. Exp. Med., 176: 1191-1195 (1992) and Shopes, J. Immunol., 148: 2918-2922 (1992). Homodimeric antibodies with enhanced anti-tumor activity may also be prepared using heterobifunctional cross-linkers as described in Wolff et al., Cancer Research, 53: 2560-2565 (1993). Alternatively, an antibody can be engineered that has dual Fc regions and may thereby have enhanced complement lysis and ADCC capabilities. *See,* Stevenson et al., Anti-Cancer Drug Design, 3: 219-230 (1989).

7. Immunoconjugates

The invention also pertains to immunoconjugates comprising an antibody conjugated to a cytotoxic agent such as a chemotherapeutic agent, toxin (e.g., an enzymatically active toxin of bacterial, fungal, plant, or animal origin, or fragments thereof), or a radioactive isotope (i.e., a radioconjugate).
Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa),* ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include $^{212}$Bi, $^{131}$I, $^{131}$In, $^{90}$Y, and $^{186}$Re.
Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azidocompounds (such as bis (p-azidobenzoyl) hexanediamine), bis-diazoniumderivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098(1987). Carbon-14-labeled 1-isothiocyanatobenzyl-3-methyldiethylene triaminepentaacetic acid (MX-DTPA) is an exemplary chelating agent for conjugation of radionucleotide to the antibody. *See,* WO94/11026.
In another embodiment, the antibody may be conjugated to a "receptor" (such as streptavidin) for utilization in tumor pretargeting wherein the antibody-receptor conjugate is administered to the patient, followed by removal of unbound conjugate from the circulation using a clearing agent and then administration of a "ligand" (e.g., avidin) that is conjugated to a cytotoxic agent (*e.g.,* a radionucleotide).

8. Immunoliposomes

The antibodies disclosed herein may also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl. Acad. Sci. USA, 77: 4030 (1980); and U.S. Pat. Nos. 4,485,045 and 4,544,545. Liposomes with enhanced circulation time are disclosed in U.S. Patent No. 5,013,556.
Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al., J. Biol. Chem., 257: 286-288 (1982) via a disulfide-interchange reaction. A chemotherapeutic agent (such as Doxorubicin) is optionally contained within the liposome. *See,* Gabizon et al., J. National Cancer Inst., 81(19): 1484 (1989).

F. Identification of Proteins Capable of Inhibiting Neoplastic Cell Growth or Proliferation

The proteins disclosed in the present application have been assayed in a panel of 60 tumor cell lines currently used in the investigational, disease-oriented, *in vitro* drug-discovery screen of the National Cancer Institute (NCI). The purpose of this screen is to identify molecules that have cytotoxic and/or cytostatic activity against different types of tumors. NCI screens more than 10,000 new molecules per year (Monks et al., J. Natl. Cancer Inst., 83:757-766 (1991); Boyd, Cancer: Princ. Pract. Oncol. Update, 3(10):1-12 ([1989]). The tumor cell lines employed in this study have been described in Monks *et al., supra.* The cell lines the growth of which has been significantly inhibited by the proteins of the present application are specified in the Examples.
The results have shown that the proteins tested show cytostatic and, in some instances and concentrations, cytotoxic activities in a variety of cancer cell lines, and therefore are useful candidates for tumor therapy.
Other cell-based assays and animal models for tumors (e.g., cancers) can also be used to verify the findings of the NCI cancer screen, and to further understand the relationship between the protein identified herein and the development and pathogenesis of neoplastic cell growth. For example, primary cultures derived from tumors in transgenic animals (as described below) can be used in the cell-based assays herein, although stable cell lines are preferred. Techniques to derive continuous cell lines from transgenic animals are well known in the art *(see, e.g.,* Small et al., Mol. Cell. Biol., 5:642-648 [1985]).

G. Animal Models

A variety of well known animal models can be used to further understand the role of the molecules identified herein in the development and pathogenesis of tumors, and to test the efficacy of candidate therapeutic agents, including antibodies, and other agonists of the native polypeptides, including small molecule agonists. The *in vivo* nature of such models makes them particularly predictive of responses in human patients. Animal models of tumors and cancers (e.g., breast cancer, colon cancer, prostate cancer, lung cancer, etc.) include both non-recombinant and recombinant (transgenic) animals. Non-recombinant animal models include, for example, rodent. *e.g.*, murine models. Such models can be generated by introducing tumor cells into syngeneic mice using standard techniques, *e.g.*, subcutaneous injection, tail vein injection, spleen implantation, intraperitoneal implantation, implantation under the renal capsule, or orthopin implantation, *e.g.*, colon cancer cells implanted in colonic tissue. *(See, e.g.,* PCT publication No. WO 97/33551, published September 18, 1997).
Probably the most often used animal species in oncological studies are immunodeficient mice and, in particular, nude mice. The observation that the nude mouse with hypo/aplasia could successfully act as a host for human tumor xenografts has lead to its widespread use for this purpose. The autosomal recessive *nu* gene has been introduced into a very large number of distinct congenic strains of nude mouse, including, for example, ASW, A/He, AKR, BALB/c, B10.LP, C17, C3H, C57BL, C57, CBA, DBA, DDD, 1/st, NC, NFR, NFS, NFS/N, NZB, NZC, NZW, P, RIII and SJL. In addition, a wide variety of other animals with inherited immunological defects other than the nude mouse have been bred and used as recipients of tumor xenografts. For further details *see, e.g.,* The Nude Mouse in Oncology Research, E. Boven and B. Winograd, eds., CRC Press, Inc., 1991.
The cells introduced into such animals can be derived from known tumor/cancer cell lines, such as, any of the above-listed tumor cell lines, and, for example, the B104-1- 1 cell line (stable NIH-3T3 cell line transfected with the *neu* protooncogene); *ras*-transfected NIH-3T3 cells; Caco-2 (ATCC HTB-37); a moderately well-differentiated grade II human colon adenocarcinoma cell line, HT-29 (ATCC HTB-38), or from tumors and cancers. Samples of tumor or cancer cells can be obtained from patients undergoing surgery, using standard conditions, involving freezing and storing in liquid nitrogen (Karmali et al., Br. J. Cancer, 48:689-696 [1983]).
Tumor cells can be introduced into animals, such as nude mice, by a variety of procedures. The subcutaneous (s.c.) space in mice is very suitable for tumor implantation. Tumors can be transplanted s.c. as solid blocks, as needle biopsies by use of a trochar, or as cell suspensions. For solid block or trochar implantation, tumor tissue fragments of suitable size are introduced into the s.c. space. Cell suspensions are freshly prepared from primary tumors or stable tumor cell lines, and injected subcutaneously. Tumor cells can also be injected as subdermal implants. In this location, the inoculum is deposited between the lower part of the dermal connective tissue and the s.c. tissue. Boven and Winograd (1991), *supra.* Animal models of breast cancer can be generated, for example, by implanting rat neuroblastoma cells (from which the *neu* oncogen was initially isolated), or *neu*-transformed NIH-3T3 cells into nude mice, essentially as described by Drebin et al., Proc. Natl. Acad. Sci. USA, 83:9129-9133 (1986).
Similarly, animal models of colon cancer can be generated by passaging colon cancer cells in animals, *e.g.,* nude mice, leading to the appearance of tumors in these animals. An orthotopic transplant model of human colon cancer in nude mice has been described, for example, by Wang et al., Cancer Research, 54:4726-4728 (1994) and Too et al., Cancer Research, 55:681-684 (1995). This model is based on the so-called "METAMOUSE™" sold by AntiCancer, Inc., (San Diego, California).
Tumors that arise in animals can be removed and cultured *in vitro.* Cells from the *in vitro* cultures can then be passaged to animals. Such tumors can serve as targets for further testing or drug screening. Alternatively, the tumors resulting from the passage can be isolated and RNA from pre-passage cells and cells isolated after one or more rounds of passage analyzed for differential expression of genes of interest. Such passaging techniques can be performed with any known tumor or cancer cell lines.
For example, Meth A. CMS4, CMS5. CMS21, and WEHI-164 are chemically induced fibrosarcomas of BALB/c female mice (DeLeo et al., J. Exp. Med., 146:720 [1977]), which provide a highly controllable model system for studying the anti-tumor activities of various agents (Palladino et al., J. Immunol., 138:4023-4032 [1987]). Briefly, tumor cells are propagated *in vitro* in cell culture. Prior to injection into the animals, the cell lines are washed and suspended in buffer, at a cell density of about $10x10^6$ to $10x10^7$ cells/ml. The animals are then infected subcutaneously with 10 to 100 μl of the cell suspension, allowing one to three weeks for a tumor to appear.

In addition, the Lewis lung (3LL) carcinoma of mice, which is one of the most thoroughly studied experimental tumors, can be used as an investigational tumor model. Efficacy in this tumor model has been correlated with beneficial effects in the treatment of human patients diagnosed with small cell carcinoma of the lung (SCCL). This tumor can be introduced in normal mice upon injection of tumor fragments from an affected mouse or of cells maintained in culture (Zupi et al., Br. J. Cancer, 41, suppl. 4:309 [1980]), and evidence indicates that tumors can be started from injection of even a single cell and that a very high proportion of infected tumor cells survive. For further information about this tumor model *see,* Zacharski, Haemostasis, 16:300-320 (1986).

One way of evaluating the efficacy of a test compound in an animal model on an implanted tumor is to measure the size of the tumor before and after treatment. Traditionally, the size of implanted tumors has been measured with a slide caliper in two or three dimensions. The measure limited to two dimensions does not accurately reflect the size of the tumor, therefore, it is usually converted into the corresponding volume by using a mathematical formula. However, the measurement of tumor size is very inaccurate. The therapeutic effects of a drug candidate can be better described as treatment-induced growth delay and specific growth delay. Another important variable in the description of tumor growth is the tumor volume doubling time. Computer programs for the calculation and description of tumor growth are also available, such as the program reported by Rygaard and Spang-Thomsen, Proc. 6th Int. Workshop on Immune-Deficient Animals. Wu and Sheng eds., Basel, 1989, 301. It is noted, however, that necrosis and inflammatory responses following treatment may actually result in an increase in tumor size, at least initially. Therefore, these changes need to be carefully monitored, by a combination of a morphometric method and flow cytometric analysis.

Recombinant (transgenic) animal models can be engineered by introducing the coding portion of the genes identified herein into the genome of animals of interest, using standard techniques for producing transgenic animals. Animals that can serve as a target for transgenic manipulation include, without limitation, mice, rats, rabbits, guinea pigs, sheep, goats, pigs, and non-human primates, *e.g.,* baboons, chimpanzees and monkeys. Techniques known in the art to introduce a transgene into such animals include pronucleic microinjection (Hoppe and Wanger, U.S. Patent No. 4,873,191): retrovirus-mediated gene transfer into germ lines (*e.g.,* Van der Putten et al., Proc. Natl. Acad. Sci. USA, 82:6148-615 [1985]); gene targeting in embryonic stem cells (Thompson et al., Cell, 56:313-321 [1989]); electroporation of embryos (Lo, Mol. Cell. Biol., 3:1803-1814 [1983]); sperm-mediated gene transfer (Lavitrano et al., Cell, 57:717-73 [1989]). For review, *see,* for example, U.S. Patent No. 4,736,866.

For the purpose of the present invention, transgenic animals include those that carry the transgene only in part of their cells ("mosaic animals"). The transgene can be integrated either as a single transgene, or in concatamers. *e.g.*, head-to-head or head-to-tail tandems. Selective introduction of a transgene into a particular cell type is also possible by following, for example, the technique of Lasko et al., Proc. Nail. Acad. Sci. USA, 89:6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as *in situ* hybridization, Northern blot analysis, PCR, or immunocytochemistry. The animals are further examined for signs of tumor or cancer development.

The efficacyof antibodies specifically binding the polypeptides identified herein and other drug candidates, can be tested also in the treatment of spontaneous animal tumors. A suitable target for such studies is the feline oral squamous cell carcinoma (SCC). Feline oral SCC is a highly invasive, malignant tumor that is the most common oral malignancy of cats, accounting for over 60% of the oral tumors reported in this species. It rarely metastasizes to distant sites, although this low incidence of metastasis may merely be a reflection of the short survival times for cats with this tumor. These tumors are usually not amenable to surgery, primarily because of the anatomy of the feline oral cavity. At present, there is no effective treatment for this tumor. Prior to entry into the study, each cat undergoes complete clinical examination, biopsy, and is scanned by computed tomography (CT). Cats diagnosed with sublingual oral squamous cell tumors are excluded from the study. The tongue can become paralyzed as a result of such tumor, and even if the treatment kills the tumor, the animals may not be able to feed themselves. Each cat is treated repeatedly, over a longer period of time. Photographs of the tumors will be taken daily during the treatment period, and at each subsequent recheck. After treatment, each cat undergoes another CT scan. CT scans and thoracic radiograms are evaluated every 8 weeks thereafter. The data are evaluated for differences in survival, response and toxicity as compared to control groups. Positive response may require evidence of tumor regression, preferably with improvement of quality of life and/or increased life span.

In addition, other spontaneous animal tumors, such as fibrosarcoma, adenocarcinoma, lymphoma, chrondroma, leiomyosarcoma of dogs, cats, and baboons can also be tested. Of these mammary adenocarcinoma in dogs and cats is a preferred model as its appearance and behavior are very similar to those in humans. However, the use of this model is limited by the rare occurrence of this type of tumor in animals.

H. Screening Assays for Drug Candidates

Screening assays for drug candidates are designed to identify compounds that competitively bind or complex with the receptor(s) of the polypeptides identified herein, or otherwise signal through such receptor(s). Such screening assays will include assays amenable to high-throughput screening of chemical libraries, making them particularly suitable for identifying small molecule drug candidates. Small molecules contemplated include synthetic organic or inorganic compounds, including peptides, preferably soluble peptides, (poly)peptide-immunoglobulin fusions, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. The assays can be performed in a variety of formats, including protein-protein binding assays, biochemical screening assays, immunoassays and cell based assays, which are well characterized in the art. In binding assays, the interaction is binding and the complex formed can be isolated or detected in the reaction mixture. In a particular embodiment, a receptor of a polypeptide encoded by the gene identified herein or the drug candidate is immobilized on a solid phase, *e.g.,* on a microtiter plate, by covalent or non-covalent attachments. Non-covalent attachment generally is accomplished by coating the solid surface with a solution of the polypeptide and drying. Alternatively, an immobilized antibody, *e.g.,* a monoclonal antibody, specific for the polypeptide to be immobilized can be used to anchor it to a solid surface. The assay is performed by adding the non-immobilized component, which may be labeled by a detectable label, to the immobilized component, *e.g.*, the coated surface containing the anchored component. When the reaction is complete, the non-reacted components are removed, *e.g.*, by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a particular receptor, its interaction with that polypeptide can be assayed by methods well known for detecting protein-protein interactions. Such assays include traditional approaches, such as, cross-linking, co-immunoprecipitation, and co-purification through gradients or chromatographic columns. In addition, protein-protein interactions can be monitored by using a yeast-based genetic system described by Fields and co-workers [Fields and Song, Nature (London), 340:245-246 (1989); Chien et al., Proc. Natl. Acad. Sci. USA, 88:9578-9582 (1991)] as disclosed by Chevray and Nathans [Proc. Natl. Acad. Sci. USA, 89:5789-5793 (1991)]. Many transcriptional activators, such as yeast GAL4, consist of two physically discrete modular domains, one acting as the DNA-binding domain, while the other one functioning as the transcription activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

I. Pharmaceutical Compositions

The polypeptides of the present invention, agonist antibodies specifically binding proteins identified herein, as well as other molecules identified by the screening assays disclosed herein, can be administered for the treatment of tumors, including cancers, in the form of pharmaceutical compositions.

Where antibody fragments are used, the smallest inhibitory fragment which specifically binds to the binding domain of the target protein is preferred. For example, based upon the variable region sequences of an antibody, peptide molecules can be designed which retain the ability to bind the target protein sequence. Such peptides can be synthesized chemically and/or produced by recombinant DNA technology *(see. e.g.,* Marasco et al., Proc. Natl. Acad. Sci. USA. 90:7889-7893 [1993]).

The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.

Therapeutic formulations of the polypeptides identified herein, or agonists thereof are prepared for storage by mixing the active ingredient having the desired degree of purity with optional pharmaceutically acceptable carriers, excipients or stabilizers (Remington's Pharmaceutical Sciences. 16th edition, Osol, A. ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine, preservatives (such as octadecyldimethylbenzyl ammonium chloride; hexamethonium chloride: benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or nonionic surfactants such as TWEEN™, PLURONICS™ or polyethylene glycol (PEG).

The formulation herein may also contain more than one active compound as necessary for the particular indication being treated, preferably those with complementary activities that do not adversely affect each other. Alternatively, or in addition, the composition may comprise a cytotoxic agent, cytokine or growth inhibitory agent. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. ed. (1980).

The formulations to be used for *in vivo* administration must be sterile. This is readily accomplished by filtration through sterile filtration membranes, prior to or following lyophilization and reconstitution.

Therapeutic compositions herein generally are placed into a container having a sterile access port, for example, an intravenous solution bag or vial having a stopper pierceable by a hypodermic injection needle.

Sustained-release preparations may be prepared. Suitable examples of sustained-release preparations include semipermeable matrices of solid hydrophobic polymers containing the antibody, which matrices are in the form of shaped articles, *e.g.*, films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and $\gamma$ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

J. Methods of Treatment

It is contemplated that the polypeptides of the present invention and their agonists, including antibodies, peptides, and small molecule agonists, may be used to treat various tumors, *e.g.*, cancers. Exemplary conditions or disorders to be treated include benign or malignant tumors (*e.g.,* renal, liver, kidney, bladder, breast, gastric, ovarian, colorectal, prostate, pancreatic, lung, vulval, thyroid, hepatic carcinomas; sarcomas; glioblastomas; and various head and neck tumors); leukemias and lymphoid malignancies; other disorders such as neuronal, glial, astrocytal, hypothalamic and other glandular, macrophagal, epithelial, stromal and blastocoelic disorders; and inflammatory, angiogenic and immunologic disorders. The anti-tumor agents of the present invention (including the polypeptides disclosed herein and agonists which mimic their activity, *e.g.*, antibodies, peptides and small organic molecules), are administered to a mammal, preferably a human, in accord with known methods, such as intravenous administration as a bolus or by continuous infusion over a period of time, or by intramuscular, intraperitoneal, intracerobrospinal, intraocular, intraarterial, intralesional, subcutaneous, intraarticular, intrasynovial, intrathecal, oral, topical, or inhalation routes.

Other therapeutic regimens may be combined with the administration of the anti-cancer agents of the instant invention. For example, the patient to be treated with such anti-cancer agents may also receive radiation therapy. Alternatively, or in addition, a chemotherapeutic agent may be administered to the patient. Preparation and dosing schedules for such chemotherapeutic agents may be used according to manufacturers' instructions or as determined empirically by the skilled practitioner. Preparation and dosing schedules for such chemotherapy are also described in Chemotherapy Service, ed., M.C. Perry, Williams & Wilkins, Baltimore, MD (1992). The chemotherapeutic agent may precede, or follow administration of the anti-tumor agent of the present invention, or may be given simultaneously therewith. The anti-cancer agents of the present invention may be combined with an anti-oestrogen compound such as tamoxifen or an anti-progesterone such as onapristone *(see,* EP 616812) in dosages known for such molecules.
It may be desirable to also administer antibodies against tumor associated antigens, such as antibodies which bind to the ErbB2, EGFR, ErbB3, ErbB4, or vascular endothelial factor (VEGF). Alternatively, or in addition, two or more antibodies binding the same or two or more different cancer-associated antigens may be co-administered to the patient. Sometimes, it may be beneficial to also administer one or more cytokines to the patient. In a preferred embodiment, the anti-cancer agents herein are co-administered with a growth inhibitory agent. For example, the growth inhibitory agent may be administered first, followed by the administration of an anti-cancer agent of the present invention. However, simultaneous administration or administration of the anti-cancer agent of the present invention first is also contemplated. Suitable dosages for the growth inhibitory agent are those presently used and may be lowered due to the combined action (synergy) of the growth inhibitory agent and the antibody herein.
For the prevention or treatment of disease, the appropriate dosage of an anti-tumor agent herein will depend on the type of disease to be treated, as defined above, the severity and course of the disease, whether the agent is administered for preventive or therapeutic purposes, previous therapy, the patient's clinical history and response to the agent, and the discretion of the attending physician. The agent is suitably administered to the patient at one time or over a series of treatments. Animal experiments provide reliable guidance for the determination of effective doses for human therapy. Interspecies scaling of effective doses can be performed following the principles laid down by Mordenti, J. and Chappell, W. "The use of interspecies scaling in toxicokinetics" in Toxicokinetics and New Drug Development, Yacobi et al., eds., Pergamon Press, New York 1989, pp. 42-96.
For example, depending on the type and severity of the disease, about 1 μg/kg to 15 mg/kg *(e.g.,* 0.1-20 mg/kg) of an antitumor agent is an initial candidate dosage for administration to the patient, whether, for example, by one or more separate administrations, or by continuous infusion. A typical daily dosage might range from about I μg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays. Guidance as to particular dosages and methods of delivery is provided in the literature; *see,* for example, U.S. Pat. Nos. 4,657,760; 5,206,344; or 5,225,212. It is anticipated that different formulations will be effective for different treatment compounds and different disorders, that administration targeting one organ or tissue, for example, may necessitate delivery in a manner different from that to another organ or tissue.

K. Articles of Manufacture

In another embodiment of the invention, an article of manufacture containing materials useful for the diagnosis or treatment of the disorders described above is provided. The article of manufacture comprises a container and a label. Suitable containers include, for example, bottles, vials, syringes, and test tubes. The containers may be formed from a variety of materials such as glass or plastic. The container holds a composition which is effective for diagnosing or treating the condition and may have a sterile access port (for example the container may be an intravenous solution bag or a vial having a stopper pierceable by a hypodermic injection needle). The active agent in the composition is an anti-tumor agent of the present invention. The label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.
The following examples are offered for illustrative purposes only, and are not intended to limit the scope of the present invention in any way.
All patent and literature references cited in the present specification are hereby incorporated by reference in their entirety.

## EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

EXAMPLE 1

Extracellular Domain Homology Screening to Identify Novel Polypeptides and cDNA Encoding Therefor

The extracellular domain (ECD) sequences (including the secretion signal sequence, if any) from about 950 known secreted proteins from the Swiss-Prot public database were used to search EST databases. The EST databases included public databases (e.g., Dayhoff, GenBank), and proprietary databases (*e.g.* LIFESEQ®, Incyte Pharmaceuticals, Palo Alto, CA). The search was performed using the computer program BLAST or BLAST-2 (Altschul *et al.,* Methods in Enzymology, 266:460-480 (1996)) as a comparison of the ECD protein sequences to a 6 frame translation of the EST sequences. Those comparisons with a BLAST score of 70 (or in some cases, 90) or greater that did not encode known proteins were clustered and assembled into consensus DNA sequences with the program "phrap" (Phil Green, University of Washington, Seattle, WA).
Using this extracellular domain homology screen, consensus DNA sequences were assembled relative to the other identified EST sequences using phrap. In addition, the consensus DNA sequences obtained were often (but not always) extended using repeated cycles of BLAST or BLAST-2 and phrap to extend the consensus sequence as far as possible using the sources of EST sequences discussed above.
Based upon the consensus sequences obtained as described above, oligonucleotides were then synthesized and used to identify by PCR a cDNA library that contained the sequence of interest and for use as probes to isolate a clone of the full-length coding sequence for a PRO polypeptide. Forward and reverse PCR primers generally range from 20 to 30 nucleotides and are often designed to give a PCR product of about 100-1000 bp in length. The probe sequences are typically 40-55 bp in length. In some cases, additional oligonucleotides are synthesized when the consensus sequence is greater than about 1-1.5 kbp. In order to screen several libraries for a full-length clone, DNA from the libraries was screened by PCR amplification, as per Ausubel *et al.,* Current Protocols in Molecular Biology, with the PCR primer pair. A positive library was then used to isolate clones encoding the gene of interest using the probe oligonucleotide and one of the primer pairs.
The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see,* Holmes et al., Science, 253:1278-1280 (1991)) in the unique XhoI and NotI sites.

EXAMPLE 2

Isolation of cDNA Clones by Amylase Screening

1. Preparation of oligo dT primed cDNA library

mRNA was isolated from a human tissue of interest using reagents and protocols from Invitrogen, San Diego, CA (Fast Track 2). This RNA was used to generate an oligo dT primed cDNA library in the vector pRK5D using reagents and protocols from Life Technologies, Gaithersburg, MD (Super Script Plasmid System). In this procedure, the double stranded cDNA was sized to greater than 1000 bp and the SalI/NotI linkered cDNA was cloned into XhoI/NotI cleaved vector. pRK5D is a cloning vector that has an sp6 transcription initiation site followed by an SfiI restriction enzyme site preceding the XhoI/NotI cDNA cloning sites.

2. Preparation of random primed cDNA library

A secondary cDNA library was generated in order to preferentially represent the 5' ends of the primary cDNA clones. Sp6 RNA was generated from the primary library (described above), and this RNA was used to generate a random primed cDNA library in the vector pSST-AMY.0 using reagents and protocols from Life Technologies (Super Script Plasmid System, referenced above). In this procedure the double stranded cDNA was sized to 500-1000 bp, linkered with blunt to NotI adaptors, cleaved with SfiI, and cloned into SfiI/NotI cleaved vector. pSST-AMY.0 is a cloning vector that has a yeast alcohol dehydrogenase promoter preceding the cDNA cloning sites and the mouse amylase sequence (the mature sequence without the secretion signal) followed by the yeast alcohol dehydrogenase terminator, after the cloning sites. Thus, cDNAs cloned into this vector that are fused in frame with amylase sequence will lead to the secretion of amylase from appropriately transfected yeast colonies.

3. Transformation and Detection

DNA from the library described in paragraph 2 above was chilled on ice to which was added electrocompetent DH10B bacteria (Life Technologies, 20 ml). The bacteria and vector mixture was then electroporated as recommended by the manufacturer. Subsequently, SOC media (Life Technologies, 1 ml) was added and the mixture was incubated at 37˚C for 30 minutes. The transformants were then plated onto 20 standard 150 mm LB plates containing ampicillin and incubated for 16 hours (37˚C). Positive colonies were scraped off the plates and the DNA was isolated from the bacterial pellet using standard protocols, *e.g.*, CsCl-gradient. The purified DNA was then carried on to the yeast protocols below. The yeast methods were divided into three categories: (1) Transformation of yeast with the plasmid/cDNA combined vector; (2) Detection and isolation of yeast clones secreting amylase; and (3) PCR amplification of the insert directly from the yeast colony and purification of the DNA for sequencing and further analysis.
The yeast strain used was HD56-5A (ATCC-90785). This strain has the following genotype: MAT alpha, ura3-52, leu2-3, leu2-112, his3-11, his3-15, $MAL^+$, $SUC^+$, $GAL^+$. Preferably, yeast mutants can be employed that have deficient post-translational pathways. Such mutants may have translocation deficient alleles in *sec*71, *sec*72, sec62, with truncated *sec*71 being most preferred. Alternatively, antagonists (including antisense nucleotides and/or ligands) which interfere with the normal operation of these genes, other proteins implicated in this post translation pathway (e.g., SEC61p, SEC72p, SEC62p, SEC63p, TDJ1p or SSA1p-4p) or the complex formation of these proteins may also be preferably employed in combination with the amylase-expressing yeast.
Transformation was performed based on the protocol outlined by Gietz et al., Nucl. Acid. Res., 20:1425 (1992). Transformed cells were then inoculated from agar into YEPD complex media broth (100 ml) and grown overnight at 30˚C. The YEPD broth was prepared as described in Kaiser et al., Methods in Yeast Genetics, Cold Spring Harbor Press, Cold Spring Harbor, NY, p. 207 (1994). The overnight culture was then diluted to about 2 x $10^6$ cells/ml (approx. $OD_{600}$=0.1) into fresh YEPD broth (500 ml) and regrown to 1 x $10^7$ cells/ml (approx. $OD_{600}$=0.4-0.5).
The cells were then harvested and prepared for transformation by transfer into GS3 rotor bottles in a Sorval GS3 rotor at 5,000 rpm for 5 minutes, the supernatant discarded, and then resuspended into sterile water, and centrifuged again in 50 ml falcon tubes at 3,500 rpm in a Beckman GS-6KR centrifuge. The supernatant was discarded and the cells were subsequently washed with LiAc/TE (10 ml, 10 mM Tris-HCl, I mM EDTA pH 7.5, 100 mM $Li_2OOCCH_3$), and resuspended into LiAc/TE (2.5 ml).
Transformation took place by mixing the prepared cells (100 μl) with freshly denatured single stranded salmon testes DNA (Lofstrand Labs, Gaithersburg, MD) and transforming DNA (1 μg, vol. < 10 μl) in microfuge tubes. The mixture was mixed briefly by vortexing, then 40% PEG/TE (600 μl, 40% polyethylene glycol-4000, 10 mM Tris-HCl, 1 mM EDTA, 100 mM $Li_2OOCCH_3$, pH 7.5) was added. This mixture was gently mixed and incubated at 30˚C while agitating for 30 minutes. The cells were then heat shocked at 42˚C for 15 minutes, and the reaction vessel centrifuged in a microfuge at 12,000 rpm for 5-10 seconds, decanted and resuspended into TE (500μl, 10 mM Tris-HCl, I mM EDTA pH 7.5) followed by recentrifugation. The cells were then diluted into TE (1 ml) and aliquots (200 μl) were spread onto the selective media previously prepared in 150 mm growth plates (VWR).
Alternatively, instead of multiple small reactions, the transformation was performed using a single, large scale reaction, wherein reagent amounts were scaled up accordingly.
The selective media used was a synthetic complete dextrose agar lacking uracil (SCD-Ura) prepared as described in Kaiser et al., Methods in Yeast Genetics, Cold Spring Harbor Press, Cold Spring Harbor, NY, p. 208-210 (1994). Transformants were grown at 30˚C for 2-3 days.
The detection of colonies secreting amylase was performed by including red starch in the selective growth media. Starch was coupled to the red dye (Reactive Red-120, Sigma) as per the procedure described by Biely et al., Anal. Biochem., 172:176-179 (1988). The coupled starch was incorporated into the SCD-Ura agar plates at a final concentration of 0.15% (w/v), and was buffered with potassium phosphate to a pH of 7.0 (50-100 mM final concentration).
The positive colonies were picked and streaked across fresh selective media (onto 150 mm plates) in order to obtain well isolated and identifiable single colonies. Well isolated single colonies positive for amylase secretion were detected by direct incorporation of red starch into buffered SCD-Ura agar. Positive colonies were determined by their ability to break down starch resulting in a clear halo around the positive colony visualized directly.

4. Isolation of DNA by PCR Amplification

When a positive colony was isolated, a portion of it was picked by a toothpick and diluted into sterile water (30 μl) in a 96 well plate. At this time, the positive colonies were either frozen and stored for subsequent analysis or immediately amplified. An aliquot of cells (5 μl) was used as a template for the PCR reaction in a 25μl volume containing: 0.5 μl Klentaq (Clontech, Palo Alto, CA); 4.0 μl 10 mM dNTP's (Perkin Elmer-Cetus); 2.5 μl Kentaq buffer (Clontech); 0.25 μl forward oligo 1; 0.25 μl reverse oligo 2; 12.5 μl distilled water. The sequence of the forward oligonucleotide 1 was:

5'-TGTAAAACGACGGCCAGTTAAATAGACCTGCAATTATTAATCT-3' (SEQ ID NO:57)
The sequence of reverse oligonucleotide 2 was:
5'-CAGGAAACAGCTATGACCACCTGCACACCTGCAAATCCATT-3' (SEQ ID NO:58)

PCR was then performed as follows:

| | | | |
|---|---|---|---|
| a. | | Denature | 92˚C, 5 minutes |
| b. | 3 cycles of: | Denature | 92˚C, 30 seconds |
| | | Anneal | 59˚C, 30 seconds |
| | | Extend | 72˚C, 60 seconds |
| c. | 3 cycles of: | Denature | 92˚C, 30 seconds |
| | | Anneal | 57˚C, 30 seconds |
| | | Extend | 72˚C, 60 seconds |
| d. | 25 cycles of: | Denature | 92˚C, 30 seconds |
| | | Anneal | 55 ˚C. 30 seconds |
| | | Extend | 72˚C, 60 seconds |
| e. | | Hold | 4˚C |

The underlined regions of the oligonucleotides annealed to the ADH promoter region and the amylase region, respectively, and amplified a 307 bp region from vector pSST-AMY.0 when no insert was present. Typically, the first 18 nucleotides of the 5' end of these oligonucleotides contained annealing sites for the sequencing primers. Thus, the total product of the PCR reaction from an empty vector was 343 bp. However, signal sequence-fused cDNA resulted in considerably longer nucleotide sequences.
Following the PCR, an aliquot of the reaction (5 µl) was examined by agarose gel electrophoresis in a 1% agarose gel using a Tris-Borate-EDTA (TBE) buffering system as described by Sambrook *et al., supra.* Clones resulting in a single strong PCR product larger than 400 bp were further analyzed by DNA sequencing after purification with a 96 Qiaquick PCR clean-up column (Qiagen Inc., Chats worth, CA).

EXAMPLE 3

Isolation of cDNA Clones Using Signal Algorithm Analysis

Various polypeptide-encoding nucleic acid sequences were identified by applying a proprietary signal sequence finding algorithm developed by Genentech, Inc., (South San Francisco, CA) upon ESTs as well as clustered and assembled EST fragments from public (e.g., GenBank) and/or private (LIFESEQ®, Incyte Pharmaceuticals, Inc., Palo Alto, CA) databases. The signal sequence algorithm computes a secretion signal score based on the character of the DNA nucleotides surrounding the first and optionally the second methionine codon(s) (ATG) at the 5'-end of the sequence or sequence fragment under consideration. The nucleotides following the first ATG must code for at least 35 unambiguous amino acids without any stop codons. If the first ATG has the required amino acids, the second is not examined. If neither meets the requirement, the candidate sequence is not scored. In order to determine whether the EST sequence contains an authentic signal sequence, the DNA and corresponding amino acid sequences surrounding the ATG codon are scored using a set of seven sensors (evaluation parameters) known to be associated with secretion signals. Use of this algorithm resulted in the identification of numerous polypeptide-encoding nucleic acid sequences.

EXAMPLE 4

Isolation of cDNA Clones Encoding Human PRO240

A consensus DNA sequence was assembled relative to other EST sequences using phrap as described in Example I above. This consensus sequence is designated herein as DNA30873. Based on the DNA30873 consensus sequence, oligonucleotides were synthesized: 1) to identify by PCR a cDNA library that contained the sequence of interest, and 2) for use as probes to isolate a clone of the full-length coding sequence for PRO240.
PCR primers (forward and reverse) were synthesized:

forward PCR primer:
5'-TCAGCTCCAGACTCTGATACTGCC-3' (SEQ ID NO:59)

reverse PCR primer:
5'-TGCCTTTCTAGGAGGCAGAGCTCC-3' (SEQ ID NO:60)
Additionally, a synthetic oligonucleotide hybridization probe was constructed from the consensus DNA30873 sequence which had the following nucleotide sequence:
hybridization probe:
5'-GGACCCAGAAATGTGTCCTGAGAATGGATCTTGTGTACCTGATGGTCCAG-3' (SEQ ID NO:61)

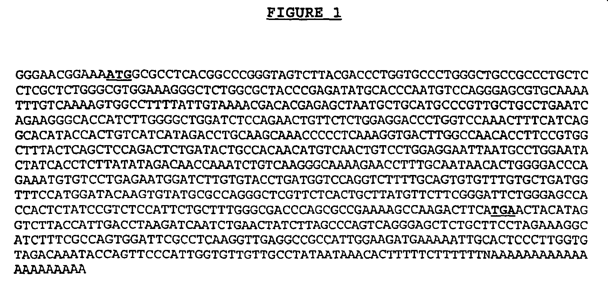

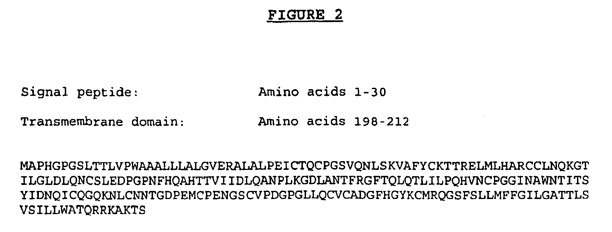

In order to screen several libraries for a source of a full-length clone, DNA from the libraries was screened by PCR amplification with the PCR primer pair identified above. A positive library was then used to isolate clones encoding the PRO240 gene using the probe oligonucleotide and one of the PCR primers.
RNA for construction of the cDNA libraries was isolated from human fetal liver tissue. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI. sized appropriately by gel electrophoresis, and cloned in a defined orientation into a suitable cloning vector (such as pRKB or pRKD; pRK5B is a precursor of pRK5D that does not contain the SfiI site; *see,* Holmes et al., Science, 253:1278-1280 (1991)) in the unique XhoI and NotI sites.
DNA sequencing of the clones isolated as described above gave the full-length DNA sequence for a full-length PRO240 polypeptide (designated herein as DNA34387-1138 [Figure 1, SEQ ID NO:1]) and the derived protein sequence for that PRO240 polypeptide.
The full length clone identified above contained a single open reading frame with an apparent translational initiation site at nucleotide positions 12-14 and a stop signal at nucleotide positions 699-701 (Figure 1, SEQ ID NO:1). The predicted polypeptide precursor is 229 amino acids long and is shown in Figure 2 (SEQ ID NO:2). Analysis of the full-length PRO240 sequence shown in Figure 2 (SEQ ID NO:2) evidences the presence of a variety of important polypeptide domains as shown in Figure 2, wherein the locations given for those important polypeptide domains are approximate as described above. Analysis of the full-length PRO240 sequence evidences the presence of the following: a signal peptide from about amino acid 1 to about amino acid 30 and a transmembrane domain from about amino acid 198 to about amino acid 212. Clone DNA34387-1138 has been deposited with ATCC on September 16, 1997 and is assigned ATCC deposit no. 209260.
An analysis of the Dayhoff database (version 35.45 SwissProt 35), using the ALIGN-2 sequence alignment analysis of the full-length sequence shown in Figure 2 (SEQ ID NO:2), evidenced sequence identity between the PRO240 amino acid sequence and the serrate precursor protein from *Drosophilia melanogaster* and the C-serrate-1 protein from *Gallus gallus* (30% and 35%, respectively).

## EXAMPLE 5

### Isolation of cDNA Clones Encoding Human PRO381

A consensus DNA sequence was assembled relative to other EST sequences using phrap as described in Example 1 above. This consensus sequence is designated herein as DNA39651. Based on the DNA39651 consensus sequence, oligonucleotides were synthesized: 1) to identify by PCR a cDNA library that contained the sequence of interest, and 2) for use as probes to isolate a clone of the full-length coding sequence for PRO381.
A pair of PCR primers (forward and reverse) were synthesized:

forward PCR primer (39651.f1):
5'-CTTTCCTTGCTTCAGCAACATGAGGC-3' (SEQ ID NO:62)
reverse PCR primer (39651.r1):
5'-GCCCAGAGCAGGAGGAATGATGAGC-3' (SEQ ID NO:63)

Additionally, a synthetic oligonucleotide hybridization probe was constructed from the consensus DNA39651 sequence which had the following nucleotide sequence:

hybridization probe (39651.p1):
5'-GTGGAACGCGGTCTTGACTCTGTTCGTCACTTCTTTGATTGGGGCTTTG-3' (SEQ ID NO:64)

In order to screen several libraries for a source of a full-length clone, DNA from the libraries was screened by PCR amplification with the PCR primer pair identified above. A positive library was then used to isolate clones encoding the PRO381 gene using the probe oligonucleotide and one of the PCR primers. RNA for construction of the cDNA libraries was isolated from human fetal kidney tissue (LIB227).

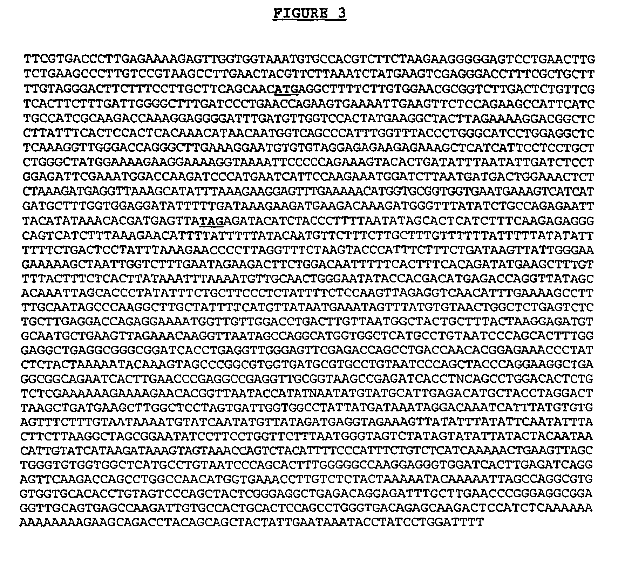

DNA sequencing of the isolated clones isolated as described above gave the full-length DNA sequence for DNA44194-1317 [Figure 3, SEQ ID NO:3]; and the derived protein sequence for PRO381.

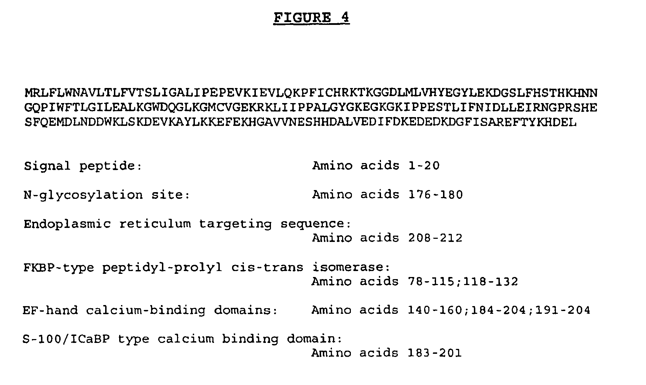

The entire coding sequence of DNA44194-1317 is included in Figure 3 (SEQ ID NO:3). Clone DNA44194-1317 contains a single open reading frame with an apparent translational initiation site at nucleotide positions 174-176, and an apparent stop codon at nucleotide positions 807-809. The predicted polypeptide precursor is 211 amino acids long. Analysis of the full-length PRO381 sequence shown in Figure 4 (SEQ ID NO:4) evidences the presence of a variety of important polypeptide domains, wherein the locations given for those important polypeptide domains are approximate as described above. Analysis of the full-length PRO381 polypeptide shown in Figure 4 evidences the presence of the following: a signal peptide from about amino acid 1 to about amino acid 20; a potential N-glycosylation site from about amino acid 176 to about amino acid 180; an endoplasmic reticulum targeting sequence from about amino acid 208 to about amino acid 212; FKBP-type peptidyl-prolyl cis-trans isomerase sites from about amino acid 78 to about amino acid 115. and from about amino acid 118 to about amino acid 132; EF-hand calcium binding domains from about amino acid 140 to about amino acid 160, from about amino acid 184 to about amino acid 204, and from about amino acid 191 to about amino acid 204; and an S-100/ICaBP type calcium binding domain from about amino acid 183 to about amino acid 201.

Clone DNA44194-1317 has been deposited with the ATCC on April 28,1998 and is assigned ATCC deposit no. 209808. The full-length PRO381 protein shown in Figure 4 has an estimated molecular weight of about 24,172 daltons and a pI of about 5.99.

Analysis of the amino acid sequence of the full-length PRO381 polypeptide suggests that it possesses sugnificant sequence similarity to FKBP immunophilin proteins, thereby indicating that PRO381 may be a novel FKBP immunophilin homolog. More specifically, an analysis of the Dayhoff database (version 35.45 SwissProt 35), using a WU-BLAST2 sequence alignment analysis of the full-length sequence shown in Figure 4 (SEQ ID NO:4), revealed sequence identity between the PRO381 amino acid sequence and the following Dayhoff sequences: AF040252_1, 149669, P_R93551, S71238, CELC05C8_1. CEU27353_1, MIP_TRYCR, CEZC455_3, FKB4_HUMAN and 140718.

EXAMPLE 6

Isolation of cDNA Clones Encoding Human PRO534

A consensus sequence was obtained relative to a variety of EST sequences as described in Example I above, wherein the consensus sequence obtained is herein designated DNA43038. Based on the 43048 consensus sequence, oligonucleotides were synthesized: 1) to identify by PCR a cDNA library that contained the sequence of interest, and 2) for use as probes to isolate a clone of the full-length coding sequence for PRO534.

A pair of PCR primers (forward and reverse) were synthesized:

forward PCR primer:
5'-CACAGAGCCAGAAGTGGCGGAATC-3' (SEQ ID NO:65)
reverse PCR primer:
5'-CCACATGTTCCTGCTCTTGTCCTGG-3' (SEQ ID NO:66)

Additionally, a synthetic oligonucleotide hybridization probe was constructed from the consensus DNA43038 sequence which had the following nucleotide sequence:

hybridization probe:
5'-CGGTAGTGACTGTACTCTAGTCCTGTnTACACCCCGTGGTGCCG-3' (SEQ ID NO:67).

In order to screen several libraries for a source of a full-length clone, DNA from the libraries was screened by PCR amplification with the PCR primer pair identified above. A positive library was then used to isolate clones encoding the PRO534 gene using the probe oligonucleotide and one of the PCR primers. RNA for construction of the cDNA libraries was isolated from human fetal lung tissue (LIB26).

DNA sequencing of the clones isolated as described above gave the full-length DNA sequence for PRO534 [herein designated as DNA48333-1321] (SEQ ID NO:5) and the derived protein sequence for PRO534.

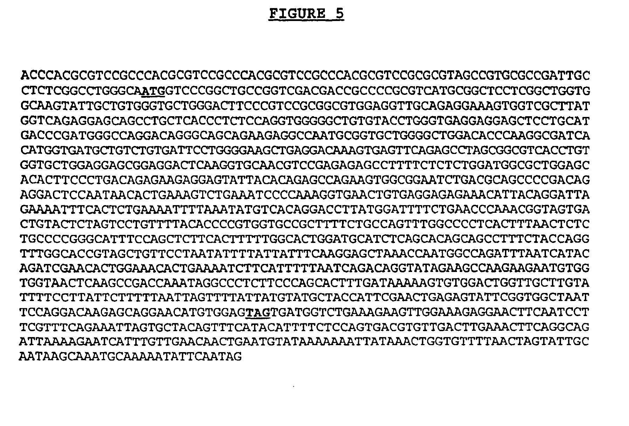

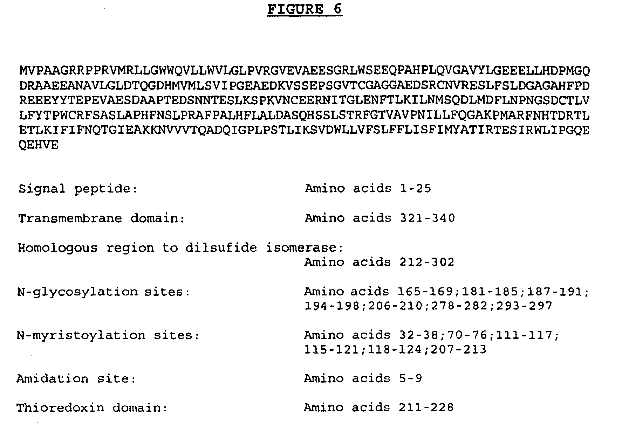

The entire nucleotide sequence of DNA48333-1321 is shown in Figure 5 (SEQ ID NO:5). Clone DNA48333-1321 contains a single open reading frame with an apparent translational initiation site at nucleotide positions 87-89 and ending at the stop codon at nucleotide positions 1167-1169 (Figure 5). The predicted polypeptide precursor is 360 amino acids long (Figure 6). The full-length PRO534 protein shown in Figure 6 has an estimated molecular weight of about 39,885 daltons and a pI of about 4.79. Clone DNA48333-1321 has been deposited with ATCC on March 26, 1998 and is assigned ATCC deposit no. 209701. It is understood that the deposited clone contains the actual sequence, and that the sequences provided herein are representative based on current sequencing techniques.

Analysis of the amino acid sequence of the full-length PRO534 polypeptide suggests that portions of it possess significant sequence identity with the protein disulfide isomerase, thereby indicating that PRO534 may be a novel disulfide isomerase.

Still analyzing the amino acid sequence of PRO534, the signal peptide is at about amino acids 1-25 of SEQ ID NO:6. The transmembrane domain is at about amino acids 321-340 of SEQ ID NO:6. The disulfide isomerase corresponding region is at about amino acids 212-302 of SEQ ID NO:6. The thioredoxin domain is at about amino acids 211-228 of SEQ ID NO:6. N-glycosylation sites are at about amino acids: 165-169, 181-185, 187-191, 194-198, 206-210, 278-282, and 293-297 of SEQ ID NO:6. N-myristoylation sites are at about amino acids: 32-38, 70-76, 111-117, 115-121, 118-124, and 207-213 of SEQ ID NO:6. An amidation site is at about amino acids 5-9 of SEQ ID NO:6. The corresponding nucleotides can routinely be determined from the sequences provided herein. PRO534 has a transmembrane domain rather than an ER retention peptide like other protein disulfide isomerases. Additionally, PRO534 may have an intron at the 5 prime end.

EXAMPLE 7

Isolation of cDNA Clones Encoding Human PRO540

A consensus DNA sequence was assembled relative to other EST sequences using phrap as described in Example 1 above. This consensus sequence is designated herein as DNA39631. Based on the DNA39631 consensus sequence, oligonucleotides were synthesized: 1) to identify by PCR a cDNA library that contained the sequence of interest, and 2) for use as probes to isolate a clone of the full-length coding sequence for PRO540.

A pair of PCR primers (forward and reverse) were synthesized:

forward PCR primer (39631.f1):
5'-CTGGGGCTACACACGGGGTGAGG-3' (SEQ ID NO:68)
reverse PCR primer (39631.r1):
5'-GGTGCCGCTGCAGAAAGTAGAGCG-3' (SEQ ID NO:69)
hybridization probe (39631.p1):
5'-GCCCCAAATGAAAACGGGCCCTACTTCCTGGCCCTCCGCGAGATG-3' (SEQ ID NO:70)

In order to screen several libraries for a source of a full-length clone, DNA from the libraries was screened by PCR amplification with one of the PCR primer pairs identified above. A positive library was then used to isolate clones encoding the PRO540 gene using the probe oligonucleotide and one of the PCR primers.

RNA for construction of the cDNA libraries was isolated from human fetal kidney tissue (LIB227). The cDNA libraries used to isolate the cDNA clones were constructed by standard methods using commercially available reagents such as those from Invitrogen, San Diego, CA. The cDNA was primed was oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; see, Holmes et al., Science, 253: 1278-1280 (1991)) in the unique XhoI and NotI sites.

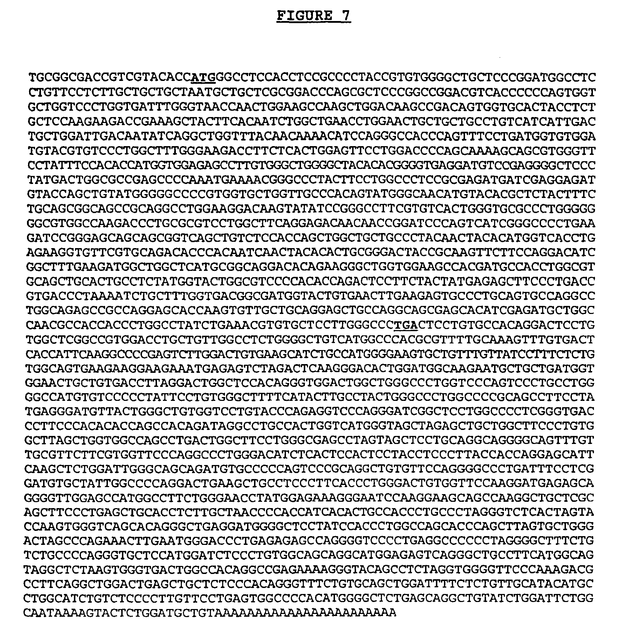

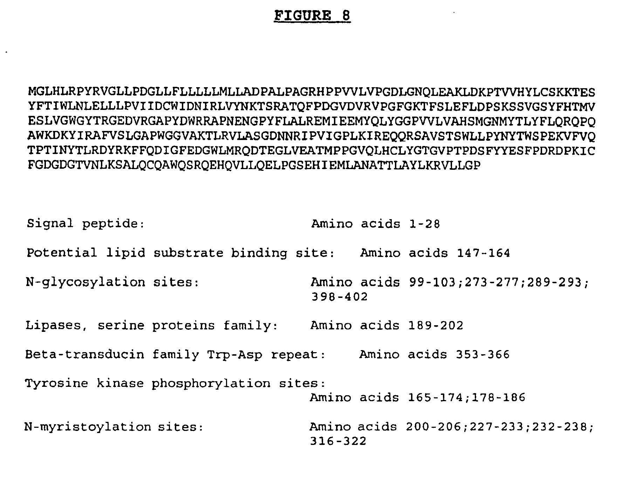

DNA sequencing of the clones isolated as described above gave the full-length DNA sequence for PRO540 herein designated as DNA44189-1322 (SEQ ID NO: 7). Clone DNA44189-1322 contains a single open reading frame with an apparent translational initiation site at nucleotide positions 21-23 and ending at the stop codon at nucleotide positions 1257-1259 (Figure 7). The predicted encoded polypeptide precursor is 412 amino acids long (Figure 8; SEQ ID NO: 8). The full-length PRO540 protein shown in Figure 8 has an estimated molecular weight of about 46,658 daltons and a pI of about 6.65. Important regions of the amino acid sequence of PRO540 (including approximate locations) include the signal peptide (residues 1-28), potential N-glycosylation sites (residues 99-103, 273-277, 289-293, 398-402), a potential lipid substrate binding site (residues 147-164), a sequence typical of lipases and serine proteins (residues 189-202), tyrosine kinase phosphorylation sites (residues 165-174 and 178-186), a beta-transducin family Trp-Asp repeat (residues 353-366) and N-myristoylation sites (residues 200-206, 227-233, 232-238 and 316-322). Clone DNA44189-1322 was deposited with the ATCC on March 26, 1998 and is assigned ATCC deposit no. 209699.

EXAMPLE 8

Isolation of cDNA Clones Encoding Human PRO698

A yeast screening assay was employed to identify cDNA clones that encoded potential secreted proteins. Use of this yeast screening assay allowed identification of a single cDNA clone herein designated as DNA39906. Based on the DNA39906 sequence, oligonucleotides were synthesized: 1) to identify by PCR a cDNA library that contained the sequence of interest, and 2) for use as probes to isolate a clone of the full-length coding sequence for PRO698. In order to screen several libraries for a full-length clone, DNA from the libraries was screened by PCR amplification, as per Ausubel *et al.,* Current Protocols in Molecular Biology, with the PCR primer pair. A positive library was then used to isolate clones encoding the gene of interest using the probe oligonucleotide and one of the primer pairs.
PCR primers (forward and reverse) were synthesized:

forward PCR primer:
5'-AGCTGTGGTCATGGTGGTGTGGTG-3' (SEQ ID NO:71)
reverse PCR primer:
5'-CTACCTTGGCCATAGGTGATCCGC.3' (SEQ ID NO:72)

Additionally, a synthetic oligonucleotide hybridization probe was constructed from the consensus DNA39906 sequence which had the following nucleotide sequence:

hybridization probe:
5'-CATCAGCAAACCGTCTGTGGTTCAGCTCAACTGGAGAGGGTT-3' (SEQ ID NO:73)
In order to screen several libraries for a source of a full-length clone, DNA from the libraries was screened by PCR amplification with the PCR primer pair identified above. A positive library was then used to isolate clones encoding the PRO698 gene using the probe oligonucleotide and one of the PCR primers. RNA for construction of the cDNA libraries was isolated from human bone marrow tissue (LIB255). The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see*, Holmes et al., Science, 253:1278-1280 (1991)) in the unique XhoI and NotI sites.

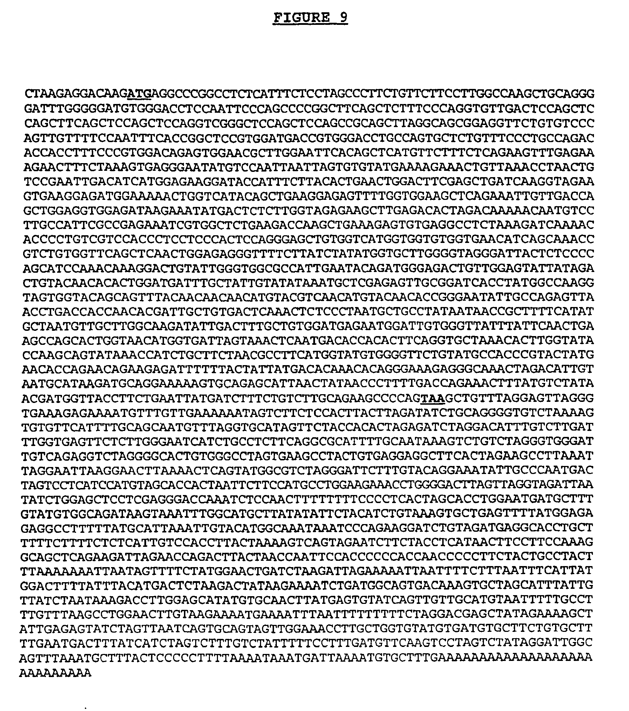

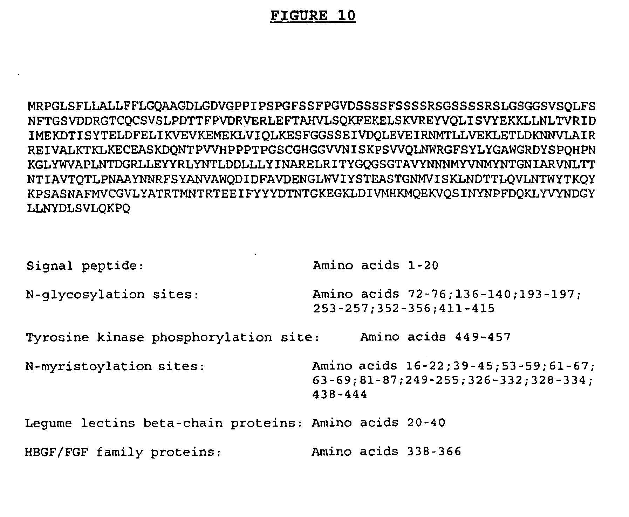

A full length clone was identified (herein designated DNA48320-1433 [SEQ ID NO:9])that contained a single open reading frame with an apparent translational initiation site at nucleotide positions 14-16 and ending at the stop codon found at nucleotide positions 1544-1546 (Figure 9, SEQ ID NO:9). The predicted polypeptide precursor is 510 amino acids long, and has a calculated molecular weight of approximately 57,280 daltons and an estimated pl of approximately 5.61. Analysis of the full-length PRO698 sequence shown in Figure 10 (SEQ ID NO: 10) evidences the presence of the following: a signal peptide from about amino acid I to about amino acid 20, potential N-glycosylation sites from about amino acid 72 to about amino acid 76, from about amino acid 136 to about amino acid 140, from about amino acid 193 to about amino acid 197, from about amino acid 253 to about amino acid 257, from about amino acid 352 to about amino acid 356, and from about amino acid 411 to about amino acid 415; a tyrosine kinase phosphorylation site from about amino acid 449 to about amino acid 457; an amino acid block having homology to legume lectin beta-chain proteins from about amino acid 20 to about amino acid 40; N-myristoylation sites from about amino acid 16 to about amino acid 22. from about amino acid 39 to about amino acid 45, from about amino acid 53 to about amino acid 59, from about amino acid 61 to about amino acid 67, from about amino acid 63 to about amino acid 69, from about amino acid 81 to about amino acid 87, from about amino acid 249 to about amino acid 255, from about amino acid 326 to about amino acid 332, from about amino acid 328 to about amino acid 334, and from about amino acid 438 to about amino acid 444; and an amino acid block having homology to the HBGF/FGF family of proteins from about amino acid 338 to about amino acid 366. Clone DNA48320-1433 has been deposited with ATCC on May 27, 1998 and is assigned ATCC deposit no. 209904.
Analysis of the amino acid sequence of the full-length PRO698 polypeptide suggests that it possesses significant sequence similarity to the olfactomedin protein, thereby indicating that PRO698 may be a novel olfactomedin homolog. More specifically, an analysis of the Dayhoff database (version 35.45 SwissProt 35) evidenced significant homology between the PRO698 amino acid sequence and the following Dayhoff sequences, OLFM_RANCA, 173637, AB006686S3_1, RNU78105_1, RNU72487_1, P_R98225, CELC48E7_4, CEF11C3_3, XLU85970_1 and S42257.

EXAMPLE 9

Isolation of cDNA Clones Encoding Human PRO982

DNA57700-1408 was identified by applying the proprietary signal sequence finding algorithm described in Example 3 above. Use of the above described signal sequence algorithm allowed identification of an ESTcluster sequence from the Incyte database, designated herein as Incyte cluster sequence no. 43715. This EST cluster sequence was then compared to a variety of expressed sequence tag (EST) databases which included public EST databases (e.g.,

GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases, 90) or greater that did not encode known proteins were clustered and assembled into aconsensus DNA sequence with the program "phrap" (Phil Green, University of Washington, Seattle, Washington). The consensus sequence obtained therefrom is herein designated DNA56095.

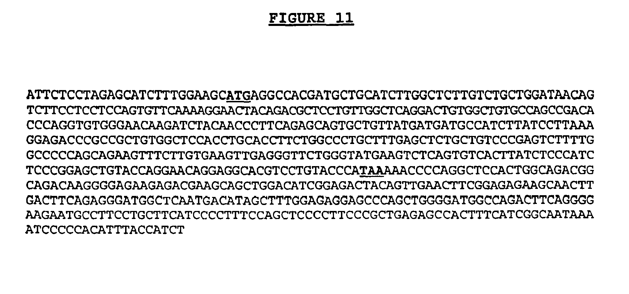

In light of an observed sequence homology between the DNA56095 consensus sequence and Merck EST no. AA024389, from the Merck database, the Merck EST no. AA024389 was purchased and the cDNA insert was obtained and sequenced. It was found herein that the cDNA insert encoded a full-length protein. The sequence of this cDNA insert is shown in Figure 11 (SEQ ID NO:11) and is herein designated as DNA57700-1408.

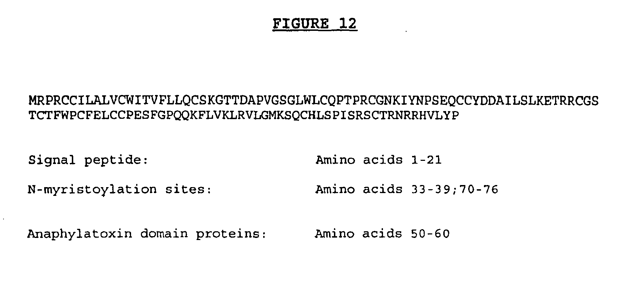

Clone DNA57700-1408 (Figure 11; SEQ ID NO:11)contains a single open reading frame with an apparent translational initiation site at nucleotide positions 26-28 and ending at the stop codon at nucleotide positions 401-403 (Figure 11; SEQ ID NO:11). The predicted polypeptide precursor is 125 amino acids long (Figure 12) and has a calculated molecular weight of approximately 14,198 daltons and an estimated pI of approximately 9.01 (Figure 12). Further analysis of the PRO982 (SEQ ID NO:12) polypeptide of Figure 12 reveals a signal peptide from about amino acid residues 1 to about amino acid 21; N-myristoylation sites from about amino acid 33 to about amino acid 39 and from about amino acid 70 to about amino acid 76; and a potential anaphylatoxin domain from about amino acid residue 50 to about amino acid 60. A cDNA clone containing DNA57700-1408 was deposited with the ATCC on January 12, 1999 and is assigned ATCC deposit No. 203583.

EXAMPLE 10

Isolation of cDNA Clones Encoding Human PRO1005

DNA57708-1411 was identified by applying the proprietary signal sequence finding algorithm described in Example 3 above. Use of the above described signal sequence algorithm allowed identification of an EST cluster sequence from the Incyte database, designated herein as Incyte cluster sequence no. 49243. This EST cluster sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymolgy, 266:460-480 (1996)). Those comparisons resulting in a BLAST score of 70 (or in some cases, 90) or greater that did not encode known proteins were clustered and assembled into a consensus DNA sequence with the program "phrap" (Phil Green, University of Washington, Seattle, Washington). The consensus sequence obtained therefrom is herein designated DNA56380.

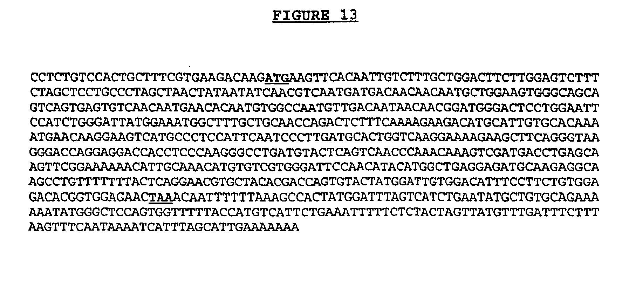

In light of an observed sequence homology between the DNA56380 consensus sequence and Merck EST no. AA256657, from the Merck database, the Merck EST no. AA256657 was purchased and the cDNA insert was obtained and sequenced. It was found herein that the cDNA insert encoded a full-length protein. The sequence of this cDNA insert is shown in Figure 13 (SEQ ID NO:13) and is herein designated as DNA57708-1411.

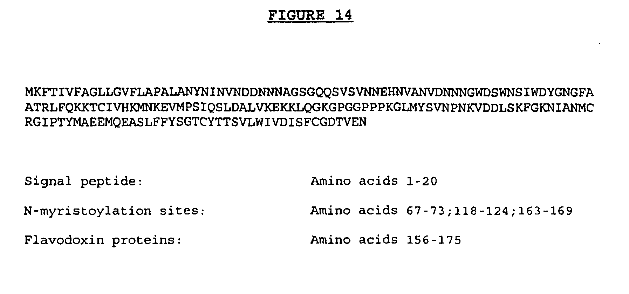

Clone DNA57708-141 (Figure 13; SEQ ID NO:13) contains a single open reading frame with an apparent translational initiation site at nucleotide positions 30-32 and ending at the stop codon at nucleotide positions 585-587 (Figure 13; SEQ ID NO:13). The predicted polypeptide precursor is 185 amino acids long (Figure 14). The full-length PRO1005 protein shown in Figure 14 (SEQ ID NO:14) has an estimated molecular weight of about 20,331 daltons and a pI of about 5.85. Analysis of the full-length PRO1005 sequence shown in Figure 14 (SEQ ID NO:14) evidences the presence of important polypeptide domains as shown in Figure 14, wherein the locations given for those important polypeptide domains are approximate as described above. Analysis of the full-length PRO1005 sequence shown in Figure 14 evidences the following: a signal peptide from about amino acid I to about amino acid 20; N-myristoylation sites from about amino acid 67 to about amino acid 73, from about amino acid 118 to about amino acid 124, and from about amino acid 163 to about amino acid 169: and a flavodoxin protein homology from about amino acid 156 to about amino acid 175. Clone DNA57708-141 has been deposited with ATCC on June 23, 1998 and is assigned ATCC deposit no. 203021.

An analysis of the Dayhoff database (version 35.45 SwissProt 35), using the ALIGN-2 sequence alignment analysis of the full-length sequence shown in Figure 14 (SEQ ID NO:14), evidenced sequence identity between the PRO1005 amino acid sequence and the following Dayhoff sequences: DDU07187_1, DDU87912_1, CELD1007_14, A42239, DDU42597_1, CYAG_DICDI, S50452, MRKC_KLEPN, P_R41998, and XYNA_RUMFL.

EXAMPLE I I

Isolation of cDNA Clones Encoding Human PRO1007

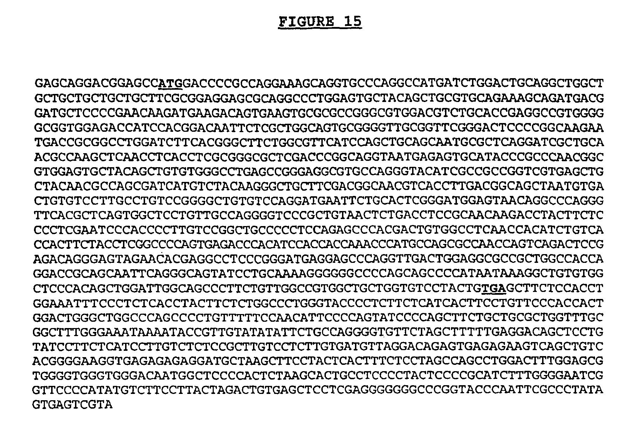

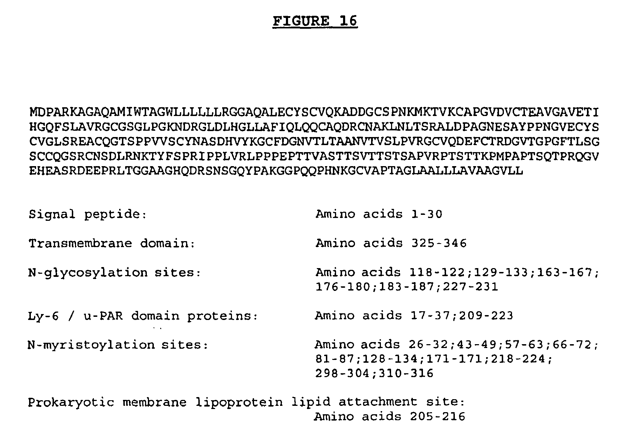

A consensus DNA sequence was assembled relative to other EST sequences using phrap as described in Example 1 above. Use of the ECD homology procedure described above resulted in the identification of an EST sequence designated Merck EST T70513, which was derived from human liver tissue (clone 83012 from library 341). Merck EST T70513 was obtained and further examined and sequenced, resulting in the isolation of the full-length DNA sequence herein designated DNA57690-1374 (Figure 15, SEQ ID NO:15) and the derived PRO1007 native sequence polypeptide (Figure 16, SEQ ID NO:16).

Clone DNA57690-1374 (SEQ ID NO: 15) contains a single open reading frame with an apparent translation initiation site at nucleotide positions 16-18 and ending at the stop codon (TGA) at nucleotide positions 1054-1056 (Figure 15), as indicated by bolded underline. The predicted PRO1007 polypeptide precursor (SEQ ID NO:16) is 346 amino acids long (Figure 16), and has a calculated molecular weight of 35.971 daltons and a pl of 8.17. A cDNA clone containing DNA57690-1374 has been deposited with the ATCC on 9 June 1998, and has been assigned deposit number 209950. Analysis of the amino acid sequence of PRO1007 (SEQ ID NO:16) reveals the putative signal peptide at about amino acid residues 1-30; a transmembrane domain at about amino acid residues 325-346: N-glycosylation sites at about amino acid residues 118-122, 129-133, 163-167, 176-180, 183-187 and 227-231; a Ly-6/u-Par domain protein at about amino acid residues 17-37 and 209-223; N-myristoylation sites at about amino acid residues 26-32, 43-49, 57-63, 66-72, 81-87, 128-134, 171-171, 218-224, 298-304 and 310-316; and a prokaryotic membrane lipoprotein lipid attachment site at about amino acid residues 205-216. The corresponding nucleotides of the amino acids presented herein can be routinely determined given the sequences provided herein.

EXAMPLE 12

Isolation of cDNA Clones Encoding Human PRO1131

A cDNA sequence isolated in the amylase screen described in Example 2 above is herein designated DNA43546. The DNA43546 sequence was then compared to a variety of expressed sequence tag (EST) databases which included public EST databases (e.g., GenBank) and a proprietary EST DNA database (LIFESEQ™, Incyte Pharmaceuticals, Palo Alto, CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases, 90) or greater that did not encode known proteins were clustered and assembled into consensus DNA sequences with the program "phrap" (Phil Green, University of Washington, Seattle, Washington). The consensus sequence obtained therefrom is herein designated DNA45627.
Based on the DNA45627 sequence, oligonucleotide probes were generated and used to screen a human library prepared as described in paragraph 1 of Example 2 above. The cloning vector was pRK5B (pRK5B is a precursor of pRK5D that does not contain the SfiI site*; see,* Holmes et al., Science, 253:1278-1280 (1991)), and the cDNA size cut was less than 2800 bp.
PCR primers (forward and 2 reverse) were synthesized:

forward PCR primer:
5'-ATGCAGGCCAAGTACAGCAGCAC-3' (SEQ ID NO:74)
reverse PCR primer 1:
5'-CATGCTGACGACTTCCTGCAAGC-3' (SEQ ID NO:75)
reverse PCR primer 2:
5'-CCACACAGTCTCTGCTTCTTGGG-3' (SEQ ID NO:76)

Additionally, a synthetic oligonucleotide hybridization probe was constructed from the DNA45627 sequence which had the following nucleotide sequence:

hybridization probe:
5'-ATGCTGGATGATGATGGGGACACCACCATGAGCCTGCATT-3' (SEQ ID NO:77)

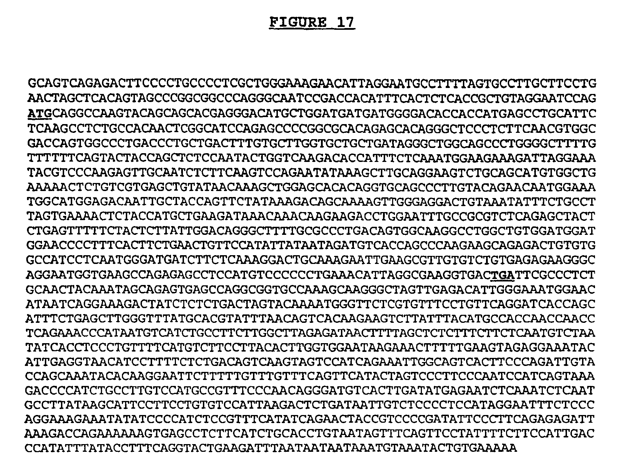

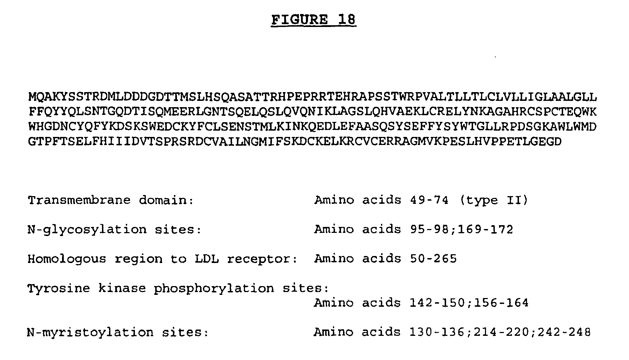

In order to screen several libraries for a source of a full-length clone, DNA from the libraries was screened by PCR amplification with the PCR primer pair identified above. A positive library was then used to isolate clones encoding the PRO1131 gene using the probe oligonucleotide and one of the PCR primers.
A full length clone was identified that contained a single open reading frame with an apparent translational initiation site at nucleotide positions 144-146, and a stop signal at nucleotide positions 984-986 (Figure 17; SEQ ID NO:17). The predicted polypeptide precursor is 280 amino acids long, and has a calculated molecular weight of approximately 31,966 daltons and an estimated pl of approximately 6.26. The transmembrane domain sequence is at about amino acid residues 49-74 of SEQ ID NO:18; N-glycosylation sites are at about amino acid residues 95-98 and 169-172 of SEQ ID NO:18; tyrosine kinase phosphorylation sites are at about amino acid residues 142-150 and 156-164 of SEQ ID NO: 18; N-myristoylation sites are at about amino acid residues 130-136.214-220 and 242-248 of SEQ ID NO:18; and the region having sequence identity with LDL receptors is about amino acid residues 50-265 of SEQ ID NO:18. Clone DNA59777-1480 has been deposited with the ATCC on August 11, 1998 and is assigned ATCC deposit no. 203111.
An analysis of the Dayhoff database (version 35.45 SwissProt 35), using a WU-BLAST2 sequence alignment analysis of the full-length sequence shown in Figure 18 (SEQ ID NO:18), evidenced some sequence identity between the PRO1131 amino acid sequence and the following Dayhoff sequences: AB010710_1, I49053, I49115, RNU56863_1, LY4A_MOUSE, 155686, MMU56404_1, 149361, AF030313_1 and MMU09739_1.

EXAMPLE 13

Isolation of cDNA Clones Encoding Human PRO1157

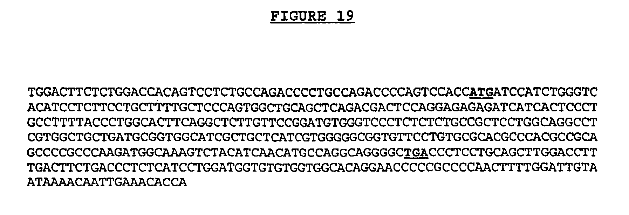

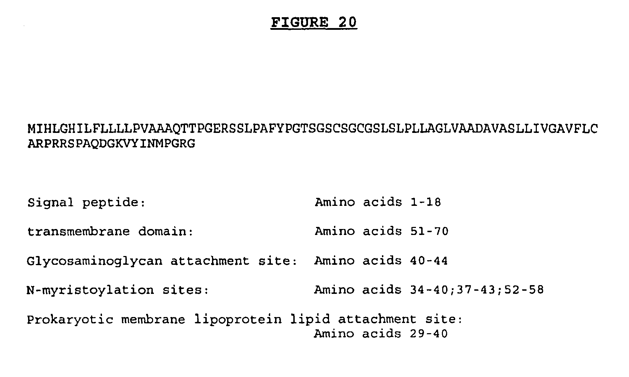

DNA60292-1506 was identified by applying the proprietary signal sequence finding algorithm described in Example 3 above. Use of the above described signal sequence algorithm allowed identification of an EST cluster sequence from the LIFESEQ® database, designated Incyte EST cluster no. 65816. This EST cluster sequence was then compared to a variety of expressed sequence tag (EST) databases which included public EST databases (e.g., GenBank) and a proprietary EST DNA database (LIFESEQ®. Incyte Pharmaceuticals, Palo Alto, CA) to identify existing homologies. One or more of the ESTs was derived from a human mast cell line from normal human prostatic epithelial cells. The homology search was performed using the computer program BLAST or BLAST2 (Altshul et al., Methods in Enzymology, 266:460-480 (1996)). Those comparisons resulting in a BLAST score of 70 (or in some cases, 90) or greater that did not encode known proteins were clustered and assembled into a consensus DNA sequence with the program "phrap" (Phil Green, University of Washington, Seattle, Washington). The consensus sequence obtained therefrom is herein designated as DNA56058.
In light of the sequence homology between the DNA56058 consensus sequence and the Merck EST no. AA516481, Merck EST no. AA516481 was purchased and the cDNA insert was obtained and sequenced. The sequence of this cDNA insert is shown in Figure 19 (SEQ ID NO:19) and is herein designated as DNA60292-1506.
The entire coding sequence of DNA60292-1506 is included in Figure 19 (SEQ ID NO:19). Clone DNA60292-1506 contains a single open reading frame with an apparent translational initiation site at nucleotide positions 56-58 and ending at the stop codon at nucleotide positions 332-334 (Figure 19). The predicted polypeptide precursor is 92 amino acids long (Figure 20; SEQ ID NO:20). The full-length PRO1157 protein shown in Figure 20 has an estimated molecular weight of about 9.360 daltons and a pl of about 9.17. Analysis of the full-length PRO1157 sequence shown in Figure 20 (SEQ ID NO:20) evidences the presence of a variety of important polypeptide domains, wherein the locations given for those important polypeptide domains are approximate as described above. Analysis of the full-length PRO1157 sequence shown in Figure 20 evidences the presence of the following: a signal peptide from about amino acid 1 to about amino acid 18; a putative transmembrane domain from about amino acid 51 to about amino acid 70; a glycosaminoglycan attachment site from about amino acid 40 to about amino acid 44; N-myristoylation sites from about amino acid 34 to about amino acid 40, from about amino acid 37 to about amino acid 43 and from about amino acid 52 to about amino acid 58; and a prokaryotic membrane lipoprotein lipid attachment site from about amino acid 29 to about amino acid 40. Clone DNA60292-1506 has been deposited with ATCC on December 15, 1998 and is assigned ATCC deposit no. 203540.
An analysis of the Dayhoff database (version 35.45 SwissProt 35), using a WU-BLAST2 sequence alignment analysis of the full-length sequence shown in Figure 20 (SEQ ID NO:20), evidenced homology between the PRO1157 amino acid sequence and the following Dayhoff sequences: PTPN_HUMAN, B69251, 151419. AF019562_1, AF019563_1, C211_HUMAN, 137577, A39171. GATS_MOUSE. ACR3_MOUSE. 5H6_RAT, P_W31512, and S58082.

EXAMPLE 14

Isolation of cDNA Clones Encoding Human PRO1199

A public expressed sequence tag (EST) DNA database (GenBank) was searched with the full-length murine m-FIZZ1 DNA (DNA53517), and an EST [designated AA311223 and renamed as DNA53028] was identified which showed homology to the m-FIZZ1 DNA.
Oligonucleotides probes based upon the above described EST sequence were then synthesized: 1) to identify by PCR a cDNA library that contained the sequence of interest, and 2) for use as probes to isolate a clone of the full-length coding sequence for PRO1199. Forward and reverse PCR primers generally range from 20 to 30 nucleotides and are often designed to give a PCR product of about 100-1000 bp in length. The probe sequences are typically 40-55 bp in length. In order to screen several libraries for a full-length clone, DNA from the libraries was screened by PCR amplification, as per Ausubel *et al.,* Current Protocols in Molecular Biology, *supra,* with the PCR primer pair. A positive library was then used to isolate clones encoding the gene of interest using the probe oligonucleotide and one of the primer pairs. The oligonucleotide probes employed were as follows:

forward primer (h-FIZZ3.f):
5'-GGATTTGGTTAGCTGAGCCCACCGAGA-3' (SEQ ID NO:78)
reverse primer (h-FIZZ3.r):
5'-GCACTGCGCGCGACCTCAGGGCTGCA-3' (SEQ ID NO:79)
probe (h-FIZZ3.p):
5'-CTTATTGCCCTAAATATTAGGGAGCCGGCGACCTCCTGGATCCTCTCATT-3' (SEQ ID NO:80)

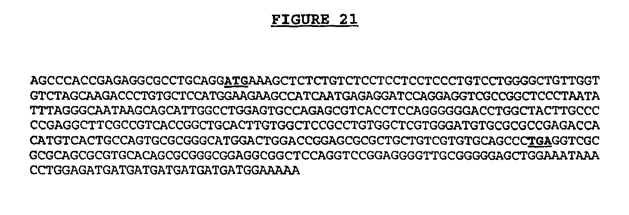

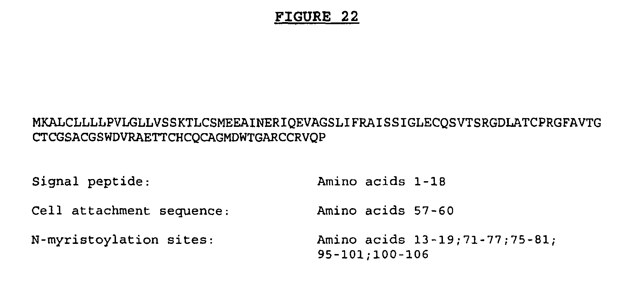

In order to screen several libraries for a source of a full-length clone, DNA from the libraries was screened by PCR amplification with the PCR primer pair identified above. A positive library was then used to isolate clones encoding the PRO1199 gene using the probe oligonucleotide and one of the PCR primers.
RNA for construction of cDNA libraries was then isolated from human bone marrow tissue. The cDNA libraries used to isolate the cDNA clones encoding human PRO1199 were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site: *see,* Holmes *et al.,* Science, 253:1278-1280 (1991)) in the unique XhoI and NotI.
A full length clone DNA65351-1366-1 was identified that contained a single open reading frame with an apparent translational initiation site at nucleotide positions 25-27 and a stop signal at nucleotide positions 349-351 (Figure 21; SEQ ID NO:21). The predicted polypeptide precursor is 108 amino acids long, and has a calculated molecular weight of approximately 11,419 daltons and an estimated pI of approximately 7.05. Analysis of the full-length PRO1199 sequence shown in Figure 22 (SEQ ID NO:22) evidences the presence of a variety of important polypeptide domains as shown in Figure 22, wherein the locations given for those important polypeptide domains are approximate as described above.
Analysis of the full-length PRO 1199 polypeptide shown in Figure 22 evidences the presence of the following: a signal peptide from about amino acid 1 to about amino acid 18; a cell attachment sequence motif (RGD) from about amino acid 57 to about amino acid 60; and N-myristoylation sites from about amino acid 13 to about amino acid 19, from about amino acid 71 to about amino acid 77, from about amino acid 75 to about amino acid 81, from about amino acid 95 to about amino acid 101, and from about amino acid 100 to about amino acid 106. Clone DNA65351-1366-1 has been deposited with ATCC on May 12, 1998 and is assigned ATCC deposit no. 209856.

EXAMPLE 15

Isolation of cDNA Clones Encoding Human PRO1265

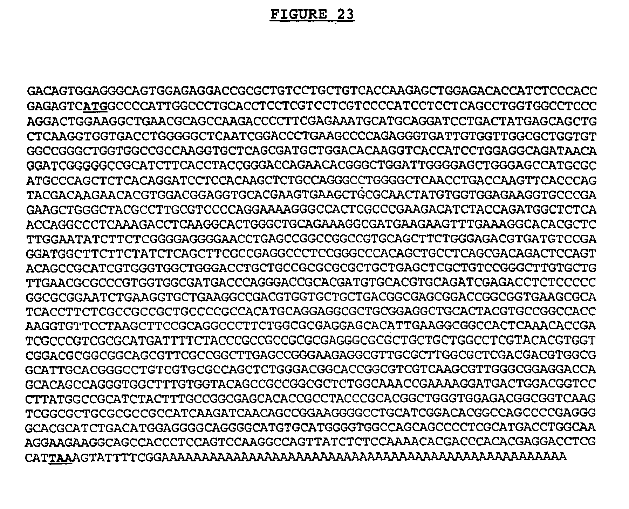

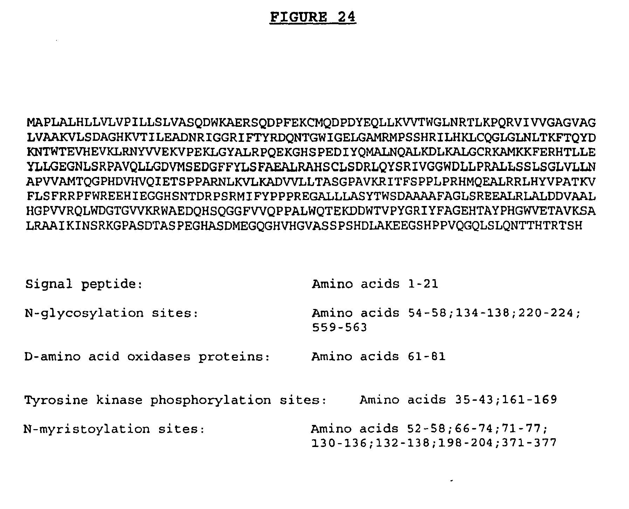

DNA60764-1533 was identified by applying the proprietary signal sequence finding algorithm described in Example 3 above. Use of the above described signal sequence algorithm allowed identification of an EST cluster sequence from the LIFESEQ® database, designated Incyte EST cluster no. 86995. This EST cluster sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases, 90) or greater that did not encode known proteins were clustered and assembled into a consensus DNA sequence with the program "phrap" (Phil Green, University of Washington, Seattle, Washington). The consensus sequence obtained therefrom is herein designated as DNA55717.
In light of the sequence homology between the DNA55717 consensus sequence and Incyte EST no. 20965, Incyte EST no. 20965 was purchased and the cDNA insert was obtained and sequenced. The sequence of this cDNA insert is shown in Figure 23 (SEQ ID NO:23) and is herein designated as DNA60764-1533.
The entire coding sequence of DNA60764-1533 is included in Figure 23 (SEQ ID NO:23). Clone DNA60764-1533 contains a single open reading frame with an apparent translational initiation site at nucleotide positions 79-81 and ending at the stop codon at nucleotide positions 1780-1782 (Figure 23). The predicted polypeptide precursor is 567 amino acids long (Figure 24; SEQ ID NO:24). The full-length PRO1265 protein shown in Figure 24 has an estimated molecular weight of about 62,881 daltons and a pI of about 8.97. Analysis of the full-length PRO1265 sequence shown in Figure 24 (SEQ ID NO:24) evidences the presence of a variety of important polypeptide domains, wherein the locations given for those important polypeptide domains are approximate as described above. Analysis of the full-length PRO1265 sequence shown in Figure 24 evidences the presence of the following: a signal peptide from about amino acid 1 to about amino acid 21; N-glycosylation sites from about amino acid 54 to about amino acid 58, from about amino acid 134 to about amino acid 138, from about amino acid 220 to about amino acid 224, and from about amino acid 559 to about amino acid 563; tyrosine kinase phosphorylation sites from about amino acid 35 to about amino acid 43, and from about amino acid 161 to about amino acid 169; N-myristoylation sites from about amino acid 52 to about amino acid 58, from about amino acid 66 to about amino acid 74, from about amino acid 71 to about amino acid 77, from about amino acid 130 to about amino acid 136, from about amino acid 132 to about amino acid 138, from about amino acid 198 to about amino acid 204, and from about amino acid 371 to about amino acid 377: and a D-amino acid oxidase protein site from about amino acid 61 to about amino acid 81. Clone DNA60764-1533 has been deposited with ATCC on November 10, 1998 and is assigned ATCC deposit no. 203452.

An analysis of the Dayhoff database (version 35.45 SwissProt 35), using a WU-BLAST2 sequence alignment analysis of the full-length sequence shown in Figure 24 (SEQ ID NO:24), evidenced significant sequence identity between the PRO1265 amino acid sequence and Dayhoff sequence no. MMU70429_1. Sequence homology was also found to exist between the full-length sequence shown in Figure 24 (SEQ ID NO:24.) and the following Dayhoff sequences: BC542A_1, E69899, S76290, MTV014_14, AOFB_HUMAN, ZMJ002204_1, 545812_1, DBRNAPD_1, and CRT1_SOYBN.

EXAMPLE 16

Isolation of cDNA Clones Encoding Human PRO1286

DNA64903-1553 was identified by applying the proprietary signal sequence finding algorithm described in Example 3 above. Use of the above described signal sequence algorithm allowed identification of an ESTcluster sequence from the LIFESEQ® database, designated Incyte EST cluster no. 86809. This EST cluster sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases, 90) or greater that did not encode known proteins were clustered and assembled into a consensus DNA sequence with the program "phrap" (Phil Green, University of Washington, Seattle, Washington). ESTs in the assembly included those identified from tumors, cell lines, or diseased tissue. One or more of the ESTs was obtained from a cDNA library constructed from RNA isolated from diseased colon tissue. The consensus sequence obtained therefrom is herein designated as DNA58822.

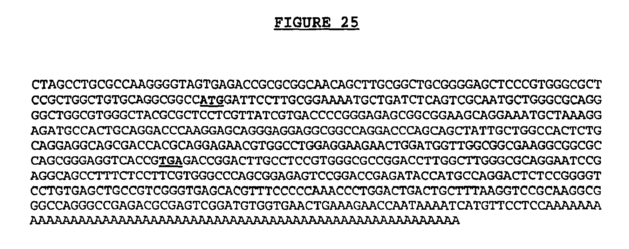

In light of the sequence homology between the DNA58822 sequence and Incyte EST clone no. 1695:134. Incyte EST no. 1695434 was purchased and the cDNA insert was obtained and sequenced. The sequence of this cDNA insert is shown in Figure 25 (SEQ ID NO:25) and is herein designated as DNA64903-1553.

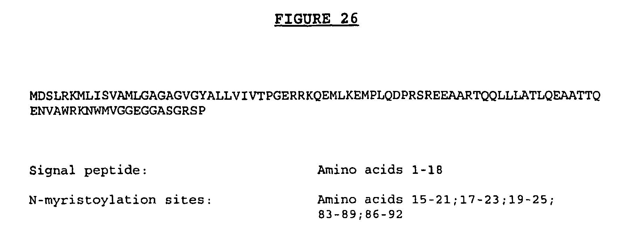

The entire coding sequence of DNA64903-1553 is included in Figure 25 (SEQ ID NO:25). Clone DNA64903-1553 contains a single open reading frame with an apparent translational initiation site at nucleotide positions 93-95 and ending at the stop codon at nucleotide positions 372-374 (Figure 25). The predicted polypeptide precursor is 93 amino acids long (Figure 26; SEQ ID NO:26). The full-length PRO1286 protein shown in Figure 26 has an estimated molecular weight of about 10,111 daltons and a pI of about 9.70. Analysis of the full-length PRO1286 sequence shown in Figure 26 (SEQ ID NO:26) evidences the presence of a variety of important polypeptide domains, wherein the locations given for those important polypeptide domains are approximate as described above. Analysis of the full-length PRO1286 sequence shown in Figure 26 evidences the presence of the following: a signal peptide from about amino acid I to about amino acid 18; and N-myristoylation sites from about amino acid 15 to about amino acid 21, from about amino acid 17 to about amino acid 23, from about amino acid 19 to about amino acid 25, from about amino acid 83 to about amino acid 89, and from about amino acid 86 to about amino acid 92. Clone DNA64903-1553 has been deposited with ATCC on September 15, 1998 and is assigned ATCC deposit no. 203223.

An analysis of the Dayhoff database (version 35.45 SwissProt 35), using a WU-BLAST2 sequence alignment analysis of the full-length sequence shown in Figure 26 (SEQ ID NO:26), revealed some homology between the PRO1286 amino acid sequence and the following Dayhoff sequences: SRSC_ARATH, CELC17H12_11, MCPD_ENTAE, JQ2283, INVO_LEMCA, P_R07309, ADEVBCAGN_4, AF020947_1, CELT23H2_1, and MDH_STRAR.

EXAMPLE 17

Isolation of cDNA Clones Encoding Human PRO1313

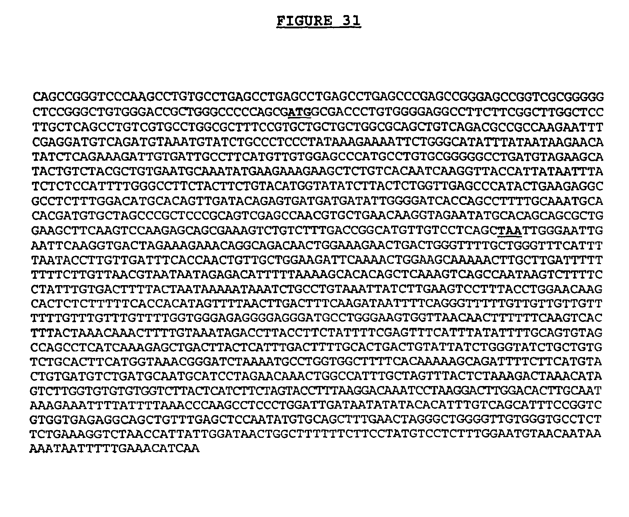

A consensus DNA sequence was assembled relative to other EST sequences using phrap as described in Example 1 above. This consensus sequence is designated herein as DNA64876. Based on the DNA64876 consensus sequence and upon a search for sequence homology with a proprietary Genentech EST sequence designated as DNA57711, a Merck/Washington University EST sequence (designated R80613) was found to have significant homology with DNA64876 and DNA57711. Therefore, the Merck/Washington University EST clone no. R80613 was purchased and the insert thereof obtained and sequenced, thereby giving rise to the DNA64966-1575 sequence shown in Figure 31 (SEQ ID NO:31), and the derived protein sequence for PRO1313.

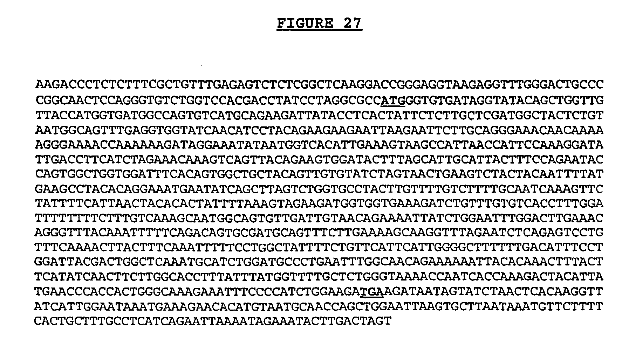

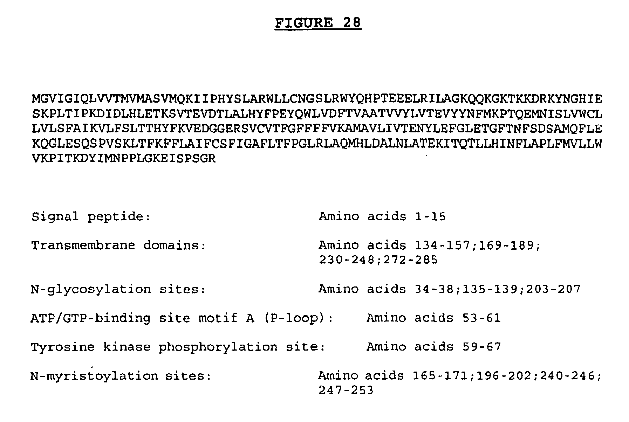

The entire coding sequence of DNA64966-1575 is included in Figure 27 (SEQ ID NO:27). Clone DNA64966-1575 contains a single open reading frame with an apparent translational initiation site at nucleotide positions 115-117, and an apparent stop codon at nucleotide positions 1036-1038. The predicted polypeptide precursor is 307 amino acids long, and has an estimated molecular weight of about 35,098 daltons and a pl of about 8.11. Analysis of the full-length PRO1313 sequence shown in Figure 28 (SEQ ID NO:28) evidences the presence of a variety of important polypeptide domains, wherein the locations given for those important polypeptide domains are approximate as described above. Analysis of the full-length PRO1313 polypeptide shown in Figure 28 evidences the presence of the following: a signal peptide from about amino acid I to about amino acid 15; transmembrane domains from about amino acid 134 to about amino acid 157. from about amino acid 169 to about amino acid 189, from about amino acid 230 to about amino acid 248, and from about amino acid 272 to about amino acid 285; N-glycosylation sites from about amino acid 34 to about amino acid 38, from about amino acid 135 to about amino acid 139, and from about amino acid 203 to about amino acid 207; a tyrosine kinase phosphorylation site from about amino acid 59 to about amino acid 67; N-myristoylation sites from about amino acid 165 to about amino acid 171, from about amino acid 196 to about amino acid 202, from about amino acid 240 to about amino acid 246, and from about amino acid 247 to about amino acid 253; and an ATP/GTP-binding site motif A (P-loop) from about amino acid 53 to about amino acid 61. Clone DNA64966-1575 has been deposited with the ATCC on January 12, 1999 and is assigned ATCC deposit no. 203575.

An analysis of the Dayhoff database (version 35.45 SwissProt 35), using a WU-BLAST2 sequence alignment analysis of the full-length sequence shown in Figure 28 (SEQ ID NO:28), evidenced significant homology between the PRO1313 amino acid sequence and the following Dayhoff sequences: CELT27A1_3, CEF09C6_7, U93688_9, H64896, YDCX_ECOLI, and RNU06101_1.

## EXAMPLE 18

## Isolation of cDNA Clones Encoding Human PRO1338

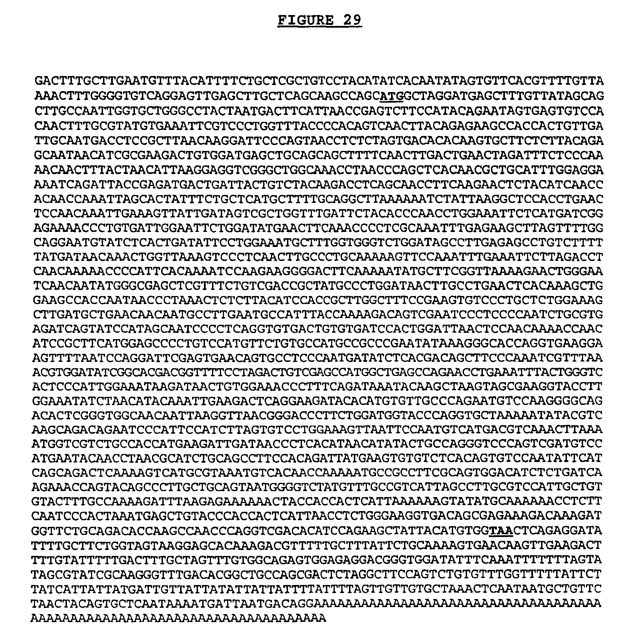

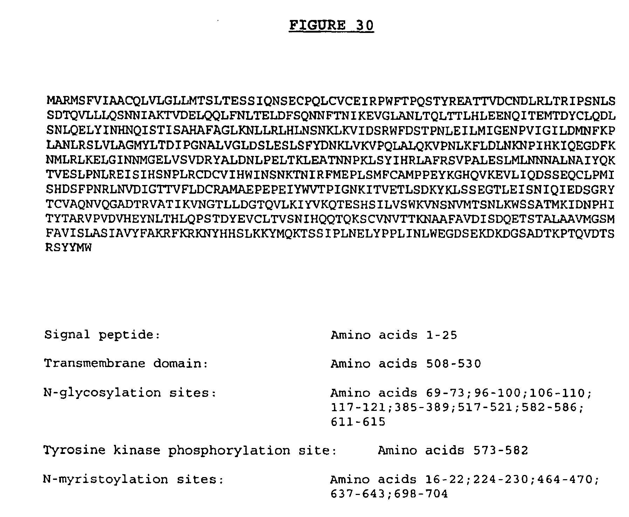

The use of yeast screens resulted in EST sequences which were then compared to various public and private EST databases in a manner similar to that described above under ECD homology (Example 1) and which resulted in the identification of Incyte EST2615184, an EST derived fromcholecystitis gall bladder tissue. Analysis of the corresponding full-length sequence ultimately resulted in the isolation of DNA66667 (SEQ ID NO:29, Figure 29) and the derived PRO1338 native sequence protein (SEQ ID NO:30, Figure 30).

DNA66667 (SEQ ID NO:29) as shown in Figure 29 contains a single open reading frame with a translation initiation site at about nucleotide residues 115-117 and ending at the stop codon (TAA) at nucleotide positions 2263-2265, as indicated by bolded underline. The predicted PRO1338 polypeptide precursor (SEQ ID NO:30) is 716 amino acids in length (Figure 30), and has a calculated molecular weight of 80,716 daltons and a pI of 6.06.

Analysis of the PRO1338 polypeptide (SEQ ID NO:30) of Figure 30 reveals a signal sequence at about amino acid residues 1 to 25; a transmembrane domain at about amino acid residues 508 to 530; N-glycosylation sites at about amino acid residues 69-73, 96-100, 106-110, 117-121, 385-389, 517-521, 582-586 and 611-615; a tyrosine kinase phosphorylation site at about residues 573-582; and N-myristoylation sites at about amino acid residues 16-22, 224-230, 464-470, 637-643 and 698-704.

A cDNA containing DNA66667 has been deposited with the ATCC under the designation DNA66667 on September 22, 1998 and has been assigned ATCC deposit number 203267.

## EXAMPLE 19

## Isolation of cDNA Clones Encoding Human PRO1375

A Merck/Wash. U. database was searched and a Merck EST was identified. This sequence was then put in a program which aligns it with other sequences from the Swiss-Prot public database, public EST databases (e.g., GenBank, Merck/Wash. U.), and a proprietary EST database (LIFESEQ®. Incyte Pharmaceuticals, Palo Alto, CA). The search was performed using the computer program BLAST or BLAST2 (Altschul et al., Methods in Enzymology, 266:460-480 (1996)] as a comparison of the extracellular domain (ECD) protein sequences to a 6 frame translation of the EST sequences. Those comparisons resulting in a BLAST score of 70 (or in some cases, 90) or greater that did not encode known proteins were clustered and assembled into consensus DNA sequences with the program "phrap" (Phil Green, University of Washington, Seattle, Washington).

A consensus DNA sequence was assembled relative to other EST sequences using phrap. This consensus sequence is designated herein "DNA67003".

Based on theDNA67003 consensus sequence, a nucleic acid was identified in a human pancreas library. DNA sequencing of the clone gave the full-length DNA67004-1614 sequence and the derived protein sequence for PRO1375.

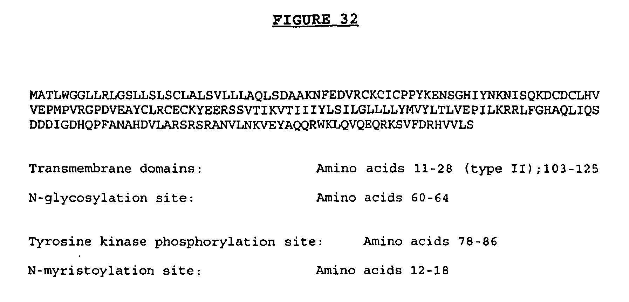

The entire coding sequence of PRO1375 is shown in Figure 31 (SEQ ID NO:31). CloneDNA67004-1614 contains a single open reading frame with an apparent translational initiation site at nucleotide positions 104-106, and an apparent stop codon at nucleotide positions 698-700. The predicted polypeptide precursor is 198 amino acids long and is shown in Figure 32 (SEQ ID NO:32). The transmembrane domains are at about amino acids 11-28 (type II) and 103-125; an N-glycosylation site is at about amino acids 60-64; a tyrosine kinase phosphorylation site is at about amino acids 78-86; and an N-myristoylation site is at about amino acids 12-18. Clone DNA67004-1614 has been deposited with ATCC on August 11, 1998 and is assigned ATCC deposit no. 203115. The full-length PRO1375 protein shown in Figure 32 has an estimated molecular weight of about 22,531 daltons and a pI of about 8.47.

An analysis of the Dayhoff database (version 35.45 SwissProt 35), using a WU-BLAST2 sequence alignment analysis of the full-length sequence shown in Figure 32, revealed sequence identity between the PRO1375 amino acid sequence and the following Dayhoff sequences: AF026198_5, CELR12C12 5, S73465, Y011_MYCPN, S64538_1, P_P8150, MUVSHPO10_1, VSH_MUMPL and CVU59751_5.

EXAMPLE 20

Isolation of cDNA Clones Encoding Human PRO1410

DNA68874-1622 was identified by applying the proprietary signal sequence finding algorithm described in Example 3 above. Use of the above described signal sequence algorithm allowed identification of an EST cluster sequence from the LIFESEQ® database, designated Incyte EST cluster sequence no. 98502. This EST cluster sequence was then compared to a variety of expressed sequence tag (EST) databases which included public EST databases *(e.g.,* GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology, 266:460-480 (1996)). Those comparisons resulting in a BLAST score of 70 (or in some cases, 90) or greater that did not encode known proteins were clustered and assembled into a consensus DNA sequence with the program "phrap" (Phil Green, University of Washington, Seattle, Washington). The consensus sequence obtained therefrom is herein designated as DNA56451.

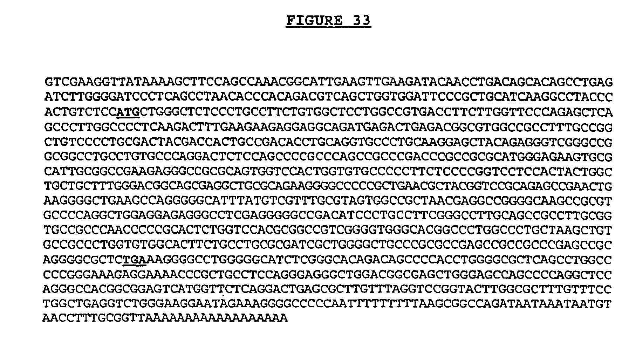

In light of the sequence homology between the DNA56451 sequence and the Incyte EST clone no. 1257046, the Incyte EST clone no. 1257046 was purchased and the cDNA insert was obtained and sequenced. The sequence of this cDNA insert is shown in Figure 33 (SEQ ID NO:33) and is herein designated as DNA68874-1622.

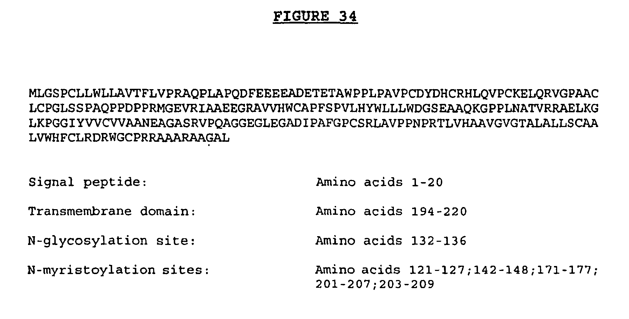

Clone DNA68874-1622 contains a single open reading frame with an apparent translational initiation site at nucleotide positions 152-154 and ending at the stop codon at nucleotide positions 866-868 (Figure 33). The predicted polypeptide precursor is 238 amino acids long (Figure 34; SEQ ID NO:34). The full-length PRO1410 protein shown in Figure 34 has an estimated molecular weight of about 25,262 daltons and a pI of about 6.44. Analysis of the full-length PRO1410 0 sequence shown in Figure 34 (SEQ ID NO:34) evidences the presence of a variety of important polypeptide domains, wherein the locations given for those important polypeptide domains are approximate as described above. Analysis of the full-length PRO1410 sequence shown in Figure 34 evidences the presence of the following: a signal peptide from about amino acid 1 to about amino acid 20; a transmembrane domain from about amino acid 194 to about amino acid 220; a potential N-glycosylation site from about amino acid 132 to about amino acid 136; and N-myristoylation sites from about amino acid 121 to about amino acid 127, from about amino acid 142 to about amino acid 148, from about amino acid 171 to about amino acid 177, from about amino acid 201 to about amino acid 207, and from about amino acid 203 to about amino acid 209. Clone DNA68874-1622 has been deposited with ATCC on September 22. 1998 and is assigned ATCC deposit no. 203277.

An analysis of the Dayhoff database (version 35.45 SwissProt 35), using a WU-BLAST2 sequence alignment analysis of the full-length sequence shown in Figure 34 (SEQ ID NO:34), evidenced significant homology between the PRO1410 amino acid sequence and the following Dayhoff sequences: 148652, P_R76466, HSMHC3W36A_2, EPB4_HUMAN, P_R14256, EPA8_MOUSE, P_R77285, P_W13569, AF000560_1, and ASF1_HELAN.

EXAMPLE 21

Isolation of cDNA Clones Encoding Human PRO1488

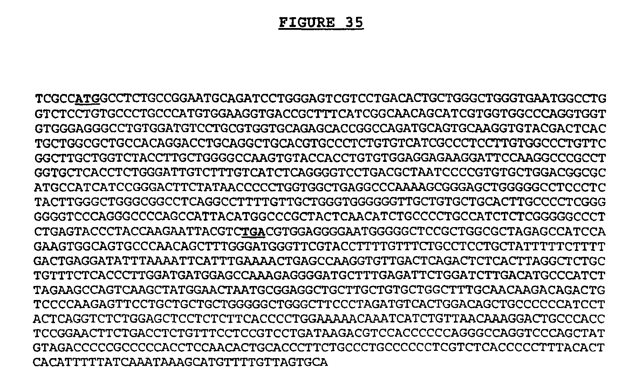

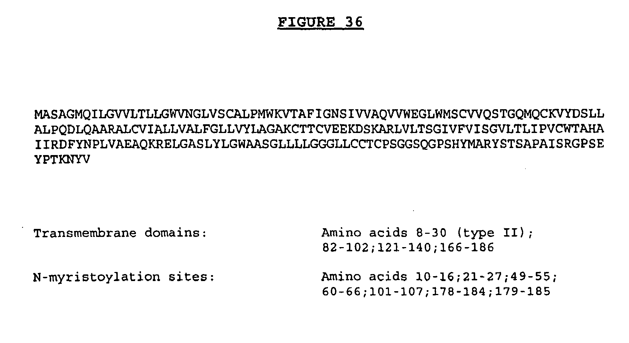

An expressed sequence tag (EST) DNA database (LIFESEQ® Incyte Pharmaceuticals, Palo Alto, CA) was searched and EST No. 3639112H1 was identified as having homology to CPE-R. EST No. 3639112H1 is designated herein as "DNA69562". EST clone 3639112H1, which was derived from a lung tissue library of a 20-week old fetus who died from Patau's syndrome, was purchased and the cDNA insert was obtained and sequenced in its entirety. The entire nucleotide sequence of PRO1488 is shown in Figure 35 (SEQ ID NO:35), and is designated herein as DNA73736-1657. DNA73736-1657 contains a single open reading frame with an apparent translational initiation site at nucleotide positions 6-8 and a stop codon at nucleotide positions 666-668 (Figure 35; SEQ ID NO:35). The predicted polypeptide precursor is 220 amino acids long.
The full-length PRO1488 protein shown in Figure 36 has an estimated molecular weight of about 23,292 daltons and a pI of about 8.43. Four transmembrane domains have been identified as being located at about amino acid positions 8-30, 82-102, 121-140, and 166-186. N-myristoylation sites are at about amino acid positions 10-16. 21-27, 49-55, 60-66, 101-107, 178-184, and 179-185.
An analysis of the Dayhoff database (version 35.45 SwissProt 35), using a WU-BLAST2 sequence alignment analysis of the full-length sequence shown in Figure 36 (SEQ ID NO:36), revealed significant homology between the PRO1488 amino acid sequence and Dayhoff sequence AB000712_1. Homology was also found between the PRO1488 amino acid sequence and the following additional Dayhoff sequences: AB000714_1, AF007189_1, AF000959_1, P_W63697, MMU82758_1, AF072127_1, AF072128_1, HSU89916_1, AF068863_1, CEAF000418_1. and AF077739_ 1.
Clone DNA73736-1657 was deposited with the ATCC on November 17, 1998, and is assigned ATCC deposit no. 203466.

EXAMPLE 22

Isolation of cDNA Clones Encoding Human PRO3438

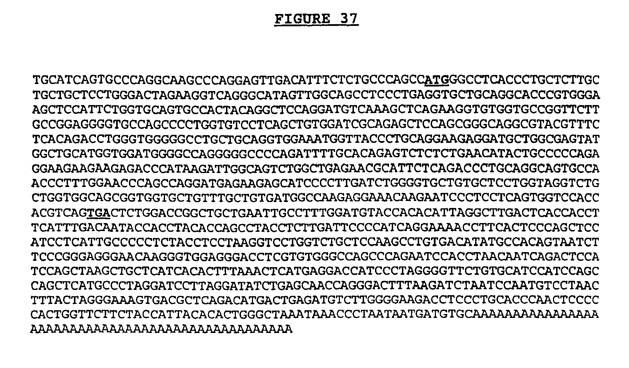

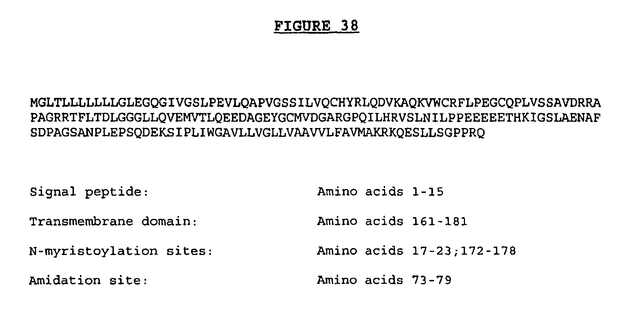

DNA82364-2538 was identified by applying the proprietary signal sequence finding algorithm described in Example 3 above. Use of the above described signal sequence algorithm allowed identification of an EST sequence from the LIFESEQ® database, designated Incyte EST187233H1. This EST sequence was then compared to a variety of expressed sequence tag (EST) databases which included public EST databases (e.g., GenBank) and a proprietary EST DNA database (LIFESEQ®, Incyte Pharmaceuticals, Palo Alto, CA) to identify existing homologies. The homology search was performed using the computer program BLAST or BLAST2 (Altshul et al., Methods in Enzymology, 266: 460-480 (1996)). Those comparisons resulting in a BLAST score of 70 (or in some cases, 90) or greater that did not encode known proteins were clustered and assembled into a consensus DNA sequence with the program "phrap" (Phil Green, University of Washington, Seattle, Washington). The consensus sequence obtained therefrom is herein designated as DNA73888.
In light of the sequence homology between the DNA73888 consensus sequence and the Incyte EST187233H 1, the clone including this EST was purchased and the cDNA insert was obtained and sequenced. The sequence of this cDNA insert is shown in Figure 37 (SEQ ID NO:37) and is herein designated as DNA82364-2538.
Clone DNA82364-2538 contains a single open reading frame with an apparent translational initiation site at nucleotide positions 50-52 and ending at the stop codon at nucleotide positions 647-649 (Figure 37). The predicted polypeptide precursor is 199 amino acids long (Figure 38; SEQ ID NO:38). The full-length PRO3438 protein shown in Figure 38 has an estimated molecular weight of about 21,323 daltons and a pI of about 5.05. Analysis of the full-length PRO3438 sequence shown in Figure 38 (SEQ ID NO:38) evidences the presence of a variety of important polypeptide domains, wherein the locations given for those important polypeptide domains are approximate as described above. Analysis of the full-length PRO3438 sequence shown in Figure 38 evidences the presence of the following: a signal peptide from about amino acid 1 to about amino acid 15; a transmembrane domain from about amino acid 161 to about amino acid 181; N-myristoylation sites from about amino acid 17 to about amino acid 23 and from about amino acid 172 to about amino acid 178; and an amidation site from about amino acid 73 to about amino acid 79. Clone DNA82364-2538 has been deposited with ATCC on January 20, 1999 and is assigned ATCC deposit no. 203603.
An analysis of the Dayhoff database (version 35.45 SwissProt 35), using a WU-BLAST2 sequence alignment analysis of the full-length sequence shown in Figure 38 (SEQ ID NO:38), evidenced homology between the PRO3438 amino acid sequence and the following Dayhoff sequences: S48841, P_W03179, P_W03178, PIGR_HUMAN, HGS_A215, AB001489_1. HGS_B471, P_W61380, P_R15068 and MML1L_1.

EXAMPLE 23

Isolation of cDNA Clones Encoding Human PRO4302

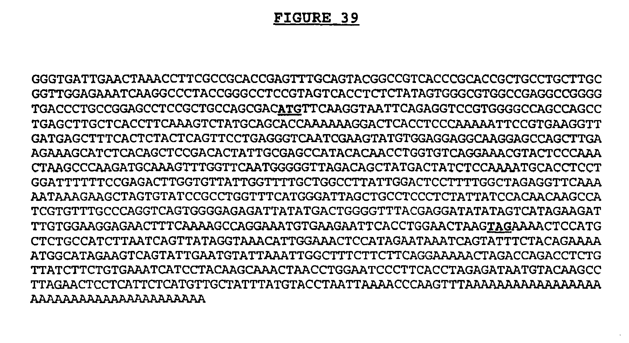

Use of the amylase screen procedure described above in Example 2 on tissue isolated from human tissue resulted in an EST sequence which was then compared against various EST databases to create a consensus sequence by a methodology as described above under the amylase yeast screen procedure and/or the ECD homology procedure. The consensus sequence obtained therefrom is herein designated DNA78875. Based upon an observed homology between the DNA78875 consensus sequence and the Incyte EST no. 2408081H1, Incyte EST no. 2408081 H I was purchased and its insert obtained and sequenced. The sequence of this cDNA insert is shown in Figure 39 (SEQ ID NO:39) and is herein designated as DNA92218-2554 and the derived PRO4302 full-length native sequence protein (SEQ ID NO:40).

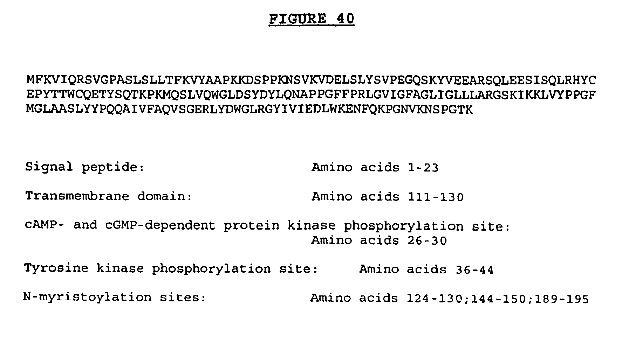

The full length clone DNA92218-2554 (SEQ ID NO:39) shown in Figure 39 has a single open reading frame with an apparent translational initiation site at nucleotide positions 174-176 and a stop signal (TAG) at nucleotide positions 768-770, as indicated by bolded underline. The predicted PRO4302 polypeptide precursor is 198 amino acids long, and has a calculated molecular weight of approximately 22,285 daltons and an estimated pI of approximately 9.35. Analysis of the full-length PRO4302 sequence shown in Figure 40 (SEQ ID NO:40) reveals a signal peptide from about amino acid residue I to about amino acid residue 23; a transmembrane domain from about amino acid residue 111 to about amino acid residue 130; a cAMP- and cGMP-dependent protein kinase phosphorylation site at amino acid residues 26-30; a tyrosine kinase phosphorylation site at amino acid residues 36-44; and N-myristoylation sites at amino acid residues 124-130, 144-150 and 189-195.

A cDNA clone containing DNA92218-2554 was deposited with the ATCC on March 9. 1999 and has been assigned deposit number 203834.

EXAMPLE 24

Isolation of cDNA Clones Encoding Human PRO4400

A consensus DNA sequence was assembled relative to other EST sequences using phrap as described in Example 1 above. The EST databases included public EST databases (e.g., GenBank), and a proprietary EST database (LIFESEQ®, Incyte Pharmaceuticals, Palo Alto, CA) and proprietary ESTs from Genentech. This consensus sequence is designated herein as DNA77634. Based on the DNA77634 consensus sequence, oligonucleotides were synthesized: 1) to identify by PCR a cDNA library that contained the sequence of interest, and 2) for use as probes to isolate a clone of the full-length coding sequence for PRO4400.

A pair of PCR primers (forward and reverse) were synthesized:

forward PCR primer:
5'-GCTGCTGCCGTCCATGCTGATG-3' (SEQ ID NO:81)
reverse PCR primer:
5'-CTCGGGGAATGTGACATCGTCGC-3' (SEQ ID NO:82)

Additionally, a synthetic oligonucleotide hybridization probe was constructed from the consensus DNA77634 sequence which had the following nucleotide sequence:

hybridization probe:
5'-GCTGCCG1'CCATGCTGATGTTTGCGGTGATCGTGG-3' (SEQ ID NO:83)

RNA for construction of the cDNA libraries was isolated from a human adenocarcinoma cell line. The cDNA libraries used to isolate the cDNA clones were constructed by standard methods using commercially available reagents such as those from Invitrogen, San Diego, CA. The cDNA was primed with oligo dT containing a NotI site, linked with blunt to SalI hemikinased adaptors, cleaved with NotI, sized appropriately by gel electrophoresis, and cloned in a defined orientation into a suitable cloning vector (such as pRKB or pRKD; pRK5B is a precursor of pRK5D that does not contain the SfiI site; *see.* Holmes et al., Science, 253: 1278-1280 (1991)) in the unique XhoI and NotI sites.

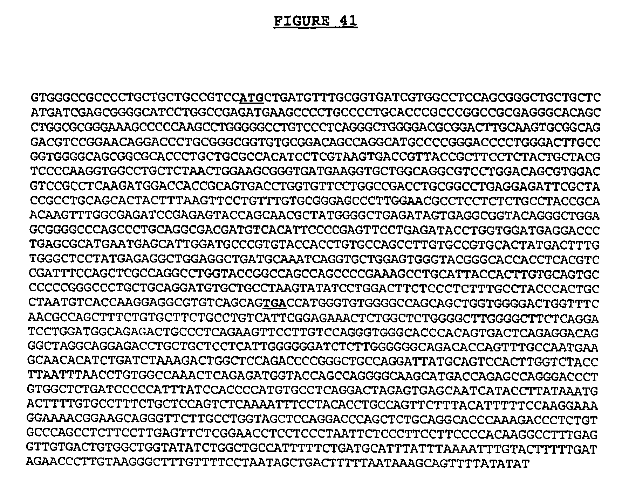

DNA sequencing of the clones isolated as described above gave the full-length DNA sequence for the PRO4400 polypeptide (designated herein as DNA87974-2609 [Figure 41, SEQ ID NO:41]) and the derived protein sequence for that PRO4400 polypeptide.

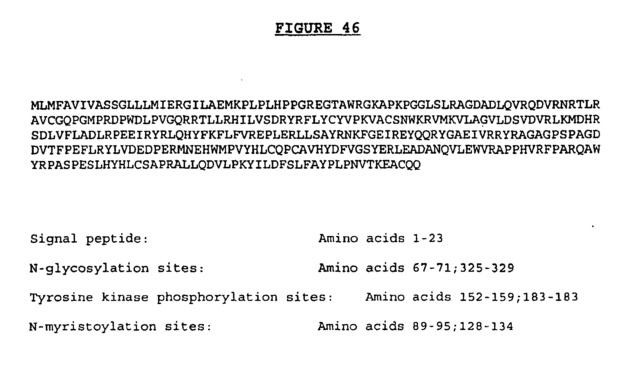

The entire coding sequence of DNA87974-2609 is included in Figure 41 (SEQ ID NO:41). Clone DNA87974-2609 contains a single open reading frame with an apparent translational initiation site at nucleotide positions 27-29, and an apparent stop codon at nucleotide positions 1026-1028. The predicted polypeptide precursor is 333 amino acids long, and has an estimated molecular weight of about 38,618 daltons and a pI of about 9.27. Analysis of the full-length PRO4400 sequence shown in Figure 42 (SEQ ID NO:42) evidences the presence of a variety of important polypeptide domains, wherein the locations given for those important polypeptide domains are approximate as described above. Analysis of the full-length PRO4400 polypeptide shown in Figure 42 evidences the presence of the following: a signal peptide from about amino acid 1 to about amino acid 23; N-gylcosylation sites from about amino acid 67 to about amino acid 71 and from about amino acid 325 to about amino acid 329; tyrosine kinase phosphorylation sites from about amino acid 152 to about amino acid 159 and at about amino acid 183; and N-myristoylation sites from about amino acid 89 to about amino acid 95, and from about amino acid 128 to about amino acid 134. Clone DNA87974-2609 has been deposited with the ATCC on April 27, 1999 and is assigned ATCC deposit no. 203963.
An analysis of the Dayhoff database (version 35.45 SwissProt 35), using a WU-BLAST2 sequence alignment analysis of the full-length sequence shown in Figure 42 (SEQ ID NO:42), evidenced significant homology between the PRO4400 amino acid sequence and the following Dayhoff sequences: AF033827_1, AF070594_1, AF022729_1, CEC34F6_4, SYFB_THETH, G70405, SD_DROME, S64023, ALK1_YEAST and VG04_HSVH.

## EXAMPLE 25

### Isolation of cDNA Clones Encoding Human PRO5725

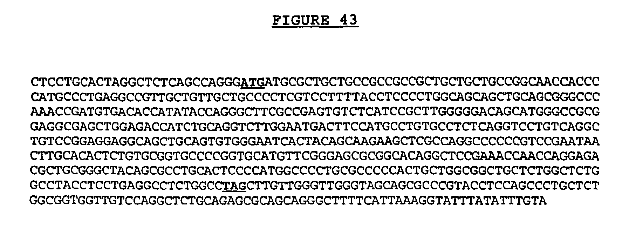

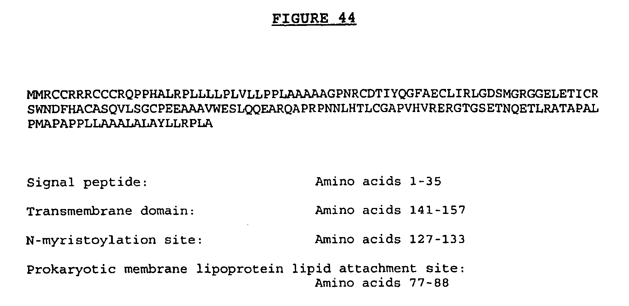

An expressed sequence tag (EST) DNA database (LIFESEQ®, Incyte Pharmaceuticals, Palo Alto, CA) was searched and an EST was identified which showed homology to Neuritin. Incyte ESTclone no. 3705684 was then purchased from LIFESEQ®, Incyte Pharmaceuticals, Palo Alto, CA and the cDNA insert of that clone designated herein as DNA92265-2669 was obtained and sequenced in entirety [Figure 43; SEQ ID NO:43].
The full-length clone DNA92265-2669 (SEQ ID NO:43) contains a single open reading frame with an apparent translational initiation site at nucleotide positions 27-29 and a stop signal at nucleotide positions 522-524 (Figure 43, SEQ ID NO:43). The predicted polypeptide precursor is 165 amino acids long and has a calculated molecular weight of approximately 17,786 daltons and an estimated pI of approximately 8.43. Analysis of the full-length PRO5725 sequence shown in Figure 44 (SEQ ID NO:44) evidences the presence of a variety of important polypeptide domains as shown in Figure 44, wherein the locations given for those important polypeptide domains are approximate as described above. Analysis of the full-length PRO5725 polypeptide shown in Figure 44 evidences the presence of the following: a signal sequence from about amino acid 1 to about amino acid 35; a transmembrane domain from about amino acid 141 to about amino acid 157; an N-myristoylation site from about amino acid 127 to about amino acid 133; and a prokaryotic membrane lipoprotein lipid attachment site from about amino acid 77 to about amino acid 88. Clone DNA92265-2669 has been deposited with ATCC on June 22, 1999 and is assigned ATCC deposit no. PTA-256.
An analysis of the Dayhoff database (version 35.45 SwissProt 35), using a WU-BLAST2 sequence alignment analysis of the full-length sequence shown in Figure 44 (SEQ ID NO:44), evidenced sequence identity between the PRO5725 amino acid sequence and the following Dayhoff sequences: RNU88958_1, P_W37859, P_W37858, JC6305, HGS_RE778, HGS_RE777, P_W27652, P_W44088, HGS_RE776, and HGS_RE425.

## EXAMPLE 26

### *In situ* Hybridization

*In situ* hybridization is a powerful and versatile technique for the detection and localization of nucleic acid sequences within cell or tissue preparations. It may be useful, for example, to identify sites of gene expression, analyze the tissue distribution of transcription, identify and localize viral infection, follow changes in specific mRNA synthesis, and aid in chromosome mapping.
*In situ* hybridization was performed following an optimized version of the protocol by Lu and Gillett, Cell Vision. 1: 169-176 (1994), using PCR-generated $^{33}$P-labeled riboprobes. Briefly, formalin-fixed, paraffin-embedded human tissues were sectioned, deparaffinized, deproteinated in proteinase K (20 g/ml) for 15 minutes at 37 ˚C, and further processed for *in situ* hybridization as described by Lu and Gillett, *supra.* A ($^{33}$-P)UTP-labeled antisense riboprobe was generated from a PCR product and hybridized at 55 ˚C overnight. The slides were dipped in Kodak NTB2™ nuclear track emulsion and exposed for 4 weeks.

### $^{33}$P-Riboprobe synthesis

6.0 μl (125 mCi) of $^{33}$P-UTP (Amersham BF 1002, SA<2000 Ci/mmol were speed-vacuum dried. To each tube containing dried $^{33}$P-UTP, the following ingredients were added:

2.0 μl 5x transcription buffer
1.0μl DTT (100 mM)
2.0 μl NTP mix (2.5 mM: 10 μl each of 10 mM GTP, CTP & ATP + 10μl $H_2O$)
1.0μl UTP (50μM)
1.0 μl RNAsin
1.0 μl DNA template (1 μg)
1.0 μl $H_2O$
1.0 μl RNA polymerase (for PCR products T3 = AS, T7 = S, usually)

The tubes were incubated at 37°C for one hour. A total of 1.0 μl RQ1 DNase was added, followed by incubation at 37 °C for 15 minutes. A total of 90 μl TE (10 mM Tris pH 7.6/1 mM EDTA pH 8.0) was added, and the mixture was pipetted onto DE81 paper. The remaining solution was loaded in a MICROCON-50™ ultrafiltration unit, and spun using program 10 (6 minutes). The filtration unit was inverted over a second tube and spun using program 2 (3 minutes). After the final recovery spin, a total of 100μl TE was added, then 1 μl of the final product was pipetted on DE81 paper and counted in 6 ml of BIOFLUOR II™.
The probe was run on a TBE/urea gel. A total of 1-3 μl of the probe or 5 μl of RNA Mrk III was added to 3 μl of loading buffer. After heating on a 95 °C heat block for three minutes, the gel was immediately placed on ice. The wells of gel were flushed, and the sample was loaded and run at 180-250 volts for 45 minutes. The gel was wrapped in plastic wrap (SARAN™ brand) and exposed to XAR film with an intensifying screen in a -70°C freezer one hour to overnight.

$^{33}$P-Hybridization

A. *Pretreatment of frozen sections*

The slides were removed from the freezer, placed on aluminum trays, and thawed at room temperature for 5 minutes. The trays were placed in a 55°C incubator for five minutes to reduce condensation. The slides were fixed for 10 minutes in 4% paraformaldehyde on ice in the fume hood, and washed in 0.5 x SSC for 5 minutes, at room temperature (25 ml 20 x SSC + 975 ml SQ $H_2O$). After deproteination in 0.5 μg/ml proteinase K for 10 minutes at 37°C (12.5 μl of 10 mg/ml stock in 250 ml prewarmed RNAse-free RNAse buffer), the sections were washed in 0.5 x SSC for 10 minutes at room temperature. The sections were dehydrated in 70%. 95%, and 100% ethanol, 2 minutes each.

B. *Pretreatment ofparaffin-embedded sections*

The slides were deparaffinized, placed in SQ $H_2O$, and rinsed twice in 2 x SSC at room temperature, for 5 minutes each time. The sections were deproteinated in 20 μg/ml proteinase K (500 μl of 10 mg/ml in 250 ml RNase-free RNase buffer; 37°C, 15 minutes) for human embryo tissue, or 8 x proteinase K (100 μl in 250 ml Rnase buffer, 37°C, 30 minutes) for formalin tissues. Subsequent rinsing in 0.5 x SSC and dehydration were performed as described above.

C. *Prehybridization*

The slides were laid out in a plastic box lined with Box buffer (4 x SSC, 50% formamide)-saturated filter paper. The tissue was covered with 50 μl of hybridization buffer (3.75 g dextran sulfate + 6 ml SQ $H_2O$), vortexed, and heated in the microwave for 2 minutes with the cap loosened. After cooling on ice, 18.75 ml formamide, 3.75 ml 20 x SSC, and 9 ml SQ $H_2O$ were added, and the tissue was vortexed well and incubated at 42°C for 1-4 hours.

D. *Hybridization*

1.0 x $10^6$ cpm probe and 1.0 μl tRNA (50 mg/ml stock) per slide were heated at 95°C for 3 minutes. The slides were cooled on ice, and 48 μl hybridization buffer was added per slide. After vortexing, 50 μl $^{33}$P mix was added to 50 μl prehybridization on the slide. The slides were incubated overnight at 55°C.

E. *Washes*

Washing was done for 2x 1 minutes with 2xSSC, EDTA at room temperature (400 ml 20 x SSC + 16 ml 0.25 M EDTA, $V_i$=4L), followed by RNAseA treatment at 37°C for 30 minutes (500 μl of 10 mg/ml in 250 ml Rnase buffer = 20 μg/ml). The slides were washed 2 x 10 minutes with 2 x SSC, EDTA at room temperature. The stringency wash conditions were as follows: 2 hours at 55 °C. 0.1 x SSC, EDTA (20 ml 20 x SSC + 16 ml EDTA, $V_i$=4L).

F. *Oligonucleotides*

*In situ* analysis was performed on four of the DNA sequences disclosed herein. The oligonucleotides employed for these analyses are as follows:

(1) DNA34387-1138 (PRO240) (Jagged/EGF homolog)

Oligo B-231 W 48mer:
5'-GGATTCTAATACGACTCACTATAGGGCCCGAGATATGCACCCAATGTC-3' (SEQ ID NO:84)
Oligo B-231-X 47mer:
5'-CTATGAAATTAACCCTCACTAAAGGGATCCCAGAATCCCGAAGAACA-3' (SEQ ID NO:85)

(2) DNA57708-1411 (PRO1005) (Novel secreted CA associated protein)

678.pl:
5'-GGA TTC TAA TAC GAC TCA CTA TAG GGC CCT CTG TCC ACT GCT TTC GTG-3' (SEQ ID NO:86)
678.p2:
5'-CTA TGA AAT TAA CCC TCA CTA AAG GGA GTT CTC CAC CGT GTC TCC ACA-3'(SEQ ID NO:87)

(3) DNA60764-1533 (PRO1265) (Fig-1 Homolog)

DNA60764-pl:
5'-GGA TTC TAA TAC GAC TCA CTA TAG GGC CGC GCT GTC CTG CTG TCA CCA-3'(SEQ ID NO:88)
DNA60764-p2:
5'-CTA TGA AAT TAA CCC TCA CTA AAG GGA GTT CCC CTC CCC GAG AAG ATA-3' (SEQ ID NO:89)

(4) DNA28498(PRO183) (FHF-2)

DNA28498-pl:
5'-GGA TTC TAA TAC GAC TCA CTA TAG GGC CAG CAA AAG AAG CGG TGG TG-3' (SEQ ID NO:90)
DNA28498-p2:
5'-CTA TGA AAT TAA CCC TCA CTA AAG GGA TTC AGC ACG CCA GAG ACA CTT-3' (SEQ ID NO:91)

G. *Results*

*In situ* analysis was performed on the above four DNA sequences disclosed herein. The results from these analysis are as follows:

(1) DNA34387-1138 (PRO240) (Jagged/EGF Homolog):

*Expression pattern in human adult and fetal tissues*

Elevated signal was observed at the following sites:

Fetal tissues: thyroid epithelium, small intestinal epithelium, gonad, pancreatic epithelium, hepatocytes in liver and renal tubules; expression was also seen in vascular tissue in developing bones.
Adult tissues: moderate signal in placental cytotrophoblast, renal tubular epithelium, bladder epithelium, parathyroid and epithelial tumors.

*Expression in lung adenocarcinoma and squamous carcinoma*

Expression was observed in all eight squamous carcinomas and in six out of eight adenocarcinomas. Expression was seen in *in-situ* and infiltrating components. Expression levels were low to moderate in the adenocarcinomas. In general, expression was higher in the squamous carcinomas and in two of these the expression was strong. No expression was seen in the tumor stroma, alveoli or normal respiratory epithelium. There was possible low level expression in lymph nodes.

(2) DNA57708-1411 (PRO1005) (Novel secreted CA associated protein):

Extremely strong expression was seen over mucus neck cells of gastric fundal (chimp) and antral (human) mucosa. These cells are important in proliferation and mucosal regeneration in the stomach. Focal expression was also seen over fetal hepatocytes and adult hepatocytes at the edge of cirrhotic nodules. Possible expression appeared over skeletal muscle of fetal extra-ocular muscles and the lower limb. No significant expression was observed in any of the 16 primary lung carcinomas (eight squamous and eight adenocarcinomas) that were examined.

(3) DNA60764-1533 (PRO1265) (Fig-1 Homolog)

Fifteen of the sixteen lung tumors examined were suitable for analysis (eight adeno and seven squamous carcinomas). Most of the tumors showed some expression of DNA60764. Expression was largely confined to mononuclear cells adjacent to the infiltrating tumor. In one squamous carcinoma, expression was seen by the malignant epithelium.

Expression was also seen over cells in the fetal thymic medulla of uncertain histogenesis. Expression was seen over mononuclear cells in damaged renal interstitium and in interstitial cells in a renal cell carcinoma. Expression was also seen over cells in a germinal center, consistent with the fact that most Fig- 1 positive cells are probably inflammatory in origin.

(4) DNA28498 (PRO183) (FHF-2)

Expression was observed over the inner aspect of the fetal retina. Strong expression was seen over spinal ganglia and over neurones in the anterior horns of the spinal cord of the human fetus. While significant expression was not observed in the human fetal brain, high expression was seen over neurones in rhesus monkey brain, including the hippocampal neurones. Expression was also observed in the spinal cord and developing hindbrain of the rat embryo.

## EXAMPLE 27

### Use of PRO as a Hybridization Probe

The following method describes use of a nucleotide sequence encoding PRO as a hybridization probe.
DNA comprising the coding sequence of full-length or mature PRO as disclosed herein or a fragment thereof is employed as a probe to screen for homologous DNAs (such as those encoding natural ly-occurring variants of PRO) in human tissue cDNA libraries or human tissue genomic libraries.
Hybridization and washing of filters containing either library DNAs is performed under the following high-stringency conditions. Hybridization of radiolabeled probe derived from the gene encoding a PRO polypeptide to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42˚C for 20 hours. Washing of the filters is performed in an aqueous solution of 0.1 xSSC and 0.1% SDS at 42˚C.
DNAs having a desired sequence identity with the DNA encoding full-length native sequence PRO can then be identified using standard techniques known in the art.

## EXAMPLE 28

### Expression of PRO in *E. coli*

This example illustrates preparation of an unglycosylated form of PRO by recombinant expression in *E. coli.* The DNA sequence encoding PRO is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli; see,* Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a poly-His leader (including the first six STII codons, poly-His sequence, and enterokinase cleavage site), the PRO coding region, lambda transcriptional terminator, and an argU gene.

The ligation mixture is then used to transform a selected *E. coli* strain using the methods described in Sambrook *et al., supra.* Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

Afterculturing the cells for several more hours, the cells can be harvested by centrifugation. The cell pellet obtained by the centrifugation can be solubilized using various agents known in the art, and the solubilized PRO protein can then be purified using a metal chelating column under conditions that allow tight binding of the protein.

PRO may be expressed in *E. coli* in a poly-His tagged form, using the following procedure. The DNA encoding PRO is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an *E. coli* host based on strain 52 (W3110 fuhA(tonA) Ion galE rpoHts(htpRts) clpP(laclq). Transformants are first grown in LB containing 50 mg/ml carbenicillin at 30˚C with shaking until an $OD_{600}$ of 3-5 is reached. Cultures are then diluted 50-100 fold into CRAP media (prepared by mixing 3.57 g $(NH_4)_2SO_4$, 0.71 g sodium citrate•$2H_2O$, 1.07 g KCl, 5.36 g Difco yeast extract, 5.36 g Sheffield hycase SF in 500 ml water, as well as 110 mM MPOS, pH 7.3, 0.55% (w/v) glucose and 7 mM $MgSO_4$) and grown for approximately 20-30 hours at 30˚C with shaking. Samples are removed to verify expression by SDS-PAGE analysis, and the bulk culture is centrifuged to pellet the cells. Cell pellets are frozen until purification and refolding.

*E. coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1M and 0.02 M, respectively, and the solution is stirred overnight at 4˚C. This step results in a denatured protein with all cysteine residues blocked by sulfitolization. The solution is centrifuged at 40.000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen $Ni^{2+}$-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4˚C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4˚C for 12-36 hours. The refolding reaction is quenched by the addition of TFA to a final concentration of 0.4% (pH of approximately 3). Before further purification of the protein, the solution is filtered through a 0.22 micron filter and acetonitrile is added to 2-10% final concentration. The refolded protein is chromatographed on a Poros R1/H reversed phase column using a mobile buffer of 0.1% TFA with elution with a gradient of acetonitrile from 10 to 80%. Aliquots of fractions with $A_{280}$ absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

Many of the PRO polypeptides disclosed herein were successfully expressed as descibed above.

EXAMPLE 29

Expression of PRO in mammalian cells

This example illustrates preparation of a potentially glycosylated form of PRO by recombinant expression in mammalian cells.

The vector, pRK5 *(see* EP 307,247, published March 15, 1989), is employed as the expression vector. Optionally, the PRO DNA is ligated into pRK5 with selected restriction enzymes to allow insertion of the PRO DNA using ligation methods such as described in Sambrook *et al., supra.* The resulting vector is called pRK5-PRO.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 μg pRK5-PRO DNA is mixed with about 1 μg DNA encoding the VA RNA gene [Thimmappaya et al., Cell, 31:543 (1982)] and dissolved in 500 μl of 1 mM Tris-HCl. 0.1 mM EDTA, 0.227 M $CaCl_2$. To this mixture is added, dropwise, 500 μl of 50 mM HEPES (pH 7.35), 280 mM NaCl, 1.5 mM $NaPO_4$, and a precipitate is allowed to form for 10 minutes at 25˚C. The precipitate is suspended and added to the 293 cells and allowed to settle for about four hours at 37˚C. The culture medium is aspirated off and 2 ml of 20% glycerol in PBS is added for 30 seconds. The 293 cells are then washed with serum free medium, fresh medium is added and the cells are incubated for about 5 days. Approximately 24 hours after the transfections, the culture medium is removed and replaced with culture medium (alone) or culture medium containing 200 μCi/ml $^{35}$S-cysteine and 200 μCi/ml $^{35}$S-methionine. After a 12 hour incubation, the conditioned medium is collected, concentrated on a spin filter, and loaded onto a 15% SDS gel. The processed gel may be dried and exposed to film for a selected period of time to reveal the presence of the PROpolypeptide. The cultures containing transfected cells may undergo further incubation (in serum free medium) and the medium is tested in selected bioassays.

In an alternative technique, PRO may be introduced into 293 cells transiently using the dextran sulfate method described by Somparyrac et al., Proc. Natl. Acad. Sci., 12:7575 (1981). 293 cells are grown to maximal density in a spinner flask and 700 μg pRK5-PRO DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and reintroduced into the spinner flask containing tissue culture medium, 5 μg/ml bovine insulin and 0.1 μg/ml bovine transferrin. After about four days, the conditioned media is centrifuged and filtered to remove cells and debris. The sample containing expressed PRO can then be concentrated and purified by any selected method, such as dialysis and/or column chromatography.

In another embodiment, PRO can be expressed in CHO cells. The pRK5-PRO can be transfected into CHO cells using known reagents such as $CaPO_4$ or DEAE-dextran. As described above, the cell cultures can be incubated, and the medium replaced with culture medium (alone) or medium containing a radiolabel such as ''S-methionine. After determining the presence of a PRO polypeptide, the culture medium may be replaced with serum free medium. Preferably, the cultures are incubated for about 6 days, and then the conditioned medium is harvested. The medium containing the expressed PRO polypeptide can then be concentrated and purified by any selected method.

Epitope-tagged PRO may also be expressed in host CHO cells. The PRO may be subcloned out of the pRK5 vector. The subclone insert can undergo PCR to fuse in frame with a selected epitope tag such as a poly-His tag into a Baculovirus expression vector. The poly-His tagged PRO insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged PRO can then be concentrated and purified by any selected method, such as by $Ni^{2+}$-chelate affinity chromatography.

PRO may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (e.g., extracellular domains) of the respective proteins are fused to an 1gGl constant region sequence containing the hinge, CH2 and CH2 domains and/or as a poly-His tagged form.

Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used in expression in CHO cells is as described in Lucas et al., Nucl. Acids Res., 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect® (Qiagen), Dosper® or Fugene® (Boehringer Mannheim). The cells are grown as described in Lucas *et al., supra.* Approximately 3 x $10^7$ cells are frozen in an ampule for further growth and production as described below.

The ampules containing the plasmid DNA are thawed by placement into a water bath and mixed by vortexing. The contents are pipetted into a centrifuge tube containing 10 ml of media and centrifuged at 1000 rpm for 5 minutes. The supernatant is aspirated and the cells are resuspended in 10 ml of selective media (0.2 μm filtered PS20 with 5% 0.2 μm diafiltered fetal bovine serum). The cells are then aliquoted into a 100 ml spinner containing 90 ml of selective media. After 1-2 days, the cells are transferred into a 250 ml spinner filled with 150 ml selective growth medium and incubated at 37˚C. After another 2-3 days, 250 ml, 500 ml and 2000 ml spinners are seeded with 3 x $10^5$ cells/ml. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x $10^6$ cells/ml. On day 0, the cell number and pH is determined. On day 1, the spinner is sampled and sparging with filtered air is commenced. On day 2, the spinner is sampled, the temperature shifted to 33˚C, and 30 ml of 500 g/L glucose and 0.6 ml of 10% antifoam *(e.g.,* 35% polydimethylsiloxane emulsion. Dow Coming 365 Medical Grade Emulsion) taken. Throughout the production, the pH is adjusted as necessary to keep it at around 7.2. After 10 days, or until the viability drops below 70%, the cell culture is harvested by centrifugation and filtering through a 0.22 μm filter. The filtrate is either stored at 4˚C or immediately loaded onto columns for purification.

For the poly-His tagged constructs, the proteins are purified using a Ni $^{2+}$-NTA column (Qiagen). Before purification, imidazole is added to the conditioned media to a concentration of 5 mM. The conditioned media is pumped onto a 6 ml Ni $^{2+}$-NTA column equilibrated in 20 mM Hepes, pH 7.4. buffer containing 0.3 M NaCl and 5 mM imidazole at a flow rate of 4-5 ml/min. at 4˚C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80˚C.

Immunoadhesin (Fc-containing) constructs are purified from the conditioned media as follows. The conditioned medium is pumped onto a 5 ml Protein A column (Pharmacia) which has been equilibrated in 20 mM Na phosphate buffer, pH 6.8. After loading, the column is washed extensively with equilibration buffer before elution with 100 mM citric acid, pH 3.5. The eluted protein is immediately neutralized by collecting I ml fractions into tubes containing 275 μl of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

Many of the PRO polypeptides disclosed herein were successfully expressed as descibed above.

## EXAMPLE 30

## Expression of PRO in Yeast

The following method describes recombinant expression of PRO in yeast.

First, yeast expression vectors are constructed for intracellular production or secretion of PRO from the ADH2/GAPDH promoter. DNA encoding PRO and the promoter is inserted into suitable restriction enzyme sites in the selected plasmid to direct intracellular expression of PRO. For secretion, DNA encoding PRO can be cloned into the selected plasmid, together with DNA encoding the ADH2/GAPDH promoter, a native PRO signal peptide or other mammalian signal peptide, or, for example, a yeast alpha-factor or invertase secretory signal/leader sequence, and linker sequences (if needed) for expression of PRO.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO can subsequently be isolated and purified by removing the yeast cells from the fermentation medium by centrifugation and then concentrating the medium using selected cartridge filters. The concentrate containing PRO may further be purified using selected column chromatography resins.

Many of the PRO polypeptides disclosed herein were successfully expressed as described above.

## EXAMPLE 31

## Expression of PRO in Baculovirus-Infected Insect Cells

The following method describes recombinant expression in Baculovirus-infected insect cells.

The sequence coding for PRO is fused upstream of an epitope tag contained within a baculovirus expression vector.

Such epitope tags include poly-His tags and immunoglobulin tags (like Fc regions of IgG). A variety of plasmids may be employed, including plasmids derived from commercially available plasmids such as pVL1393 (Novagen). Briefly, the sequence encoding PRO or the desired portion of the coding sequence of PRO (such as the sequence encoding the extracellular domain of a transmembrane protein or the sequence encoding the mature protein if the protein is extracellular) is amplified by PCR with primers complementary to the 5' and 3' regions. The 5' primer may incorporate flanking (selected) restriction enzyme sites. The product is then digested with those selected restriction enzymes and subcloned into the expression vector.
Recombinant baculovirus is generated by co-transfecting the above plasmid and BaculoGold™ virus DNA (Pharmingen) into *Spodoptera frugiperda* ("Sf9") cells (ATCCCRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).
Expressed poly-His tagged PRO can then be purified, for example, by $Ni^{2+}$-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 ml Hepes, pH 7.9; 12.5 mM $MgCl_2$; 0.1 mM EDTA; 10% glycerol; 0.1% NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 μm filter. A $Ni^{2+}$-NTA agarose column (commercially available from Qiagen) is prepared with a bed volume of 5 ml, washed with 25 ml of water and equilibrated with 25 ml of loading buffer. The filtered cell extract is loaded onto the column at 0.5 ml per minute. The column is washed to baseline $A_{280}$ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching $A_{280}$ baseline again, the column is developed with a 0 to 500 mM imidazole gradient in the secondary wash buffer. One ml fractions are collected and analyzed by SDS-PAGE and silver staining or Western blot with $Ni^{2+}$-NTA-conjugated to alkaline phosphatase (Qiagen). Fractions containing the eluted $His_{10}$-tagged PRO are pooled and dialyzed against loading buffer.
Alternatively, purification of the IgG tagged (or Fc tagged) PRO can be performed using known chromatography techniques, including for instance, Protein A or protein G column chromatography.
Many of the PRO polypeptides disclosed herein were successfully expressed as described above.

## EXAMPLE 32

### Preparation of Antibodies that Bind PRO

This example illustrates preparation of monoclonal antibodies which can specifically bind PRO.
Techniques for producing the monoclonal antibodies are known in the art and are described, for instance, in Goding, *supra.* Immunogens that may be employed include purified PRO. fusion proteins containing PRO. and cells expressing recombinant PRO on the cell surface. Selection of the immunogen can be made by the skilled artisan without undue experimentation.
Mice, such as Balb/c, are immunized with the PRO immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO antibodies.
After a suitable antibody titer has been detected, the animals "positive" for antibodies can be injected with a final intravenous injection of PRO. Three to four days later, the mice are sacrificed and the spleen cells are harvested. The spleen cells are then fused (using 35% polyethylene glycol) to a selected murine myeloma cell line such as P3X63AgU. 1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.
The hybridoma cells will be screened in an ELISA for reactivity against PRO. Determination of "positive" hybridoma cells secreting the desired monoclonal antibodies against PRO is within the skill in the art.
The positive hybridoma cells can be injected intraperitoneally into syngeneic Balb/c mice to produce ascites containing the anti-PRO monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

EXAMPLE 33

Purification of PRO Polypeptides Using Specific Antibodies

Native or recombinant PRO polypeptides may be purified by a variety of standard techniques in the art of protein purification. For example, pro-PRO polypeptide, mature PRO polypeptide, or pre-PRO polypeptide is purified by immunoaffinity chromatography using antibodies specific for the PRO polypeptide of interest. In general, an immunoaffinity column is constructed by covalently coupling the anti-PRO polypeptide antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE™ (Pharmacia LKB Biotechnology). The antibody is coupled to the resin, the resin is blocked, and the derivative resin is washed according to the manufacturer's instructions.

Such an immunoaffinity column is utilized in the purification of the PRO polypeptide by preparing a fraction from cells containing the PRO polypeptide in a soluble form. This preparation is derived by-solubilization of the whole cell or of a subcellular fraction obtained via differential centrifugation by the addition of detergent or by other methods well known in the art. Alternatively, soluble PRO polypeptide containing a signal sequence may be secreted in useful quantity into the medium in which the cells are grown.

A soluble PRO polypeptide-containing preparation is passed over the immunoaffinity column, and the column is washed under conditions that allow the preferential absorbance of the PRO polypeptide *(e.g.,* high ionic strength buffers in the presence of detergent). Then, the column is eluted under conditions that disrupt antibody/PRO polypeptide binding (e.g., a low pH buffer such as approximately pH 2-3, or a high concentration of a chaotrope such as urea or thiocyanate ion), and the PRO polypeptide is collected.

EXAMPLE 34

Drug Screening

This invention is particularly useful for screening compounds by using PRO polypeptides or a binding fragment thereof in any of a variety of drug screening techniques. The PRO polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PRO polypeptide or fragment. Drugs are screened against such transformed cells in competitive binding assays. Such cells, either in viable or fixed form, can be used for standard binding assays. One may measure, for example, the formation of complexes between a PRO polypeptide or a fragment and the agent being tested. Alternatively, one can examine the diminution in complex formation between the PRO polypeptide and its target cell or target receptors caused by the agent being tested.

Thus, the present invention provides methods of screening for drugs or any other agents which can affect a PRO polypeptide-associated disease or disorder. These methods comprise contacting such an agent with a PRO polypeptide or fragment thereof and assaying (i) for the presence of a complex between the agent and the PRO polypeptide or fragment, or (ii) for the presence of a complex between the PRO polypeptide or fragment and the cell, by methods well known in the art. In such competitive binding assays, the PRO polypeptide or fragment is typically labeled. After suitable incubation, the free PRO polypeptide or fragment is separated from that present in bound form, and the amount of free or uncomplexed label is a measure of the ability of the particular agent to bind to the PRO polypeptide or to interfere with the PRO polypeptide/cell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PRO polypeptide, the peptide test compounds are reacted with the PRO polypeptide and washed. Bound PRO polypeptide is detected by methods well known in the art. Purified PRO polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable of binding a PRO polypeptide specifically compete with a test compound for binding to the PRO polypeptide or fragments thereof. In this manner, the antibodies can be used to detect the presence of any peptide which shares one or more antigenic determinants with a PRO polypeptide.

EXAMPLE 35

Rational Drug Design

The goal of rational drug design is to produce structural analogs of a biologically active polypeptide of interest (i.e., a PRO polypeptide) or of small molecules with which they interact, *e.g.*, agonists, antagonists, or inhibitors. Any of these examples can be used to fashion drugs which are more active or stable forms of the PRO polypeptide or which enhance or interfere with the function of the PRO polypeptide *in vivo* (*c.f.*, Hodgson, Bio/Technology, 9: 19-21 (1991)).
In one approach, the three-dimensional structure of the PRO polypeptide, or of a PRO polypeptide-inhibitor complex, is determined by x-ray crystallography, by computer modeling or, most typically, by a combination of the two approaches. Both the shape and charges of the PRO polypeptide must be ascertained to elucidate the structure and to determine active site(s) of the molecule. Less often, useful information regarding the structure of the PRO polypeptide may be gained by modeling based on the structure of homologous proteins. In both cases, relevant structural information is used to design analogous PRO polypeptide-like molecules or to identify efficient inhibitors. Useful examples of rational drug design may include molecules which have improved activity or stability as shown by Braxton and Wells, Biochemistry. 31:7796-7801 (1992) or which act as inhibitors, agonists, or antagonists of native peptides as shown by Athauda et al., J. Biochem., 113:742-746 (1993).
It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.
By virtue of the present invention, sufficient amounts of the PRO polypeptide may be made available to perform such analytical studies as X-ray crystallography. In addition, knowledge of the PRO polypeptide amino acid sequence provided herein will provide guidance to those employing computer modeling techniques in place of or in addition to x-ray crystallography.

EXAMPLE 36

*In Vitro* Antitumor Assay

The antiproliferative activity of the PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO4400, PRO5725, PRO183, PRO202, PRO542, PRO861, PRO1096 or PRO3562 polypeptide was determined in the investigational, disease-oriented *in vitro* anti-cancer drug discovery assay of the National Cancer Institute (NCI), using a sulforhodamine B (SRB) dye binding assay essentially as described by Skehan et al., J. Natl. Cancer Inst., 82:1107-1112 (1990). The 60 tumor cell lines employed in this study ("the NCI panel"), as well as conditions for their maintenance and culture *in vitro* have been described by Monks et al., J. Natl. Cancer Inst., 83:757-766 (1991). The purpose of this screen is to initially evaluate the cytotoxic and/or cytostatic activity of the test compounds against different types of tumors (Monks et al., supra: Boyd, Cancer: Princ. Pract. Oncol. Update, 3(10): 1-12 [1989]).
Cells from approximately 60 human tumor cell lines were harvested with trypsin/EDTA (Gibco), washed once, resuspended in IMEM and their viability was determined. The cell suspensions were added by pipet (100 μl volume) into separate 96-well microtiter plates. The cell density for the 6-day incubation was less than for the 2-day incubation to prevent overgrowth. Inoculates were allowed a preincubation period of 24 hours at 37°C for stabilization. Dilutions at twice the intended test concentration were added at time zero in 100 μl aliquots to the microtiter plate wells (1:2 dilution). Test compounds were evaluated at five half-log dilutions (1000 to 100,000-fold). Incubations took place for two days and six days in a 5% $CO_2$ atmosphere and 100% humidity.
After incubation, the medium was removed and the cells were fixed in 0.1 ml of 10% trichloroacetic acid at 40°C. The plates were rinsed five times with deionized water, dried, stained for 30 minutes with 0.1 ml of 0.4% sulforhodamine B dye (Sigma) dissolved in 1% acetic acid, rinsed four times with 1% acetic acid to remove unbound dye, dried, and the stain was extracted for five minutes with 0.1 ml of 10 mM Tris base [tris(hydroxymethyl)aminomethane], pH 10.5. The absorbance (OD) of sulforhodamine B at 492 nm was measured using a computer-interfaced, 96-well microtiter plate reader.
A test sample is considered positive if it shows at least 40% growth inhibitory effect at one or more concentrations. The results are shown in the following Table 7, where the tumor cell type abbreviations are as follows:
NSCL = non-small cell lung carcinoma; CNS = central nervous system

Table 7

| Test-compound | Days | Tumor Cell Line Type | Cell Line Designation |
|---|---|---|---|
| PRO240 | 6 | Leukemia | MOLT-4; RPMI-8226 |
| PRO240 | 6 | Colon Cancer | COLO 205; HCT-116; KM12 |
| PRO240 | 6 | Breast Cancer | MDA-MB-435 |
| PRO240 | 6 | NSCL | NCI-H322M; HOP-92 |
| PRO240 | 6 | Prostate Cancer | DU-145 |
| PRO240 | 6 | CNS-Cancer | SNB-75 |
| PRO240 | 2 | Leukemia | RPM1-8226 |
| PRO240 | 2 | NSCL | HOP92 |
| PRO240 | 2 | CNS Cancer | SF-539 |
| PRO240 | 2 | Breast Cancer | NCI/ADR-RES* |
| PRO240 | 2 | Leukemia | HL-60(TB) |
| PRO240 | 2 | Breast Cancer | MDA-MB-231/ATCC |
| PRO240 | N/A | Leukemia | HL-60(TB); MOLT-4 |
| PRO240 | N/A | Leukemia | RPMI-8226 |
| PRO381 | N/A | NSCL | A549/ATCC; EKVX; HOP-62* |
| PRO381 | N/A | NSCL | NCI-H226*: NCI-H23 |
| PRO381 | N/A | NSCL | NCI-H322M; NCI-H460* |
| PRO381 | N/A | NSCL | NCI-H522* |
| PRO381 | N/A | NSCL | HOP-92* |
| PRO381 | N/A | Colon Cancer | COLO 205*; HCC-2998* |
| PRO381 | N/A | Colon Cancer | HCT-116; HCT-15; HT29 |
| PRO381 | N/A | Colon Cancer | SW620; KM12 |
| PRO381 | N/A | Breast Cancer | BT-549*; HS 578T*; MCF7 |
| PRO381 | N/A | Breast Cancer | MDA-MB-435; MDA-N |
| PRO381 | N/A | Breast Cancer | T-47D: MDA-MB-231/ATCC |
| PRO381 | N/A | Ovarian Cancer | IGROVI*; OVCAR-3 |
| PRO381 | N/A | Ovarian Cancer | OVCAR-5*; OVCAR-8 |
| PRO381 | N/A | Ovarian Cancer | SK-OV-3; OVCAR-4 |
| PRO381 | N/A | Leukemia | CCRF-CEM*; HL-60(TB)* |
| PRO381 | N/A | Leukemia | K-562; MOLT-4 |
| PRO381 | N/A | Renal Cancer | 786-0*; A498; ACHN; CAKI- |
| PRO381 | N/A | Renal Cancer | RXF 393; SN 12C; TK-10* |
| PRO381 | N/A | Renal Cancer | UO-31* |
| PRO381 | N/A | Melanoma | LOX IMVI*; M14 |
| PRO381 | N/A | Melanoma | MALME-3M; UACC-62* |
| PRO381 | N/A | Melanoma | SK-MEL-2; SK-MEL-28 |
| PRO381 | N/A | Melanoma | SK-MEL-5; UACC-257 |
| PRO381 | N/A | Prostate Cancer | DU-145; PC-3* |

(continued)

| Test-compound | Days | Tumor Cell Line Type | Cell Line Designation |
|---|---|---|---|
| PRO381 | N/A | CNS Cancer | SF-268; SNB- 19*; U251* |
| PRO381 | N/A | CNS Cancer | SF-295; S-539; SNB-75 |
| PRO534 | N/A | NSCL | A549/ATCC: HOP-62; HOP-92 |
| PRO534 | N/A | NSCL | NCI-H23; NCI-H322M |
| PRO534 | N/A | NSCL | NCI-H460; NCI-H522 |
| PRO534 | N/A | NSCL | NCI-H266 |
| PRO534 | N/A | Colon Cancer | HCT-116; HT29; KM 12 |
| PRO534 | N/A | Colon Cancer | SW620 |
| PRO534 | N/A | Breast Cancer | BT-549; HS 578T; MCF7 |
| PRO534 | N/A | Breast Cancer | MDA-MB-231/ATCC |
| PRO534 | N/A | Breast Cancer | MDA-MB-435: MDA-N |
| PRO534 | N/A | Breast Cancer | T-47D |
| PRO534 | N/A | Ovarian Cancer | IGROVI; OVCAR-3* |
| PRO534 | N/A | Ovarian Cancer | OVCAR-8 |
| PRO534 | N/A | Leukemia | HL-60(TB); K-562; MOLT-4* |
| PRO534 | N/A | Leukemia | RPMI-8226*; CCRF-CEM |
| PRO534 | N/A | Renal Cancer | 786-0; A498: ACHN; RXF 393 |
| PRO534 | N/A | Renal Cancer | SN 12C; TK-10 |
| PRO534 | N/A | Melanoma | LOX IMVI; M 14; SK-MEL-28 |
| PRO534 | N/A | Melanoma | SK-MEL-2; UACC-257 |
| PRO534 | N/A | Melanoma | UACC-62 |
| PRO534 | N/A | Prostate Cancer | DU-145 |
| PRO534 | N/A | CNS Cancer | SF-268; SF-295; SNB-19 |
| PRO534 | N/A | CNS Cancer | SNB-75 |
| PRO540 | 6 | Leukemia | HL-60(TB) |
| PRO540 | 6 | Colon Cancer | KM12 |
| PRO540 | 6 | CNS Cancer | SF-295 |
| PRO540 | 6 | Melanoma | LOX IMVI |
| PRO540 | 6 | Ovarian Cancer | SK-OV-3 |
| PRO540 | 6 | Renal Cancer | 786-0; CAKI-1; UO-31; TK-10 |
| PRO540 | 2 | NSCL | HOP-62; HOP-92; NCI-H460 |
| PRO540 | 2 | Colon Cancer | HCC-2998 |
| PRO540 | 2 | CNS Cancer | SF-295; SN-75 |
| PRO540 | 2 | Ovarian Cancer | SK-OV-3 |
| PRO698 | N/A | NSCL | EKVX; HOP-62; NCI-H322M |
| PRO698 | N/A | NSCL | NCI-H522 |
| PRO698 | N/A | Colon Cancer | HCT-116 |

(continued)

| Test-compound | Days | Tumor Cell Line Type | Cell Line Designation |
|---|---|---|---|
| PRO698 | N/A | Breast Cancer | MDA-MB-231/ATCC |
| PRO698 | N/A | Breast Cancer | MDA-MB-435; MDA-N |
| PRO698 | N/A | Ovarian Cancer | OVCAR-3; OVCAR-1 |
| PRO698 | N/A | Ovarian Cancer | OVCAR-5; OVCAR-8 |
| PRO698 | N/A | Ovarian Cancer | SK-OV-3 |
| PRO698 | N/A | Renal Cancer | ACHN; RXF 393; SN 12C |
| PRO698 | N/A | Renal Cancer | TK-10 |
| PRO698 | N/A | CNS Cancer | SF-268; SF-295; SNB-19 |
| PRO698 | N/A | CNS Cancer | SNB-75*: U251 |
| PRO982 | 6 | NSCL | HOP-62 |
| PRO982 | 6 | Leukemia | CCRF-CEM; RPMI-8226 |
| PRO982 | 6 | Melanoma | LOX IMVI |
| PRO982 | N/A | NSCL | HOP-92; NCI-H522 |
| PRO982 | N/A | Colon Cancer | COLO 205 |
| PRO982 | N/A | Breast Cancer | BT-549; MDA-MB-231/ATCC |
| PRO982 | N/A | Ovarian Cancer | IGROVI; OVCAR-5 |
| PRO982 | N/A | Leukemia | MOLT-4; RPMI-8226 |
| PRO982 | N/A | Renal Cancer | 786-0; CAKI-1; RXF 393 |
| PRO982 | N/A | Renal Cancer | TK-10 |
| PRO982 | N/A | Prostate Cancer | PC-3 |
| PRO982 | N/A | CNS Cancer | SNB-19; U251 |
| PRO1005 | N/A | NSCL | A549/ATCC |
| PRO1005 | N/A | Renal Cancer | TK-10 |
| PRO1005 | N/A | CNS Cancer | SNB-19 |
| PRO1007 | 6 | Leukemia | CCRF-CEM |
| PRO1007 | 6 | Colon Cancer | HCT-116; KM 12 |
| PRO1007 | N/A | NSCL | NCI-H522 |
| PRO1007 | N/A | Colon Cancer | KM12 |
| PRO1007 | N/A | Breast Cancer | HS 578T |
| PRO1007 | N/A | Breast Cancer | MDA-MB-231/ATCC |

(continued)

| Test-compound | Days | Tumor Cell Line Type | Cell Line Designation |
|---|---|---|---|
| PRO1007 | N/A | Ovarian Cancer | IGROVI |
| PRO1007 | N/A | Leukemia | RPMI-8226 |
| PRO1007 | N/A | Melanoma | SK-MEL-628 |
| PRO1131 | N/A | Leukemia | MOLT-4; CCRF-CEM |
| PRO1131 | N/A | NSCL | HOP-62; NCI-H23; NCI-H522 |
| PRO1131 | N/A | Breast Cancer | MCF7; MDA-MB-231/ATCC |
| PRO1131 | N/A | Ovarian Cancer | OVCAR-3; OVCAR-4 |
| PRO1131 | N/A | Ovarian Cancer | OVCAR-8 |
| PRO1131 | N/A | Melanoma | LOX IMVI; UACC-257 |
| PRO1131 | N/A | CNS Cancer | SNB-19; U251 |
| PRO1157 | N/A | NSCL | A549/ATCC; HOP-62* |
| PRO1157 | N/A | NSCL | HOP-92*; NCI-H23 |
| PRO1157 | N/A | NSCL | NCI-H322M; NCI-H522* |
| PRO1157 | N/A | NSCL | NCI-H226 |
| PRO1157 | N/A | Colon Cancer | COLO 205; HCT-116; HCT-15 |
| PRO1157 | N/A | Colon Cancer | HT29; KM12*; SW620 |
| PRO1157 | N/A | Breast Cancer | BT-549*; HS 578T; MCF7 |
| PRO1157 | N/A | Breast Cancer | MDA-MB-231/ATCC* |
| PRO1157 | N/A | Breast Cancer | MDA-MB-435; MDA-N |
| PRO1157 | N/A | Ovarian Cancer | IGROVI; OVCAR-3* |
| PRO1157 | N/A | Ovarian Cancer | OVCAR-5*; OVCAR-8 |
| PRO1157 | N/A | Ovarian Cancer | SK-OV-3 |
| PRO1157 | N/A | Leukemia | HL-60(TB); K-562; MOLT-4 |
| PRO 1157 | N/A | Leukemia | RPMI-8226; CCRF-CEM; SR |
| PRO1157 | N/A | Renal Cancer | 786-0*; A498; ACHN |
| PRO1157 | N/A | Renal Cancer | CAKI-1 *; RXF 393*; TK-10* |
| PRO1157 | N/A | Renal Cancer | UO-31 * |
| PRO1157 | N/A | Melanoma | LOX IMVI; M14 |
| PRO1157 | N/A | Melanoma | MALME-3M; SK-MEL-28 |
| PRO1157 | N/A | Melanoma | SK-MEL-2; SK-MEL-5 |
| PRO1157 | N/A | Melanoma | UACC-257; UACC-62 |
| PRO1157 | N/A | Prostate Cancer | DU-145; PC-3* |

(continued)

| Test-compound | Days | Tumor Cell Line Type | Cell Line Designation |
|---|---|---|---|
| PRO1157 | N/A | CNS Cancer | SF-268; SF-295; S-539* |
| PRO1157 | N/A | CNS Cancer | SNB-19; SNB-75*; U251* |
| PRO1199 | N/A | NSCL | A549/ATCC; HOP-62 |
| PRO1199 | N/A | NSCL | HOP-92; NCI-H23 |
| PRO1199 | N/A | NSCL | NCI-H322M; NCI-H522 |
| PRO1199 | N/A | Colon Cancer | HCC-2998*; SW620 |
| PRO1199 | N/A | Breast Cancer | HS 578T; MCF7 |
| PRO1199 | N/A | Breast Cancer | MDA-MB-435; MDA-N |
| PRO1 199 | N/A | Breast Cancer | T-47D |
| PRO1199 | N/A | Ovarian Cancer | IGROVI; OVCAR-3 |
| PRO1199 | N/A | Ovarian Cancer | OVCAR-4; OVCAR-5 |
| PRO1199 | N/A | Ovarian Cancer | OVCAR-8 |
| PRO1199 | N/A | Leukemia | CCRF-CEM*; RPMI-8226* |
| PRO1199 | N/A | Renal Cancer | RXF 393; SN 12C; UO-31 |
| PRO1199 | N/A | Melanoma | LOX IMVI; M14; SK-MEL-28 |
| PRO1199 | N/A | Melanoma | UACC-257 |
| PRO1199 | N/A | Prostate Cancer | PC-3 |
| PRO1199 | N/A | CNS Cancer | SNB-19; SNB-75; U25 |
| PRO1265 | N/A | NSCL | EKVX |
| PRO1265 | N/A | Colon Cancer | COLO 205; SW620 |
| PRO1265 | N/A | Breast Cancer | HS 578T; MCF7 |
| PRO1265 | N/A | Breast Cancer | MDA-MB-231/ATCC |
| PRO1265 | N/A | Breast Cancer | MDA-MB-435; MDA-N* |
| PRO1265 | N/A | Breast Cancer | BT 549 |
| PRO1265 | N/A | Ovarian Cancer | IGROVI; OVCAR-3 |
| PRO1265 | N/A | Ovarian Cancer | OVCAR-4; OVCAR-5 |
| PRO1265 | N/A | Ovarian Cancer | OVCAR-8 |
| PRO1265 | N/A | Leukemia | CCRF-CEM*; HL-60(TB) |
| PRO1265 | N/A | Leukemia | K-562; MOLT-4; RPMI-8226 |
| PRO1265 | N/A | Renal Cancer | ACHN; CAKI-1; SN 12C |
| PRO1265 | N/A | Renal Cancer | RXF-393 |
| PRO1265 | N/A | Melanoma | LOX IMVI; UACC-257 |
| PRO1265 | N/A | CNS Cancer | SF-295; SNB-19; U251 |

(continued)

| Test-compound | Days | Tumor Cell Line Type | Cell Line Designation |
|---|---|---|---|
| PRO1286 | N/A | NSCL | A549/ATCC: EKVX; HOP-92 |
| PRO1286 | N/A | NSCL | NCI-H23; NCI-H322M |
| PRO1286 | N/A | NSCL | NCI-H522*; NCI-H226* |
| PRO1286 | N/A | Colon Cancer | HCT-1116*; SW620 |
| PRO1286 | N/A | Breast Cancer | BT-549; MCF7: MDA-N |
| PRO1286 | N/A | Breast Cancer | NCI/ADR-RES*; T-47D |
| PRO1286 | N/A | Ovarian Cancer | OVCAR-4*; OVCAR-5* |
| PRO1286 | N/A | Ovarian Cancer | OVCAR-8*; SK-OV-3 |
| PRO1286 | N/A | Renal Cancer | 786-0; ACHN: CAKI-1 |
| PRO1286 | N/A | Renal Cancer | RXF 393*; SN 12C*; TK-10* |
| PRO1286 | N/A | Melanoma | LOX IMVI; SK-MEL-2 |
| PRO1286 | N/A | Melanoma | SK-MEL-5: UACC-257 |
| PRO1286 | N/A | Melanoma | MEL-14 |
| PRO1286 | N/A | Prostate Cancer | PC-3 |
| PRO1286 | N/A | CNS Cancer | SF-268;SF-295: S-539* |
| PRO1286 | N/A | CNS Cancer | SNB-19; SNB-75* |
| PRO1313 | N/A | NSCL | A549/ATCC; HOP-62; HOP-92 |
| PRO1313 | N/A | NSCL | NCI-H23; NCI-H322M |
| PRO1313 | N/A | NSCL | NCI-H460; NCI-H522 |
| PRO1313 | N/A | NSCL | NCI-H226 |
| PRO1313 | N/A | Colon Cancer | COLO 205; HCT-116; HCT-15 |
| PRO1313 | N/A | Colon Cancer | HT29; SW620 |
| PRO1313 | N/A | Breast Cancer | BT-549*; HS 578T; MCF7 |
| PRO1313 | N/A | Breast Cancer | MDA-MB-231/ATCC |
| PRO1313 | N/A | Breast Cancer | MDA-MB-435; MDA-N |
| PRO1313 | N/A | Breast Cancer | NCI/ADR-RES |
| PRO1313 | N/A | Ovarian Cancer | IGROVI*; OVCAR-3 |
| PRO1313 | N/A | Ovarian Cancer | OVCAR-4: OVCAR-5 |
| PRO1313 | N/A | Ovarian Cancer | OVCAR-8; SK-OV-3 |
| PRO1313 | N/A | Leukemia | CCRF-CEM: HL-60(TB)* |
| PRO1313 | N/A | Leukemia | K-562; MOLT-4; RPMI-8226 |
| PRO1313 | N/A | Renal Cancer | 786-0; A498: ACHN; CAKI-1 |
| PRO1313 | N/A | Renal Cancer | RXF 393; SN 12C; TK-10* |
| PRO1313 | N/A | Renal Cancer | UO-31 |
| PRO1313 | N/A | Melanoma | LOX IMVI: M14; MALME-3M |

(continued)

| Test-compound | Days | Tumor Cell Line Type | Cell Line Designation |
|---|---|---|---|
| PRO1313 | N/A | Melanoma | SK-MEL-28: SK-MEL-5 |
| PRO1313 | N/A | Melanoma | UACC-257: SK-MEL-2 |
| PRO1313 | N/A | Prostate Cancer | DU-145; PC-3 |
| PRO1313 | N/A | CNS Cancer | SF-268; SF-295; SNB-19 |
| PRO1313 | N/A | CNS Cancer | U251 * |
| PRO1338 | N/A | NSCL | A549/ATCC; EKVX*; HOP-62 |
| PRO1338 | N/A | NSCL | HOP-92*; NCI-H226* |
| PRO1338 | N/A | NSCL | NCI-H23*; NCI-H322M |
| PRO1338 | N/A | NSCL | NCI-460; NCI-H522* |
| PRO1338 | N/A | Colon Cancer | HCC-2998*; HCT-116* |
| PRO1338 | N/A | Colon Cancer | HCT-15; HT29; SW620 |
| PRO1338 | N/A | Breast Cancer | BT-549*; MCF7 |
| PRO1338 | N/A | Breast Cancer | MDA-MB-435; MDA-N |
| PRO1338 | N/A | Breast Cancer | NCI/ADR-RES* |
| PRO1338 | N/A | Ovarian Cancer | OVCAR-3; OVCAR-4* |
| PRO1338 | N/A | Ovarian Cancer | OVCAR-5*; OVCAR-8* |
| PRO1338 | N/A | Ovarian Cancer | SK-OV-3 |
| PRO1338 | N/A | Renal Cancer | 786-0: ACHN; CAKI-1 |
| PRO1338 | N/A | Renal Cancer | RXF-393*; SN 12C*; TK-10* |
| PRO1338 | N/A | Renal Cancer | UO-31 * |
| PRO1338 | N/A | Melanoma | LOX IMVI; M14; SK-MEL-2* |
| PRO1338 | N/A | Melanoma | SK-MEL-5*; UACC-257* |
| PRO1338 | N/A | Melanoma | UACC-62 |
| PRO1338 | N/A | Prostate Cancer | DU-145; PC-3 |
| PRO1338 | N/A | CNS Cancer | SF-268; SF-295; S-539* |
| PRO1338 | N/A | CNS Cancer | SNB-19; SNB-75*; U251* |
| PRO1375 | N/A | NSCL | NCI-H23 |
| PRO1375 | N/A | Colon Cancer | SW620 |
| PRO1375 | N/A | Breast Cancer | NCI/ADR-RES |
| PRO1375 | N/A | Ovarian Cancer | OVCAR-5 |
| PRO1375 | N/A | Renal Cancer | SN 12C |
| PRO1375 | N/A | Melanoma | LOX IMVI |
| PRO1375 | N/A | CNS Cancer | SF-268 |

(continued)

| Test-compound | Days | Tumor Cell Line Type | Cell Line Designation |
|---|---|---|---|
| PRO1410 | N/A | Renal Cancer | UO-31 |
| PRO1410 | N/A | NSCL | NCI-H522 |
| PRO1410 | N/A | Colon Cancer | HCC-2998; KM12 |
| PRO1410 | N/A | Ovarian Cancer | IGROVI |
| PRO1410 | N/A | Leukemia | SR |
| PRO1410 | N/A | Renal Cancer | A498*; TK-10 |
| PRO1410 | N/A | Melanoma | LOX IMVI |
| PRO1410 | N/A | CNS Cancer | S-539 |
| PRO1488 | N/A | NSCL | A549/ATCC*; EKVX* |
| PRO1488 | N/A | NSCL | HOP-62; HOP-92; NCI-H23 |
| PRO1488 | N/A | NSCL | NCI-H322M; NCI-H460 |
| PRO1488 | N/A | NSCL | NCI-H522; NCI-H226 |
| PRO1488 | N/A | Colon Cancer | COLO 205; HCC-2998 |
| PRO1488 | N/A | Colon Cancer | HCT-116*; HCT-15 |
| PRO1488 | N/A | Colon Cancer | KM12: SW620 |
| PRO1488 | N/A | Breast Cancer | HS 578T |
| PRO1488 | N/A | Breast Cancer | MDA-MB-231/ATCC |
| PRO1488 | N/A | Breast Cancer | MDA-MB-435; MDA-N |
| PRO1488 | N/A | Breast Cancer | NCI/ADR-RES; T-47D |
| PRO1488 | N/A | Breast Cancer | BT-549 |
| PRO1488 | N/A | Ovarian Cancer | IGROVI; OVCAR-3 |
| PRO1488 | N/A | Ovarian Cancer | OVCAR-4; OVCAR-5* |
| PRO1488 | N/A | Ovarian Cancer | OVCAR-8; SK-OV-3 |
| PRO1488 | N/A | Leukemia | CCRF-CEM; K-562 |
| PRO1488 | N/A | Leukemia | RPMI-8226; SR |
| PRO1488 | N/A | Renal Cancer | 786-0*; A498; ACHN; CAKI-1 |
| PRO1488 | N/A | Renal Cancer | RXF 393; SN 12C*, TK-10* |
| PRO1488 | N/A | Renal Cancer | UO-31 |
| PRO1488 | N/A | Melanoma | LOX IMVI; M14; SK-MEL-2* |
| PRO1488 | N/A | Melanoma | SK-MEL-28; SK-MEL-5 |
| PRO1488 | N/A | Melanoma | UACC-257*; UACC-62* |
| PRO1488 | N/A | Prostate Cancer | DU-145;PC-3* |
| PRO1488 | N/A | CNS Cancer | SF-268; SF-295; S-539 |

(continued)

| Test-compound | Days | Tumor Cell Line Type | Cell Line Designation |
|---|---|---|---|
| PRO1488 | N/A | CNS Cancer | SNB-19: SNB-75 |
| PRO3438 | N/A | Colon Cancer | HCC-2998; KM12 |
| PRO3438 | N/A | Leukemia | SR |
| PRO3438 | N/A | Renal Cancer | RXF 393; TK- 10 |
| PRO3438 | N/A | Prostate Cancer | PC-3 |
| PRO3438 | N/A | Ovarian Cancer | IGROVI |
| PRO4302 | N/A | NSCL | A549/ATCC*; EKVX* |
| PRO4302 | N/A | NSCL | HOP-62; HOP-92*; NCI-H23 |
| PRO4302 | N/A | NSCL | NCI-H322M; NCI-H460 |
| PRO4302 | N/A | NSCL | NCI-H522 |
| PRO4302 | N/A | Colon Cancer | COLO 205; HCC-2998 |
| PRO4302 | N/A | Colon Cancer | HCT-15; KM12; SW620 |
| PRO4302 | N/A | Breast Cancer | BT-549*; HS 578T |
| PRO4302 | N/A | Breast Cancer | MDA-MB-435; MDA-N |
| PRO4302 | N/A | Breast Cancer | NCI/ADR-RES; T-47D |
| PRO4302 | N/A | Ovarian Cancer | IGROVI; OVCAR-3 |
| PRO4302 | N/A | Ovarian Cancer | OVCAR-4; OVCAR-5* |
| PRO4302 | N/A | Ovarian Cancer | OVCAR-8; SK-OV-3 |
| PRO4302 | N/A | Leukemia | CCRF-CEM; HL-60(TB) |
| PRO4302 | N/A | Leukemia | K-562; SR; RPMI-8226 |
| PRO4302 | N/A | Renal Cancer | 786-0*; A498*; ACHN |
| PRO4302 | N/A | Renal Cancer | CAKI-1; RXF 393; SN 12C* |
| PRO4302 | N/A | Renal Cancer | TK-10*; UO-31 * |
| PRO4302 | N/A | Melanoma | LOX IMVI; M14; SK-MEL-2* |
| PRO4302 | N/A | Melanoma | SK-MEL-28; SK-MEL-5 |
| PRO4302 | N/A | Melanoma | UACC-257*; UACC-62* |
| PRO4302 | N/A | Prostate Cancer | DU-1455; PC-3* |
| PRO4302 | N/A | CNS Cancer | SF-268; SF-295: S-539 |
| PRO4302 | N/A | CNS Cancer | SNB-19; SNB-75 |
| PRO4400 | N/A | NSCL | HOP-92; NCI-H226 |
| PRO4400 | N/A | NSCL | A549/ATCC; EKVX; HOP-62 |
| PRO4400 | N/A | NSCL | NCI-H23; NCI-H322 |
| PRO4400 | N/A | NSCL | NCI-H522 |
| PRO4400 | N/A | Colon Cancer | HCC-2998; HCT-15; HT29 |

(continued)

| Test-compound | Days | Tumor Cell Line Type | Cell Line Designation |
|---|---|---|---|
| PRO4400 | N/A | Colon Cancer | KM12: SW620 |
| PRO4400 | N/A | Breast Cancer | HS 578T; BT-549; MCF7 |
| PRO4400 | N/A | Breast Cancer | MDA-MB-231/ATCC |
| PRO4400 | N/A | Breast Cancer | NCI/ADR-RES |
| PRO4400 | N/A | Ovarian Cancer | IGROVI*; OVCAR-3 |
| PRO4400 | N/A | Ovarian Cancer | OVCAR-4; OVCAR-5 |
| PRO4400 | N/A | Ovarian Cancer | OVCAR-8 |
| PRO4400 | N/A | Leukemia | CCRF-CEM; HL-60(TB); SR |
| PRO4400 | N/A | Leukemia | RPMI-8226; K-562; MOLT-4 |
| PRO4400 | N/A | Prostate Cancer | PC-3 |
| PRO4400 | N/A | Melanoma | MALME-3M; M 14; UACC-257 |
| PRO4400 | N/A | Renal Cancer | A498; ACHN; CAKI- I |
| PRO4400 | N/A | Renal Cancer | RXF 393; SN 12C; TK-10 |
| PRO4400 | N/A | Renal Cancer | 786-0 |
| PRO4400 | N/A | CNS Cancer | SF-268; SNB-19; U251 |
| PRO5725 | N/A | NSCL | A549/ATCC; EKVX; HOP-92 |
| PRO5725 | N/A | NSCL | NCI-H23; NCI-H322M |
| PRO5725 | N/A | NSCL | NCI-H522; NCI-H226 |
| PRO5725 | N/A | NSCL | NCI-H460 |
| PRO5725 | N/A | Colon Cancer | COLO 205; HCC-2998 |
| PRO5725 | N/A | Colon Cancer | HCT-15;HT29;KM12;SW620 |
| PRO5725 | N/A | Breast Cancer | BT-549; HS 578T; MCF7 |
| PRO5725 | N/A | Breast Cancer | MDA-MB231/ATCC |
| PRO5725 | N/A | Breast Cancer | NCI/ADR-RES; T-47D |
| PRO5725 | N/A | Ovarian Cancer | OVCAR-3; OVCAR-4 |
| PRO5725 | N/A | Ovarian Cancer | OVCAR-8 |
| PRO5725 | N/A | Leukemia | CCRF-CEM; HL-60(TB)* |
| PRO5725 | N/A | Leukemia | K-562; MOLT-4; RPMI-8226 |
| PRO5725 | N/A | Leukemia | SR |
| PRO5725 | N/A | Renal Cancer | 786-0; ACHN; CAKI-1 |
| PRO5725 | N/A | Renal Cancer | RXF 393; SN 12C; TK-10 |
| PRO5725 | N/A | Renal Cancer | UO-31; A498 |
| PRO5725 | N/A | Melanoma | LOX IMVI; M14; SK-MEL-28 |
| PRO5725 | N/A | Melanoma | UACC-257 |
| PRO5725 | N/A | Prostate Cancer | PC-3 |

(continued)

| Test-compound | Days | Tumor Cell Line Type | Cell Line Designation |
|---|---|---|---|
| PRO5725 | N/A | CNS Cancer | SF-295; SNB-19; SNB-75 |
| PRO5725 | N/A | CNS Cancer | U251; SF-268 |
| PRO183 | 6 | Leukemia | HL-60(TB); SR; CCRF-CEM |
| PRO183 | 6 | Leukemia | RPMI-8226 |
| PRO183 | 6 | Colon Cancer | KM12; COLO 205; SW620 |
| PRO183 | 6 | Colon Cancer | HCT-15; HT-29 |
| PRO183 | 6 | Breast Cancer | MDA-N; MDA-MB-435 |
| PRO183 | 6 | NSCL | HOP-62; A549/ATCC; EKVX |
| PRO183 | 6 | NSCL | NCI-H23; NCI-H322M |
| PRO183 | 6 | Ovarian Cancer | IGROVI; OVCAR-8 |
| PRO183 | 6 | Melanoma | LOX IMVI; UACC-62 |
| PRO183 | 6 | Melanoma | UACC-257 |
| PRO183 | 6 | CNS Cancer | SF-295; S-539; U251 |
| PRO183 | 6 | Renal Cancer | SN 12C |
| PRO202 | 6 | NSCL | HOP-62; NCI-H322M |
| PRO202 | 6 | NSCL | NCI-H460; NCI-H522 |
| PRO202 | 6 | CNS Cancer | SF-268; SF-295; SNB-19 |
| PRO202 | 6 | CNS Cancer | U251 |
| PRO202 | 6 | BreastCancer | MDA-N;MDA-MB 231/ATCC |
| PRO202 | 6 | Breast Cancer | MCF7 |
| PRO202 | 6 | Colon Cancer | KM 12: HCC-2998 |
| PRO202 | 6 | Renal Cancer | SN 12C |
| PRO202 | 6 | Leukemia | CCRF-CEM; RPMI-8226 |
| PRO202 | 6 | Leukemia | MOLT-4 |
| PRO202 | 6 | Melanoma | LOX IMVI |
| PRO202 | 2 | NSCL | NCI-H322M |
| PRO202 | 2 | Colon Cancer | KM-12; HCC-2998* |
| PRO202 | 2 | CNS Cancer | SNB-19 |
| PRO202 | 2 | Leukemia | K-562; HL-60(TB) |
| PRO540 | 6 | Leukemia | CCRF-CEM; K-562; MOLT-4 |
| PRO540 | 6 | NSCL | EKVX; HOP-92; NCI-H23 |
| PRO540 | 6 | NSCL | NCI-H322M |
| PRO540 | 6 | Colon Cancer | COLO 205; HCT-116; HCT-15 |
| PRO540 | 6 | Colon Cancer | SW620 |
| PRO540 | 6 | CNS Cancer | SF-295; SNB-19; SNB-75 |
| PRO540 | 6 | CNS Cancer | U251 |
| PRO540 | 6 | Melanoma | M14 |
| PRO540 | 6 | Ovarian Cancer | IGROVI; OVCAR-4 |
| PRO540 | 6 | Ovarian Cancer | OVCAR-5 |
| PRO540 | 6 | Renal Cancer | RXF 393; SN 12C |
| PRO540 | 6 | Breast Cancer | MDA-N; BT-549 |
| PRO542 | 2 | Leukemia | SR |
| PRO542 | 2 | Breast Cancer | MDA-MB-231/ATCC |

(continued)

| Test-compound | Days | Tumor Cell Line Type | Cell Line Designation |
|---|---|---|---|
| PRO542 | 2 | Breast Cancer | MDA-MB-435 |
| PRO542 | 2 | NSCL | EKVX; HOP-92; NCI-H226 |
| PRO542 | 2 | Colon Cancer | HCT- 116; HCT- 15 |
| PRO542 | 2 | CNS Cancer | SNB-75 |
| PRO542 | 2 | Ovarian Cancer | OVCAR-5 |
| PRO542 | 2 | Renal Cancer | RXF 393 |
| PRO861 | 6 | Leukemia | CCRF-CEM; HL-60(TB)* |
| PRO861 | 6 | Leukemia | MOLT-4*; SR |
| PRO861 | 6 | NSCL | HOP-92; NCI-H23; NCI-H522 |
| PRO861 | 6 | NSCL | NCI-H322M; EKVX |
| PRO861 | 6 | Colon Cancer | COLO 205; HCC-2998; HT29 |
| PRO861 | 6 | Colon Cancer | KM 12: SW620 |
| PRO861 | 6 | CNS Cancer | SF-268; U251 |
| PRO861 | 6 | Melanoma | LOX IMVI; SK-MEL-2 |
| PRO861 | 6 | Melanoma | SK-MEL-28; UACC-257 |
| PRO861 | 6 | Ovarian Cancer | IGROVI; OVCAR-3 |
| PRO861 | 6 | Ovarian Cancer | OVCAR-4; OVCAR-8 |
| PRO861 | 6 | Renal Cancer | SN 12C |
| PRO861 | 6 | Prostate Cancer | PC-3 |
| PRO861 | 6 | Breast Cancer | MCF7; MDA-MB-231/ATCC |
| PRO861 | 6 | Breast Cancer | MDA-MB-435; MDA-N |
| PRO861 | 6 | Breast Cancer | T-47D |
| PRO861 | 2 | Leukemia | CCRF-CEM; HL-60(TB); SR |
| PRO861 | 2 | NSCL | HOP-92 |
| PRO861 | 2 | Colon Cancer | COLO 205; HT29 |
| PRO861 | 2 | Melanoma | MALME-3M |
| PRO861 | 2 | Ovarian Cancer | OVCAR-5 |
| PRO861 | 2 | Breast Cancer | MCF7 |
| PRO1096 | 6 | Leukemia | CCRF-CEM; HL-60(TB)* |
| PRO1096 | 6 | Leukemia | K-562*; MOLT-4 |
| PRO1096 | 6 | Leukemia | RPMI-8226*; SR |
| PRO1096 | 6 | NSCL | A549/ATCC; EKVX; HOP-62* |
| PRO1096 | 6 | NSCL | NCI-H226*; NCI-H322M |
| PRO1096 | 6 | NSCL | NCI-H460*; NCI-H522 |
| PRO1096 | 6 | NSCL | HOP-92*; NCI-H23 |
| PRO1096 | 6 | Colon Cancer | COLO 205*; HCC-2998* |
| PRO1096 | 6 | Colon Cancer | HCT-15*; KM12; HT29 |
| PRO1096 | 6 | Colon Cancer | SW620; HCT-116* |
| PRO1096 | 6 | CNS Cancer | SF-295*; U251; S-539; SF-268 |
| PRO1096 | 6 | Melanoma | M14*; MALME-3M* |
| PRO1096 | 6 | Melanoma | SK-MEL-2; LOX IMVI |
| PRO1096 | 6 | Melanoma | SK-MEL-28; SK-MEL-5 |
| PRO1096 | 6 | Melanoma | UACC-257; UACC-62 |
| PRO1096 | 6 | Ovarian Cancer | OVCAR-3*; OVCAR-4* |
| PRO1096 | 6 | Ovarian Cancer | OVCAR-5; SK-OV-3 |
| PRO1096 | 6 | Ovarian Cancer | OVCAR-8 |
| PRO1096 | 6 | Renal Cancer | A498; ACHN*; CAKI-1 |

(continued)

| Test-compound | Days | Tumor Cell Line Type | Cell Line Designation |
|---|---|---|---|
| PRO1096 | 6 | Renal Cancer | RXF 393*; SN 12C; TK-10* |
| PRO1096 | 6 | Renal Cancer | 786-0; UO-31 |
| PRO1096 | 6 | Prostate Cancer | DU-145; PC-3 |
| PRO1096 | 6 | Breast Cancer | MDA-MB-231/ATCC* |
| PRO1096 | 6 | Breast Cancer | MDA-MB-435; MDA-N |
| PRO1096 | 6 | Breast Cancer | BT-549; MCF7; HS 578T |
| PRO1096 | 2 | Leukemia | HL-60(TB)*; K-562* |
| PRO1096 | 2 | Leukemia | RPMI-8226*; SR; MOLT-4 |
| PRO1096 | 2 | Leukemia | CCRF-CEM |
| PRO1096 | 2 | NSCL | A549/ATCC; EKVX; HOP-92* |
| PRO1096 | 2 | NSCL | NCI-H226*; NCI-H322M* |
| PRO1096 | 2 | NSCL | NCI-H460*; NCI-H522 |
| PRO1096 | 2 | NSCL | HOP-62* |
| PRO1096 | 2 | Colon Cancer | COLO 205*; HCC-2998* |
| PRO1096 | 2 | Colon Cancer | HCT-15*; HCT-116*; KM12 |
| PRO1096 | 2 | Colon Cancer | HT29; SW620 |
| PRO1096 | 2 | CNS Cancer | SF-295*; S-539*; U251 |
| PRO1096 | 2 | Melanoma | M14*; MALME-3M* |
| PRO1096 | 2 | Melanoma | SK-MEL-28*; UACC-62 |
| PRO1096 | 2 | Melanoma | SK-MEL-2; LOX IMVI* |
| PRO1096 | 2 | Melanoma | SK-MEL-5 |
| PRO1096 | 2 | Ovarian Cancer | OVCAR-3*; OVCAR-4* |
| PRO1096 | 2 | Ovarian Cancer | OVCAR-5*; SK-OV-3* |
| PRO1096 | 2 | Renal Cancer | A498*; ACHN*; CAKI-1 |
| PRO1096 | 2 | Renal Cancer | RXF 393*; SN 12C; TK-10* |
| PRO1096 | 2 | Renal Cancer | UO-31 |
| PRO1096 | 2 | Prostate Cancer | DU-145; PC-3* |
| PRO1096 | 2 | Breast Cancer | MCF7; MDA-MB-231/ATCC* |
| PRO1096 | 2 | Breast Cancer | MDA-MB-435; MDA-N |
| PRO1096 | 2 | Breast Cancer | BT-549; HS 578T |
| PRO1096 | N/A | NSCL | A549/ATCC*: EKVX |
| PRO1096 | N/A | NSCL | HOP-62*; HOP-92*; NCI-H23 |
| PRO1096 | N/A | NSCL | NCI-H322M*; NCI-H460 |
| PRO1096 | N/A | NSCL | NCI-H522 |
| PRO1096 | N/A | Colon Cancer | COLO 205; HCT-116* |
| PRO1096 | N/A | Colon Cancer | HCT-15; HT29*; KM12* |
| PRO1096 | N/A | Colon Cancer | SW620; HCC-2998* |
| PRO1096 | N/A | Breast Cancer | HS 578T; MCF7 |
| PRO1096 | N/A | Breast Cancer | MDA-MB-231/ATCC* |
| PRO1096 | N/A | Breast Cancer | MDA-MB-435*; MDA-N* |
| PRO1096 | N/A | Breast Cancer | T-47D |
| PRO1096 | N/A | Ovarian Cancer | OVCAR-3*; OVCAR-4* |
| PRO1096 | N/A | Ovarian Cancer | OVCAR-5*; SK-OV-3 |
| PRO1096 | N/A | Leukemia | HL-60(TB); MOLT-4 |

(continued)

| Test-compound | Days | Tumor Cell Line Type | Cell Line Designation |
|---|---|---|---|
| PRO1096 | N/A | Leukemia | RPMI-8226 |
| PRO1096 | N/A | Renal Cancer | A498; ACHN*; RXF 393* |
| PRO1096 | N/A | Renal Cancer | SN 12C*; TK-10; UO-31 |
| PRO1096 | N/A | Renal Cancer | CAKI-1 |
| PRO1096 | N/A | Melanoma | M14*; MALME-3M |
| PRO1096 | N/A | Melanoma | SK-MEL-28; SK-MEL-2 |
| PRO1096 | N/A | Melanoma | UACC-257; UACC-62 |
| PRO1096 | N/A | Melanoma | LOX IMVI |
| PRO1096 | N/A | Prostate Cancer | DU-145; PC-3* |
| PRO1096 | N/A | CNS Cancer | SF-268; SF-295; S-539 |
| PRO1096 | N/A | CNS Cancer | SNB-75*;U251 |
| PRO3562 | N/A | Colon Cancer | HCC-2998 |
| PRO3562 | N/A | NSCL | HOP-62 |
| PRO3562 | N/A | Ovarian Cancer | IGROVI; OVCAR-3 |
| PRO3562 | N/A | Ovarian Cancer | OVCAR-8 |
| PRO3562 | N/A | Leukemia | MOLT-4 |
| PRO3562 | N/A | CNS Cancer | SNB-19 |

*= CYTOTOXIC

Deposit of Material

The following materials have been deposited with the American Type Culture Collection, 10801 University Blvd., Manassas, VA 20110-2209. USA (ATCC):

| Material | ATCC Dep. No. | Deposit Date |
|---|---|---|
| DNA34387-1138 | 209260 | September 16, 1997 |
| DNA44194-1317 | 209808 | April 28, 1998 |
| DNA48333-1321 | 209701 | March 26, 1998 |
| DNA44189-1322 | 209699 | March 26, 1998 |
| DNA48320-1433 | 209904 | May 27, 1998 |
| DNA57700-1408 | 203583 | January 12, 1999 |
| DNA57708-141 | 203021 | June 23, 1998 |
| DNA57690-1374 | 209950 | June 9, 1998 |
| DNA59777-1480 | 203111 | August 11, 1998 |
| DNA60292-1506 | 203540 | December 15, 1998 |
| DNA65351-1366-1 | 209856 | May 12, 1998 |
| DNA60764-1533 | 203452 | November 10, 1998 |
| DNA64903-1553 | 203223 | September 15, 1998 |
| DNA64966-1575 | 203575 | January 12, 1999 |
| DNA66667 | 203267 | September 22, 1998 |
| DNA67004-1614 | 203115 | August 11, 1998 |

(continued)

| Material | ATCC Dep. No. | Deposit Date |
|---|---|---|
| DNA68874-1622 | 203277 | September 22, 1998 |
| DNA73736-1657 | 203466 | November 17. 1998 |
| DNA82364-2538 | 203603 | January 20, 1999 |
| DNA92218-2554 | 203834 | March 9, 1999 |
| DNA87974-2609 | 203963 | April 27, 1999 |
| DNA92265-2669 | PTA-256 | June 22, 1999 |

These deposits were made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposits will be made available by ATCC under the terms of the Budapest Treaty, and subject to an agreement between Genentech, Inc., and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 U.S.C. § 122 and the Commissioner's rules pursuant thereto (including 37 CFR § 1.14 with particular reference to 886 OG 638).
The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.
The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.
The following numbered paragraphs (paras.) contain further statements of various aspects of the present invention:-

1. A composition of matter useful for the inhibition of neoplastic cell growth, said composition comprising an effective amount of a PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO4400, PRO5725, PRO183, PRO202, PRO542, PRO861, PRO1096 or PRO3562 polypeptide, or an agonist thereof, in admixture with a pharmaceutically acceptable carrier.

2. The composition of matter of Para. 1 comprising a growth inhibitory amount of a PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO141 0, PRO1488, PRO3438, PRO4302, PRO4400, PRO5725, PRO183, PRO202, PRO542, PRO861, PRO109 or PRO3562 polypeptide, or an agonist thereof.

3. The composition of matter of Para. 1 comprising a cytotoxic amount of a PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO4400, PRO5725, PRO183, PRO202, PRO542, PRO861, PRO1096 or PRO3562 polypeptide, or an agonist thereof.

4. The composition of matter of Para. 1 additionally comprising a further growth inhibitory agent, cytotoxic agent or chemotherapeutic agent.

5. A composition of matter useful for the treatment of a tumor in a mammal, said composition comprising a therapeutically effective amount of a PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO4400, PRO5725, PRO183, PRO202, PRO542, PRO861, PRO1096 or PRO3562 polypeptide, or an agonist thereof.

6. The composition of matter of Para. 5, wherein said tumor is a cancer.

7. The composition of matter of Para. 6, wherein the cancer is selected from the group consisting of breast cancer, ovarian cancer, renal cancer, colorectal cancer, uterine cancer, prostate cancer, lung cancer, bladder cancer, central nervous system cancer, melanoma and leukemia.

8. A method for inhibiting the growth of a tumor cell comprising exposing said tumor cell to an effective amount of a PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO4400, PRO5725, PRO183, PRO202, PRO542, PRO861, PRO1096 or PRO3562 polypeptide, or an agonist thereof.

9. The method of Para. 8, wherein said agonist is an anti-PRO240, anti-PRO381, anti-PRO534, anti-PRO540, anti-PRO698, anti-PRO982, anti-PRO1005, anti-PRO1007 anti-PRO113 anti-PRO1157, anti-PRO1199, anti-PRO1265, anti-PRO1286, anti-PRO1313, anti-PRO1338, anti-PRO1375, anti-PRO1410, anti-PRO1488, anti-PRO3438, anti-PRO4302, anti-PRO4400, anti-PRO5725, anti-PRO183, anti-PRO202, anti-PRO542, anti-PRO861, anti-PRO1096 or anti-PRO3562 agonist antibody.

10. The method of Para. 8, wherein said agonist is a small molecule mimicking the biological activity of a PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO4400, PRO5725, PRO183, PRO202, PRO542, PRO861, PRO1096 or PRO3562 polypeptide.

11. The method of Para. 8, wherein said step of exposing occurs *in vitro.*

12. The method of Para. 8, wherein said step of exposing occurs *in vivo.*

13. An article of manufacture comprising:

a container; and
a composition comprising an active agent contained within the container; wherein said active agent in the composition is a PRO240, PRO381, PRO534, PRO540, PRO698, PRO982, PRO1005 PRO1007 PRO1131, PRO1157 PRO1199 PRO1265, PRO1286, PRO1313, PRO1338, PRO1375, PRO1410, PRO1488, PRO3438, PRO4302, PRO4400, PRO5725, PRO183, PRO202, PRO542, PRO861, PRO1096 or PRO3562 polypeptide, or an agonist thereof.

14. The article of manufacture of Para. 13, further comprising a label affixed to said container, or a package insert included in said container, referring to the use of said composition for the inhibition of neoplastic cell growth.

15. The article of manufacture of Para. 13, wherein said agonist is an anti-PRO240, anti-PRO381 anti-PRO534, anti-PRO540, anti-PRO698, anti-PRO982, anti-PRO1005, anti-PRO1007 anti-PRO1131, anti-PRO1157, anti-PRO1199, anti-PRO1265, anti-PRO1286, anti-PRO1313, anti-PRO1338, anti-PRO1375, anti-PRO1410, anti-PRO1488, anti-PRO3438, anti-PRO4302, anti-PRO4400, anti-PRO5725, anti-PRO183, anti-PRO202, anti-PRO542, anti-PRO861, anti-PRO1096 or anti-PRO3562 agonist antibody.

16. The article of manufacture of Para. 13, wherein said agonist is a small molecule mimicking the biological activity of a PRO240, PRO381, PRO534, PRO540, PRO698, PRO982, PRO1005, PRO1007, PRO113, PRO1157, PRO119, PRO1265, PRO1286, PRO1313, PRO1338, PRO1375, PRO1410, PRO1488, PRO3438, PRO4302, PRO4400, PRO5725, PRO183, PRO202, PRO542, PRO861, PRO1096 or PRO3562 polypeptide.

17. The article of manufacture of Para. 13, wherein said active agent is present in an amount that is effective for the treatment of tumor in a mammal.

18. The article of manufacture of Para. 13, wherein said composition additionally comprises a further growth inhibitory agent, cytotoxic agent or chemotherapeutic agent.

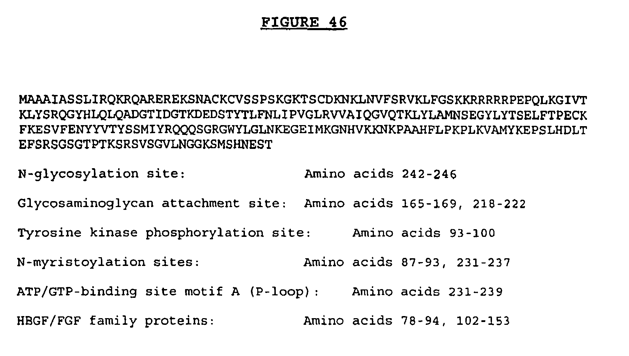

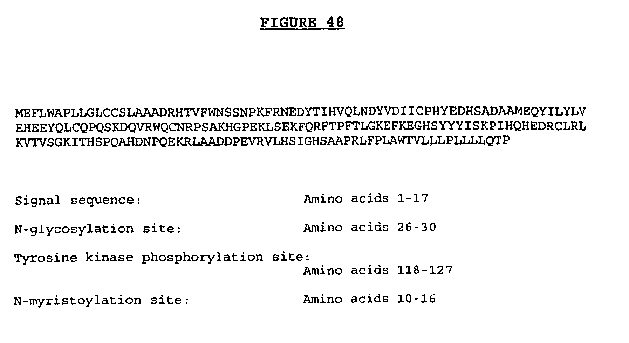

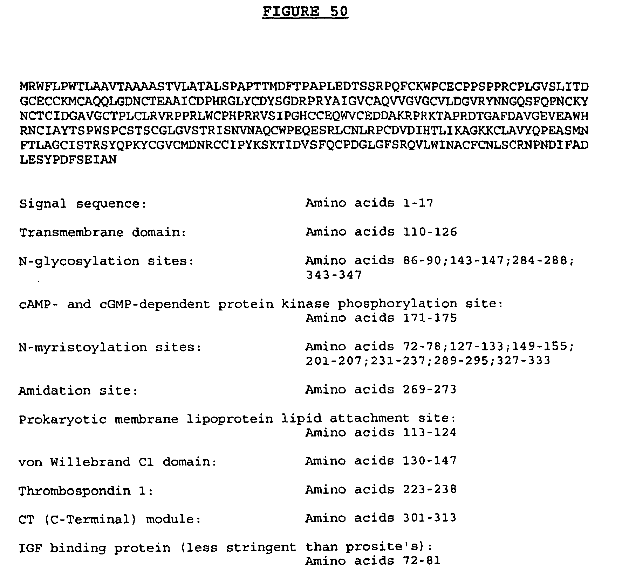

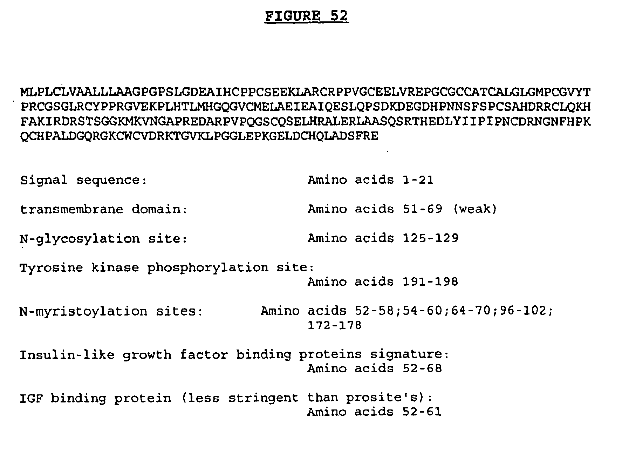

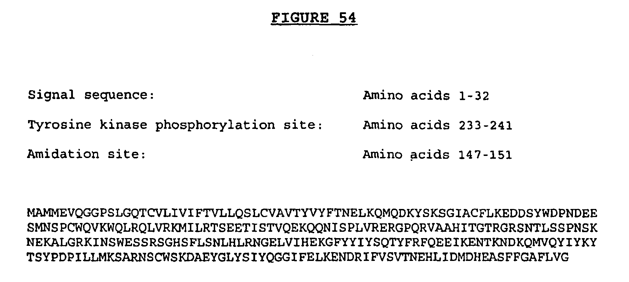

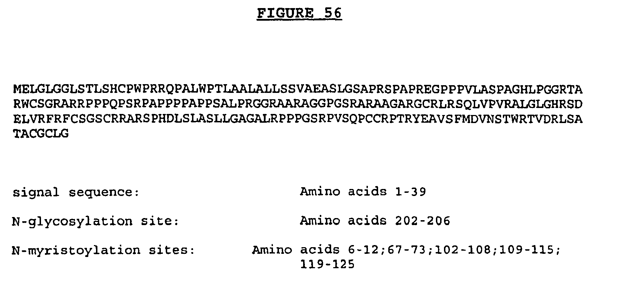

19. Isolated nucleic acid having at least 80% nucleic acid sequence identity to a nucleotide sequence that encodes an amino acid sequence selected from the group consisting of the amino acid sequence shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56).

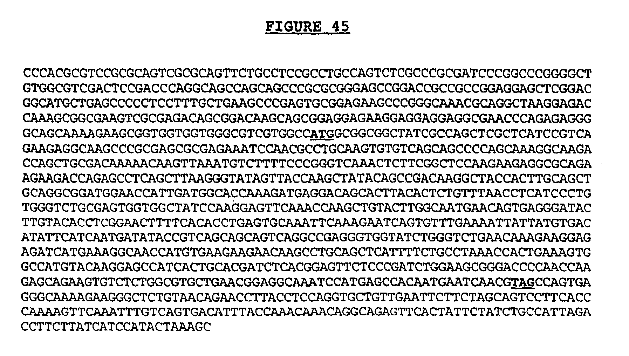

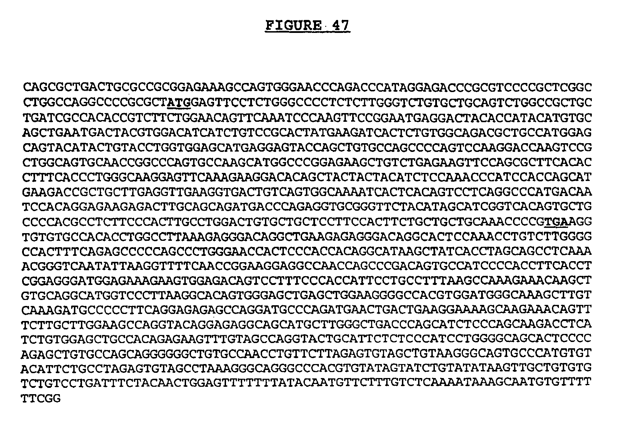

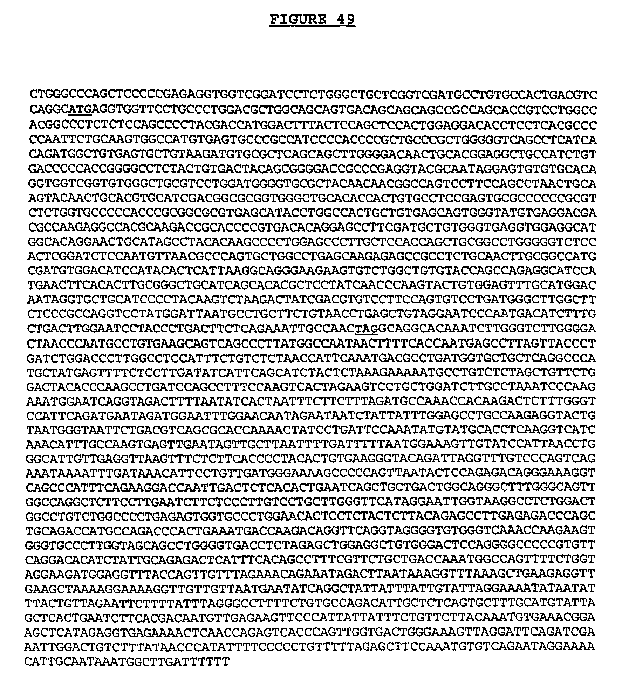

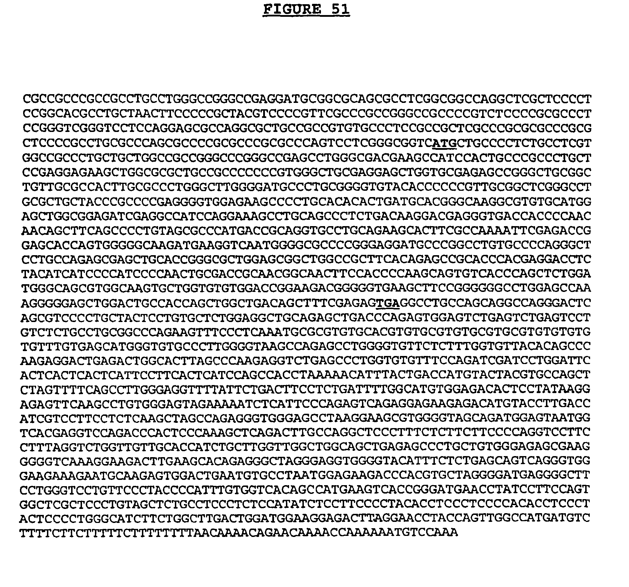

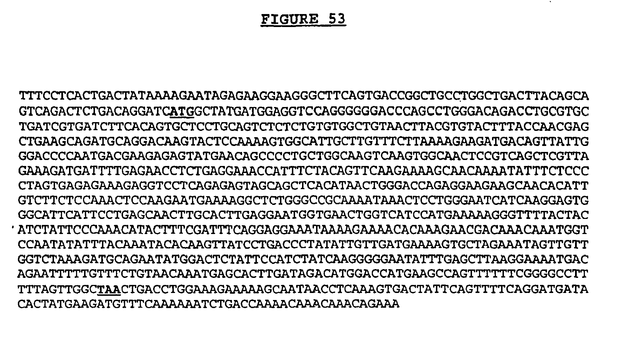

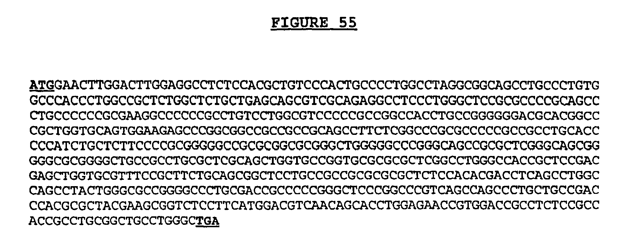

20. Isolated nucleic acid having at least 80% nucleic acid sequence identity to a nucleotide sequence selected from the group consisting of the nucleotide sequence shown in Figure 1 (SEQ ID NO:1), Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 13 (SEQ ID NO:13), Figure 15 (SEQ ID NO:15), Figure 17 (SEQ ID NO:17), Figure 19 (SEQ ID NO:19), Figure 21 (SEQ ID NO:21), Figure 23 (SEQ ID NO:23), Figure 25 (SEQ ID NO:25), Figure 27 (SEQ ID NO:27), Figure 29 (SEQ ID NO: 29), Figure 31 (SEQ ID NO:31), Figure 33 (SEQ ID NO:33), Figure 35 (SEQ ID NO:35), Figure 37 (SEQ ID NO:37), Figure 39 (SEQ ID NO:39), Figure 41 (SEQ ID NO:41), Figure 43 (SEQ ID NO:43), Figure 45 (SEQ ID NO:45), Figure 47 (SEQ ID NO:47), Figure 49 (SEQ ID NO:49), Figure 51 (SEQ ID NO:51), Figure 53 (SEQ ID NO:53), and Figure 55 (SEQ ID NO:55).

21. Isolated nucleic acid having at least 80% nucleic acid sequence identity to a nucleotide sequence selected from the group consisting of the full-length coding sequence of the nucleotide sequence shown in Figure 1 (SEQ ID NO: 1), Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 13 (SEQ ID NO:13), Figure 15 (SEQ ID NO:15), Figure 17 (SEQ ID NO:17), Figure 19 (SEQ ID NO:19), Figure 21 (SEQ ID NO:21), Figure 23 (SEQ ID NO:23), Figure 25 (SEQ ID NO:25), Figure 27 (SEQ ID NO:27), Figure 29 (SEQ ID NO:29), Figure 31 (SEQ ID NO:31), Figure 33 (SEQ ID NO:33), Figure 35 (SEQ ID NO:35), Figure 37 (SEQ ID NO:37), Figure 39 (SEQ ID NO:39), Figure 41 (SEQ ID NO:41), Figure 43 (SEQ ID NO:43), Figure 45 (SEQ ID NO:45), Figure 47 (SEQ ID NO:47), Figure 49 (SEQ ID NO:49), Figure 51 (SEQ ID NO:51), Figure 53 (SEQ ID NO:53), and Figure 55 (SEQ ID NO:55).

22. Isolated nucleic acid having at least 80% nucleic acid sequence identity to the full-length coding sequence of the DNA deposited under ATCC accession number 209260, 209808, 209701, 209699, 209904, 203583, 203021, 209950, 203111, 203540, 209856, 203452, 203223, 203575, 203267, 203115, 203277, 203466, 203603, 203834, 203963 or PTA-256.

23. A vector comprising the nucleic acid of any one of Paras. 19 to 22.

24. The vector of Para. 23 operably linked to control sequences recognized by a host cell transformed with the vector.

25. A host cell comprising the vector of Para. 23.

26. The host cell of Para. 25, wherein said cell is a CHO cell.

27. The host cell of Para. 25, wherein said cell is an *E. coli.*

28. The host cell of Para. 25, wherein said cell is a yeast cell.

29. The host cell of Para. 25, wherein said cell is a Baculovirus-infected insect cell.

30. A process for producing a PRO240, PRO381, PRO534, PRO540, PRO698, PRO982, PRO1005, PRO1007, PRO1131, PRO1157, PRO1199, PRO1265 PRO1286 PRO1313 PRO1338, PRO1375 PRO1410 PRO1488 PRO3438, PRO4302, PRO4400, PRO5725, PRO183 PRO202, PRO542, PRO861, PRO109 or PRO3562 polypeptide comprising culturing the host cell of Para. 25 under conditions suitable for expression of said polypeptide and recovering said polypeptide from the cell culture.

31. An isolated polypeptide having at least 80% amino acid sequence identity to an amino acid sequence selected from the group consisting of the amino acid sequence shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56).

32. An isolated polypeptide scoring at least 80% positives when compared to an amino acid sequence selected from the group consisting of the amino acid sequence shown in Figure 2 (SEQ ID NO:2), Figure 4 (SEQ ID NO:4), Figure 6 (SEQ ID NO:6), Figure 8 (SEQ ID NO:8), Figure 10 (SEQ ID N0:10), Figure 12 (SEQ ID N0:12), Figure 14 (SEQ.ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56).

33. An isolated polypeptide having at least 80% amino acid sequence identity to an amino acid sequence encoded by the full-length coding sequence of the DNA deposited under ATCC accession number 209260, 209808, 209701, 209699, 209904, 203583, 203021, 209950, 203111, 203540, 209856, 203452, 203223, 203575, 203267, 203115, 203277, 203466, 203603, 203834, 203963 or PTA-256.

34. A chimeric molecule comprising a polypeptide according to any one of Paras. 31 to 33 fused to a heterologous amino acid sequence.

35. The chimeric molecule of Para. 34, wherein said heterologous amino acid sequence is an epitope tag sequence.

36. The chimeric molecule of Para. 34, wherein said heterologous amino acid sequence is a Fc region of an immunoglobulin.

37. An antibody which specifically binds to a polypeptide according to any one of Paras. 31 to 33.

38. The antibody of Para. 37, wherein said antibody is a monoclonal antibody, a humanized antibody or a single-chain antibody.

39. Isolated nucleic acid having at least 80% nucleic acid sequence identity to:

(a) a nucleotide sequence encoding the polypeptide shown in Figure 2 (SEQ ID NO:2), Figure 4 (SEQ ID NO: 4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56), lacking its associated signal peptide:
(b) a nucleotide sequence encoding an extracellular domain of the polypeptide shown in Figure 2 (SEQ ID NO: 2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56), with its associated signal peptide; or
(c) a nucleotide sequence encoding an extracellular domain of the polypeptide shown in Figure 2 (SEQ ID NO: 2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56), lacking its associated signal peptide.

40. An isolated polypeptide having at least 80% amino acid sequence identity to:

(a) the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56), lacking its associated signal peptide;
(b) an extracellular domain of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56), with its associated signal peptide; or
(c) an extracellular domain of the polypeptide shown in Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 42 (SEQ ID NO:42), Figure 44 (SEQ ID NO:44), Figure 46 (SEQ ID NO:46), Figure 48 (SEQ ID NO:48), Figure 50 (SEQ ID NO:50), Figure 52 (SEQ ID NO:52), Figure 54 (SEQ ID NO:54), and Figure 56 (SEQ ID NO:56), lacking its associated signal peptide.

NOTICE

The content of the present application is not to be construed as limited to that of the claims as filed, but includes all subject matter disclosed in the specification.

The applicant maintains the right to file amendments and/or further divisional applications from this one, directed to any subject matter that is disclosed in the present specification, irrespective of whether that subject matter is in the claims of this application as filed, and irrespective of the fate of any European patent application from which this application is divided.

**Claims**

1. A composition of matter useful for the inhibition of neoplastic cell growth, said composition comprising an effective amount of a PRO202, PRO240, PRO381, PRO534, PRO540, PRO698, PRO982, PRO1005 PRO1007 PRO1131, PRO1157, PRO1199, PRO1265, PRO1286, PRO1313, PRO1338, PRO1375, PRO1410, PRO1488, PRO3438, PRO4302, PRO5725, PRO183, PRO542, PRO861, PRO1096 or PRO3562 polypeptide, or an agonist thereof, in admixture with a pharmaceutically acceptable carrier.

2. The composition of matter of Claim 1 comprising a growth inhibitory amount of a PRO202, PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO5725, PRO183, PRO542, PRO861, PRO1096 or PRO3562 polypeptide, or an agonist thereof.

3. The composition of matter of Claim 1 comprising a cytotoxic amount of a PRO202, PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO5725, PRO183, PRO542, PRO861, PRO1096 or PRO3562 polypeptide, or an agonist thereof.

4. The composition of matter of Claim 1 additionally comprising a further growth inhibitory agent, cytotoxic agent or chemotherapeutic agent.

5. A composition of matter useful for the treatment of a tumor in a mammal, said composition comprising a therapeutically effective amount of a PRO202, PRO240, PRO381, PRO534, PRO540, PRO698, PRO982, PRO1005 PRO1007 PRO1131, PRO115 PRO1199 PRO1265 PRO1286 PRO1313, PRO1338 PRO1375 PRO1410, PRO1488, PRO3438, PRO4302, PRO5725, PRO183 PRO542, PRO861, PRO1096 or PRO3562 polypeptide, or an agonist thereof.

6. The composition of matter of Claim 5, wherein said tumor is a cancer.

7. The composition of matter of Claim 6, wherein the cancer is selected from the group consisting of breast cancer, ovarian cancer, renal cancer, colorectal cancer, uterine cancer, prostate cancer, lung cancer, bladder cancer, central nervous system cancer, melanoma and leukemia.

8. A method for inhibiting the growth of a tumor cell comprising exposing said tumor cell to an effective amount of a PRO202, PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO5725, PRO183, PRO542, PRO861, PRO1096 or PRO3562 polypeptide, or an agonist thereof.

**9.** The method of Claim 8, wherein said agonist is an anti-PRO202, anti-PRO240, anti-PRO381, anti-PRO534, anti-PRO540, anti-PRO698, anti-PRO982, anti-PRO1005, anti-PRO1007, anti-PRO1131, anti-PRO1157, anti-PRO1199, anti-PRO1265, anti-PRO1286, anti-PRO1313, anti-PRO1338, anti-PRO1375, anti-PRO1410, anti-PRO1488, anti-PRO3438, anti-PRO4302, anti-PRO5725, anti-PRO183, anti-PRO542, anti-PRO861, anti-PRO1096 or anti-PRO3562 agonist antibody.

**10.** The method of Claim 8, wherein said agonist is a small molecule mimicking the biological activity of a PRO202, PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO5725, PRO183, PRO542, PRO861, PRO1096 or PRO3562 polypeptide.

**11.** The method of Claim 8, wherein said step of exposing occurs *in vitro.*

**12.** The method of Claim 8, wherein said step of exposing occurs *in vivo.*

**13.** An article of manufacture comprising:

a container; and
a composition comprising an active agent contained within the container; wherein said active agent in the composition is a PRO202, PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO5725, PRO183, PRO542, PRO861, PRO1096 or PRO3562 polypeptide, or an agonist thereof.

**14.** The article of manufacture of Claim 13, further comprising a label affixed to said container, or a package insert included in said container, referring to the use of said composition for the inhibition of neoplastic cell growth.

**15.** The article of manufacture of Claim 13, wherein said agonist is an anti-PRO202, anti-PRO240, anti-PRO381, anti-PRO534, anti-PRO540, anti-PRO698, anti-PRO982, anti-PRO1005, anti-PRO1007, anti-PRO1131, anti-PRO1157, anti-PRO1199, anti-PRO1265, anti-PRO1286, anti-PRO1313, anti-PRO1338, anti-PRO1375, anti-PRO1410, anti-PRO1488, anti-PRO3438, anti-PRO4302, anti-PRO5725, anti-PRO183, anti-PRO542, anti-PRO861, anti-PRO1096 or anti-PRO3562 agonist antibody.

**16.** The article of manufacture of Claim 13, wherein said agonist is a small molecule mimicking the biological activity of a PRO202, PRO240, PRO381, PRO534, PRO540, PRO698, PRO982, PRO1005 PRO100 PRO113 PRO115 PRO1 PRO1265 PRO1286, PRO1313, PRO1338 PRO1375 PRO1410 PRO1488 PRO3438, PRO4302, PRO5725, PRO183 PRO542, PRO861, PRO109 or PRO3562 polypeptide.

**17.** The article of manufacture of Claim 13, wherein said active agent is present in an amount that is effective for the treatment of tumor in a mammal.

**18.** The article of manufacture of Claim 13, wherein said composition additionally comprises a further growth inhibitory agent, cytotoxic agent or chemotherapeutic agent.

**19.** Isolated nucleic acid having at least 80% nucleic acid sequence identity to a nucleotide sequence that encodes an amino acid sequence selected from the group consisting of the amino acid sequence shown in Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46), Figure 50 (SEQ ID NO:50), Figure 52 (SEQ ID NO:52), Figure 54 (SEQ ID NO:54), and Figure 56 (SEQ ID NO:56).

**20.** Isolated nucleic acid having at least 80% nucleic acid sequence identity to a nucleotide sequence selected from the group consisting of the nucleotide sequence shown in Figure 47 (SEQ ID NO:47), Figure 1 (SEQ ID NO:1), Figure 3 (SEQ ID NO:3), Figure 5 (SEQ ID NO:5), Figure 7 (SEQ ID NO:7), Figure 9 (SEQ ID NO:9), Figure 11 (SEQ ID N0:11), Figure 13 (SEQ ID NO:13), Figure 15 (SEQ ID NO:15), Figure 17 (SEQ ID NO:17), Figure 19 (SEQ ID NO:19), Figure 21 (SEQ ID NO:21), Figure 23 (SEQ ID NO:23), Figure 25 (SEQ ID NO:25), Figure 27 (SEQ ID NO:27), Figure 29 (SEQ ID NO:29), Figure 31 (SEQ ID NO:31), Figure 33 (SEQ ID NO:33), Figure 35 (SEQ ID NO: 35), Figure 37 (SEQ ID NO:37), Figure 39 (SEQ ID NO:39), Figure 43 (SEQ ID NO:43), Figure 45 (SEQ ID NO:45), Figure 49 (SEQ ID NO:49), Figure 51 (SEQ ID NO:51), Figure 53 (SEQ ID NO:53), and Figure 55 (SEQ ID NO:55).

**21.** Isolated nucleic acid having at least 80% nucleic acid sequence identity to a nucleotide sequence selected from the group consisting of the full-length coding sequence of the nucleotide sequence shown in Figure 47 (SEQ ID NO:47), Figure 1 (SEQ ID NO:1), Figure 3 (SEQ ID NO:3), Figure 5 (SEQ ID NO:5), Figure 7 (SEQ ID NO:7), Figure 9 (SEQ ID NO:9), Figure 11 (SEQ ID NO:11), Figure 13 (SEQ ID NO:13), Figure 15 (SEQ ID NO:15), Figure 17 (SEQ ID NO:17), Figure 19 (SEQ ID NO:19), Figure 21 (SEQ ID NO:21), Figure 23 (SEQ ID NO:23), Figure 25 (SEQ ID NO:25), Figure 27 (SEQ ID NO:27), Figure 29 (SEQ ID NO:29), Figure 31 (SEQ ID NO:31), Figure 33 (SEQ ID NO:33), Figure 35 (SEQ ID NO:35), Figure 37 (SEQ ID NO:37), Figure 39 (SEQ ID NO:39), Figure 43 (SEQ ID NO:43), Figure 45 (SEQ ID NO:45), Figure 49 (SEQ ID NO:49), Figure 51 (SEQ ID NO:51), Figure 53 (SEQ ID NO:53), and Figure 55 (SEQ ID NO:55).

**22.** Isolated nucleic acid having at least 80% nucleic acid sequence identity to the full-length coding sequence of the DNA deposited under ATCC accession number 209260, 209808, 209701, 209699, 209904, 203583, 203021, 209950, 203111, 203540, 209856, 203452, 203223, 203575, 203267, 203115, 203277, 203466, 203603, 203834, or PTA-256.

**23.** A vector comprising the nucleic acid of any one of Claims 19 to 22.

**24.** The vector of Claim 23 operably linked to control sequences recognized by a host cell transformed with the vector.

**25.** A host cell comprising the vector of Claim 23.

**26.** The host cell of Claim 25, wherein said cell is a CHO cell.

**27.** The host cell of Claim 25, wherein said cell is an *E. coli.*

**28.** The host cell of Claim 25, wherein said cell is a yeast cell.

**29.** The host cell of Claim 25, wherein said cell is a Baculovirus-infected insect cell.

**30.** A process for producing a PRO202, PRO240, PRO381, PRO534, PRO540, PRO698, PRO982, PRO1005, PRO1007, PRO1131, PRO1157, PRO1199, PRO1265, PRO1286, PRO1313, PRO1338, PRO1375, PRO1410, PRO1488, PRO3438, PRO4302, PRO5725, PRO183, PRO542, PRO861, PRO1096 or PRO3562 polypeptide comprising culturing the host cell of Claim 25 under conditions suitable for expression of said polypeptide and recovering said polypeptide from the cell culture.

**31.** An isolated polypeptide having at least 80% amino acid sequence identity to an amino acid sequence selected from the group consisting of the amino acid sequence shown in Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46), Figure 50 (SEQ ID NO:50), Figure 52 (SEQ ID NO:52), Figure 54 (SEQ ID NO:54), and Figure 56 (SEQ ID NO:56).

**32.** An isolated polypeptide scoring at least 80% positives when compared to an amino acid sequence selected from the group consisting of the amino acid sequence shown in Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46), Figure 50 (SEQ ID NO:50), Figure 52 (SEQ ID NO:52), Figure 54 (SEQ ID NO:54), and Figure 56 (SEQ ID NO:56).

**33.** An isolated polypeptide having at least 80% amino acid sequence identity to an amino acid sequence encoded by the full-length coding sequence of the DNA deposited under ATCC accession number 209260, 209808, 209701, 209699, 209904, 203583, 203021, 209950, 203111, 203540, 209856, 203452, 203223, 203575, 203267, 203115, 203277, 203466, 203603, 203834, or PTA-256.

**34.** A chimeric molecule comprising a polypeptide according to any one of Claims 31 to 33 fused to a heterologous amino acid sequence.

**35.** The chimeric molecule of Claim 34, wherein said heterologous amino acid sequence is an epitope tag sequence.

**36.** The chimeric molecule of Claim 34, wherein said heterologous amino acid sequence is a Fc region of an immunoglobulin.

**37.** An antibody which specifically binds to a polypeptide according to any one of Claims 31 to 33.

**38.** The antibody of Claim 37, wherein said antibody is a monoclonal antibody, a humanized antibody or a single-chain antibody.

**39.** Isolated nucleic acid having at least 80% nucleic acid sequence identity to:

(a) a nucleotide sequence encoding the polypeptide shown in Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46), Figure 50 (SEQ ID NO:50), Figure 52 (SEQ ID NO:52), Figure 54 (SEQ ID NO:54), and Figure 56 (SEQ ID NO:56), lacking its associated signal peptide:
(b) a nucleotide sequence encoding an extracellular domain of the polypeptide shown in Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46), Figure 50 (SEQ ID NO:50), Figure 52 (SEQ ID NO:52), Figure 54 (SEQ ID NO:54), and Figure 56 (SEQ ID NO:56), with its associated signal peptide; or
(c) a nucleotide sequence encoding an extracellular domain of the polypeptide shown in Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46), Figure 50 (SEQ ID NO:50), Figure 52 (SEQ ID NO:52), Figure 54 (SEQ ID NO:54), and Figure 56 (SEQ ID NO:56), lacking its associated signal peptide.

**40.** An isolated polypeptide having at least 80% amino acid sequence identity to:

(a) the polypeptide shown in Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46), Figure 50 (SEQ ID NO:50), Figure 52 (SEQ ID NO:52), Figure 54 (SEQ ID NO:54), and Figure 56 (SEQ ID NO:

56), lacking its associated signal peptide;
(b) an extracellular domain of the polypeptide shown in Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46), Figure 50 (SEQ ID NO:50), Figure 52 (SEQ ID NO:52), Figure 54 (SEQ ID NO:54), and Figure 56 (SEQ ID NO:56), with its associated signal peptide; or
(c) an extracellular domain of the polypeptide shown in Figure 48 (SEQ ID NO:48), Figure 2 (SEQ ID NO:2), Figure 4 (SEQ ID NO:4), Figure 6 (SEQ ID NO:6), Figure 8 (SEQ ID NO:8), Figure 10 (SEQ ID NO:10), Figure 12 (SEQ ID NO:12), Figure 14 (SEQ ID NO:14), Figure 16 (SEQ ID NO:16), Figure 18 (SEQ ID NO:18), Figure 20 (SEQ ID NO:20), Figure 22 (SEQ ID NO:22), Figure 24 (SEQ ID NO:24), Figure 26 (SEQ ID NO:26), Figure 28 (SEQ ID NO:28), Figure 30 (SEQ ID NO:30), Figure 32 (SEQ ID NO:32), Figure 34 (SEQ ID NO:34), Figure 36 (SEQ ID NO:36), Figure 38 (SEQ ID NO:38), Figure 40 (SEQ ID NO:40), Figure 44 (SEQ ID NO:44), Figure 46 (SEQ ID NO:46), Figure 50 (SEQ ID NO:50), Figure 52 (SEQ ID NO:52), Figure 54 (SEQ ID NO:54), and Figure 56 (SEQ ID NO:56), lacking its associated signal peptide.

**FIGURE 1**

GGGAACGGAAA<u>ATG</u>GCGCCTCACGGCCCGGGTAGTCTTACGACCCTGGTGCCCTGGGCTGCCGCCCTGCTC
CTCGCTCTGGGCGTGGAAAGGGCTCTGGCGCTACCCGAGATATGCACCCAATGTCCAGGGAGCGTGCAAAA
TTTGTCAAAAGTGGCCTTTTATTGTAAAACGACACGAGAGCTAATGCTGCATGCCCGTTGCTGCCTGAATC
AGAAGGGCACCATCTTGGGGCTGGATCTCCAGAACTGTTCTCTGGAGGACCCTGGTCCAAACTTTCATCAG
GCACATACCACTGTCATCATAGACCTGCAAGCAAACCCCCTCAAAGGTGACTTGGCCAACACCTTCCGTGG
CTTTACTCAGCTCCAGACTCTGATACTGCCACAACATGTCAACTGTCCTGGAGGAATTAATGCCTGGAATA
CTATCACCTCTTATATAGACAACCAAATCTGTCAAGGGCAAAAGAACCTTTGCAATAACACTGGGGACCCA
GAAATGTGTCCTGAGAATGGATCTTGTGTACCTGATGGTCCAGGTCTTTTGCAGTGTGTTTGTGCTGATGG
TTTCCATGGATACAAGTGTATGCGCCAGGGCTCGTTCTCACTGCTTATGTTCTTCGGGATTCTGGGAGCCA
CCACTCTATCCGTCTCCATTCTGCTTTGGGCGACCCAGCGCCGAAAAGCCAAGACTTCA<u>TGA</u>ACTACATAG
GTCTTACCATTGACCTAAGATCAATCTGAACTATCTTAGCCCAGTCAGGGAGCTCTGCTTCCTAGAAAGGC
ATCTTTCGCCAGTGGATTCGCCTCAAGGTTGAGGCCGCCATTGGAAGATGAAAAATTGCACTCCCTTGGTG
TAGACAAATACCAGTTCCCATTGGTGTTGTTGCCTATAATAAACACTTTTTCTTTTTTNAAAAAAAAAAAA
AAAAAAAAA

## FIGURE 2

Signal peptide: Amino acids 1-30

Transmembrane domain: Amino acids 198-212

MAPHGPGSLTTLVPWAAALLLALGVERALALPEICTQCPGSVQNLSKVAFYCKTTRELMLHARCCLNQKGT
ILGLDLQNCSLEDPGPNFHQAHTTVIIDLQANPLKGDLANTFRGFTQLQTLILPQHVNCPGGINAWNTITS
YIDNQICQGQKNLCNNTGDPEMCPENGSCVPDGPGLLQCVCADGFHGYKCMRQGSFSLLMFFGILGATTLS
VSILLWATQRRKAKTS

FIGURE 3

TTCGTGACCCTTGAGAAAAGAGTTGGTGGTAAATGTGCCACGTCTTCTAAGAAGGGGGAGTCCTGAACTTG
TCTGAAGCCCTTGTCCGTAAGCCTTGAACTACGTTCTTAAATCTATGAAGTCGAGGGACCTTTCGCTGCTT
TTGTAGGGACTTCTTTCCTTGCTTCAGCAAC<u>ATG</u>AGGCTTTTCTTGTGGAACGCGGTCTTGACTCTGTTCG
TCACTTCTTTGATTGGGGCTTTGATCCCTGAACCAGAAGTGAAAATTGAAGTTCTCCAGAAGCCATTCATC
TGCCATCGCAAGACCAAAGGAGGGGATTTGATGTTGGTCCACTATGAAGGCTACTTAGAAAAGGACGGCTC
CTTATTTCACTCCACTCACAAACATAACAATGGTCAGCCCATTTGGTTTACCCTGGGCATCCTGGAGGCTC
TCAAAGGTTGGGACCAGGGCTTGAAAGGAATGTGTGTAGGAGAGAAGAGAAAGCTCATCATTCCTCCTGCT
CTGGGCTATGGAAAAGAAGGAAAAGGTAAAATTCCCCCAGAAAGTACACTGATATTTAATATTGATCTCCT
GGAGATTCGAAATGGACCAAGATCCCATGAATCATTCCAAGAAATGGATCTTAATGATGACTGGAAACTCT
CTAAAGATGAGGTTAAAGCATATTTAAAGAAGGAGTTTGAAAAACATGGTGCGGTGGTGAATGAAAGTCATCAT
GATGCTTTGGTGGAGGATATTTTTGATAAAGAAGATGAAGACAAAGATGGGTTTATATCTGCCAGAGAATT
TACATATAAACACGATGAGTTA<u>TAG</u>AGATACATCTACCCTTTTAATATAGCACTCATCTTTCAAGAGAGGG
CAGTCATCTTTAAAGAACATTTTATTTTTATACAATGTTCTTTCTTGCTTTGTTTTTTATTTTTATATATT
TTTTCTGACTCCTATTTAAAGAACCCCTTAGGTTTCTAAGTACCCATTTCTTTCTGATAAGTTATTGGGAA
GAAAAAGCTAATTGGTCTTTGAATAGAAGACTTCTGGACAATTTTTCACTTTCACAGATATGAAGCTTTGT
TTTACTTTCTCACTTATAAATTTAAAATGTTGCAACTGGGAATATACCACGACATGAGACCAGGTTATAGC
ACAAATTAGCACCCTATATTTCTGCTTCCCTCTATTTTCTCCAAGTTAGAGGTCAACATTTGAAAAGCCTT
TTGCAATAGCCCAAGGCTTGCTATTTTCATGTTATAATGAAATAGTTTATGTGTAACTGGCTCTGAGTCTC
TGCTTGAGGACCAGAGGAAAATGGTTGTTGGACCTGACTTGTTAATGGCTACTGCTTTACTAAGGAGATGT
GCAATGCTGAAGTTAGAAACAAGGTTAATAGCCAGGCATGGTGGCTCATGCCTGTAATCCCAGCACTTTGG
GAGGCTGAGGCGGGCGGATCACCTGAGGTTGGGAGTTCGAGACCAGCCTGACCAACACGGAGAAACCCTAT
CTCTACTAAAAATACAAAGTAGCCCGGCGTGGTGATGCGTGCCTGTAATCCCAGCTACCCAGGAAGGCTGA
GGCGGCAGAATCACTTGAACCCGAGGCCGAGGTTGCGGTAAGCCGAGATCACCTNCAGCCTGGACACTCTG
TCTCGAAAAAAGAAAAGAACACGGTTAATACCATATNAATATGTATGCATTGAGACATGCTACCTAGGACT
TAAGCTGATGAAGCTTGGCTCCTAGTGATTGGTGGCCTATTATGATAAATAGGACAAATCATTTATGTGTG
AGTTTCTTTGTAATAAAATGTATCAATATGTTATAGATGAGGTAGAAAGTTATATTTATATTCAATATTTA
CTTCTTAAGGCTAGCGGAATATCCTTCCTGGTTCTTTAATGGGTAGTCTATAGTATATTATACTACAATAA
CATTGTATCATAAGATAAAGTAGTAAACCAGTCTACATTTTCCCATTTCTGTCTCATCAAAAACTGAAGTTAGC
TGGGTGTGGTGGCTCATGCCTGTAATCCCAGCACTTTGGGGGCCAAGGAGGGTGGATCACTTGAGATCAGG
AGTTCAAGACCAGCCTGGCCAACATGGTGAAACCTTGTCTCTACTAAAAATACAAAAATTAGCCAGGCGTG
GTGGTGCACACCTGTAGTCCCAGCTACTCGGGAGGCTGAGACAGGAGATTTGCTTGAACCCGGGAGGCGGA
GGTTGCAGTGAGCCAAGATTGTGCCACTGCACTCCAGCCTGGGTGACAGAGCAAGACTCCATCTCAAAAAA
AAAAAAAAGAAGCAGACCTACAGCAGCTACTATTGAATAAATACCTATCCTGGATTTT

## FIGURE 4

MRLFLWNAVLTLFVTSLIGALIPEPEVKIEVLQKPFICHRKTKGGDLMLVHYEGYLEKDGSLFHSTHKHNN
GQPIWFTLGILEALKGWDQGLKGMCVGEKRKLIIPPALGYGKEGKGKIPPESTLIFNIDLLEIRNGPRSHE
SFQEMDLNDDWKLSKDEVKAYLKKEFEKHGAVVNESHHDALVEDIFDKEDEDKDGFISAREFTYKHDEL

Signal peptide: Amino acids 1-20

N-glycosylation site: Amino acids 176-180

Endoplasmic reticulum targeting sequence: Amino acids 208-212

FKBP-type peptidyl-prolyl cis-trans isomerase: Amino acids 78-115;118-132

EF-hand calcium-binding domains: Amino acids 140-160;184-204;191-204

S-100/ICaBP type calcium binding domain: Amino acids 183-201

## FIGURE 5

ACCCACGCGTCCGCCCACGCGTCCGCCCACGCGTCCGCCCACGCGTCCGCGCGTAGCCGTGCGCCGATTGC
CTCTCGGCCTGGGCA**ATG**GTCCCGGCTGCCGGTCGACGACCGCCCCGCGTCATGCGGCTCCTCGGCTGGTG
GCAAGTATTGCTGTGGGTGCTGGGACTTCCCGTCCGCGGCGTGGAGGTTGCAGAGGAAAGTGGTCGCTTAT
GGTCAGAGGAGCAGCCTGCTCACCCTCTCCAGGTGGGGGCTGTGTACCTGGGTGAGGAGGAGCTCCTGCAT
GACCCGATGGGCCAGGACAGGGCAGCAGAAGAGGCCAATGCGGTGCTGGGGCTGGACACCCAAGGCGATCA
CATGGTGATGCTGTCTGTGATTCCTGGGGAAGCTGAGGACAAAGTGAGTTCAGAGCCTAGCGGCGTCACCTGT
GGTGCTGGAGGAGCGGAGGACTCAAGGTGCAACGTCCGAGAGAGCCTTTTCTCTCTGGATGGCGCTGGAGC
ACACTTCCCTGACAGAGAAGAGGAGTATTACACAGAGCCAGAAGTGGCGGAATCTGACGCAGCCCCGACAG
AGGACTCCAATAACACTGAAAGTCTGAAATCCCCAAAGGTGAACTGTGAGGAGAGAAACATTACAGGATTA
GAAAATTTCACTCTGAAAATTTTAAATATGTCACAGGACCTTATGGATTTTCTGAACCCAAACGGTAGTGA
CTGTACTCTAGTCCTGTTTTACACCCCGTGGTGCCGCTTTTCTGCCAGTTTGGCCCCTCACTTTAACTCTC
TGCCCCGGGCATTTCCAGCTCTTCACTTTTTGGCACTGGATGCATCTCAGCACAGCAGCCTTTCTACCAGG
TTTGGCACCGTAGCTGTTCCTAATATTTTATTATTTCAAGGAGCTAAACCAATGGCCAGATTTAATCATAC
AGATCGAACACTGGAAACACTGAAAATCTTCATTTTTAATCAGACAGGTATAGAAGCCAAGAAGAATGTGG
TGGTAACTCAAGCCGACCAAATAGGCCCTCTTCCCAGCACTTTGATAAAAAGTGTGGACTGGTTGCTTGTA
TTTTCCTTATTCTTTTTAATTAGTTTTATTATGTATGCTACCATTCGAACTGAGAGTATTCGGTGGCTAAT
TCCAGGACAAGAGCAGGAACATGTGGAG**TAG**TGATGGTCTGAAAGAAGTTGGAAAGAGGAACTTCAATCCT
TCGTTTCAGAAATTAGTGCTACAGTTTCATACATTTTCTCCAGTGACGTGTTGACTTGAAACTTCAGGCAG
ATTAAAAGAATCATTTGTTGAACAACTGAATGTATAAAAAAATTATAAACTGGTGTTTTAACTAGTATTGC
AATAAGCAAATGCAAAAATATTCAATAG

## FIGURE 6

MVPAAGRRPPRVMRLLGWWQVLLWVLGLPVRGVEVAEESGRLWSEEQPAHPLQVGAVYLGEEELLHDPMGQ
DRAAEEANAVLGLDTQGDHMVMLSVIPGEAEDKVSSEPSGVTCGAGGAEDSRCNVRESLFSLDGAGAHFPD
REEEYYTEPEVAESDAAPTEDSNNTESLKSPKVNCEERNITGLENFTLKILNMSQDLMDFLNPNGSDCTLV
LFYTPWCRFSASLAPHFNSLPRAFPALHFLALDASQHSSLSTRFGTVAVPNILLFQGAKPMARFNHTDRTL
ETLKIFIFNQTGIEAKKNVVVTQADQIGPLPSTLIKSVDWLLVFSLFFLISFIMYATIRTESIRWLIPGQE
QEHVE

| | |
|---|---|
| Signal peptide: | Amino acids 1-25 |
| Transmembrane domain: | Amino acids 321-340 |
| Homologous region to dilsufide isomerase: | Amino acids 212-302 |
| N-glycosylation sites: | Amino acids 165-169;181-185;187-191; 194-198;206-210;278-282;293-297 |
| N-myristoylation sites: | Amino acids 32-38;70-76;111-117; 115-121;118-124;207-213 |
| Amidation site: | Amino acids 5-9 |
| Thioredoxin domain: | Amino acids 211-228 |

## FIGURE 7

TGCGGCGACCGTCGTACACCATGGGCCTCCACCTCCGCCCCTACCGTGTGGGGCTGCTCCCGGATGGCCTC
CTGTTCCTCTTGCTGCTGCTAATGCTGCTCGCGGACCCAGCGCTCCCGGCCGGACGTCACCCCCCAGTGGT
GCTGGTCCCTGGTGATTTGGGTAACCAACTGGAAGCCAAGCTGGACAAGCCGACAGTGGTGCACTACCTCT
GCTCCAAGAAGACCGAAAGCTACTTCACAATCTGGCTGAACCTGGAACTGCTGCTGCCTGTCATCATTGAC
TGCTGGATTGACAATATCAGGCTGGTTTACAACAAAACATCCAGGGCCACCCAGTTTCCTGATGGTGTGGA
TGTACGTGTCCCTGGCTTTGGGAAGACCTTCTCACTGGAGTTCCTGGACCCCAGCAAAAGCAGCGTGGGTT
CCTATTTCCACACCATGGTGGAGAGCCTTGTGGGCTGGGGCTACACACGGGGTGAGGATGTCCGAGGGGCTCCC
TATGACTGGCGCCGAGCCCCAAATGAAAACGGGCCCTACTTCCTGGCCCTCCGCGAGATGATCGAGGAGAT
GTACCAGCTGTATGGGGGCCCCGTGGTGCTGGTTGCCCACAGTATGGGCAACATGTACACGCTCTACTTTC
TGCAGCGGCAGCCGCAGGCCTGGAAGGACAAGTATATCCGGGCCTTCGTGTCACTGGGTGCGCCCTGGGGG
GGCGTGGCCAAGACCCTGCGCGTCCTGGCTTCAGGAGACAACAACCGGATCCCAGTCATCGGGCCCCTGAA
GATCCGGGAGCAGCAGCGGTCAGCTGTCTCCACCAGCTGGCTGCTGCCCTACAACTACACATGGTCACCTG
AGAAGGTGTTCGTGCAGACACCCACAATCAACTACACACTGCGGGACTACCGCAAGTTCTTCCAGGACATC
GGCTTTGAAGATGGCTGGCTCATGCGGCAGGACACAGAAGGGCTGGTGGAAGCCACGATGCCACCTGGCGT
GCAGCTGCACTGCCTCTATGGTACTGGCGTCCCCACACCAGACTCCTTCTACTATGAGAGCTTCCCTGACC
GTGACCCTAAAATCTGCTTTGGTGACGGCGATGGTACTGTGAACTTGAAGAGTGCCCTGCAGTGCCAGGCC
TGGCAGAGCCGCCAGGAGCACCAAGTGTTGCTGCAGGAGCTGCCAGGCAGCGAGCACATCGAGATGCTGGC
CAACGCCACCACCCTGGCCTATCTGAAACGTGTGCTCCTTGGGCCCTGACTCCTGTGCCACAGGACTCCTG
TGGCTCGGCCGTGGACCTGCTGTTGGCCTCTGGGGCTGTCATGGCCCACGCGTTTTGCAAAGTTTGTGACT
CACCATTCAAGGCCCCGAGTCTTGGACTGTGAAGCATCTGCCATGGGGAAGTGCTGTTTGTTATCCTTTCTCTG
TGGCAGTGAAGAAGGAAGAAATGAGAGTCTAGACTCAAGGGACACTGGATGGCAAGAATGCTGCTGATGGT
GGAACTGCTGTGACCTTAGGACTGGCTCCACAGGGTGGACTGGCTGGGCCCTGGTCCCAGTCCCTGCCTGG
GGCCATGTGTCCCCCTATTCCTGTGGGCTTTTCATACTTGCCTACTGGGCCCTGGCCCCGCAGCCTTCCTA
TGAGGGATGTTACTGGGCTGTGGTCCTGTACCCAGAGGTCCCAGGGATCGGCTCCTGGCCCCTCGGGTGAC
CCTTCCCACACACCAGCCACAGATAGGCCTGCCACTGGTCATGGGTAGCTAGAGCTGCTGGCTTCCCTGTG
GCTTAGCTGGTGGCCAGCCTGACTGGCTTCCTGGGCGAGCCTAGTAGCTCCTGCAGGCAGGGGCAGTTTGT
TGCGTTCTTCGTGGTTCCCAGGCCCTGGGACATCTCACTCCACTCCTACCTCCCTTACCACCAGGAGCATT
CAAGCTCTGGATTGGGCAGCAGATGTGCCCCCAGTCCCGCAGGCTGTGTTCCAGGGGCCCTGATTTCCTCG
GATGTGCTATTGGCCCCAGGACTGAAGCTGCCTCCCTTCACCCTGGGACTGTGGTTCCAAGGATGAGAGCA
GGGGTTGGAGCCATGGCCTTCTGGGAACCTATGGAGAAAGGGAATCCAAGGAAGCAGCCAAGGCTGCTCGC
AGCTTCCCTGAGCTGCACCTCTTGCTAACCCCACCATCACACTGCCACCCTGCCCTAGGGTCTCACTAGTA
CCAAGTGGGTCAGCACAGGGCTGAGGATGGGGCTCCTATCCACCCTGGCCAGCACCCAGCTTAGTGCTGGG
ACTAGCCCAGAAACTTGAATGGGACCCTGAGAGAGCCAGGGGTCCCCTGAGGCCCCCCTAGGGGCTTTCTG
TCTGCCCCAGGGTGCTCCATGGATCTCCCTGTGGCAGCAGGCATGGAGAGTCAGGGCTGCCTTCATGGCAG
TAGGCTCTAAGTGGGTGACTGGCCACAGGCCGAGAAAAGGGTACAGCCTCTAGGTGGGGTTCCCAAAGACG
CCTTCAGGCTGGACTGAGCTGCTCTCCCACAGGGTTTCTGTGCAGCTGGATTTTCTCTGTTGCATACATGC
CTGGCATCTGTCTCCCCTTGTTCCTGAGTGGCCCCACATGGGGCTCTGAGCAGGCTGTATCTGGATTCTGG
CAATAAAAGTACTCTGGATGCTGTAAAAAAAAAAAAAAAAAAAAA

## FIGURE 8

MGLHLRPYRVGLLPDGLLFLLLLLMLLADPALPAGRHPPVVLVPGDLGNQLEAKLDKPTVVHYLCSKKTES
YFTIWLNLELLLPVIIDCWIDNIRLVYNKTSRATQFPDGVDVRVPGFGKTFSLEFLDPSKSSVGSYFHTMV
ESLVGWGYTRGEDVRGAPYDWRRAPNENGPYFLALREMIEEMYQLYGGPVVLVAHSMGNMYTLYFLQRQPQ
AWKDKYIRAFVSLGAPWGGVAKTLRVLASGDNNRIPVIGPLKIREQQRSAVSTSWLLPYNYTWSPEKVFVQ
TPTINYTLRDYRKFFQDIGFEDGWLMRQDTEGLVEATMPPGVQLHCLYGTGVPTPDSFYYESFPDRDPKIC
FGDGDGTVNLKSALQCQAWQSRQEHQVLLQELPGSEHIEMLANATTLAYLKRVLLGP

| | |
|---|---|
| Signal peptide: | Amino acids 1-28 |
| Potential lipid substrate binding site: | Amino acids 147-164 |
| N-glycosylation sites: | Amino acids 99-103;273-277;289-293; 398-402 |
| Lipases, serine proteins family: | Amino acids 189-202 |
| Beta-transducin family Trp-Asp repeat: | Amino acids 353-366 |
| Tyrosine kinase phosphorylation sites: | Amino acids 165-174;178-186 |
| N-myristoylation sites: | Amino acids 200-206;227-233;232-238; 316-322 |

## FIGURE 9

CTAAGAGGACAAGATGAGGCCCGGCCTCTCATTTCTCCTAGCCCTTCTGTTCTTCCTTGGCCAAGCTGCAGGG
GATTTGGGGGATGTGGGACCTCCAATTCCCAGCCCCGGCTTCAGCTCTTTCCCAGGTGTTGACTCCAGCTC
CAGCTTCAGCTCCAGCTCCAGGTCGGGCTCCAGCTCCAGCCGCAGCTTAGGCAGCGGAGGTTCTGTGTCCC
AGTTGTTTTCCAATTTCACCGGCTCCGTGGATGACCGTGGGACCTGCCAGTGCTCTGTTTCCCTGCCAGAC
ACCACCTTTCCCGTGGACAGAGTGGAACGCTTGGAATTCACAGCTCATGTTCTTTCTCAGAAGTTTGAGAA
AGAACTTTCTAAAGTGAGGGAATATGTCCAATTAATTAGTGTGTATGAAAAGAAACTGTTAAACCTAACTG
TCCGAATTGACATCATGGAGAAGGATACCATTTCTTACACTGAACTGGACTTCGAGCTGATCAAGGTAGAA
GTGAAGGAGATGGAAAAACTGGTCATACAGCTGAAGGAGAGTTTTGGTGGAAGCTCAGAAATTGTTGACCA
GCTGGAGGTGGAGATAAGAAATATGACTCTCTTGGTAGAGAAGCTTGAGACACTAGACAAAAACAATGTCC
TTGCCATTCGCCGAGAAATCGTGGCTCTGAAGACCAAGCTGAAAGAGTGTGAGGCCTCTAAAGATCAAAAC
ACCCCTGTCGTCCACCCTCCTCCCACTCCAGGGAGCTGTGGTCATGGTGGTGTGGTGAACATCAGCAAACC
GTCTGTGGTTCAGCTCAACTGGAGAGGGTTTTCTTATCTATATGGTGCTTGGGGTAGGGATTACTCTCCCC
AGCATCCAAACAAAGGACTGTATTGGGTGGCGCCATTGAATACAGATGGGAGACTGTTGGAGTATTATAGA
CTGTACAACACACTGGATGATTTGCTATTGTATATAAATGCTCGAGAGTTGCGGATCACCTATGGCCAAGG
TAGTGGTACAGCAGTTTACAACAACAACATGTACGTCAACATGTACAACACCGGGAATATTGCCAGAGTTA
ACCTGACCACCAACACGATTGCTGTGACTCAAACTCTCCCTAATGCTGCCTATAATAACCGCTTTTCATAT
GCTAATGTTGCTTGGCAAGATATTGACTTTGCTGTGGATGAGAATGGATTGTGGGTTATTTATTCAACTGA
AGCCAGCACTGGTAACATGGTGATTAGTAAACTCAATGACACCACACTTCAGGTGCTAAACACTTGGTATA
CCAAGCAGTATAAACCATCTGCTTCTAACGCCTTCATGGTATGTGGGGTTCTGTATGCCACCCGTACTATG
AACACCAGAACAGAAGAGATTTTTTACTATTATGACACAAACACAGGGAAAGAGGGCAAACTAGACATTGT
AATGCATAAGATGCAGGAAAAAGTGCAGAGCATTAACTATAACCCTTTTGACCAGAAACTTTATGTCTATA
ACGATGGTTACCTTCTGAATTATGATCTTTCTGTCTTGCAGAAGCCCCAGTAAGCTGTTTAGGAGTTAGGG
TGAAAGAGAAAATGTTTGTTGAAAAAATAGTCTTCTCCACTTACTTAGATATCTGCAGGGGTGTCTAAAAG
TGTGTTCATTTTGCAGCAATGTTTAGGTGCATAGTTCTACCACACTAGAGATCTAGGACATTTGTCTTGAT
TTGGTGAGTTCTCTTGGGAATCATCTGCCTCTTCAGGCGCATTTTGCAATAAAGTCTGTCTAGGGTGGGAT
TGTCAGAGGTCTAGGGGCACTGTGGCCTAGTGAAGCCTACTGTGAGGAGGCTTCACTAGAAGCCTTAAAT
TAGGAATTAAGGAACTTAAAACTCAGTATGGCGTCTAGGGATTCTTTGTACAGGAAATATTGCCCAATGAC
TAGTCCTCATCCATGTAGCACCACTAATTCTTCCATGCCTGGAAGAAACCTGGGGACTTAGTTAGGTAGATTAA
TATCTGGAGCTCCTCGAGGGACCAAATCTCCAACTTTTTTTCCCCTCACTAGCACCTGGAATGATGCTTT
GTATGTGGCAGATAAGTAAATTTGGCATGCTTATATATTCTACATCTGTAAAGTGCTGAGTTTTATGGAGA
GAGGCCTTTTTATGCATTAAATTGTACATGGCAAATAAATCCCAGAAGGATCTGTAGATGAGGCACCTGCT
TTTTCTTTTCTCTCATTGTCCACCTTACTAAAAGTCAGTAGAATCTTCTACCTCATAACTTCCTTCCAAAG
GCAGCTCAGAAGATTAGAACCAGACTTACTAACCAATTCCACCCCCCACCAACCCCCTTCTACTGCCTACT
TTAAAAAAATTAATAGTTTTCTATGGAACTGATCTAAGATTAGAAAAATTAATTTTCTTTAATTTCATTAT
GGACTTTTATTTACATGACTCTAAGACTATAAGAAAATCTGATGGCAGTGACAAAGTGCTAGCATTTATTG
TTATCTAATAAAGACCTTGGAGCATATGTGCAACTTATGAGTGTATCAGTTGTTGCATGTAATTTTTGCCT
TTGTTTAAGCCTGGAACTTGTAAGAAAATGAAAATTTAATTTTTTTTTCTAGGACGAGCTATAGAAAAGCT
ATTGAGAGTATCTAGTTAATCAGTGCAGTAGTTGGAAACCTTGCTGGTGTATGTGATGTGCTTCTGTGCTT
TTGAATGACTTTATCATCTAGTCTTTGTCTATTTTTCCTTTGATGTTCAAGTCCTAGTCTATAGGATTGGC
AGTTTAAATGCTTTACTCCCCCTTTTAAAATAAATGATTAAAATGTGCTTTGAAAAAAAAAAAAAAAAAAA
AAAAAAAAA

## FIGURE 10

MRPGLSFLLALLFFLGQAAGDLGDVGPPIPSPGFSSFPGVDSSSSFSSSSRSGSSSSRSLGSGGSVSQLFS
NFTGSVDDRGTCQCSVSLPDTTFPVDRVERLEFTAHVLSQKFEKELSKVREYVQLISVYEKKLLNLTVRID
IMEKDTISYTELDFELIKVEVKEMEKLVIQLKESFGGSSEIVDQLEVEIRNMTLLVEKLETLDKNNVLAIR
REIVALKTKLKECEASKDQNTPVVHPPPTPGSCGHGGVVNISKPSVVQLNWRGFSYLYGAWGRDYSPQHPN
KGLYWVAPLNTDGRLLEYYRLYNTLDDLLLYINARELRITYGQGSGTAVYNNNMYVNMYNTGNIARVNLTT
NTIAVTQTLPNAAYNNRFSYANVAWQDIDFAVDENGLWVIYSTEASTGNMVISKLNDTTLQVLNTWYTKQY
KPSASNAFMVCGVLYATRTMNTRTEEIFYYYDTNTGKEGKLDIVMHKMQEKVQSINYNPFDQKLYVYNDGY
LLNYDLSVLQKPQ

| | |
|---|---|
| Signal peptide: | Amino acids 1-20 |
| N-glycosylation sites: | Amino acids 72-76;136-140;193-197; 253-257;352-356;411-415 |
| Tyrosine kinase phosphorylation site: | Amino acids 449-457 |
| N-myristoylation sites: | Amino acids 16-22;39-45;53-59;61-67; 63-69;81-87;249-255;326-332;328-334; 438-444 |
| Legume lectins beta-chain proteins: | Amino acids 20-40 |
| HBGF/FGF family proteins: | Amino acids 338-366 |

**FIGURE 11**

ATTCTCCTAGAGCATCTTTGGAAGC**ATG**AGGCCACGATGCTGCATCTTGGCTCTTGTCTGCTGGATAACAG
TCTTCCTCCTCCAGTGTTCAAAAGGAACTACAGACGCTCCTGTTGGCTCAGGACTGTGGCTGTGCCAGCCGACA
CCCAGGTGTGGGAACAAGATCTACAACCCTTCAGAGCAGTGCTGTTATGATGATGCCATCTTATCCTTAAA
GGAGACCCGCCGCTGTGGCTCCACCTGCACCTTCTGGCCCTGCTTTGAGCTCTGCTGTCCCGAGTCTTTTG
GCCCCCAGCAGAAGTTTCTTGTGAAGTTGAGGGTTCTGGGTATGAAGTCTCAGTGTCACTTATCTCCCATC
TCCCGGAGCTGTACCAGGAACAGGAGGCACGTCCTGTACCCA**TAA**AAACCCCAGGCTCCACTGGCAGACGG
CAGACAAGGGGAGAAGAGACGAAGCAGCTGGACATCGGAGACTACAGTTGAACTTCGGAGAGAAGCAACTT
GACTTCAGAGGGATGGCTCAATGACATAGCTTTGGAGAGGAGCCCAGCTGGGGATGGCCAGACTTCAGGGG
AAGAATGCCTTCCTGCTTCATCCCCTTTCCAGCTCCCCTTCCCGCTGAGAGCCACTTTCATCGGCAATAAA
ATCCCCCACATTTACCATCT

## FIGURE 12

MRPRCCILALVCWITVFLLQCSKGTTDAPVGSGLWLCQPTPRCGNKIYNPSEQCCYDDAILSLKETRRCGS
TCTFWPCFELCCPESFGPQQKFLVKLRVLGMKSQCHLSPISRSCTRNRRHVLYP

| | |
|---|---|
| Signal peptide: | Amino acids 1-21 |
| N-myristoylation sites: | Amino acids 33-39;70-76 |
| Anaphylatoxin domain proteins: | Amino acids 50-60 |

FIGURE 13

CCTCTGTCCACTGCTTTCGTGAAGACAAGATGAAGTTCACAATTGTCTTTGCTGGACTTCTTGGAGTCTTT
CTAGCTCCTGCCCTAGCTAACTATAATATCAACGTCAATGATGACAACAACAATGCTGGAAGTGGGCAGCA
GTCAGTGAGTGTCAACAATGAACACAATGTGGCCAATGTTGACAATAACAACGGATGGGACTCCTGGAATT
CCATCTGGGATTATGGAAATGGCTTTGCTGCAACCAGACTCTTTCAAAAGAAGACATGCATTGTGCACAAA
ATGAACAAGGAAGTCATGCCCTCCATTCAATCCCTTGATGCACTGGTCAAGGAAAAGAAGCTTCAGGGTAA
GGGACCAGGAGGACCACCTCCCAAGGGCCTGATGTACTCAGTCAACCCAAACAAAGTCGATGACCTGAGCA
AGTTCGGAAAAAACATTGCAAACATGTGTCGTGGGATTCCAACATACATGGCTGAGGAGATGCAAGAGGCA
AGCCTGTTTTTTACTCAGGAACGTGCTACACGACCAGTGTACTATGGATTGTGGACATTTCCTTCTGTGGA
GACACGGTGGAGAACTAAACAATTTTTTAAAGCCACTATGGATTTAGTCATCTGAATATGCTGTGCAGAAA
AAATATGGGCTCCAGTGGTTTTTACCATGTCATTCTGAAATTTTTCTCTACTAGTTATGTTTGATTTCTTT
AAGTTTCAATAAAATCATTTAGCATTGAAAAAAA

## FIGURE 14

MKFTIVFAGLLGVFLAPALANYNINVNDDNNNAGSGQQSVSVNNEHNVANVDNNNGWDSWNSIWDYGNGFA
ATRLFQKKTCIVHKMNKEVMPSIQSLDALVKEKKLQGKGPGGPPPKGLMYSVNPNKVDDLSKFGKNIANMC
RGIPTYMAEEMQEASLFFYSGTCYTTSVLWIVDISFCGDTVEN

| | |
|---|---|
| Signal peptide: | Amino acids 1-20 |
| N-myristoylation sites: | Amino acids 67-73;118-124;163-169 |
| Flavodoxin proteins: | Amino acids 156-175 |

# FIGURE 15

GAGCAGGACGGAGCC**ATG**GACCCCGCCAGGAAAGCAGGTGCCCAGGCCATGATCTGGACTGCAGGCTGGCT
GCTGCTGCTGCTGCTTCGCGGAGGAGCGCAGGCCCTGGAGTGCTACAGCTGCGTGCAGAAAGCAGATGACG
GATGCTCCCCGAACAAGATGAAGACAGTGAAGTGCGCGCCGGGCGTGGACGTCTGCACCGAGGCCGTGGGG
GCGGTGGAGACCATCCACGGACAATTCTCGCTGGCAGTGCGGGGTTGCGGTTCGGGACTCCCCGGCAAGAA
TGACCGCGGCCTGGATCTTCACGGGCTTCTGGCGTTCATCCAGCTGCAGCAATGCGCTCAGGATCGCTGCA
ACGCCAAGCTCAACCTCACCTCGCGGGCGCTCGACCCGGCAGGTAATGAGAGTGCATACCCGCCCAACGGC
GTGGAGTGCTACAGCTGTGTGGGCCTGAGCCGGGAGGCGTGCCAGGGTACATCGCCGCCGGTCGTGAGCTG
CTACAACGCCAGCGATCATGTCTACAAGGGCTGCTTCGACGGCAACGTCACCTTGACGGCAGCTAATGTGA
CTGTGTCCTTGCCTGTCCGGGGCTGTGTCCAGGATGAATTCTGCACTCGGGATGGAGTAACAGGCCCAGGG
TTCACGCTCAGTGGCTCCTGTTGCCAGGGGTCCCGCTGTAACTCTGACCTCCGCAACAAGACCTACTTCTC
CCCTCGAATCCCACCCCTTGTCCGGCTGCCCCCTCCAGAGCCCACGACTGTGGCCTCAACCACATCTGTCA
CCACTTCTACCTCGGCCCCAGTGAGACCCACATCCACCACCAAACCCATGCCAGCGCCAACCAGTCAGACTCCG
AGACAGGGAGTAGAACACGAGGCCTCCCGGGATGAGGAGCCCAGGTTGACTGGAGGCGCCGCTGGCCACCA
GGACCGCAGCAATTCAGGGCAGTATCCTGCAAAAGGGGGCCCCAGCAGCCCCATAATAAAGGCTGTGTGG
CTCCCACAGCTGGATTGGCAGCCCTTCTGTTGGCCGTGGCTGCTGGTGTCCTACTG**TGA**GCTTCTCCACCT
GGAAATTTCCCTCTCACCTACTTCTCTGGCCCTGGGTACCCCTCTTCTCATCACTTCCTGTTCCCACCACT
GGACTGGGCTGGCCCAGCCCCTGTTTTTCCAACATTCCCCAGTATCCCCAGCTTCTGCTGCGCTGGTTTGC
GGCTTTGGGAAATAAAATACCGTTGTATATATTCTGCCAGGGGTGTTCTAGCTTTTTGAGGACAGCTCCTG
TATCCTTCTCATCCTTGTCTCTCCGCTTGTCCTCTTGTGATGTTAGGACAGAGTGAGAGAAGTCAGCTGTC
ACGGGGAAGGTGAGAGAGAGGATGCTAAGCTTCCTACTCACTTTCTCCTAGCCAGCCTGGACTTTGGAGCG
TGGGGTGGGTGGGACAATGGCTCCCCACTCTAAGCACTGCCTCCCCTACTCCCCGCATCTTTGGGGAATCG
GTTCCCCATATGTCTTCCTTACTAGACTGTGAGCTCCTCGAGGGGGGGCCCGGTACCCAATTCGCCCTATA
GTGAGTCGTA

## FIGURE 16

MDPARKAGAQAMIWTAGWLLLLLLLRGGAQALECYSCVQKADDGCSPNKMKTVKCAPGVDVCTEAVGAVETI
HGQFSLAVRGCGSGLPGKNDRGLDLHGLLAFIQLQQCAQDRCNAKLNLTSRALDPAGNESAYPPNGVECYS
CVGLSREACQGTSPPVVSCYNASDHVYKGCFDGNVTLTAANVTVSLPVRGCVQDEFCTRDGVTGPGFTLSG
SCCQGSRCNSDLRNKTYFSPRIPPLVRLPPPEPTTVASTTSVTTSTSAPVRPTSTTKPMPAPTSQTPRQGV
EHEASRDEEPRLTGGAAGHQDRSNSGQYPAKGGPQQPHNKGCVAPTAGLAALLLAVAAGVLL

| | |
|---|---|
| Signal peptide: | Amino acids 1-30 |
| Transmembrane domain: | Amino acids 325-346 |
| N-glycosylation sites: | Amino acids 118-122;129-133;163-167;<br>176-180;183-187;227-231 |
| Ly-6 / u-PAR domain proteins: | Amino acids 17-37;209-223 |
| N-myristoylation sites: | Amino acids 26-32;43-49;57-63;66-72;<br>81-87;128-134;171-171;218-224;<br>298-304;310-316 |

Prokaryotic membrane lipoprotein lipid attachment site:
Amino acids 205-216

**FIGURE 17**

GCAGTCAGAGACTTCCCCTGCCCCTCGCTGGGAAAGAACATTAGGAATGCCTTTTAGTGCCTTGCTTCCTG
AACTAGCTCACAGTAGCCCGGCGGCCCAGGGCAATCCGACCACATTTCACTCTCACCGCTGTAGGAATCCAG
ATGCAGGCCAAGTACAGCAGCACGAGGGACATGCTGGATGATGATGGGGACACCACCATGAGCCTGCATTC
TCAAGCCTCTGCCACAACTCGGCATCCAGAGCCCCGGCGCACAGAGCACAGGGCTCCCTCTTCAACGTGGC
GACCAGTGGCCCTGACCCTGCTGACTTTGTGCTTGGTGCTGCTGATAGGGCTGGCAGCCCTGGGGCTTTTG
TTTTTTCAGTACTACCAGCTCTCCAATACTGGTCAAGACACCATTTCTCAAATGGAAGAAAGATTAGGAAA
TACGTCCCAAGAGTTGCAATCTCTTCAAGTCCAGAATATAAAGCTTGCAGGAAGTCTGCAGCATGTGGCTG
AAAAACTCTGTCGTGAGCTGTATAACAAAGCTGGAGCACACAGGTGCAGCCCTTGTACAGAACAATGGAAA
TGGCATGGAGACAATTGCTACCAGTTCTATAAAGACAGCAAAAGTTGGGAGGACTGTAAATATTTCTGCCT
TAGTGAAAACTCTACCATGCTGAAGATAAACAAACAAGAAGACCTGGAATTTGCCGCGTCTCAGAGCTACT
CTGAGTTTTTCTACTCTTATTGGACAGGGCTTTTGCGCCCTGACAGTGGCAAGGCCTGGCTGTGGATGGAT
GGAACCCCTTTCACTTCTGAACTGTTCCATATTATAATAGATGTCACCAGCCCAAGAAGCAGAGACTGTGTG
GCCATCCTCAATGGGATGATCTTCTCAAAGGACTGCAAAGAATTGAAGCGTTGTGTCTGTGAGAGAAGGGC
AGGAATGGTGAAGCCAGAGAGCCTCCATGTCCCCCCTGAAACATTAGGCGAAGGTGACTGATTCGCCCTCT
GCAACTACAAATAGCAGAGTGAGCCAGGCGGTGCCAAAGCAAGGGCTAGTTGAGACATTGGGAAATGGAAC
ATAATCAGGAAAGACTATCTCTCTGACTAGTACAAAATGGGTTCTCGTGTTTCCTGTTCAGGATCACCAGC
ATTTCTGAGCTTGGGTTTATGCACGTATTTAACAGTCACAAGAAGTCTTATTTACATGCCACCAACCAACC
TCAGAAACCCATAATGTCATCTGCCTTCTTGGCTTAGAGATAACTTTTAGCTCTCTTTCTTCTCAATGTCTAA
TATCACCTCCCTGTTTTCATGTCTTCCTTACACTTGGTGGAATAAGAAACTTTTTGAAGTAGAGGAAATAC
ATTGAGGTAACATCCTTTTCTCTGACAGTCAAGTAGTCCATCAGAAATTGGCAGTCACTTCCCAGATTGTA
CCAGCAAATACACAAGGAATTCTTTTTGTTTGTTTCAGTTCATACTAGTCCCTTCCCAATCCATCAGTAAA
GACCCCATCTGCCTTGTCCATGCCGTTTCCCAACAGGGATGTCACTTGATATGAGAATCTCAAATCTCAAT
GCCTTATAAGCATTCCTTCCTGTGTCCATTAAGACTCTGATAATTGTCTCCCCTCCATAGGAATTTCTCCC
AGGAAAGAAATATATCCCCATCTCCGTTTCATATCAGAACTACCGTCCCCGATATTCCCTTCAGAGAGATT
AAAGACCAGAAAAAAGTGAGCCTCTTCATCTGCACCTGTAATAGTTTCAGTTCCTATTTTCTTCCATTGAC
CCATATTTATACCTTTCAGGTACTGAAGATTTAATAATAATAAATGTAAATACTGTGAAAAA

## FIGURE 18

MQAKYSSTRDMLDDDGDTTMSLHSQASATTRHPEPRRTEHRAPSSTWRPVALTLLTLCLVLLIGLAALGLL
FFQYYQLSNTGQDTISQMEERLGNTSQELQSLQVQNIKLAGSLQHVAEKLCRELYNKAGAHRCSPCTEQWK
WHGDNCYQFYKDSKSWEDCKYFCLSENSTMLKINKQEDLEFAASQSYSEFFYSYWTGLLRPDSGKAWLWMD
GTPFTSELFHIIIDVTSPRSRDCVAILNGMIFSKDCKELKRCVCERRAGMVKPESLHVPPETLGEGD

Transmembrane domain: Amino acids 49-74 (type II)

N-glycosylation sites: Amino acids 95-98;169-172

Homologous region to LDL receptor: Amino acids 50-265

Tyrosine kinase phosphorylation sites:
Amino acids 142-150;156-164

N-myristoylation sites: Amino acids 130-136;214-220;242-248

## FIGURE 19

TGGACTTCTCTGGACCACAGTCCTCTGCCAGACCCCTGCCAGACCCCAGTCCACC**ATG**ATCCATCTGGGTC
ACATCCTCTTCCTGCTTTTGCTCCCAGTGGCTGCAGCTCAGACGACTCCAGGAGAGAGATCATCACTCCCT
GCCTTTTACCCTGGCACTTCAGGCTCTTGTTCCGGATGTGGGTCCCTCTCTCTGCCGCTCCTGGCAGGCCT
CGTGGCTGCTGATGCGGTGGCATCGCTGCTCATCGTGGGGGCGGTGTTCCTGTGCGCACGCCCACGCCGCA
GCCCCGCCCAAGATGGCAAAGTCTACATCAACATGCCAGGCAGGGGC**TGA**CCCTCCTGCAGCTTGGACCTT
TGACTTCTGACCCTCTCATCCTGGATGGTGTGTGGTGGCACAGGAACCCCCGCCCCAACTTTTGGATTGTA
ATAAAACAATTGAAACACCA

**FIGURE 20**

MIHLGHILFLLLLPVAAAQTTPGERSSLPAFYPGTSGSCSGCGSLSLPLLAGLVAADAVASLLIVGAVFLC
ARPRRSPAQDGKVYINMPGRG

| | |
|---|---|
| Signal peptide: | Amino acids 1-18 |
| transmembrane domain: | Amino acids 51-70 |
| Glycosaminoglycan attachment site: | Amino acids 40-44 |
| N-myristoylation sites: | Amino acids 34-40;37-43;52-58 |
| Prokaryotic membrane lipoprotein lipid attachment site: | Amino acids 29-40 |

## FIGURE 21

AGCCCACCGAGAGGCGCCTGCAGG**ATG**AAAGCTCTCTGTCTCCTCCTCCTCCCTGTCCTGGGGCTGTTGGT
GTCTAGCAAGACCCTGTGCTCCATGGAAGAAGCCATCAATGAGAGGATCCAGGAGGTCGCCGGCTCCCTAATA
TTTAGGGCAATAAGCAGCATTGGCCTGGAGTGCCAGAGCGTCACCTCCAGGGGGGACCTGGCTACTTGCCC
CCGAGGCTTCGCCGTCACCGGCTGCACTTGTGGCTCCGCCTGTGGCTCGTGGGATGTGCGCGCCGAGACCA
CATGTCACTGCCAGTGCGCGGGCATGGACTGGACCGGAGCGCGCTGCTGTCGTGTGCAGCCC**TGA**GGTCGC
GCGCAGCGCGTGCACAGCGCGGGCGGAGGCGGCTCCAGGTCCGGAGGGGTTGCGGGGGAGCTGGAAATAAA
CCTGGAGATGATGATGATGATGATGATGGAAAAA

**FIGURE 22**

MKALCLLLLPVLGLLVSSKTLCSMEEAINERIQEVAGSLIFRAISSIGLECQSVTSRGDLATCPRGFAVTG
CTCGSACGSWDVRAETTCHCQCAGMDWTGARCCRVQP

| | |
|---|---|
| Signal peptide: | Amino acids 1-18 |
| Cell attachment sequence: | Amino acids 57-60 |
| N-myristoylation sites: | Amino acids 13-19;71-77;75-81; 95-101;100-106 |

## FIGURE 23

GACAGTGGAGGCAGTGGAGAGGACCGCGCTGTCCTGCTGTCACCAAGAGCTGGAGACACCATCTCCCACC
GAGAGTC**ATG**GCCCCATTGGCCCTGCACCTCCTCGTCCTCGTCCCCATCCTCCTCAGCCTGGTGGCCTCCC
AGGACTGGAAGGCTGAACGCAGCCAAGACCCCTTCGAGAAATGCATGCAGGATCCTGACTATGAGCAGCTG
CTCAAGGTGGTGACCTGGGGGCTCAATCGGACCCTGAAGCCCCAGAGGGTGATTGTGGTTGGCGCTGGTGT
GGCCGGGCTGGTGGCCGCCAAGGTGCTCAGCGATGCTGGACACAAGGTCACCATCCTGGAGGCAGATAACA
GGATCGGGGGCCGCATCTTCACCTACCGGGACCAGAACACGGGCTGGATTGGGGAGCTGGAGCCATGCGC
ATGCCCAGCTCTCACAGGATCCTCCACAAGCTCTGCCAGGGCCTGGGGCTCAACCTGACCAAGTTCACCCAG
TACGACAAGAACACGTGGACGGAGGTGCACGAAGTGAAGCTGCGCAACTATGTGGTGGAGAAGGTGCCCGA
GAAGCTGGGCTACGCCTTGCGTCCCCAGGAAAAGGGCCACTCGCCCGAAGACATCTACCAGATGGCTCTCA
ACCAGGCCCTCAAAGACCTCAAGGCACTGGGCTGCAGAAAGGCGATGAAGAAGTTTGAAAGGCACACGCTC
TTGGAATATCTTCTCGGGGAGGGGAACCTGAGCCGGCCGGCCGTGCAGCTTCTGGGAGACGTGATGTCCGA
GGATGGCTTCTTCTATCTCAGCTTCGCCGAGGCCCTCCGGGCCCACAGCTGCCTCAGCGACAGACTCCAGT
ACAGCCGCATCGTGGGTGGCTGGGACCTGCTGCCGCGCGCGCTGCTGAGCTCGCTGTCCGGGCTTGTGCTG
TTGAACGCGCCCGTGGTGGCGATGACCCAGGGACCGCACGATGTGCACGTGCAGATCGAGACCTCTCCCCC
GGCGCGGAATCTGAAGGTGCTGAAGGCCGACGTGGTGCTGCTGACGGCGAGCGGACCGGCGGTGAAGCGCA
TCACCTTCTCGCCGCCGCTGCCCCGCCACATGCAGGAGGCGCTGCGGAGGCTGCACTACGTGCCGGCCACC
AAGGTGTTCCTAAGCTTCCGCAGGCCCTTCTGGCGCGAGGAGCACATTGAAGGCGGCCACTCAAACACCGA
TCGCCCGTCGCGCATGATTTTCTACCCGCCGCCGCGCGAGGGCGCGCTGCTGCTGGCCTCGTACACGTGGT
CGGACGCGGCGGCAGCGTTCGCCGGCTTGAGCCGGGAAGAGGCGTTGCGCTTGGCGCTCGACGACGTGGCG
GCATTGCACGGGCCTGTCGTGCGCCAGCTCTGGGACGGCACCGGCGTCGTCAAGCGTTGGGCGGAGGACCA
GCACAGCCAGGGTGGCTTTGTGGTACAGCCGCCGGCGCTCTGGCAAACCGAAAAGGATGACTGGACGGTCC
CTTATGGCCGCATCTACTTTGCCGGCGAGCACACCGCCTACCCGCACGGCTGGGTGGAGACGGCGGTCAAG
TCGGCGCTGCGCGCCGCCATCAAGATCAACAGCCGGAAGGGGCCTGCATCGGACACGGCCAGCCCCGAGGG
GCACGCATCTGACATGGAGGGGCAGGGGCATGTGCATGGGGTGGCCAGCAGCCCCTCGCATGACCTGGCAA
AGGAAGAAGGCAGCCACCCTCCAGTCCAAGGCCAGTTATCTCTCCAAAACACGACCCACACGAGGACCTCG
CATTAAAGTATTTTCGGAAAAAAAAAAAAAAAAAAAAAAAAAAAAAAAAAAAAAAAAAAAAAAAAAAAAAA

## FIGURE 24

MAPLALHLLVLVPILLSLVASQDWKAERSQDPFEKCMQDPDYEQLLKVVTWGLNRTLKPQRVIVVGAGVAG
LVAAKVLSDAGHKVTILEADNRIGGRIFTYRDQNTGWIGELGAMRMPSSHRILHKLCQGLGLNLTKFTQYD
KNTWTEVHEVKLRNYVVEKVPEKLGYALRPQEKGHSPEDIYQMALNQALKDLKALGCRKAMKKFERHTLLE
YLLGEGNLSRPAVQLLGDVMSEDGFFYLSFAEALRAHSCLSDRLQYSRIVGGWDLLPRALLSSLSGLVLLN
APVVAMTQGPHDVHVQIETSPPARNLKVLKADVVLLTASGPAVKRITFSPPLPRHMQEALRRLHYVPATKV
FLSFRRPFWREEHIEGGHSNTDRPSRMIFYPPPREGALLLASYTWSDAAAAFAGLSREEALRLALDDVAAL
HGPVVRQLWDGTGVVKRWAEDQHSQGGFVVQPPALWQTEKDDWTVPYGRIYFAGEHTAYPHGWVETAVKSA
LRAAIKINSRKGPASDTASPEGHASDMEGQGHVHGVASSPSHDLAKEEGSHPPVQGQLSLQNTTHTRTSH

| | |
|---|---|
| Signal peptide: | Amino acids 1-21 |
| N-glycosylation sites: | Amino acids 54-58;134-138;220-224;<br>559-563 |
| D-amino acid oxidases proteins: | Amino acids 61-81 |
| Tyrosine kinase phosphorylation sites: | Amino acids 35-43;161-169 |
| N-myristoylation sites: | Amino acids 52-58;66-74;71-77;<br>130-136;132-138;198-204;371-377 |

**FIGURE 25**

CTAGCCTGCGCCAAGGGGTAGTGAGACCGCGCGGCAACAGCTTGCGGCTGCGGGGAGCTCCCGTGGGCGCT
CCGCTGGCTGTGCAGGCGGCC**ATG**GATTCCTTGCGGAAAATGCTGATCTCAGTCGCAATGCTGGGCGCAGG
GGCTGGCGTGGGCTACGCGCTCCTCGTTATCGTGACCCCGGGAGAGCGGCGGAAGCAGGAAATGCTAAAGG
AGATGCCACTGCAGGACCCAAGGAGCAGGGAGGAGGCGGCCAGGACCCAGCAGCTATTGCTGGCCACTCTG
CAGGAGGCAGCGACCACGCAGGAGAACGTGGCCTGGAGGAAGAACTGGATGGTTGGCGGCGAAGGCGGCGC
CAGCGGGAGGTCACCG**TGA**GACCGGACTTGCCTCCGTGGGCGCCGGACCTTGGCTTGGGCGCAGGAATCCG
AGGCAGCCTTTCTCCTTCGTGGGCCCAGCGGAGAGTCCGGACCGAGATACCATGCCAGGACTCTCCGGGGT
CCTGTGAGCTGCCGTCGGGTGAGCACGTTTCCCCCAAACCCTGGACTGACTGCTTTAAGGTCCGCAAGGCG
GGCCAGGGCCGAGACGCGAGTCGGATGTGGTGAACTGAAAGAACCAATAAAATCATGTTCCTCCAAAAAAA
AAAAAAAAAAAAAAAAAAAAAAAAAAAAAAAAAAAAAAAAAAAAAAAAAAAAA

FIGURE 26

MDSLRKMLISVAMLGAGAGVGYALLVIVTPGERRKQEMLKEMPLQDPRSREEAARTQQLLLATLQEAATTQ
ENVAWRKNWMVGGEGGASGRSP

| | |
|---|---|
| Signal peptide: | Amino acids 1-18 |
| N-myristoylation sites: | Amino acids 15-21;17-23;19-25;<br>83-89;86-92 |

## FIGURE 27

AAGACCCTCTCTTTCGCTGTTTGAGAGTCTCTCGGCTCAAGGACCGGGAGGTAAGAGGTTTGGGACTGCCC
CGGCAACTCCAGGGTGTCTGGTCCACGACCTATCCTAGGCGCC**ATG**GGTGTGATAGGTATACAGCTGGTTG
TTACCATGGTGATGGCCAGTGTCATGCAGAAGATTATACCTCACTATTCTCTTGCTCGATGGCTACTCTGT
AATGGCAGTTTGAGGTGGTATCAACATCCTACAGAAGAAGAATTAAGAATTCTTGCAGGGAAACAACAAAA
AGGGAAAACCAAAAAAGATAGGAAATATAATGGTCACATTGAAAGTAAGCCATTAACCATTCCAAAGGATA
TTGACCTTCATCTAGAAACAAAGTCAGTTACAGAAGTGGATACTTTAGCATTGCATTACTTTCCAGAATAC
CAGTGGCTGGTGGATTTCACAGTGGCTGCTACAGTTGTGTATCTAGTAACTGAAGTCTACTACAATTTTAT
GAAGCCTACACAGGAAATGAATATCAGCTTAGTCTGGTGCCTACTTGTTTTGTCTTTTGCAATCAAAGTTC
TATTTTCATTAACTACACACTATTTTAAAGTAGAAGATGGTGGTGAAAGATCTGTTTGTGTCACCTTTGGA
TTTTTTTTCTTTGTCAAAGCAATGGCAGTGTTGATTGTAACAGAAAATTATCTGGAATTTGGACTTGAAAC
AGGGTTTACAAATTTTTCAGACAGTGCGATGCAGTTTCTTGAAAAGCAAGGTTTAGAATCTCAGAGTCCTG
TTTCAAAACTTACTTTCAAATTTTTCCTGGCTATTTTCTGTTCATTCATTGGGGCTTTTTTGACATTTCCT
GGATTACGACTGGCTCAAATGCATCTGGATGCCCTGAATTTGGCAACAGAAAAAATTACACAAACTTTACT
TCATATCAACTTCTTGGCACCTTTATTTATGGTTTTGCTCTGGGTAAAACCAATCACCAAAGACTACATTA
TGAACCCACCACTGGGCAAAGAAATTTCCCCATCTGGAAGA**TGA**AGATAATAGTATCTAACTCACAAGGTT
ATCATTGGAATAAATGAAAGAACACATGTAATGCAACCAGCTGGAATTAAGTGCTTAATAAATGTTCTTTT
CACTGCTTTGCCTCATCAGAATTAAAATAGAAATACTTGACTAGT

## FIGURE 28

MGVIGIQLVVTMVMASVMQKIIPHYSLARWLLCNGSLRWYQHPTEEELRILAGKQQKGKTKKDRKYNGHIE
SKPLTIPKDIDLHLETKSVTEVDTLALHYFPEYQWLVDFTVAATVVYLVTEVYYNFMKPTQEMNISLVWCL
LVLSFAIKVLFSLTTHYFKVEDGGERSVCVTFGFFFFVKAMAVLIVTENYLEFGLETGFTNFSDSAMQFLE
KQGLESQSPVSKLTFKFFLAIFCSFIGAFLTFPGLRLAQMHLDALNLATEKITQTLLHINFLAPLFMVLLW
VKPITKDYIMNPPLGKEISPSGR

| | |
|---|---|
| Signal peptide: | Amino acids 1-15 |
| Transmembrane domains: | Amino acids 134-157;169-189; 230-248;272-285 |
| N-glycosylation sites: | Amino acids 34-38;135-139;203-207 |
| ATP/GTP-binding site motif A (P-loop): | Amino acids 53-61 |
| Tyrosine kinase phosphorylation site: | Amino acids 59-67 |
| N-myristoylation sites: | Amino acids 165-171;196-202;240-246; 247-253 |

## FIGURE 29

GACTTTGCTTGAATGTTTACATTTTCTGCTCGCTGTCCTACATATCACAATATAGTGTTCACGTTTTGTTA
AAACTTTGGGGTGTCAGGAGTTGAGCTTGCTCAGCAAGCCAGC**ATG**GCTAGGATGAGCTTTGTTATAGCAG
CTTGCCAATTGGTGCTGGGCCTACTAATGACTTCATTAACCGAGTCTTCCATACAGAATAGTGAGTGTCCA
CAACTTTGCGTATGTGAAATTCGTCCCTGGTTTACCCCACAGTCAACTTACAGAGAAGCCACCACTGTTGA
TTGCAATGACCTCCGCTTAACAAGGATTCCCAGTAACCTCTCTAGTGACACACAAGTGCTTCTCTTACAGA
GCAATAACATCGCGAAGACTGTGGATGAGCTGCAGCAGCTTTTCAACTTGACTGAACTAGATTTCTCCCAA
AACAACTTTACTAACATTAAGGAGGTCGGGCTGGCAAACCTAACCCAGCTCACAACGCTGCATTTGGAGGA
AAATCAGATTACCGAGATGACTGATTACTGTCTACAAGACCTCAGCAACCTTCAAGAACTCTACATCAACC
ACAACCAAATTAGCACTATTTCTGCTCATGCTTTTGCAGGCTTAAAAAATCTATTAAGGCTCCACCTGAAC
TCCAACAAATTGAAAGTTATTGATAGTCGCTGGTTTGATTCTACACCCAACCTGGAAATTCTCATGATCGG
AGAAAACCCTGTGATTGGAATTCTGGATATGAACTTCAAACCCCTCGCAAATTTGAGAAGCTTAGTTTTGG
CAGGAATGTATCTCACTGATATTCCTGGAAATGCTTTGGTGGGTCTGGATAGCCTTGAGAGCCTGTCTTTT
TATGATAACAAACTGGTTAAAGTCCCTCAACTTGCCCTGCAAAAAGTTCCAAATTTGAAATTCTTAGACCT
CAACAAAAACCCCATTCACAAAATCCAAGAAGGGGACTTCAAAAATATGCTTCGGTTAAAAGAACTGGGAA
TCAACAATATGGGCGAGCTCGTTTCTGTCGACCGCTATGCCCTGGATAACTTGCCTGAACTCACAAAGCTG
GAAGCCACCAATAACCCTAAACTCTCTTACATCCACCGCTTGGCTTTCCGAAGTGTCCCTGCTCTGGAAAG
CTTGATGCTGAACAACAATGCCTTGAATGCCATTTACCAAAAGACAGTCGAATCCCTCCCCAATCTGCGTG
AGATCAGTATCCATAGCAATCCCCTCAGGTGTGACTGTGTGATCCACTGGATTAACTCCAACAAAACCAAC
ATCCGCTTCATGGAGCCCCTGTCCATGTTCTGTGCCATGCCGCCCGAATATAAAGGGCACCAGGTGAAGGA
AGTTTTAATCCAGGATTCGAGTGAACAGTGCCTCCCAATGATATCTCACGACAGCTTCCCAAATCGTTTAA
ACGTGGATATCGGCACGACGGTTTTCCTAGACTGTCGAGCCATGGCTGAGCCAGAACCTGAAATTTACTGGGTC
ACTCCCATTGGAAATAAGATAACTGTGGAAACCCTTTCAGATAAATACAAGCTAAGTAGCGAAGGTACCTT
GGAAATATCTAACATACAAATTGAAGACTCAGGAAGATACACATGTGTTGCCCAGAATGTCCAAGGGGCAG
ACACTCGGGTGGCAACAATTAAGGTTAACGGGACCCTTCTGGATGGTACCCAGGTGCTAAAAATATACGTC
AAGCAGACAGAATCCCATTCCATCTTAGTGTCCTGGAAAGTTAATTCCAATGTCATGACGTCAAACTTAAA
ATGGTCGTCTGCCACCATGAAGATTGATAACCCTCACATAACATATACTGCCAGGGTCCCAGTCGATGTCC
ATGAATACAACCTAACGCATCTGCAGCCTTCCACAGATTATGAAGTGTGTCTCACAGTGTCCAATATTCAT
CAGCAGACTCAAAAGTCATGCGTAAATGTCACAACCAAAAATGCCGCCTTCGCAGTGGACATCTCTGATCA
AGAAACCAGTACAGCCCTTGCTGCAGTAATGGGGTCTATGTTTGCCGTCATTAGCCTTGCGTCCATTGCTGT
GTACTTTGCCAAAAGATTTAAGAGAAAAAACTACCACCACTCATTAAAAAAGTATATGCAAAAAACCTCTT
CAATCCCACTAAATGAGCTGTACCCACCACTCATTAACCTCTGGGAAGGTGACAGCGAGAAAGACAAAGAT
GGTTCTGCAGACACCAAGCCAACCCAGGTCGACACATCCAGAAGCTATTACATGTGG**TAA**CTCAGAGGATA
TTTTGCTTCTGGTAGTAAGGAGCACAAAGACGTTTTTGCTTTATTCTGCAAAAGTGAACAAGTTGAAGACT
TTTGTATTTTTGACTTTGCTAGTTTGTGGCAGAGTGGAGAGGACGGGTGGATATTTCAAATTTTTTTAGTA
TAGCGTATCGCAAGGGTTTGACACGGCTGCCAGCGACTCTAGGCTTCCAGTCTGTGTTTGGTTTTTATTCT
TATCATTATTATGATTGTTATTATATTATTATTTTATTTTAGTTGTTGTGCTAAACTCAATAATGCTGTTC
TAACTACAGTGCTCAATAAAATGATTAATGACAGGAAAAAAAAAAAAAAAAAAAAAAAAAAAAAAAAAAA
AAAAAAAAAAAAAAAAAAAAAAAAAAAAAAAAAAAA

## FIGURE 30

MARMSFVIAACQLVLGLLMTSLTESSIQNSECPQLCVCEIRPWFTPQSTYREATTVDCNDLRLTRIPSNLS
SDTQVLLLQSNNIAKTVDELQQLFNLTELDFSQNNFTNIKEVGLANLTQLTTLHLEENQITEMTDYCLQDL
SNLQELYINHNQISTISAHAFAGLKNLLRLHLNSNKLKVIDSRWFDSTPNLEILMIGENPVIGILDMNFKP
LANLRSLVLAGMYLTDIPGNALVGLDSLESLSFYDNKLVKVPQLALQKVPNLKFLDLNKNPIHKIQEGDFK
NMLRLKELGINNMGELVSVDRYALDNLPELTKLEATNNPKLSYIHRLAFRSVPALESLMLNNNALNAIYQK
TVESLPNLREISIHSNPLRCDCVIHWINSNKTNIRFMEPLSMFCAMPPEYKGHQVKEVLIQDSSEQCLPMI
SHDSFPNRLNVDIGTTVFLDCRAMAEPEPEIYWVTPIGNKITVETLSDKYKLSSEGTLEISNIQIEDSGRY
TCVAQNVQGADTRVATIKVNGTLLDGTQVLKIYVKQTESHSILVSWKVNSNVMTSNLKWSSATMKIDNPHI
TYTARVPVDVHEYNLTHLQPSTDYEVCLTVSNIHQQTQKSCVNVTTKNAAFAVDISDQETSTALAAVMGSM
FAVISLASIAVYFAKRFKRKNYHHSLKKYMQKTSSIPLNELYPPLINLWEGDSEKDKDGSADTKPTQVDTS
RSYYMW

| | |
|---|---|
| Signal peptide: | Amino acids 1-25 |
| Transmembrane domain: | Amino acids 508-530 |
| N-glycosylation sites: | Amino acids 69-73;96-100;106-110;<br>117-121;385-389;517-521;582-586;<br>611-615 |
| Tyrosine kinase phosphorylation site: | Amino acids 573-582 |
| N-myristoylation sites: | Amino acids 16-22;224-230;464-470;<br>637-643;698-704 |

## FIGURE 31

CAGCCGGGTCCCAAGCCTGTGCCTGAGCCTGAGCCTGAGCCTGAGCCCGAGCCGGGAGCCGGTCGCGGGGG
CTCCGGGCTGTGGGACCGCTGGGCCCCCAGCG**ATG**GCGACCCTGTGGGGAGGCCTTCTTCGGCTTGGCTCC
TTGCTCAGCCTGTCGTGCCTGGCGCTTTCCGTGCTGCTGCTGGCGCAGCTGTCAGACGCCGCCAAGAATTT
CGAGGATGTCAGATGTAAATGTATCTGCCCTCCCTATAAAGAAAATTCTGGGCATATTTATAATAAGAACA
TATCTCAGAAAGATTGTGATTGCCTTCATGTTGTGGAGCCCATGCCTGTGCGGGGGCCTGATGTAGAAGCA
TACTGTCTACGCTGTGAATGCAAATATGAAGAAAGAAGCTCTGTCACAATCAAGGTTACCATTATAATTTA
TCTCTCCATTTTGGGCCTTCTACTTCTGTACATGGTATATCTTACTCTGGTTGAGCCCATACTGAAGAGGC
GCCTCTTTGGACATGCACAGTTGATACAGAGTGATGATGATATTGGGGATCACCAGCCTTTTGCAAATGCA
CACGATGTGCTAGCCCGCTCCCGCAGTCGAGCCAACGTGCTGAACAAGGTAGAATATGCACAGCAGCGCTG
GAAGCTTCAAGTCCAAGAGCAGCGAAAGTCTGTCTTTGACCGGCATGTTGTCCTCAGC**TAA**TTGGGAATTG
AATTCAAGGTGACTAGAAAGAAACAGGCAGACAACTGGAAAGAACTGACTGGGTTTTGCTGGGTTTCATTT
TAATACCTTGTTGATTTCACCAACTGTTGCTGGAAGATTCAAAACTGGAAGCAAAAACTTGCTTGATTTTT
TTTTCTTGTTAACGTAATAATAGAGACATTTTTAAAAGCACACAGCTCAAAGTCAGCCAATAAGTCTTTTC
CTATTTGTGACTTTTACTAATAAAAATAAATCTGCCTGTAAATTATCTTGAAGTCCTTTACCTGGAACAAG
CACTCTCTTTTTCACCACATAGTTTTAACTTGACTTTCAAGATAATTTTCAGGGTTTTTGTTGTTGTTGTT
TTTTGTTTGTTTGTTTTGGTGGGAGAGGGGAGGGATGCCTGGGAAGTGGTTAACAACTTTTTTCAAGTCAC
TTTACTAAACAAACTTTTGTAAATAGACCTTACCTTCTATTTTCGAGTTTCATTTATATTTTGCAGTGTAG
CCAGCCTCATCAAAGAGCTGACTTACTCATTTGACTTTTGCACTGACTGTATTATCTGGGTATCTGCTGTG
TCTGCACTTCATGGTAAACGGGATCTAAAATGCCTGGTGGCTTTTCACAAAAAGCAGATTTTCTTCATGTA
CTGTGATGTCTGATGCAATGCATCCTAGAACAAACTGGCCATTTGCTAGTTTACTCTAAAGACTAAACATA
GTCTTGGTGTGTGTGGTCTTACTCATCTTCTAGTACCTTTAAGGACAAATCCTAAGGACTTGGACACTTGCAAT
AAAGAAATTTTATTTTAAACCCAAGCCTCCCTGGATTGATAATATATACACATTTGTCAGCATTTCCGGTC
GTGGTGAGAGGCAGCTGTTTGAGCTCCAATATGTGCAGCTTTGAACTAGGGCTGGGGTTGTGGGTGCCTCT
TCTGAAAGGTCTAACCATTATTGGATAACTGGCTTTTTTCTTCCTATGTCCTCTTTGGAATGTAACAATAA
AAATAATTTTTGAAACATCAA

## FIGURE 32

MATLWGGLLRLGSLLSLSCLALSVLLLAQLSDAAKNFEDVRCKCICPPYKENSGHIYNKNISQKDCDCLHV
VEPMPVRGPDVEAYCLRCECKYEERSSVTIKVTIIIYLSILGLLLLYMVYLTLVEPILKRRLFGHAQLIQS
DDDIGDHQPFANAHDVLARSRSRANVLNKVEYAQQRWKLQVQEQRKSVFDRHVVLS

Transmembrane domains: Amino acids 11-28 (type II);103-125

N-glycosylation site: Amino acids 60-64

Tyrosine kinase phosphorylation site: Amino acids 78-86

N-myristoylation site: Amino acids 12-18

## FIGURE 33

GTCGAAGGTTATAAAAGCTTCCAGCCAAACGGCATTGAAGTTGAAGATACAACCTGACAGCACAGCCTGAG
ATCTTGGGGATCCCTCAGCCTAACACCCACAGACGTCAGCTGGTGGATTCCCGCTGCATCAAGGCCTACCC
ACTGTCTCC**ATG**CTGGGCTCTCCCTGCCTTCTGTGGCTCCTGGCCGTGACCTTCTTGGTTCCCAGAGCTCA
GCCCTTGGCCCCTCAAGACTTTGAAGAAGAGGAGGCAGATGAGACTGAGACGGCGTGGCCGCCTTTGCCGG
CTGTCCCCTGCGACTACGACCACTGCCGACACCTGCAGGTGCCCTGCAAGGAGCTACAGAGGGTCGGGCCG
GCGGCCTGCCTGTGCCCAGGACTCTCCAGCCCCGCCCAGCCGCCCGACCCGCCGCGCATGGGAGAAGTGCG
CATTGCGGCCGAAGAGGGCCGCGCAGTGGTCCACTGGTGTGCCCCCTTCTCCCCGGTCCTCCACTACTGGC
TGCTGCTTTGGGACGGCAGCGAGGCTGCGCAGAAGGGGCCCCCGCTGAACGCTACGGTCCGCAGAGCCGAACTG
AAGGGGCTGAAGCCAGGGGGCATTTATGTCGTTTGCGTAGTGGCCGCTAACGAGGCCGGGGCAAGCCGCGT
GCCCCAGGCTGGAGGAGAGGGCCTCGAGGGGGCCGACATCCCTGCCTTCGGGCCTTGCAGCCGCCTTGCGG
TGCCGCCCAACCCCCGCACTCTGGTCCACGCGGCCGTCGGGGTGGGCACGGCCCTGGCCCTGCTAAGCTGT
GCCGCCCTGGTGTGGCACTTCTGCCTGCGCGATCGCTGGGGCTGCCCGCGCCGAGCCGCCGCCCGAGCCGC
AGGGGCGCTC**TGA**AAGGGGCCTGGGGGCATCTCGGGCACAGACAGCCCCACCTGGGGCGCTCAGCCTGGCC
CCCGGGAAAGAGGAAAACCCGCTGCCTCCAGGGAGGGCTGGACGGCGAGCTGGGAGCCAGCCCCAGGCTCC
AGGGCCACGGCGGAGTCATGGTTCTCAGGACTGAGCGCTTGTTTAGGTCCGGTACTTGGCGCTTTGTTTCC
TGGCTGAGGTCTGGGAAGGAATAGAAAGGGGCCCCCAATTTTTTTTTAAGCGGCCAGATAATAAATAATGT
AACCTTTGCGGTTAAAAAAAAAAAAAAAAAA

## FIGURE 34

MLGSPCLLWLLAVTFLVPRAQPLAPQDFEEEEADETETAWPPLPAVPCDYDHCRHLQVPCKELQRVGPAAC
LCPGLSSPAQPPDPPRMGEVRIAAEEGRAVVHWCAPFSPVLHYWLLLWDGSEAAQKGPPLNATVRRAELKG
LKPGGIYVVCVVAANEAGASRVPQAGGEGLEGADIPAFGPCSRLAVPPNPRTLVHAAVGVGTALALLSCAA
LVWHFCLRDRWGCPRRAAARAAGAL

| | |
|---|---|
| Signal peptide: | Amino acids 1-20 |
| Transmembrane domain: | Amino acids 194-220 |
| N-glycosylation site: | Amino acids 132-136 |
| N-myristoylation sites: | Amino acids 121-127;142-148;171-177;<br>201-207;203-209 |

**FIGURE 35**

TCGCC**ATG**GCCTCTGCCGGAATGCAGATCCTGGGAGTCGTCCTGACACTGCTGGGCTGGGTGAATGGCCTG
GTCTCCTGTGCCCTGCCCATGTGGAAGGTGACCGCTTTCATCGGCAACAGCATCGTGGTGGCCCAGGTGGT
GTGGGAGGGCCTGTGGATGTCCTGCGTGGTGCAGAGCACCGGCCAGATGCAGTGCAAGGTGTACGACTCAC
TGCTGGCGCTGCCACAGGACCTGCAGGCTGCACGTGCCCTCTGTGTCATCGCCCTCCTTGTGGCCCTGTTC
GGCTTGCTGGTCTACCTTGCTGGGGCCAAGTGTACCACCTGTGTGGAGGAGAAGGATTCCAAGGCCCGCCT
GGTGCTCACCTCTGGGATTGTCTTTGTCATCTCAGGGGTCCTGACGCTAATCCCCGTGTGCTGGACGGCGC
ATGCCATCATCCGGGACTTCTATAACCCCCTGGTGGCTGAGGCCCAAAAGCGGGAGCTGGGGGCCTCCCTC
TACTTGGGCTGGGCGGCCTCAGGCCTTTTGTTGCTGGGTGGGGGGTTGCTGTGCTGCACTTGCCCCTCGGG
GGGGTCCCAGGGCCCCAGCCATTACATGGCCCGCTACTCAACATCTGCCCCTGCCATCTCTCGGGGGCCCT
CTGAGTACCCTACCAAGAATTACGTC**TGA**CGTGGAGGGGAATGGGGGCTCCGCTGGCGCTAGAGCCATCCA
GAAGTGGCAGTGCCCAACAGCTTTGGGATGGGTTCGTACCTTTTGTTTCTGCCTCCTGCTATTTTTCTTTT
GACTGAGGATATTTAAAATTCATTTGAAAACTGAGCCAAGGTGTTGACTCAGACTCTCACTTAGGCTCTGC
TGTTTCTCACCCTTGGATGATGGAGCCAAAGAGGGGATGCTTTGAGATTCTGGATCTTGACATGCCCATCT
TAGAAGCCAGTCAAGCTATGGAACTAATGCGGAGGCTGCTTGCTGTGCTGGCTTTGCAACAAGACAGACTG
TCCCCAAGAGTTCCTGCTGCTGCTGGGGGCTGGGCTTCCCTAGATGTCACTGGACAGCTGCCCCCCATCCT
ACTCAGGTCTCTGGAGCTCCTCTCTTCACCCCTGGAAAAACAAATCATCTGTTAACAAAGGACTGCCCACC
TCCGGAACTTCTGACCTCTGTTTCCTCCGTCCTGATAAGACGTCCACCCCCCAGGGCCAGGTCCCAGCTAT
GTAGACCCCCGCCCCCACCTCCAACACTGCACCCTTCTGCCCTGCCCCCCTCGTCTCACCCCCCTTTACACT
CACATTTTTATCAAATAAAGCATGTTTTGTTAGTGCA

## FIGURE 36

MASAGMQILGVVLTLLGWVNGLVSCALPMWKVTAFIGNSIVVAQVVWEGLWMSCVVQSTGQMQCKVYDSLL
ALPQDLQAARALCVIALLVALFGLLVYLAGAKCTTCVEEKDSKARLVLTSGIVFVISGVLTLIPVCWTAHA
IIRDFYNPLVAEAQKRELGASLYLGWAASGLLLLGGGLLCCTCPSGGSQGPSHYMARYSTSAPAISRGPSE
YPTKNYV

| | |
|---|---|
| Transmembrane domains: | Amino acids 8-30 (type II); 82-102;121-140;166-186 |
| N-myristoylation sites: | Amino acids 10-16;21-27;49-55; 60-66;101-107;178-184;179-185 |

## FIGURE 37

TGCATCAGTGCCCAGGCAAGCCCAGGAGTTGACATTTCTCTGCCCAGCC**ATG**GGCCTCACCCTGCTCTTGC
TGCTGCTCCTGGGACTAGAAGGTCAGGGCATAGTTGGCAGCCTCCCTGAGGTGCTGCAGGCACCCGTGGGA
AGCTCCATTCTGGTGCAGTGCCACTACAGGCTCCAGGATGTCAAAGCTCAGAAGGTGTGGTGCCGGTTCTT
GCCGGAGGGGTGCCAGCCCCTGGTGTCCTCAGCTGTGGATCGCAGAGCTCCAGCGGGCAGGCGTACGTTTC
TCACAGACCTGGGTGGGGGCCTGCTGCAGGTGGAAATGGTTACCCTGCAGGAAGAGGATGCTGGCGAGTAT
GGCTGCATGGTGGATGGGGCCAGGGGGCCCCAGATTTTGCACAGAGTCTCTCTGAACATACTGCCCCCAGA
GGAAGAAGAAGAGACCCATAAGATTGGCAGTCTGGCTGAGAACGCATTCTCAGACCCTGCAGGCAGTGCCA
ACCCTTTGGAACCCAGCCAGGATGAGAAGAGCATCCCCTTGATCTGGGGTGCTGTGCTCCTGGTAGGTCTG
CTGGTGGCAGCGGTGGTGCTGTTTGCTGTGATGGCCAAGAGGAAACAAGAATCCCTCCTCAGTGGTCCACC
ACGTCAG**TGA**CTCTGGACCGGCTGCTGAATTGCCTTTGGATGTACCACACATTAGGCTTGACTCACCACCT
TCATTTGACAATACCACCTACACCAGCCTACCTCTTGATTCCCCATCAGGAAAACCTTCACTCCCAGCTCC
ATCCTCATTGCCCCCTCTACCTCCTAAGGTCCTGGTCTGCTCCAAGCCTGTGACATATGCCACAGTAATCT
TCCCGGGAGGGAACAAGGGTGGAGGGACCTCGTGTGGGCCAGCCCAGAATCCACCTAACAATCAGACTCCA
TCCAGCTAAGCTGCTCATCACACTTTAAACTCATGAGGACCATCCCTAGGGGTTCTGTGCATCCATCCAGC
CAGCTCATGCCCTAGGATCCTTAGGATATCTGAGCAACCAGGGACTTTAAGATCTAATCCAATGTCCTAAC
TTTACTAGGGAAAGTGACGCTCAGACATGACTGAGATGTCTTGGGGAAGACCTCCCTGCACCCAACTCCCC
CACTGGTTCTTCTACCATTACACACTGGGCTAAATAAACCCTAATAATGATGTGCAAAAAAAAAAAAAAAA
AAAAAAAAAAAAAAAAAAAAAAAAAAAAAAAA

FIGURE 38

MGLTLLLLLLLGLEGQGIVGSLPEVLQAPVGSSILVQCHYRLQDVKAQKVWCRFLPEGCQPLVSSAVDRRA
PAGRRTFLTDLGGGLLQVEMVTLQEEDAGEYGCMVDGARGPQILHRVSLNILPPEEEEETHKIGSLAENAF
SDPAGSANPLEPSQDEKSIPLIWGAVLLVGLLVAAVVLFAVMAKRKQESLLSGPPRQ

| | |
|---|---|
| Signal peptide: | Amino acids 1-15 |
| Transmembrane domain: | Amino acids 161-181 |
| N-myristoylation sites: | Amino acids 17-23;172-178 |
| Amidation site: | Amino acids 73-79 |

**FIGURE 39**

GGGTGATTGAACTAAACCTTCGCCGCACCGAGTTTGCAGTACGGCCGTCACCCGCACCGCTGCCTGCTTGC
GGTTGGAGAAATCAAGGCCCTACCGGGCCTCCGTAGTCACCTCTCTATAGTGGGCGTGGCCGAGGCCGGGG
TGACCCTGCCGGAGCCTCCGCTGCCAGCGAC<u>ATG</u>TTCAAGGTAATTCAGAGGTCCGTGGGGCCAGCCAGCC
TGAGCTTGCTCACCTTCAAAGTCTATGCAGCACCAAAAAAGGACTCACCTCCCAAAAATTCCGTGAAGGTT
GATGAGCTTTCACTCTACTCAGTTCCTGAGGGTCAATCGAAGTATGTGGAGGAGGCAAGGAGCCAGCTTGA
AGAAAGCATCTCACAGCTCCGACACTATTGCGAGCCATACACAACCTGGTGTCAGGAAACGTACTCCCAAA
CTAAGCCCAAGATGCAAAGTTTGGTTCAATGGGGGTTAGACAGCTATGACTATCTCCAAAATGCACCTCCT
GGATTTTTTCCGAGACTTGGTGTTATTGGTTTTGCTGGCCTTATTGGACTCCTTTTGGCTAGAGGTTCAAA
AATAAAGAAGCTAGTGTATCCGCCTGGTTTCATGGGATTAGCTGCCTCCCTCTATTATCCACAACAAGCCA
TCGTGTTTGCCCAGGTCAGTGGGGAGAGATTATATGACTGGGGTTTACGAGGATATATAGTCATAGAAGAT
TTGTGGAAGGAGAACTTTCAAAAGCCAGGAAATGTGAAGAATTCACCTGGAACTAAG<u>TAG</u>AAAACTCCATG
CTCTGCCATCTTAATCAGTTATAGGTAAACATTGGAAACTCCATAGAATAAATCAGTATTTCTACAGAAAA
ATGGCATAGAAGTCAGTATTGAATGTATTAAATTGGCTTTCTTCTTCAGGAAAAACTAGACCAGACCTCTG
TTATCTTCTGTGAAATCATCCTACAAGCAAACTAACCTGGAATCCCTTCACCTAGAGATAATGTACAAGCC
TTAGAACTCCTCATTCTCATGTTGCTATTTATGTACCTAATTAAAACCCAAGTTTAAAAAAAAAAAAAAAA
AAAAAAAAAAAAAAAAAAAAA

## FIGURE 40

MFKVIQRSVGPASLSLLTFKVYAAPKKDSPPKNSVKVDELSLYSVPEGQSKYVEEARSQLEESISQLRHYC
EPYTTWCQETYSQTKPKMQSLVQWGLDSYDYLQNAPPGFFPRLGVIGFAGLIGLLLARGSKIKKLVYPPGF
MGLAASLYYPQQAIVFAQVSGERLYDWGLRGYIVIEDLWKENFQKPGNVKNSPGTK

Signal peptide: Amino acids 1-23

Transmembrane domain: Amino acids 111-130

cAMP- and cGMP-dependent protein kinase phosphorylation site: Amino acids 26-30

Tyrosine kinase phosphorylation site: Amino acids 36-44

N-myristoylation sites: Amino acids 124-130;144-150;189-195

**FIGURE 41**

GTGGGCCGCCCCTGCTGCTGCCGTCC<u>**ATG**</u>CTGATGTTTGCGGTGATCGTGGCCTCCAGCGGGCTGCTGCTC
ATGATCGAGCGGGGCATCCTGGCCGAGATGAAGCCCCTGCCCCTGCACCCGCCCGGCCGCGAGGGCACAGC
CTGGCGCGGGAAAGCCCCCAAGCCTGGGGGCCTGTCCCTCAGGGCTGGGGACGCGGACTTGCAAGTGCGGCAG
GACGTCCGGAACAGGACCCTGCGGGCGGTGTGCGGACAGCCAGGCATGCCCCGGGACCCCTGGGACTTGCC
GGTGGGGCAGCGGCGCACCCTGCTGCGCCACATCCTCGTAAGTGACCGTTACCGCTTCCTCTACTGCTACG
TCCCCAAGGTGGCCTGCTCTAACTGGAAGCGGGTGATGAAGGTGCTGGCAGGCGTCCTGGACAGCGTGGAC
GTCCGCCTCAAGATGGACCACCGCAGTGACCTGGTGTTCCTGGCCGACCTGCGGCCTGAGGAGATTCGCTA
CCGCCTGCAGCACTACTTTAAGTTCCTGTTTGTGCGGGAGCCCTTGGAACGCCTCCTCTCTGCCTACCGCA
ACAAGTTTGGCGAGATCCGAGAGTACCAGCAACGCTATGGGGCTGAGATAGTGAGGCGGTACAGGGCTGGA
GCGGGGCCCAGCCCTGCAGGCGACGATGTCACATTCCCCGAGTTCCTGAGATACCTGGTGGATGAGGACCC
TGAGCGCATGAATGAGCATTGGATGCCCGTGTACCACCTGTGCCAGCCTTGTGCCGTGCACTATGACTTTG
TGGGCTCCTATGAGAGGCTGGAGGCTGATGCAAATCAGGTGCTGGAGTGGGTACGGGCACCACCTCACGTC
CGATTTCCAGCTCGCCAGGCCTGGTACCGGCCAGCCAGCCCCGAAAGCCTGCATTACCACTTGTGCAGTGC
CCCCCGGGCCCTGCTGCAGGATGTGCTGCCTAAGTATATCCTGGACTTCTCCCTCTTTGCCTACCCACTGC
CTAATGTCACCAAGGAGGCGTGTCAGCAG<u>**TGA**</u>CCATGGGTGTGGGGCCAGCAGCTGGTGGGGACTGGTTTC
AACGCCAGCTTTCTGTGCTTCTGCCTGTCATTCGGAGAAACTCTGGCTCTGGGGCTTGGGGCTTCTCAGGA
TCCTGGATGGCAGAGACTGCCCTCAGAAGTTCCTTGTCCAGGGTGGGCACCCACAGTGACTCAGAGGACAG
GGCTAGGCAGGAGACCTGCTGCTCCTCATTGGGGGGATCTCTTGGGGGGCAGACACCAGTTTGCCAATGAA
GCAACACATCTGATCTAAAGACTGGCTCCAGACCCCGGGCTGCCAGGATTATGCAGTCCACTTGGTCTACC
TTAATTTAACCTGTGGCCAAACTCAGAGATGGTACCAGCCAGGGGCAAGCATGACCAGAGCCAGGGACCCT
GTGGCTCTGATCCCCCATTTATCCACCCCATGTGCCTCAGGACTAGAGTGAGCAATCATACCTTATAAATG
ACTTTTGTGCCTTTCTGCTCCAGTCTCAAAATTTCCTACACCTGCCAGTTCTTTACATTTTTCCAAGGAAA
GGAAAACGGAAGCAGGGTTCTTGCCTGGTAGCTCCAGGACCCAGCTCTGCAGGCACCCAAAGACCCTCTGT
GCCCAGCCTCTTCCTTGAGTTCTCGGAACCTCCTCCCTAATTCTCCCTTCCTTCCCCACAAGGCCTTTGAG
GTTGTGACTGTGGCTGGTATATCTGGCTGCCATTTTTCTGATGCATTTATTTAAAATTTGTACTTTTTGAT
AGAACCCTTGTAAGGGCTTTGTTTTCCTAATAGCTGACTTTTTAATAAAGCAGTTTTATATAT

## FIGURE 46

MLMFAVIVASSGLLLMIERGILAEMKPLPLHPPGREGTAWRGKAPKPGGLSLRAGDADLQVRQDVRNRTLR
AVCGQPGMPRDPWDLPVGQRRTLLRHILVSDRYRFLYCYVPKVACSNWKRVMKVLAGVLDSVDVRLKMDHR
SDLVFLADLRPEEIRYRLQHYFKFLFVREPLERLLSAYRNKFGEIREYQQRYGAEIVRRYRAGAGPSPAGD
DVTFPEFLRYLVDEDPERMNEHWMPVYHLCQPCAVHYDFVGSYERLEADANQVLEWVRAPPHVRFPARQAW
YRPASPESLHYHLCSAPRALLQDVLPKYILDFSLFAYPLPNVTKEACQQ

Signal peptide: Amino acids 1-23

N-glycosylation sites: Amino acids 67-71;325-329

Tyrosine kinase phosphorylation sites: Amino acids 152-159;183-183

N-myristoylation sites: Amino acids 89-95;128-134

## FIGURE 43

CTCCTGCACTAGGCTCTCAGCCAGGGATGATGCGCTGCTGCCGCCGCCGCTGCTGCTGCCGGCAACCACCC
CATGCCCTGAGGCCGTTGCTGTTGCTGCCCCTCGTCCTTTTACCTCCCCTGGCAGCAGCTGCAGCGGGCCC
AAACCGATGTGACACCATATACCAGGGCTTCGCCGAGTGTCTCATCCGCTTGGGGGACAGCATGGGCCGCG
GAGGCGAGCTGGAGACCATCTGCAGGTCTTGGAATGACTTCCATGCCTGTGCCTCTCAGGTCCTGTCAGGC
TGTCCGGAGGAGGCAGCTGCAGTGTGGGAATCACTACAGCAAGAAGCTCGCCAGGCCCCCCGTCCGAATAA
CTTGCACACTCTGTGCGGTGCCCCGGTGCATGTTCGGGAGCGCGGCACAGGCTCCGAAACCAACCAGGAGA
CGCTGCGGGCTACAGCGCCTGCACTCCCCATGGCCCCTGCGCCCCCACTGCTGGCGGCTGCTCTGGCTCTG
GCCTACCTCCTGAGGCCTCTGGCCTAGCTTGTTGGGTTGGGTAGCAGCGCCCGTACCTCCAGCCCTGCTCT
GGCGGTGGTTGTCCAGGCTCTGCAGAGCGCAGCAGGGCTTTTCATTAAAGGTATTTATATTTGTA

## FIGURE 44

MMRCCRRRCCCRQPPHALRPLLLLPLVLLPPLAAAAAGPNRCDTIYQGFAECLIRLGDSMGRGGELETICR
SWNDFHACASQVLSGCPEEAAAVWESLQQEARQAPRPNNLHTLCGAPVHVRERGTGSETNQETLRATAPAL
PMAPAPPLLAAALALAYLLRPLA

Signal peptide: Amino acids 1-35

Transmembrane domain: Amino acids 141-157

N-myristoylation site: Amino acids 127-133

Prokaryotic membrane lipoprotein lipid attachment site: Amino acids 77-88

**FIGURE 45**

CCCACGCGTCCGCGCAGTCGCGCAGTTCTGCCTCCGCCTGCCAGTCTCGCCCGCGATCCCGGCCCGGGGCT
GTGGCGTCGACTCCGACCCAGGCAGCCAGCAGCCCGCGCGGGAGCCGGACCGCCGCCGGAGGAGCTCGGAC
GGCATGCTGAGCCCCCTCCTTTGCTGAAGCCCGAGTGCGGAGAAGCCCGGGCAAACGCAGGCTAAGGAGAC
CAAAGCGGCGAAGTCGCGAGACAGCGGACAAGCAGCGGAGGAGAAGGAGGAGGAGGCGAACCCAGAGAGGG
GCAGCAAAAGAAGCGGTGGTGGTGGGCGTCGTGGCC<u>ATG</u>GCGGCGGCTATCGCCAGCTCGCTCATCCGTCA
GAAGAGGCAAGCCCGCGAGCGCGAGAAATCCAACGCCTGCAAGTGTGTCAGCAGCCCCAGCAAAGGCAAGA
CCAGCTGCGACAAAAACAAGTTAAATGTCTTTTCCCGGGTCAAACTCTTCGGCTCCAAGAAGAGGCGCAGA
AGAAGACCAGAGCCTCAGCTTAAGGGTATAGTTACCAAGCTATACAGCCGACAAGGCTACCACTTGCAGCT
GCAGGCGGATGGAACCATTGATGGCACCAAAGATGAGGACAGCACTTACACTCTGTTTAACCTCATCCCTG
TGGGTCTGCGAGTGGTGGCTATCCAAGGAGTTCAAACCAAGCTGTACTTGGCAATGAACAGTGAGGGATAC
TTGTACACCTCGGAACTTTTCACACCTGAGTGCAAATTCAAAGAATCAGTGTTTGAAAATTATTATGTGAC
ATATTCATCAATGATATACCGTCAGCAGCAGTCAGGCCGAGGGTGGTATCTGGGTCTGAACAAAGAAGGAG
AGATCATGAAAGGCAACCATGTGAAGAAGAACAAGCCTGCAGCTCATTTTCTGCCTAAACCACTGAAAGTG
GCCATGTACAAGGAGCCATCACTGCACGATCTCACGGAGTTCTCCCGATCTGGAAGCGGGACCCCAACCAA
GAGCAGAAGTGTCTCTGGCGTGCTGAACGGAGGCAAATCCATGAGCCACAATGAATCAACG<u>TAG</u>CCAGTGA
GGGCAAAAGAAGGGCTCTGTAACAGAACCTTACCTCCAGGTGCTGTTGAATTCTTCTAGCAGTCCTTCACC
CAAAAGTTCAAATTTGTCAGTGACATTTACCAAACAAACAGGCAGAGTTCACTATTCTATCTGCCATTAGA
CCTTCTTATCATCCATACTAAAGC

## FIGURE 46

MAAAIASSLIRQKRQAREREKSNACKCVSSPSKGKTSCDKNKLNVFSRVKLFGSKKRRRRRPEPQLKGIVT
KLYSRQGYHLQLQADGTIDGTKDEDSTYTLFNLIPVGLRVVAIQGVQTKLYLAMNSEGYLYTSELFTPECK
FKESVFENYYVTYSSMIYRQQQSGRGWYLGLNKEGEIMKGNHVKKNKPAAHFLPKPLKVAMYKEPSLHDLT
EFSRSGSGTPTKSRSVSGVLNGGKSMSHNEST

| | |
|---|---|
| N-glycosylation site: | Amino acids 242-246 |
| Glycosaminoglycan attachment site: | Amino acids 165-169, 218-222 |
| Tyrosine kinase phosphorylation site: | Amino acids 93-100 |
| N-myristoylation sites: | Amino acids 87-93, 231-237 |
| ATP/GTP-binding site motif A (P-loop): | Amino acids 231-239 |
| HBGF/FGF family proteins: | Amino acids 78-94, 102-153 |

## FIGURE 47

CAGCGCTGACTGCGCCGCGGAGAAAGCCAGTGGGAACCCAGACCCATAGGAGACCCGCGTCCCCGCTCGGC
CTGGCCAGGCCCCGCGCT**ATG**GAGTTCCTCTGGGCCCCTCTCTTGGGTCTGTGCTGCAGTCTGGCCGCTGC
TGATCGCCACACCGTCTTCTGGAACAGTTCAAATCCCAAGTTCCGGAATGAGGACTACACCATACATGTGC
AGCTGAATGACTACGTGGACATCATCTGTCCGCACTATGAAGATCACTCTGTGGCAGACGCTGCCATGGAG
CAGTACATACTGTACCTGGTGGAGCATGAGGAGTACCAGCTGTGCCAGCCCCAGTCCAAGGACCAAGTCCG
CTGGCAGTGCAACCGGCCCAGTGCCAAGCATGGCCCGGAGAAGCTGTCTGAGAAGTTCCAGCGCTTCACAC
CTTTCACCCTGGGCAAGGAGTTCAAAGAAGGACACAGCTACTACTACATCTCCAAACCCATCCACCAGCAT
GAAGACCGCTGCTTGAGGTTGAAGGTGACTGTCAGTGGCAAAATCACTCACAGTCCTCAGGCCCATGACAA
TCCACAGGAGAAGAGACTTGCAGCAGATGACCCAGAGGTGCGGGTTCTACATAGCATCGGTCACAGTGCTG
CCCCACGCCTCTTCCCACTTGCCTGGACTGTGCTGCTCCTTCCACTTCTGCTGCTGCAAACCCCG**TGA**AGG
TGTGTGCCACACCTGGCCTTAAAGAGGGACAGGCTGAAGAGAGGGACAGGCACTCCAAACCTGTCTTGGGG
CCACTTTCAGAGCCCCCAGCCCTGGGAACCACTCCCACCACAGGCATAAGCTATCACCTAGCAGCCTCAAA
ACGGGTCAATATTAAGGTTTTCAACCGGAAGGAGGCCAACCAGCCCGACAGTGCCATCCCCACCTTCACCT
CGGAGGGATGGAGAAAGAAGTGGAGACAGTCCTTTCCCACCATTCCTGCCTTTAAGCCAAAGAAACAAGCT
GTGCAGGCATGGTCCCTTAAGGCACAGTGGGAGCTGAGCTGGAAGGGGCCACGTGGATGGGCAAAGCTTGT
CAAAGATGCCCCCTTCAGGAGAGAGCCAGGATGCCCAGATGAACTGACTGAAGGAAAAGCAAGAAACAGTT
TCTTGCTTGGAAGCCAGGTACAGGAGAGGCAGCATGCTTGGGCTGACCCAGCATCTCCCAGCAAGACCTCA
TCTGTGGAGCTGCCACAGAGAAGTTTGTAGCCAGGTACTGCATTCTCTCCCATCCTGGGGCAGCACTCCCC
AGAGCTGTGCCAGCAGGGGGGCTGTGCCAACCTGTTCTTAGAGTGTAGCTGTAAGGGCAGTGCCCATGTGT
ACATTCTGCCTAGAGTGTAGCCTAAAGGGCAGGGCCCACGTGTATAGTATCTGTATATAAGTTGCTGTGTG
TCTGTCCTGATTTCTACAACTGGAGTTTTTTATACAATGTTCTTTGTCTCAAAATAAAGCAATGTGTTTT
TTCGG

## FIGURE 48

MEFLWAPLLGLCCSLAAADRHTVFWNSSNPKFRNEDYTIHVQLNDYVDIICPHYEDHSADAAMEQYILYLV
EHEEYQLCQPQSKDQVRWQCNRPSAKHGPEKLSEKFQRFTPFTLGKEFKEGHSYYYISKPIHQHEDRCLRL
KVTVSGKITHSPQAHDNPQEKRLAADDPEVRVLHSIGHSAAPRLFPLAWTVLLLPLLLLQTP

| | |
|---|---|
| Signal sequence: | Amino acids 1-17 |
| N-glycosylation site: | Amino acids 26-30 |
| Tyrosine kinase phosphorylation site: | Amino acids 118-127 |
| N-myristoylation site: | Amino acids 10-16 |

**FIGURE 49**

CTGGGCCCAGCTCCCCCGAGAGGTGGTCGGATCCTCTGGGCTGCTCGGTCGATGCCTGTGCCACTGACGTC
CAGGC<u>ATG</u>AGGTGGTTCCTGCCCTGGACGCTGGCAGCAGTGACAGCAGCAGCCGCCAGCACCGTCCTGGCC
ACGGCCCTCTCTCCAGCCCCTACGACCATGGACTTTACTCCAGCTCCACTGGAGGACACCTCCTCACGCCC
CCAATTCTGCAAGTGGCCATGTGAGTGCCCGCCATCCCCACCCCGCTGCCCGCTGGGGTCAGCCTCATCA
CAGATGGCTGTGAGTGCTGTAAGATGTGCGCTCAGCAGCTTGGGGACAACTGCACGGAGGCTGCCATCTGT
GACCCCCACCGGGGCCTCTACTGTGACTACAGCGGGGACCGCCCGAGGTACGCAATAGGAGTGTGTGCACA
GGTGGTCGGTGTGGGCTGCGTCCTGGATGGGGTGCGCTACAACAACGGCCAGTCCTTCCAGCCTAACTGCA
AGTACAACTGCACGTGCATCGACGGCGCGGTGGGCTGCACACCACTGTGCCTCCGAGTGCGCCCCCCGCGT
CTCTGGTGCCCCCACCCGCGGCGCGTGAGCATACCTGGCCACTGCTGTGAGCAGTGGGTATGTGAGGACGA
CGCCAAGAGGCCACGCAAGACCGCACCCCGTGACACAGGAGCCTTCGATGCTGTGGGTGAGGTGGAGGCAT
GGCACAGGAACTGCATAGCCTACACAAGCCCCTGGAGCCCTTGCTCCACCAGCTGCGGCCTGGGGGTCTCC
ACTCGGATCTCCAATGTTAACGCCCAGTGCTGGCCTGAGCAAGAGAGCCGCCTCTGCAACTTGCGGCCATG
CGATGTGGACATCCATACACTCATTAAGGCAGGGAAGAAGTGTCTGGCTGTGTACCAGCCAGAGGCATCCA
TGAACTTCACACTTGCGGGCTGCATCAGCACACGCTCCTATCAACCCAAGTACTGTGGAGTTTGCATGGAC
AATAGGTGCTGCATCCCCTACAAGTCTAAGACTATCGACGTGTCCTTCCAGTGTCCTGATGGGCTTGGCTT
CTCCCGCCAGGTCCTATGGATTAATGCCTGCTTCTGTAACCTGAGCTGTAGGAATCCCAATGACATCTTTG
CTGACTTGGAATCCTACCCTGACTTCTCAGAAATTGCCAAC<u>TAG</u>GCAGGCACAAATCTTGGGTCTTGGGGA
CTAACCCAATGCCTGTGAAGCAGTCAGCCCTTATGGCCAATAACTTTTCACCAATGAGCCTTAGTTACCCT
GATCTGGACCCTTGGCCTCCATTTCTGTCTCTAACCATTCAAATGACGCCTGATGGTGCTGCTCAGGCCCA
TGCTATGAGTTTTCTCCTTGATATCATTCAGCATCTACTCTAAAGAAAAATGCCTGTCTCTAGCTGTTCTG
GACTACACCCAAGCCTGATCCAGCCTTTCCAAGTCACTAGAAGTCCTGCTGGATCTTGCCTAAATCCCAAG
AAATGGAATCAGGTAGACTTTTAATATCACTAATTTCTTCTTTAGATGCCAAACCACAAGACTCTTTGGGT
CCATTCAGATGAATAGATGGAATTTGGAACAATAGAATAATCTATTATTTGGAGCCTGCCAAGAGGTACTG
TAATGGGTAATTCTGACGTCAGCGCACCAAAACTATCCTGATTCCAAATATGTATGCACCTCAAGGTCATC
AAACATTTGCCAAGTGAGTTGAATAGTTGCTTAATTTTGATTTTTAATGGAAAGTTGTATCCATTAACCTG
GGCATTGTTGAGGTTAAGTTTCTCTTCACCCCTACACTGTGAAGGGTACAGATTAGGTTTGTCCCAGTCAG
AAATAAAATTTGATAAACATTCCTGTTGATGGAAAAGCCCCCAGTTAATACTCCAGAGACAGGGAAAGGT
CAGCCCATTTCAGAAGGACCAATTGACTCTCACACTGAATCAGCTGCTGACTGGCAGGGCTTTGGGCAGTT
GGCCAGGCTCTTCCTTGAATCTTCTCCCTTGTCCTGCTTGGGTTCATAGGAATTGGTAAGGCCTCTGGACT
GGCCTGTCTGGCCCCTGAGAGTGGTGCCCTGGAACACTCCTCTACTCTTACAGAGCCTTGAGAGACCCAGC
TGCAGACCATGCCAGACCCACTGAAATGACCAAGACAGGTTCAGGTAGGGGTGTGGGTCAAACCAAGAAGT
GGGTGCCCTTGGTAGCAGCCTGGGGTGACCTCTAGAGCTGGAGGCTGTGGGACTCCAGGGGCCCCCGTGTT
CAGGACACATCTATTGCAGAGACTCATTTCACAGCCTTTCGTTCTGCTGACCAAATGGCCAGTTTTCTGGT
AGGAAGATGGAGGTTTACCAGTTGTTTAGAAACAGAAATAGACTTAATAAAGGTTTAAAGCTGAAGAGGTT
GAAGCTAAAAGGAAAAGGTTGTTGTTAATGAATATCAGGCTATTATTTATTGTATTAGGAAAATATAATAT
TTACTGTTAGAATTCTTTTATTTAGGGCCTTTTCTGTGCCAGACATTGCTCTCAGTGCTTTGCATGTATTA
GCTCACTGAATCTTCACGACAATGTTGAGAAGTTCCCATTATTATTTCTGTTCTTACAAATGTGAAACGGA
AGCTCATAGAGGTGAGAAAACTCAACCAGAGTCACCCAGTTGGTGACTGGGAAAGTTAGGATTCAGATCGA
AATTGGACTGTCTTTATAACCCATATTTTCCCCCTGTTTTTAGAGCTTCCAAATGTGTCAGAATAGGAAAA
CATTGCAATAAATGGCTTGATTTTTT

## FIGURE 50

MRWFLPWTLAAVTAAAASTVLATALSPAPTTMDFTPAPLEDTSSRPQFCKWPCECPPSPPRCPLGVSLITD
GCECCKMCAQQLGDNCTEAAICDPHRGLYCDYSGDRPRYAIGVCAQVVGVGCVLDGVRYNNGQSFQPNCKY
NCTCIDGAVGCTPLCLRVRPPRLWCPHPRRVSIPGHCCEQWVCEDDAKRPRKTAPRDTGAFDAVGEVEAWH
RNCIAYTSPWSPCSTSCGLGVSTRISNVNAQCWPEQESRLCNLRPCDVDIHTLIKAGKKCLAVYQPEASMN
FTLAGCISTRSYQPKYCGVCMDNRCCIPYKSKTIDVSFQCPDGLGFSRQVLWINACFCNLSCRNPNDIFAD
LESYPDFSEIAN

| | |
|---|---|
| Signal sequence: | Amino acids 1-17 |
| Transmembrane domain: | Amino acids 110-126 |
| N-glycosylation sites: | Amino acids 86-90;143-147;284-288; 343-347 |
| cAMP- and cGMP-dependent protein kinase phosphorylation site: | Amino acids 171-175 |
| N-myristoylation sites: | Amino acids 72-78;127-133;149-155; 201-207;231-237;289-295;327-333 |
| Amidation site: | Amino acids 269-273 |
| Prokaryotic membrane lipoprotein lipid attachment site: | Amino acids 113-124 |
| von Willebrand C1 domain: | Amino acids 130-147 |
| Thrombospondin 1: | Amino acids 223-238 |
| CT (C-Terminal) module: | Amino acids 301-313 |
| IGF binding protein (less stringent than prosite's): | Amino acids 72-81 |

**FIGURE 51**

CGCCGCCCGCCGCCTGCCTGGGCCGGGCCGAGGATGCGGCGCAGCGCCTCGGCGGCCAGGCTCGCTCCCCT
CCGGCACGCCTGCTAACTTCCCCCGCTACGTCCCCGTTCGCCCGCCGGGCCGCCCCGTCTCCCCGCGCCCT
CCGGGTCGGGTCCTCCAGGAGCGCCAGGCGCTGCCGCCGTGTGCCCTCCGCCGCTCGCCCGCGCGCCCGCG
CTCCCCGCCTGCGCCCAGCGCCCCGCGCCCGCGCCCAGTCCTCGGGCGGTC**ATG**CTGCCCCTCTGCCTCGT
GGCCGCCCTGCTGCTGGCCGCCGGGCCCGGGCCGAGCCTGGGCGACGAAGCCATCCACTGCCCGCCCTGCT
CCGAGGAGAAGCTGGCGCGCTGCCGCCCCCCCGTGGGCTGCGAGGAGCTGGTGCGAGAGCCGGGCTGCGGC
TGTTGCGCCACTTGCGCCCTGGGCTTGGGGATGCCCTGCGGGGTGTACACCCCCCGTTGCGGCTCGGGCCT
GCGCTGCTACCCGCCCCGAGGGGTGGAGAAGCCCCTGCACACACTGATGCACGGGCAAGGCGTGTGCATGG
AGCTGGCGGAGATCGAGGCCATCCAGGAAAGCCTGCAGCCCTCTGACAAGGACGAGGGTGACCACCCCAAC
AACAGCTTCAGCCCCTGTAGCGCCCATGACCGCAGGTGCCTGCAGAAGCACTTCGCCAAAATTCGAGACCG
GAGCACCAGTGGGGGCAAGATGAAGGTCAATGGGGCGCCCCGGGAGGATGCCCGGCCTGTGCCCCAGGGCT
CCTGCCAGAGCGAGCTGCACCGGGCGCTGGAGCGGCTGGCCGCTTCACAGAGCCGCACCCACGAGGACCTC
TACATCATCCCCATCCCCAACTGCGACCGCAACGGCAACTTCCACCCCAAGCAGTGTCACCCAGCTCTGGA
TGGGCAGCGTGGCAAGTGCTGGTGTGTGGACCGGAAGACGGGGGTGAAGCTTCCGGGGGGCCTGGAGCCAA
AGGGGGAGCTGGACTGCCACCAGCTGGCTGACAGCTTTCGAGAG**TGA**GGCCTGCCAGCAGGCCAGGGACTC
AGCGTCCCCTGCTACTCCTGTGCTCTGGAGGCTGCAGAGCTGACCCAGAGTGGAGTCTGAGTCTGAGTCCT
GTCTCTGCCTGCGGCCCAGAAGTTTCCCTCAAATGCGCGTGTGCACGTGTGCGTGTGCGTGCGTGTGTGTG
TGTTTGTGAGCATGGGTGTGCCCTTGGGGTAAGCCAGAGCCTGGGGTGTTCTCTTTGGTGTTACACAGCCC
AAGAGGACTGAGACTGGCACTTAGCCCAAGAGGTCTGAGCCCTGGTGTGTTTCCAGATCGATCCTGGATTC
ACTCACTCACTCATTCCTTCACTCATCCAGCCACCTAAAAACATTTACTGACCATGTACTACGTGCCAGCT
CTAGTTTTCAGCCTTGGGAGGTTTTATTCTGACTTCCTCTGATTTTGGCATGTGGAGACACTCCTATAAGG
AGAGTTCAAGCCTGTGGGAGTAGAAAAATCTCATTCCCAGAGTCAGAGGAGAAGAGACATGTACCTTGACC
ATCGTCCTTCCTCTCAAGCTAGCCAGAGGGTGGGAGCCTAAGGAAGCGTGGGGTAGCAGATGGAGTAATGG
TCACGAGGTCCAGACCCACTCCCAAAGCTCAGACTTGCCAGGCTCCCTTTCTCTTCTTCCCCAGGTCCTTC
CTTTAGGTCTGGTTGTTGCACCATCTGCTTGGTTGGCTGGCAGCTGAGAGCCCTGCTGTGGGAGAGCGAAG
GGGGTCAAAGGAAGACTTGAAGCACAGAGGGCTAGGGAGGTGGGGTACATTTCTCTGAGCAGTCAGGGTGG
GAAGAAAGAATGCAAGAGTGGACTGAATGTGCCTAATGGAGAAGACCCACGTGCTAGGGGATGAGGGGCTT
CCTGGGTCCTGTTCCCTACCCCATTTGTGGTCACAGCCATGAAGTCACCGGGATGAACCTATCCTTCCAGT
GGCTCGCTCCCTGTAGCTCTGCCTCCCTCTCCATATCTCCTTCCCCTACACCTCCCTCCCCACACCTCCCT
ACTCCCCTGGGCATCTTCTGGCTTGACTGGATGGAAGGAGACTTAGGAACCTACCAGTTGGCCATGATGTC
TTTTCTTCTTTTTCTTTTTTTTAACAAAACAGAACAAAACCAAAAAATGTCCAAA

## FIGURE 52

MLPLCLVAALLLAAGPGPSLGDEAIHCPPCSEEKLARCRPPVGCEELVREPGCGCCATCALGLGMPCGVYT
PRCGSGLRCYPPRGVEKPLHTLMHGQGVCMELAEIEAIQESLQPSDKDEGDHPNNSFSPCSAHDRRCLQKH
FAKIRDRSTSGGKMKVNGAPREDARPVPQGSCQSELHRALERLAASQSRTHEDLYIIPIPNCDRNGNFHPK
QCHPALDGQRGKCWCVDRKTGVKLPGGLEPKGELDCHQLADSFRE

Signal sequence: Amino acids 1-21

transmembrane domain: Amino acids 51-69 (weak)

N-glycosylation site: Amino acids 125-129

Tyrosine kinase phosphorylation site: Amino acids 191-198

N-myristoylation sites: Amino acids 52-58;54-60;64-70;96-102; 172-178

Insulin-like growth factor binding proteins signature: Amino acids 52-68

IGF binding protein (less stringent than prosite's): Amino acids 52-61

**FIGURE 53**

TTTCCTCACTGACTATAAAAGAATAGAGAAGGAAGGGCTTCAGTGACCGGCTGCCTGGCTGACTTACAGCA
GTCAGACTCTGACAGGATC**ATG**GCTATGATGGAGGTCCAGGGGGGACCCAGCCTGGGACAGACCTGCGTGC
TGATCGTGATCTTCACAGTGCTCCTGCAGTCTCTCTGTGTGGCTGTAACTTACGTGTACTTTACCAACGAG
CTGAAGCAGATGCAGGACAAGTACTCCAAAAGTGGCATTGCTTGTTTCTTAAAAGAAGATGACAGTTATTG
GGACCCCAATGACGAAGAGAGTATGAACAGCCCCTGCTGGCAAGTCAAGTGGCAACTCCGTCAGCTCGTTA
GAAAGATGATTTTGAGAACCTCTGAGGAAACCATTTCTACAGTTCAAGAAAAGCAACAAAATATTTCTCCC
CTAGTGAGAGAAAGAGGTCCTCAGAGAGTAGCAGCTCACATAACTGGGACCAGAGGAAGAAGCAACACATT
GTCTTCTCCAAACTCCAAGAATGAAAAGGCTCTGGGCCGCAAAATAAACTCCTGGGAATCATCAAGGAGTG
GGCATTCATTCCTGAGCAACTTGCACTTGAGGAATGGTGAACTGGTCATCCATGAAAAAGGGTTTTACTAC
ATCTATTCCCAAACATACTTTCGATTTCAGGAGGAAATAAAAGAAAACACAAAGAACGACAAACAAATGGT
CCAATATATTTACAAATACACAAGTTATCCTGACCCTATATTGTTGATGAAAAGTGCTAGAAATAGTTGTT
GGTCTAAAGATGCAGAATATGGACTCTATTCCATCTATCAAGGGGGAATATTTGAGCTTAAGGAAAATGAC
AGAATTTTTGTTTCTGTAACAAATGAGCACTTGATAGACATGGACCATGAAGCCAGTTTTTTCGGGGCCTT
TTTAGTTGGC**TAA**CTGACCTGGAAAGAAAAAGCAATAACCTCAAAGTGACTATTCAGTTTTCAGGATGATA
CACTATGAAGATGTTTCAAAAAATCTGACCAAAACAAACAAACAGAAA

## FIGURE 54

Signal sequence: Amino acids 1-32

Tyrosine kinase phosphorylation site: Amino acids 233-241

Amidation site: Amino acids 147-151

MAMMEVQGGPSLGQTCVLIVIFTVLLQSLCVAVTYVYFTNELKQMQDKYSKSGIACFLKEDDSYWDPNDEE
SMNSPCWQVKWQLRQLVRKMILRTSEETISTVQEKQQNISPLVRERGPQRVAAHITGTRGRSNTLSSPNSK
NEKALGRKINSWESSRSGHSFLSNLHLRNGELVIHEKGFYYIYSQTYFRFQEEIKENTKNDKQMVQYIYKY
TSYPDPILLMKSARNSCWSKDAEYGLYSIYQGGIFELKENDRIFVSVTNEHLIDMDHEASFFGAFLVG

## FIGURE 55

**ATG**GAACTTGGACTTGGAGGCCTCTCCACGCTGTCCCACTGCCCCTGGCCTAGGCGGCAGCCTGCCCTGTG
GCCCACCCTGGCCGCTCTGGCTCTGCTGAGCAGCGTCGCAGAGGCCTCCCTGGGCTCCGCGCCCCGCAGCC
CTGCCCCCCGCGAAGGCCCCCCGCCTGTCCTGGCGTCCCCCGCCGGCCACCTGCCGGGGGGACGCACGGCC
CGCTGGTGCAGTGGAAGAGCCCGGCGGCCGCCGCCGCAGCCTTCTCGGCCCGCGCCCCCGCCGCCTGCACC
CCCATCTGCTCTTCCCCGCGGGGGCCGCGCGGCGCGGGCTGGGGGCCCGGGCAGCCGCGCTCGGGCAGCGG
GGGCGCGGGGCTGCCGCCTGCGCTCGCAGCTGGTGCCGGTGCGCGCGCTCGGCCTGGGCCACCGCTCCGAC
GAGCTGGTGCGTTTCCGCTTCTGCAGCGGCTCCTGCCGCCGCGCGCGCTCTCCACACGACCTCAGCCTGGC
CAGCCTACTGGGCGCCGGGGCCCTGCGACCGCCCCCGGGCTCCCGGCCCGTCAGCCAGCCCTGCTGCCGAC
CCACGCGCTACGAAGCGGTCTCCTTCATGGACGTCAACAGCACCTGGAGAACCGTGGACCGCCTCTCCGCC
ACCGCCTGCGGCTGCCTGGGC**TGA**

**FIGURE 56**

MELGLGGLSTLSHCPWPRRQPALWPTLAALALLSSVAEASLGSAPRSPAPREGPPPVLASPAGHLPGGRTA
RWCSGRARRPPPQPSRPAPPPPAPPSALPRGGRAARAGGPGSRARAAGARGCRLRSQLVPVRALGLGHRSD
ELVRFRFCSGSCRRARSPHDLSLASLLGAGALRPPPGSRPVSQPCCRPTRYEAVSFMDVNSTWRTVDRLSA
TACGCLG

| | |
|---|---|
| signal sequence: | Amino acids 1-39 |
| N-glycosylation site: | Amino acids 202-206 |
| N-myristoylation sites: | Amino acids 6-12;67-73;102-108;109-115; 119-125 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4675187 A
- US 4816567 A
- EP 404097 A
- WO 9311161 A
- US 4275149 A
- US 5364934 A
- WO 8705330 A
- US 4640835 A
- US 4496689 A
- US 4301144 A
- US 4670417 A
- US 4791192 A
- US 4179337 A
- US 5428130 A
- WO 8905859 A
- US 4399216 A
- DD 266710
- US 4946783 A
- EP 139383 A
- US 4943529 A
- EP 402226 A
- EP 183070 A
- EP 244234 A
- EP 394538 A
- WO 9100357 A
- US 5010182 A
- EP 362179 A
- WO 9013646 A
- EP 36776 A
- EP 73657 A
- GB 2211504 A
- EP 117060 A
- EP 117058 A
- US 5545807 A
- US 5545806 A
- US 5569825 A
- US 5625126 A
- US 5633425 A
- US 5661016 A
- WO 9308829 A
- WO 9627011 A
- US 4676980 A
- WO 9100360 A
- WO 92200373 A
- EP 03089 A
- WO 9411026 A
- US 4485045 A
- US 4544545 A
- US 5013556 A
- WO 9733551 A
- US 4873191 A, Hoppe and Wanger
- US 4736866 A
- US 3773919 A
- EP 616812 A
- US 4657760 A
- US 5206344 A
- US 5225212 A
- EP 307247 A
- US 5122469 A
- WO 8403564 A


### Non-patent literature cited in the description


- **BORING et al.** *CA Cancel J. Clin.,* 1993, vol. 43, 7
- **NIELSEN et al.** *Prot. Eng.,* 1997, vol. 10, 1-6
- **VON HEINJE et al.** *Nucl. Acids Res.,* 1986, vol. 14, 4683-4690
- **ALTSCHUL et al.** *Nucleic Acids Res.,* 1997, vol. 25, 3389-3402
- **ALTSCHUL et al.** *Methods in Enzymology,* 1996, vol. 266, 460-480
- **AUSUBEL et al.** Current Protocols in Molecular Biology. Wiley Interscience Publishers, 1995
- **SAMBROOK et al.** *Molecular Cloning: A Laboratory Manual,* 1989
- The Molecular Basis of Cancer. Cell cycle regulation, oncogens, and antineoplastic drugs
- **MURAKAMI et al.** *WB Saunders: Philadelphia,* 1995
- **WILMAN.** Prodrugs in Cancer Chemotherapy. *Biochemical Society Transactions,* 1986, vol. 14, 375-382
- Prodrugs: A Chemical Approach to Targeted Drug Delivery. **STELLA et al.** Directed Drug Delivery. Humana Press, 1985, 247-267
- **KABAT et al.** *NIH Publ. No.91-3242,* 1991, vol. I, 647-669
- Sequences of Proteins of Immunological Interest. **KABAT et al.** National Institute of Health. 1991
- **CLOTHIA ; LESK.** *J. Mol. Biol,* 1987, vol. 196, 901-917

- **ZAPATA et al.** *Protein Eng.,* 1995, vol. 8 (10), 1057-1062
- **KOHLER et al.** *Nature,* 1975, vol. 256, 495
- **CLACKSON et al.** *Nature,* 1991, vol. 352, 624-628
- **MARKS et al.** *J. Mol. Biol,* 1991, vol. 222, 581-597
- **MORRISON et al.** *Proc. Natl. Acad. Sci. USA,* 1984, vol. 81, 6851-6855
- **JONES et al.** *Nature,* 1986, vol. 321, 522-525
- **REICHMANN et al.** *Nature,* 1988, vol. 332, 323-329
- **PRESTA.** *Curr. Op. Struct. Biol.,* 1992, vol. 2, 593-596
- **PLUCKTHUN.** The Pharmacology of Monoclonal Antibodies. *Springer-Verlag,* 1994, vol. 113, 269-315
- **HOLLINGER et al.** *Proc. Natl. Acad. Sci. USA,* 1993, vol. 90, 6444-6448
- **CARTER et al.** *Nucl. Acids Res.,* 1986, vol. 13, 4331
- **ZOLLER et al.** *Nucl. Acids Res.,* 1987, vol. 10, 6487
- **WELLS et al.** *Gene,* 1985, vol. 34, 315
- **WELLS et al.** *Philos. Trans. R. Soc. London SerA,* 1986, vol. 317, 415
- **CUNNINGHAM ; WELLS.** *Science,* 1989, vol. 244, 1081-1085
- **CREIGHTON.** The Proteins. W.H. Freeman & Co, 1976, vol. 150, 1
- *J. Mol. Biol,* 1976, vol. 150, 1
- **T.E. CREIGHTON.** Proteins: Structure and Molecular Properties. W.H. Freeman & Co, 1983, 79-86
- **APLIN ; WRISTON.** *CRC Crit. Rev. Biochem.,* 1981, 259-306
- **HAKIMUDDIN et al.** *Arch. Biochem. Biophys.,* 1987, vol. 259, 52
- **EDGE et al.** *Anal. Biochem.,* 1981, vol. 118, 131
- **THOTAKURA et al.** *Meth. Enzymol.,* 1987, vol. 138, 350
- **FIELD et al.** *Mol. Cell. Biol.,* 1988, vol. 8, 2159-2165
- **EVAN et al.** *Molecular and Cellular Biology,* 1985, vol. 5, 3610-3616
- **PABORSKY et al.** *Protein Engineering,* 1990, vol. 3 (6), 547-553
- **HOPP et al.** *BioTechnology,* 1988, vol. 6, 1204-1210
- **MARTIN et al.** *Science,* 1992, vol. 255, 192-194
- **SKINNER et al.** *J. Biol. Chem.,* 1991, vol. 266, 15163-15166
- **LUTZ-FREYERMUTH et al.** *Proc. Natl. Acad. Sci. USA,* 1990, vol. 87, 6393-6397
- **STEWART et al.** Solid-Phase Peptide Synthesis. W.H. Freeman Co, 1969
- **MERRIFIELD.** *J. Am. Chem. Soc.,* 1963, vol. 85, 2149-2154
- **SAMBROOK et al.** Molecular Cloning: A Laboratory Manual. Cold Spring Harbor Laboratory Press, 1989
- **DIEFFENBACH et al.** PCR Primer: A Laboratory Manual. Cold Spring Harbor Laboratory Press, 1995
- **SHAW et al.** *Gene,* 1983, vol. 23, 315
- **GRAHAM ; VAN DER EB.** *Virology,* 1978, vol. 52, 456-457
- **SOLINGEN et al.** *J. Bact.,* 1977, vol. 130, 946
- **HSIAO et al.** *Proc. Natl. Acad. Sci. (USA),* 1979, vol. 76, 3829
- **KEOWN et al.** *Methods in Enzymology,* 1990, vol. 185, 527-537
- **MANSOUR et al.** *Nature,* 1988, vol. 336, 348-352
- **BEACH ; NURSE.** *Nature,* 1981, vol. 290, 140
- **FLEER et al.** *Bio/Technology,* 1991, vol. 9, 968-975
- **LOUVENCOURT et al.** *J. Bacteriol.,* 1983, vol. 737
- **VAN DEN BERG et al.** *Bio/Technology,* 1990, vol. 8, 135
- **SREEKRISHNA et al.** *J. Basic Microbiol.,* 1988, vol. 28, 265-278
- **CASE et al.** Neurospora crassa. *Proc. Natl. Acad. Sci. USA,* 1979, vol. 76, 5259-5263
- **BALLANCE et al.** *Biochem. Biophys. Res. Commun.,* 1983, vol. 112, 284-289
- **TILBURN et al.** *Gene,* vol. 26 (119831), 205-221
- **YELTON et al.** *Proc. Natl. Acad. Sci. USA,* 1984, vol. 81, 1470-1474
- **KELLY ; HYNES.** *EMBO J.,* 1985, vol. 4, 475-479
- **C. ANTHONY.** *The Biochemistry of Methylotrophs,* 1982, vol. 269
- **URLAUB et al.** *Proc. Natl. Acad. Sci. USA,* 1980, vol. 77, 4216
- **STINCHCOMB et al.** *Nature,* 1979, vol. 282, 39
- **KINGSMAN et al.** *Gene,* 1979, vol. 7, 141
- **TSCHEMPER et al.** *Gene,* 1980, vol. 10, 157
- **JONES.** *Genetics,* 1977, vol. 85, 12
- **CHANG et al.** *Nature,* 1978, vol. 275, 615
- **GOEDDEL et al.** *Nature,* 1979, vol. 281, 544
- **GOEDDEL.** *Nucleic Acids Res.,* 1980, vol. 8, 4057
- **DEBOER et al.** *Proc. Natl. Acad. Sci. USA,* 1983, vol. 80, 21-25
- **HITZEMAN et al.** *J. Biol. Chem.,* 1980, vol. 255, 2073

- **HESS et al.** *J. Adv. Enzyme Reg.,* 1968, vol. 7, 149
- **HOLLAND.** *Biochemistry,* 1978, vol. 17, 4900
- **GETHING et al.** *Nature,* 1981, vol. 293, 620-625
- **MANTEL et al.** 281. *Nature,* 1979, vol. 281, 40-46
- **THOMAS.** *Proc. Natl. Acad. Sci. USA,* 1980, vol. 77, 5201-5205
- *Methods in Enzymology,* 1990, vol. 182
- **SCOPES.** Protein Purification: Principles and Practice. Springer-Verlag, 1982
- **GODING.** Monoclonal Antibodies: Principles and Practice. Academic Press, 1986, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **MUNSON ; POLLARD.** *Anal. Biochem.,* 1980, vol. 107, 220
- **RIECHMANN et al.** *Nature,* 1988, vol. 332, 323-329
- **RIECHMANN et al.** *Nature,* 1988, vol. 332, 323-327
- **VERHOEYEN et al.** *Science,* 1988, vol. 239, 1534-1536
- **HOOGENBOOM ; WINTER.** *J. Mol. Biol.,* 1991, vol. 227, 381
- **MARKS et al.** *J. Mol. Biol.,* 1991, vol. 222, 581
- **COLE et al.** *Monoclonal Antibodies and Cancer Therapy, Alan R. Liss,* 1985, 77
- **BOERNER et al.** *J. Immunol.,* 1991, vol. 147 (1), 86-95
- **MARKS et al.** *Bio/Technology,* 1992, vol. 10, 779-783
- **LONBERG et al.** *Nature,* 1994, vol. 368, 856-859
- **MORRISON.** *Nature,* 1994, vol. 368, 812-13
- **FISHWILD et al.** *Nature Biotechnology,* 1996, vol. 14, 845-51
- **NEUBERGER.** *Nature Biotechnology,* 1996, vol. 14, 826
- **LONBERG ; HUSZAR.** *Intern. Rev. Immunol.,* 1995, vol. 13, 65-93
- **MILSTEIN ; CUELLO.** *Nature,* 1983, vol. 305, 537-539
- **TRAUNECKER et al.** *EMBO J.,* vol. 10, 3655-3659
- **SURESH et al.** *Methods in Enzymology,* 1986, vol. 121, 210
- **BRENNAN et al.** *Science,* 1985, vol. 229, 81
- **SHALABY et al.** *J. Exp. Med.,* 1992, vol. 175, 217-225
- **KOSTELNY et al.** *J. Immunol.,* 1992, vol. 148 (5), 1547-1553
- **GRUBER et al.** *J. Immunol.,* 1994, vol. 152, 5368
- **TUTT et al.** *J. Immunol.,* 1991, vol. 147, 60
- **CARON et al.** *J. Exp. Med.,* 1992, vol. 176, 1191-1195
- **SHOPES.** *J. Immunol.,* 1992, vol. 148, 2918-2922
- **WOLFF et al.** *Cancer Research,* 1993, vol. 53, 2560-2565
- **STEVENSON et al.** *Anti-Cancer Drug Design,* 1989, vol. 3, 219-230
- **VITETTA et al.** *Science,* 1987, vol. 238, 1098
- **EPSTEIN et al.** *Proc. Natl. Acad. Sci. USA,* 1985, vol. 82, 3688
- **HWANG et al.** *Proc. Natl. Acad. Sci. USA,* 1980, vol. 77, 4030
- **MARTIN et al.** *J. Biol. Chem.,* 1982, vol. 257, 286-288
- **GABIZON et al.** *J. National Cancer Inst.,* 1989, vol. 81 (19), 1484
- **MONKS et al.** *J. Natl. Cancer Inst.,* 1991, vol. 83, 757-766
- **BOYD.** *Cancer: Princ. Pract. Oncol. Update,* 1989, vol. 3 (10), 1-12
- **SMALL et al.** *Mol. Cell. Biol.,* 1985, vol. 5, 642-648
- **KARMALI et al.** *Br. J. Cancer,* 1983, vol. 48, 689-696
- **DREBIN et al.** *Proc. Natl. Acad. Sci. USA,* 1986, vol. 83, 9129-9133
- **WANG et al.** *Cancer Research,* 1994, vol. 54, 4726-4728
- **TOO et al.** *Cancer Research,* 1995, vol. 55, 681-684
- **DELEO et al.** *J. Exp. Med.,* 1977, vol. 146, 720
- **PALLADINO et al.** *J. Immunol.,* 1987, vol. 138, 4023-4032
- **ZUPI et al.** *Br. J. Cancer,* 1980, vol. 41 (4), 309
- **ZACHARSKI.** *Haemostasis,* 1986, vol. 16, 300-320
- **RYGAARD ; SPANG-THOMSEN.** Proc. 6th Int. Workshop on Immune-Deficient Animals. 1989, 301
- **VAN DER PUTTEN et al.** *Proc. Natl. Acad. Sci. USA,* 1985, vol. 82, 6148-615
- **THOMPSON et al.** *Cell,* 1989, vol. 56, 313-321
- **LO.** *Mol. Cell. Biol.,* 1983, vol. 3, 1803-1814
- **LAVITRANO et al.** *Cell,* 1989, vol. 57, 717-73
- **LASKO et al.** *Proc. Nail. Acad. Sci. USA,* 1992, vol. 89, 6232-636
- **FIELDS ; SONG.** *Nature (London),* 1989, vol. 340, 245-246
- **CHIEN et al.** *Proc. Natl. Acad. Sci. USA,* 1991, vol. 88, 9578-9582
- **CHEVRAY ; NATHANS.** *Proc. Natl. Acad. Sci. USA,* 1991, vol. 89, 5789-5793
- **MARASCO et al.** *Proc. Natl. Acad. Sci. USA.,* 1993, vol. 90, 7889-7893
- Remington's Pharmaceutical Sciences. 1980


- Remington's Pharmaceutical Sciences. 1980

- he use of interspecies scaling in toxicokinetics. **MORDENTI, J. ; CHAPPELL, W. et al.** Toxicokinetics and New Drug Development. Pergamon Press, 1989, 42-96
- **HOLMES et al.** *Science,* 1991, vol. 253, 1278-1280
- **GIETZ et al.** *Nucl. Acid. Res.,* 1992, vol. 20, 1425
- **KAISER et al.** Methods in Yeast Genetics. Cold Spring Harbor Press, 1994, 207
- **KAISER et al.** Methods in Yeast Genetics. Cold Spring Harbor Press, 1994, 208-210
- **BIELY et al.** *Anal. Biochem.,* 1988, vol. 172, 176-179
- **ALTSHUL et al.** *Methods in Enzymology,* 1996, vol. 266, 460-480
- **ALTSHUL et al.** *Methods in Enzymolgy,* 1996, vol. 266, 460-480
- **LU ; GILLETT.** *Cell Vision.,* 1994, vol. 1, 169-176
- **BOLIVAR et al.** *Gene,* 1977, vol. 2, 95
- **THIMMAPPAYA et al.** *Cell,* 1982, vol. 31, 543
- **SOMPARYRAC et al.** *Proc. Natl. Acad. Sci.,* 1981, vol. 12, 7575
- **AUSUBEL et al.** Current Protocols of Molecular Biology. John Wiley and Sons, 1997
- **LUCAS et al.** *Nucl. Acids Res.,* 1996, vol. 24 (9), 1774-1779
- **O'REILLEY et al.** Baculovirus expression vectors: A Laboratory Manual. Oxford University Press, 1994
- **RUPERT et al.** *Nature,* 1993, vol. 362, 175-179
- **BRAXTON ; WELLS.** *Biochemistry,* 1992, vol. 31, 7796-7801
- **ATHAUDA et al.** *Biochem.,* 1993, vol. 113, 742-746
- **SKEHAN et al.** *J. Natl. Cancer Inst.,* 1990, vol. 82, 1107-1112
- **MONKS et al.** *Boyd, Cancer: Princ. Pract. Oncol. Update,* 1989, vol. 3 ((10)), 1-12